# EUROPEAN PATENT APPLICATION

(11) **EP 1 262 488 A2**
(43) Date of publication of application: **04.12.2002**
(21) Application number: 02001168.0
(22) Date of filing: 28.01.2002
(51) Int. Cl.: C07K 14/47

(54) **Human LCCL-domain containing protein**

(30) Priority: 30.01.2001 WO PCT/US01/00663; 30.01.2001 WO PCT/US01/00664; 30.01.2001 WO PCT/US01/00665; 30.01.2001 WO PCT/US01/00667; 30.01.2001 WO PCT/US01/00668; 30.01.2001 WO PCT/US01/00669; 23.05.2001 US 864761; 25.09.2001 US 325062
(71) Applicant: Aeomica, Inc., Sunnyvale, CA 94086-4520 (US)
(72) Inventor: Gu, Yizhong, Cupertino, California 95014 (US); Nguyen, Cung-Tuong, San Jose, California 95131 (US)
(74) Representative: Rollins, Anthony John

(57) **Abstract**

The invention provides isolated nucleic acids that encode LCP (LCCL_domain containing protein), including two isoforms, and fragments thereof, vectors for propagating and expressing LCP nucleic acids, host cells comprising the nucleic acids and vectors of the present invention, proteins, protein fragments, and protein fusions of the novel LCP isoforms, and antibodies thereto. The invention further provides transgenic cells and non-human organisms comprising human LCP nucleic acids, and transgenic cells and non-human organisms with targeted disruption of the endogenous orthologue of the human LCP gene. The invention further provides pharmaceutical formulations of the nucleic acids, proteins, and antibodies of the present invention, and diagnostic, investigational, and therapeutic methods based on the LCP nucleic acids, proteins, and antibodies of the present invention.

## Description

### FIELD OF THE INVENTION

The present invention relates to a human LCCL_domain containing protein, which has two isforms. More specifically, the invention provides isolated nucleic acid molecules encoding human LCCL_domain containing protein, fragments thereof, vectors and host cells comprising isolated nucleic acid molecules encoding human LCCL_domain containing protein, human LCCL_domain containing protein polypeptides, antibodies, transgenic cells and non-human organisms, and diagnostic, therapeutic, and investigational methods of using the same.

### BACKGROUND OF THE INVENTION

The function of proteins can often be predicted and characterized through the functional domains within the protein sequences. The LCCL domain was first identified in *Limulus* Factor C, COCH and Lgl 1 genes. *Limulus* Factor C is an endotoxin-sensitive, trypsin type serine protease, COCH is the candidate gene for the deafness disorder DFNA9 and Lgl 1 is a late gestation lung protein. The LCCL domain is about 93 amino acids in length and contains multiple conserved cysteines (Cys). These three proteins have been grouped together as an LCCL domain containing protein family. DFNA9 is an autosomal dominant, nonsyndromic, progressive sensorineural hearing loss with vestibular pathology. Three point mutations were identified that change conserved amino acid sequence within the LCCL domain of the COCH protein in DFNA9 patients. Robertson et al, *Nature Genetics* 20:299-302 (1998). The pattern of COCH gene expression in chicken inner ear correlates well with histopathological findings in DFNA9 patients. The high COCH expression in cochlea and vestibule, the only two organ systems identified to be affected in DFNA9 patients, further indicate COCH gene as the candidate gene for DFNA9.

The CUB domain is a 110-amino acid module that was first identified in the complement subcomponents Cls and Clr. Bork *FEBS Lett.* 282:9-12 (1991). Later, this domain was also identified in bone morphogenetic protein 1 (BMP1), an embryonic sea urchin protein Uegf as well as other proteins. Although there is no clear indication of the function of the CUB domain, it is believed to be involved in the developmental and differentiation processes. Chen et al, *J*. *Biol*. *Chem*. 274:32215-32224 (1999). The Coagulation factor 5/8 C-terminal (FA58C)/discoidin (DSD) domain is a cell surface-attached carbohydrate-binding domain. Members of the discoidin (DSD) domain family, which includes the C1 and C2 repeats of blood coagulation factors V and VIII, occur in a great variety of eukaryotic proteins, most of which have been implicated in cell-adhesion or developmental processes. Baumgartner et al, *Protein Sci.* 7(7):1626-1631 (1998).

The fluid-mosaic model for the structure of the cell membrane holds that the membrane consists of a protein-embedded bilayer of phospholipids. The proteins embedded within the lipid bilayer can exist in several different positions: extending through both layers, projecting out one side or the other, or totally contained within the bilayer. Jacobson K. *et al, Science* 268:1441-1442 (1995). The plasma membrane is directly involved in dynamic cellular functions, such as growth, movement, and signaling. Plasma membrane proteins play central roles in such functions. Due to their critical role in cell signaling, membrane proteins are increasingly considered as excellent drug intervention targets. An obvious approach is to develop humanized monoclonal antibodies and use them against membrane targets, which are overexpressed in certain tumors (Mokbel K. and Hassanally D., *Curr. Med. Res. Opin.* 17:51-59 (2001)).

Recent reports suggest that at least one-third, and likely a higher percentage, of human genes are alternatively spliced. Hanke *et al., Trends Genet.* 15(1):389 - 390 (1999); Mironov *et al., Genome Res.* 9:1288-93 (1999); Brett et *al., FEBS Lett.* 474(1):83-6 (2000). Alternative splicing has been proposed to account for at least part of the difference between the number of genes recently called from the completed human genome draft sequence - 30,000 to 40,000 (Genome International Sequencing Consortium, *Nature* 409:860-921 (15 February 2001) - and earlier predictions of human gene number that routinely ranged as high as 120,000, Liang *et al*., *Nature Genet.* 25(2):239-240 (2000). With the *Drosophila* homolog of one human gene reported to have 38,000 potential alternatively spliced variants, Schmucker *et al*., *Cell* 101:671 (2000), it now appears that alternative splicing may permit the relatively small number of human coding regions to encode millions, perhaps tens of millions, of structurally distinct proteins and protein isoforms.

Due to the critical role of the COCH gene in DFNA9, and the potential role of CUB and DSD/FA58C in cell-cell adhesion and development, there is a need to identify and to characterize new members of the LCCL domain containing gene family as they have potential therapeutic as well as diagnostic roles for neurological and developmental disorders, as well as diseases involving cell-cell adhesion process.

### SUMMARY OF THE INVENTION

The present invention solves these and other needs in the art by providing isolated nucleic acids that encode human LCCL_domain containing protein (LCP), which has two isoforms (LCP1 and LCP2), and fragments thereof.

In other aspects, the invention provides vectors for propagating and expressing the nucleic acids of the present invention, host cells comprising the nucleic acids and vectors of the present invention, proteins, protein fragments, and protein fusions of the LCP, and antibodies thereto.

The invention further provides pharmaceutical formulations of the nucleic acids, proteins, and antibodies of the present invention.

In other aspects, the invention provides transgenic cells and non-human organisms comprising LCP nucleic acids, and transgenic cells and non-human organisms with targeted disruption of the endogenous orthologue of the LCP.

The invention additionally provides diagnostic, investigational, and therapeutic methods based on the LCP nucleic acids, proteins, and antibodies of the present invention.

The invention further provides medical and diagnostic uses of the LCP nucleic acids, proteins and antibodies.

### BRIEF DESCRIPTION OF THE DRAWINGS

The above and other objects and advantages of the present invention will be apparent upon consideration of the following detailed description taken in conjunction with the accompanying drawings, in which like characters refer to like parts throughout, and in which:
FIG. 1 (A) schematizes the protein domain structure of LCP1, FIG. 1 (B) presents the alignment of the CUB domain of LCP1 with that of other proteins, FIG. 1 (C) presents the alignment of the LCCL domain of LCP with that of other proteins, and FIG. 1 (D) presents the alignment of the DSD/FA58C domain of LCP with that of other proteins;
FIG. 2 is a map showing the genomic structure of LCP encoded at chromosome 3q12.1;
FIG. 3 presents the nucleotide and predicted amino acid sequences of LCP1;
FIG. 4 presents the nucleotide and predicted amino acid sequences of LCP2; and
FIG. 5 presents the expression profile of LCP by RT_PCR analysis.

### DETAILED DESCRIPTION OF THE INVENTION

Mining the sequence of the human genome for novel human genes, the present inventors have identified LCP, a membrane protein potentially associated with neurological and developmental disorders, as well as diseases involving cell-cell adhesion process.

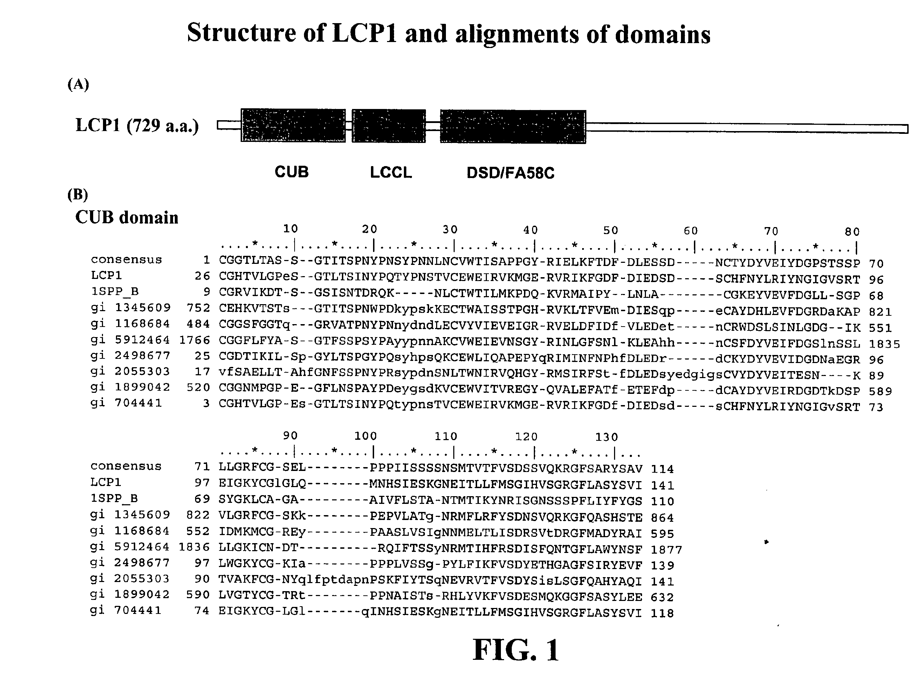

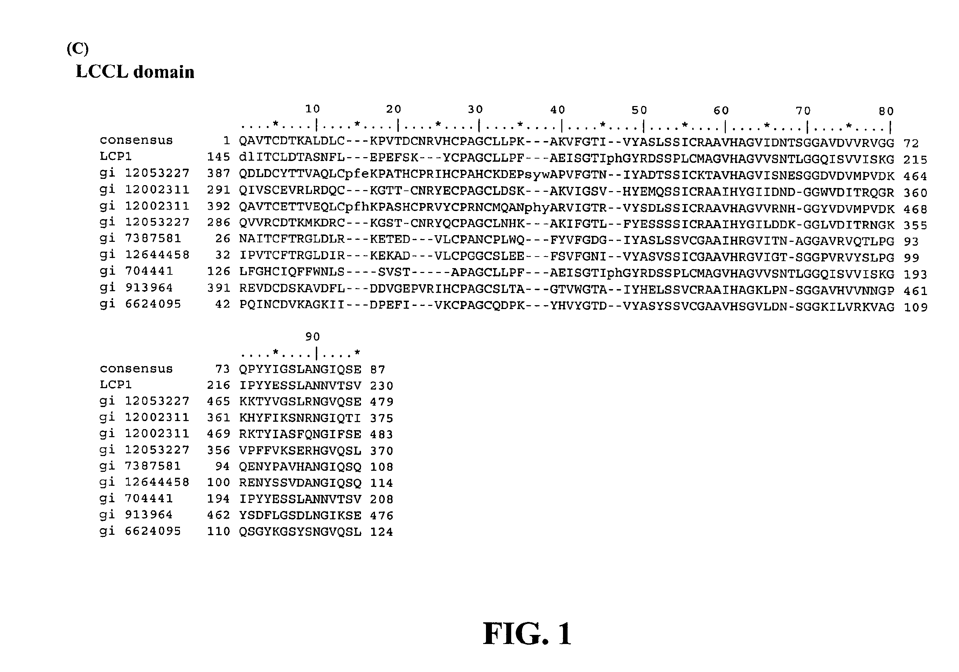

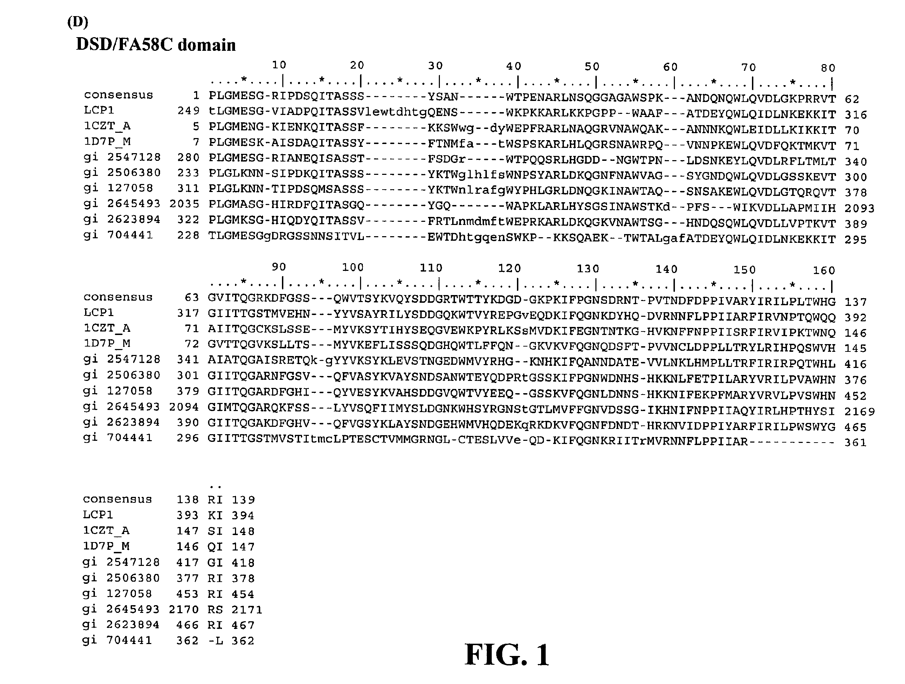

The newly isolated membrane protein LCP contains three distinct protein domains, including a CUB, an LCCL and a DSD/FA58C domain, respectively. The following four paragraphs describe the protein structure of LCP using LCP1 as an example. However, such description is also true for LCP2 except that, in comparison to LCP1, the LCP2 protein product lacks amino acid sequence 23 - 98 of LCP1, and therefore has a partial CUB domain the N-terminal of which is truncated. The structural features of LCP1 are schematized in FIG. 1.

LCP1 contains a CUB domain at residues 26 - 138 (http://smart.embl-heidelberg.de/) or alternatively at residues 26 - 141 (http://pfam.wustl.edu/). CUB is a protein domain with a predicted beta-barrel structure similar to that of immunoglobulins. It is an extracellular domain found in functionally diverse, mostly developmentally regulated proteins.

LCP1 has an LCCL domain at residues 147 - 230 (http://smart.embl-heidelberg.de/). First identified in Limulus factor C, Coch-5b2 and Lg11, the LCCL domain is hypothesized to have an antimicrobial function. Mutations in the LCCL domain have been shown to cause the deafness disorder DFNA9 in humans.

LCP1 also has a discoidin domain, also known as a F5/8 type C domain or an FA58C domain. In LCP1, the discoidin/FA58C domain occurs at residues 250 - 394 (discoidin domain, http://smart.embl-heidelberg.de/) or alternatively at residues 250 - 400 (F5/8 type C domain, http://pfam.wustl.edu/) or at residues 248-403 (FA58C, http://smart.embl-heidelberg.de/). The discoidin domain is a protein domain with a predicted amphipathic, membrane binding alpha helical structure at the C-terminal. This domain is found in a number of coagulation factors and has been shown to be responsible for phosphatidylserine-binding and essential for phosphatidylserine activity. The discoidin domain is also present in a subset of the tyrosine kinase receptor family known as discoidin domain receptors that are putatively involved in tumor progression.

The LCP1 protein contains a signal peptide consisting of the first 20 amino acid sequence of the protein. It also contains a transmembrane domain between amino acids 487 and 506 (http://www.ch.embnet.org/software/TMPRED_form.html). Other signatures of the newly isolated LCP1 proteins were identified by searching the PROSITE database (http://www.expasy.ch/tools/scnpsitl.html). These include six *N*-glycosylation sites (49-52, 109-112, 226-229, 428-431, 470-473, and 476-479), two cAMP- and cGMP-dependent protein kinase phosphorylation sites (313-316 and 512-515), six protein kinase C phosphorylation sites (130-132, 240-242, 279-281, 560-562, 592-594, and 654-656), thirteen casein kinase II phosphorylation sites, a single tyrosine kinase phosphorylation site (512-519), and twenty *N*-myristoylation sites.

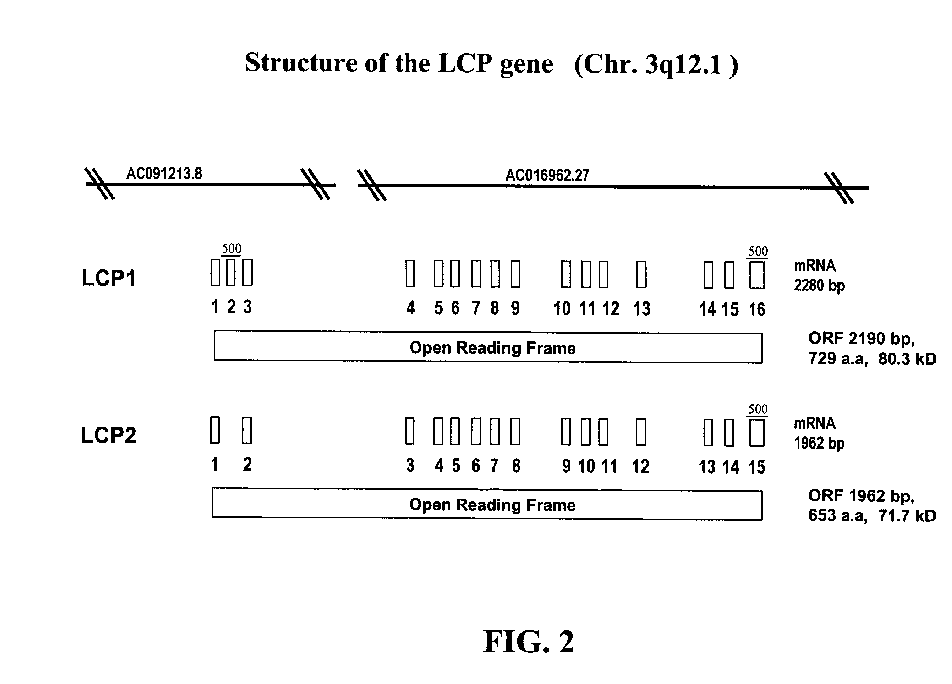

FIG. 2 shows the genomic organization of LCP.

At the top is shown the two bacterial artificial chromosomes (BACs), with GenBank accession numbers (AC091213.8, AC016962.27), which span the LCP locus. The genome-derived single-exon probes first used to demonstrate expression from this locus are shown above the exons and labeled "500".

As shown in FIG. 2, LCP1 encodes a protein of 729 amino acids and is comprised of exons 1 - 16. LCP1 has a predicted molecular weight, prior to any post-translational modification, of 80.3 kD. LCP2 encodes a protein of 653 amino acids and is lacking exon 2 of LCP1. LCP2 has a predicted molecular weight, prior to any post-translational modification, of 80.3 kD.

As further discussed in the examples herein, expression of LCP was assessed using hybridization to genome-derived single exon microarrays and RT-PCR. Microarray analysis of exons two and sixteen of LCP1 showed expression in adrenal, adult liver, bone marrow, brain, fetal liver, heart, kidney, lung, placenta and prostate as well as a cell line, hela. RT-PCR confirmed microarray data, and further provided expression data for skeletal muscle and colon.

As more fully described below, the present invention provides isolated nucleic acids that encode LCP and fragments thereof. The invention further provides vectors for propagation and expression of the nucleic acids of the present invention, host cells comprising the nucleic acids and vectors of the present invention, proteins, protein fragments, and protein fusions of the present invention, and antibodies specific for all or any one of the isoforms. The invention provides pharmaceutical formulations of the nucleic acids, proteins, and antibodies of the present invention. The invention further provides transgenic cells and non-human organisms comprising human LCP nucleic acids, and transgenic cells and non-human organisms with targeted disruption of the endogenous orthologue of the human LCP. The invention additionally provides diagnostic, investigational, and therapeutic methods based on the LCP nucleic acids, proteins, and antibodies of the present invention.

### DEFINITIONS

Unless defined otherwise, all technical and scientific terms used herein have the meaning commonly understood by one of ordinary skill in the art to which this invention belongs.

As used herein, "nucleic acid" (synonymously, "polynucleotide") includes polynucleotides having natural nucleotides in native 5'-3' phosphodiester linkage - *e.g.,* DNA or RNA - as well as polynucleotides that have nonnatural nucleotide analogues, nonnative internucleoside bonds, or both, so long as the nonnatural polynucleotide is capable of sequence-discriminating basepairing under experimentally desired conditions. Unless otherwise specified, the term "nucleic acid" includes any topological conformation; the term thus explicitly comprehends single-stranded, double-stranded, partially duplexed, triplexed, hairpinned, circular, and padlocked conformations.

As used herein, an "isolated nucleic acid" is a nucleic acid molecule that exists in a physical form that is nonidentical to any nucleic acid molecule of identical sequence as found in nature; "isolated" does not require, although it does not prohibit, that the nucleic acid so described has itself been physically removed from its native environment.

For example, a nucleic acid can be said to be "isolated" when it includes nucleotides and/or internucleoside bonds not found in nature. When instead composed of natural nucleosides in phosphodiester linkage, a nucleic acid can be said to be "isolated" when it exists at a purity not found in nature, where purity can be adjudged with respect to the presence of nucleic acids of other sequence, with respect to the presence of proteins, with respect to the presence of lipids, or with respect the presence of any other component of a biological cell, or when the nucleic acid lacks sequence that flanks an otherwise identical sequence in an organism's genome, or when the nucleic acid possesses sequence not identically present in nature.

As so defined, "isolated nucleic acid" includes nucleic acids integrated into a host cell chromosome at a heterologous site, recombinant fusions of a native fragment to a heterologous sequence, recombinant vectors present as episomes or as integrated into a host cell chromosome.

As used herein, an isolated nucleic acid "encodes" a reference polypeptide when at least a portion of the nucleic acid, or its complement, can be directly translated to provide the amino acid sequence of the reference polypeptide, or when the isolated nucleic acid can be used, alone or as part of an expression vector, to express the reference polypeptide *in vitro,* in a prokaryotic host cell, or in a eukaryotic host cell.

As used herein, the term "exon" refers to a nucleic acid sequence found in genomic DNA that is bioinformatically predicted and/or experimentally confirmed to contribute contiguous sequence to a mature mRNA transcript.

As used herein, the phrase "open reading frame" and the equivalent acronym "ORF" refer to that portion of a transcript-derived nucleic acid that can be translated in its entirety into a sequence of contiguous amino acids. As so defined, an ORF has length, measured in nucleotides, exactly divisible by 3. As so defined, an ORF need not encode the entirety of a natural protein.

As used herein, the phrase "ORF-encoded peptide" refers to the predicted or actual translation of an ORF.

As used herein, the phrase "degenerate variant" of a reference nucleic acid sequence intends all nucleic acid sequences that can be directly translated, using the standard genetic code, to provide an amino acid sequence identical to that translated from the reference nucleic acid sequence.

As used herein, the term "microarray" and the equivalent phrase "nucleic acid microarray" refer to a substrate-bound collection of plural nucleic acids, hybridization to each of the plurality of bound nucleic acids being separately detectable. The substrate can be solid or porous, planar or non-planar, unitary or distributed.

As so defined, the term "microarray" and phrase "nucleic acid microarray" include all the devices so called in Schena (ed.), DNA Microarrays: A Practical Approach (Practical Approach Series), Oxford University Press (1999) (ISBN: 0199637768); *Nature Genet.* 21(1) (suppl):1 - 60 (1999); and Schena (ed.), Microarray Biochip: Tools and Technology, Eaton Publishing Company/BioTechniques Books Division (2000) (ISBN: 1881299376), the disclosures of which are incorporated herein by reference in their entireties.

As so defined, the term "microarray" and phrase "nucleic acid microarray" also include substrate-bound collections of plural nucleic acids in which the plurality of nucleic acids are distributably disposed on a plurality of beads, rather than on a unitary planar substrate, as is described, *inter alia,* in Brenner *et al*., *Proc*. *Natl*. *Acad*. *Sci*. *USA* 97(4):166501670 (2000), the disclosure of which is incorporated herein by reference in its entirety; in such case, the term "microarray" and phrase "nucleic acid microarray" refer to the plurality of beads in aggregate.

As used herein with respect to solution phase hybridization, the term "probe", or equivalently, "nucleic acid probe" or "hybridization probe", refers to an isolated nucleic acid of known sequence that is, or is intended to be, detectably labeled. As used herein with respect to a nucleic acid microarray, the term "probe" (or equivalently "nucleic acid probe" or "hybridization probe") refers to the isolated nucleic acid that is, or is intended to be, bound to the substrate. In either such context, the term "target" refers to nucleic acid intended to be bound to probe by sequence complementarity.

As used herein, the expression "probe comprising SEQ ID NO:X", and variants thereof, intends a nucleic acid probe, at least a portion of which probe has either (i) the sequence directly as given in the referenced SEQ ID NO:X, or (ii) a sequence complementary to the sequence as given in the referenced SEQ ID NO:X, the choice as between sequence directly as given and complement thereof dictated by the requirement that the probe be complementary to the desired target.

As used herein, the phrases "expression of a probe" and "expression of an isolated nucleic acid" and their linguistic equivalents intend that the probe or, (respectively, the isolated nucleic acid), or a probe (or, respectively, isolated nucleic acid) complementary in sequence thereto,can hybridize detectably under high stringency conditions to a sample of nucleic acids that derive from mRNA transcripts from a given source. For example, and by way of illustration only, expression of a probe in "liver" means that the probe can hybridize detectably under high stringency conditions to a sample of nucleic acids that derive from mRNA obtained from liver.

As used herein, "a single exon probe" comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and that consist of one or more exons that are found adjacent to the reference exon in the genome.

For purposes herein, "high stringency conditions" are defined for solution phase hybridization as aqueous hybridization (*i*.*e*., free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. "Moderate stringency conditions" are defined for solution phase hybridization as aqueous hybridization (i.e., free of formamide) in 6X SSC, 1% SDS at 65°C for at least 8 hours, followed by one or more washes in 2x SSC, 0.1% SDS at room temperature.

For microarray-based hybridization, standard "high stringency conditions" are defined as hybridization in 50% formamide, 5X SSC, 0.2 µg/µl poly(dA), 0.2 µg/µl human cot1 DNA, and 0.5% SDS, in a humid oven at 42°C overnight, followed by successive washes of the microarray in 1X SSC, 0.2% SDS at 55°C for 5 minutes, and then 0.1X SSC, 0.2% SDS, at 55°C for 20 minutes. For microarray-based hybridization, "moderate stringency conditions", suitable for cross-hybridization to mRNA encoding structurally- and functionally-related proteins, are defined to be the same as those for high stringency conditions but with reduction in temperature for hybridization and washing to room temperature (approximately 25°C).

As used herein, the terms "protein", "polypeptide", and "peptide" are used interchangeably to refer to a naturally-occurring or synthetic polymer of amino acid monomers (residues), irrespective of length, where amino acid monomer here includes naturally-occurring amino acids, naturally-occurring amino acid structural variants, and synthetic non-naturally occurring analogs that are capable of participating in peptide bonds. The terms "protein", "polypeptide", and "peptide" explicitly permits of post-translational and post-synthetic modifications, such as glycosylation.

The term "oligopeptide" herein denotes a protein, polypeptide, or peptide having 25 or fewer monomeric subunits.

The phrases "isolated protein", "isolated polypeptide", "isolated peptide" and "isolated oligopeptide" refer to a protein (or respectively to a polypeptide, peptide, or oligopeptide) that is nonidentical to any protein molecule of identical amino acid sequence as found in nature; "isolated" does not require, although it does not prohibit, that the protein so described has itself been physically removed from its native environment.

For example, a protein can be said to be "isolated" when it includes amino acid analogues or derivatives not found in nature, or includes linkages other than standard peptide bonds.

When instead composed entirely of natural amino acids linked by peptide bonds, a protein can be said to be "isolated" when it exists at a purity not found in nature - where purity can be adjudged with respect to the presence of proteins of other sequence, with respect to the presence of non-protein compounds, such as nucleic acids, lipids, or other components of a biological cell, or when it exists in a composition not found in nature, such as in a host cell that does not naturally express that protein.

A "purified protein" (equally, a purified polypeptide, peptide, or oligopeptide) is an isolated protein, as above described, present at a concentration of at least 95%, as measured on a weight basis with respect to total protein in a composition. A "substantially purified protein" (equally, a substantially purified polypeptide, peptide, or oligopeptide) is an isolated protein, as above described, present at a concentration of at least 70%, as measured on a weight basis with respect to total protein in a composition.

As used herein, the phrase "protein isoforms" refers to a plurality of proteins having nonidentical primary amino acid sequence but that share amino acid sequence encoded by at least one common exon.

As used herein, the phrase "alternative splicing" and its linguistic equivalents includes all types of RNA processing that lead to expression of plural protein isoforms from a single gene; accordingly, the phrase "splice variant(s)" and its linguistic equivalents embraces mRNAs transcribed from a given gene that, however processed, collectively encode plural protein isoforms. For example, and by way of illustration only, splice variants can include exon insertions, exon extensions, exon truncations, exon deletions, alternatives in the 5' untranslated region ("5' UT") and alternatives in the 3' untranslated region ("3' UT"). Such 3' alternatives include, for example, differences in the site of RNA transcript cleavage and site of poly(A) addition. See, *e.g.,* Gautheret *et al., Genome Res.* 8:524-530 (1998).

As used herein, "orthologues" are separate occurrences of the same gene in multiple species. The separate occurrences have similar, albeit nonidentical, amino acid sequences, the degree of sequence similarity depending, in part, upon the evolutionary distance of the species from a common ancestor having the same gene.

As used herein, the term "paralogues" indicates separate occurrences of a gene in one species. The separate occurrences have similar, albeit nonidentical, amino acid sequences, the degree of sequence similarity depending, in part, upon the evolutionary distance from the gene duplication event giving rise to the separate occurrences.

As used herein, the term "homologues" is generic to "orthologues" and "paralogues".

As used herein, the term "antibody" refers to a polypeptide, at least a portion of which is encoded by at least one immunoglobulin gene, or fragment thereof, and that can bind specifically to a desired target molecule. The term includes naturally-occurring forms, as well as fragments and derivatives.

Fragments within the scope of the term "antibody" include those produced by digestion with various proteases, those produced by chemical cleavage and/or chemical dissociation, and those produced recombinantly, so long as the fragment remains capable of specific binding to a target molecule. Among such fragments are Fab, Fab', Fv, F(ab)'₂, and single chain Fv (scFv) fragments.

Derivatives within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, *e.g.*, Marasco (ed.), Intracellular Antibodies: Research and Disease Applications, Springer-Verlag New York, Inc. (1998) (ISBN: 3540641513), the disclosure of which is incorporated herein by reference in its entirety).

As used herein, antibodies can be produced by any known technique, including harvest from cell culture of native B lymphocytes, harvest from culture of hybridomas, recombinant expression systems, and phage display.

As used herein, "antigen" refers to a ligand that can be bound by an antibody; an antigen need not itself be immunogenic. The portions of the antigen that make contact with the antibody are denominated "epitopes".

"Specific binding" refers to the ability of two molecular species concurrently present in a heterogeneous (inhomogeneous) sample to bind to one another in preference to binding to other molecular species in the sample. Typically, a specific binding interaction will discriminate over adventitious binding interactions in the reaction by at least two-fold, more typically by at least 10-fold, often at least 100-fold; when used to detect analyte, specific binding is sufficiently discriminatory when determinative of the presence of the analyte in a heterogeneous (inhomogeneous) sample. Typically, the affinity or avidity of a specific binding reaction is least about 10⁻⁷ M, with specific binding reactions of greater specificity typically having affinity or avidity of at least 10⁻⁸ M to at least about 10⁻⁹ M.

As used herein, "molecular binding partners" - and equivalently, "specific binding partners" - refer to pairs of molecules, typically pairs of biomolecules, that exhibit specific binding. Nonlimiting examples are receptor and ligand, antibody and antigen, and biotin to any of avidin, streptavidin, neutrAvidin and captAvidin.

The term "antisense", as used herein, refers to a nucleic acid molecule sufficiently complementary in sequence, and sufficiently long in that complementary sequence, as to hybridize under intracellular conditions to (i) a target mRNA transcript or (ii) the genomic DNA strand complementary to that transcribed to produce the target mRNA transcript.

The term "portion", as used with respect to nucleic acids, proteins, and antibodies, is synonymous with "fragment".

### NUCLEIC ACID MOLECULES

In a first aspect, the invention provides isolated nucleic acids that encode LCP, variants having at least 65% sequence identity thereto, degenerate variants thereof, variants that encode LCP proteins having conservative or moderately conservative substitutions, cross-hybridizing nucleic acids, and fragments thereof.

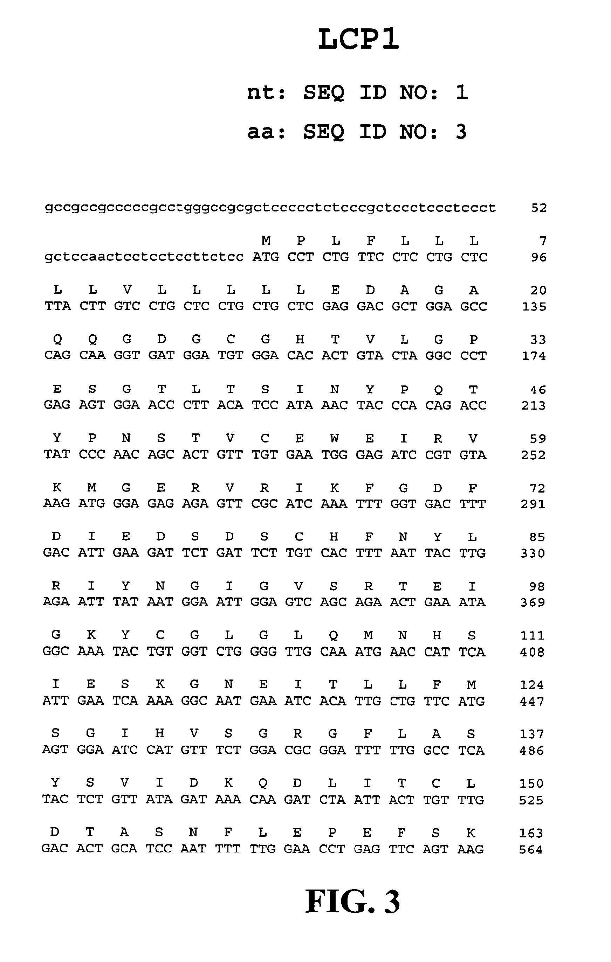

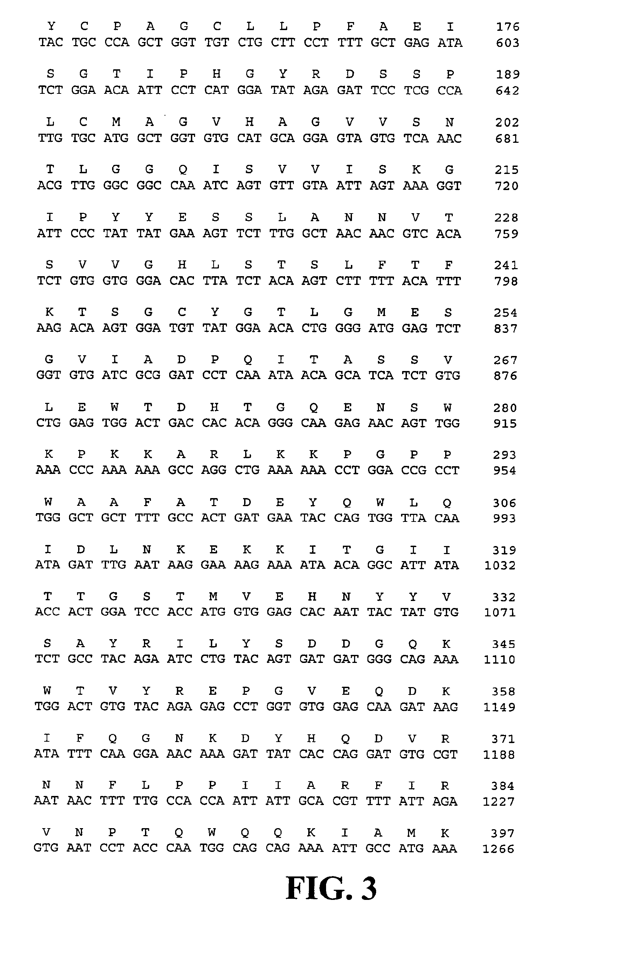

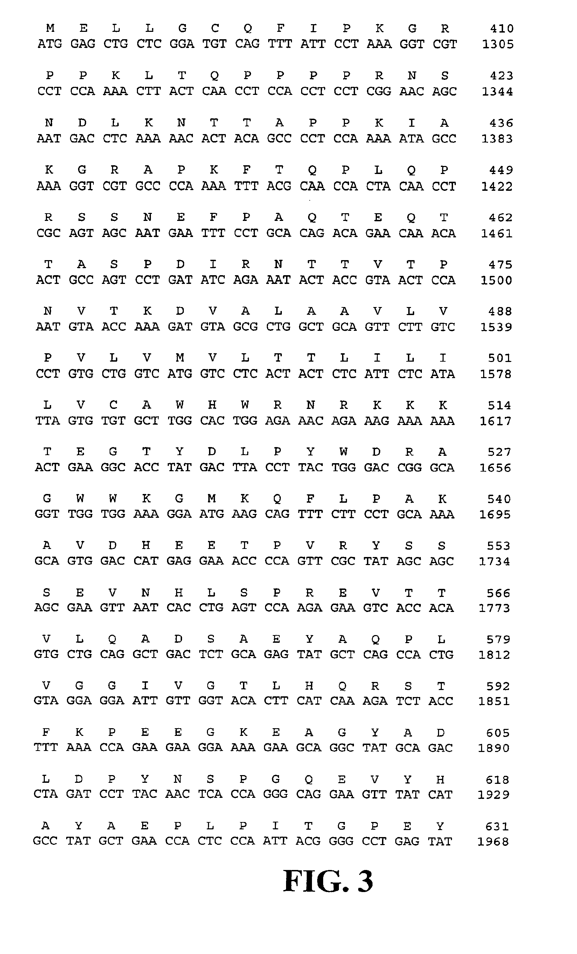

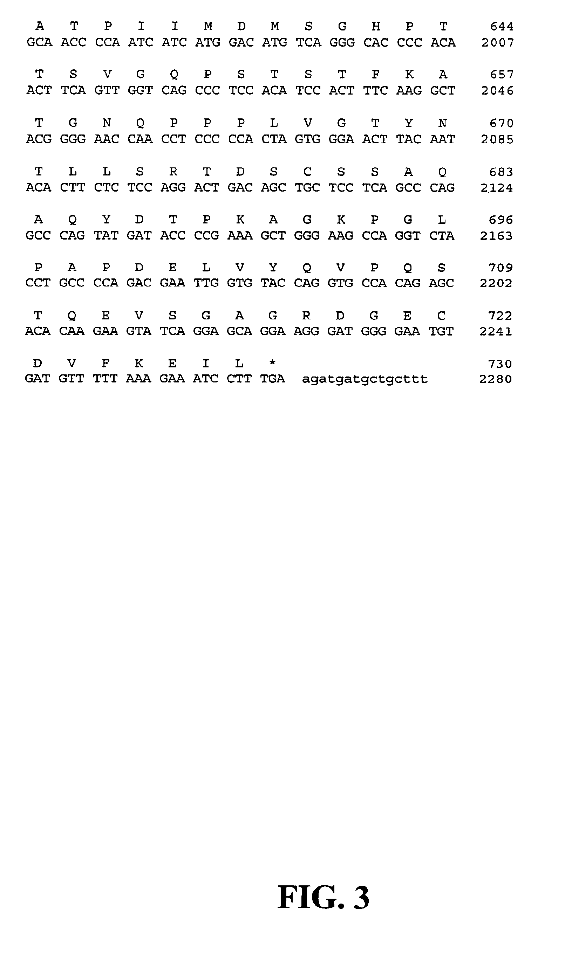

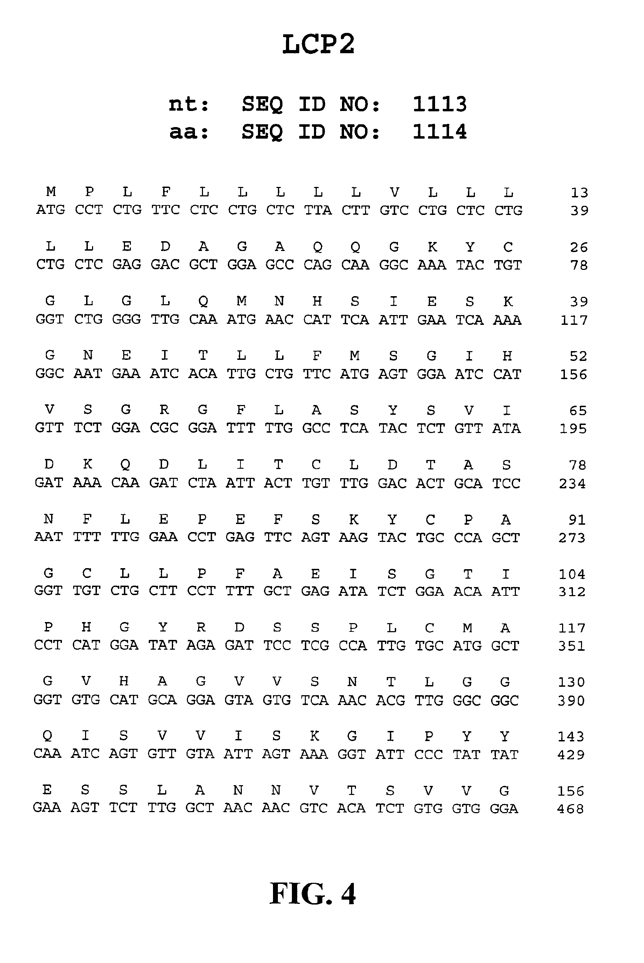

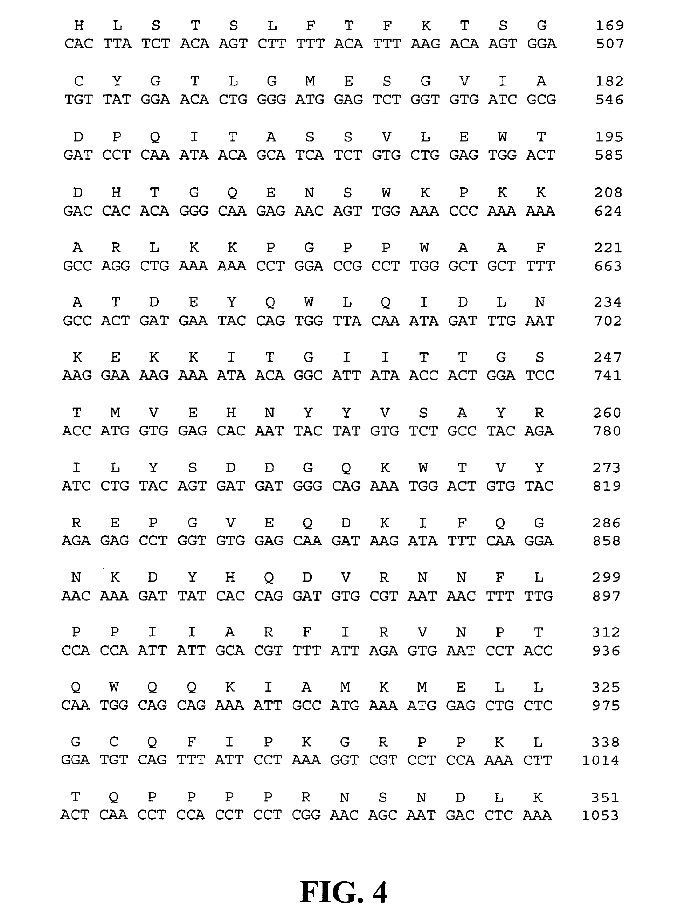

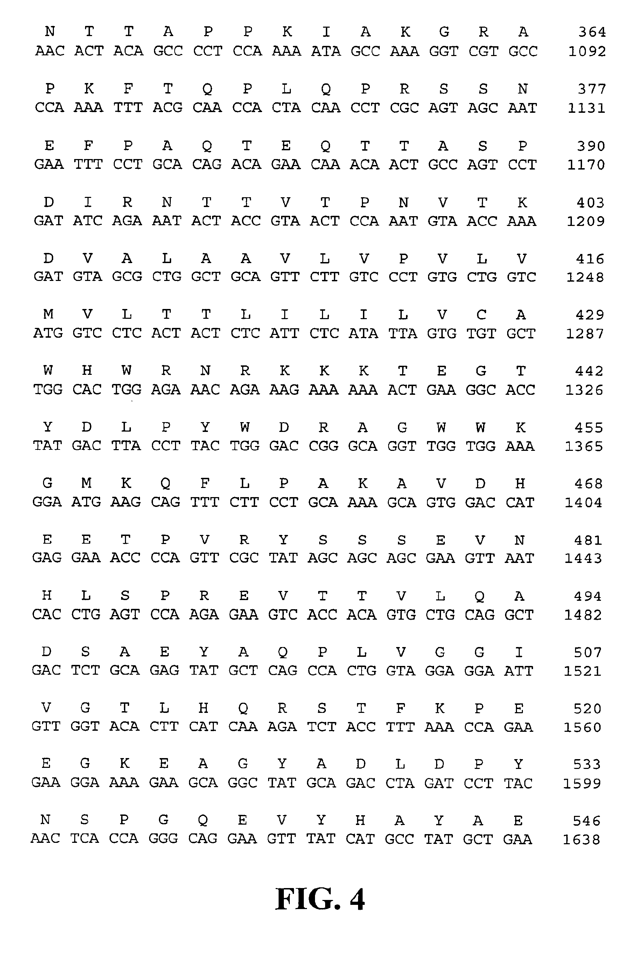

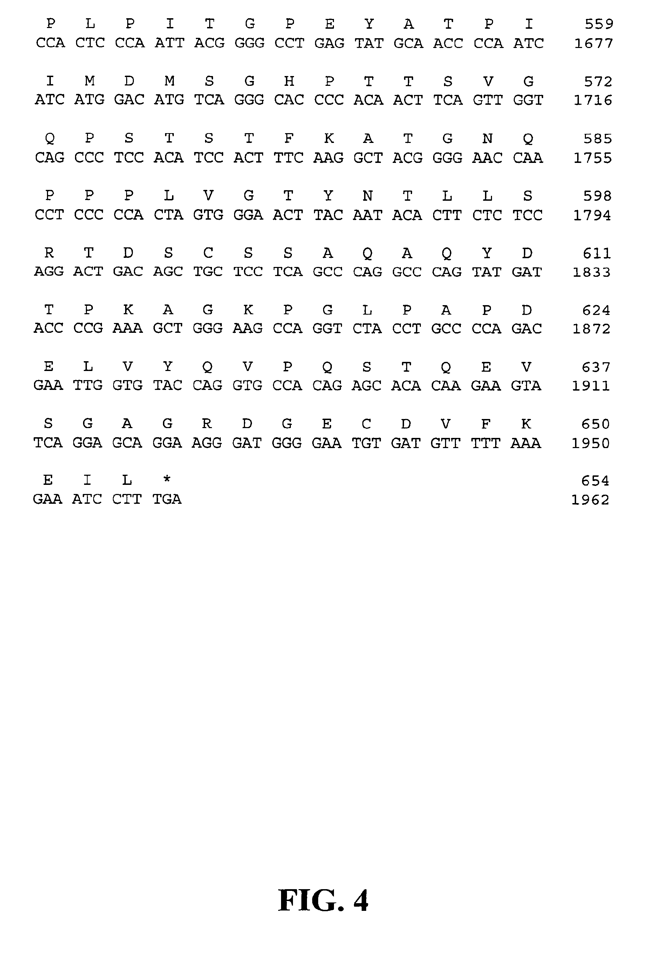

FIGs. 3 and 4 present the nucleotide sequence of the LCP cDNA clones, with predicted amino acid translation; the sequences are further presented in the Sequence Listing, incorporated herein by reference in its entirety, in SEQ ID NOs: 1 (full length nucleotide sequence of human LCP1 cDNA), 3 (full length amino acid coding sequence of human LCP1), 1113 (full length nucleotide sequence of human LCP2 cDNA) and 1114 (full length amino acid coding sequence of human LCP2).

Unless otherwise indicated, each nucleotide sequence is set forth herein as a sequence of deoxyribonucleotides. It is intended, however, that the given sequence be interpreted as would be appropriate to the polynucleotide composition: for example, if the isolated nucleic acid is composed of RNA, the given sequence intends ribonucleotides, with uridine substituted for thymidine.

Unless otherwise indicated, nucleotide sequences of the isolated nucleic acids of the present invention were determined by sequencing a DNA molecule that had resulted, directly or indirectly, from at least one enzymatic polymerization reaction *(e.g.,* reverse transcription and/or polymerase chain reaction) using an automated sequencer (such as the MegaBACE™ 1000, Molecular Dynamics, Sunnyvale, CA, USA), or by reliance upon such sequence or upon genomic sequence prior-accessioned into a public database. Unless otherwise indicated, all amino acid sequences of the polypeptides of the present invention were predicted by translation from the nucleic acid sequences so determined.

As a consequence, any nucleic acid sequence presented herein may contain errors introduced by erroneous incorporation of nucleotides during polymerization, by erroneous base calling by the automated sequencer (although such sequencing errors have been minimized for the nucleic acids directly determined herein, unless otherwise indicated, by the sequencing of each of the complementary strands of a duplex DNA), or by similar errors accessioned into the public database. Such errors can readily be identified and corrected by resequencing of the genomic locus using standard techniques.

Single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Additionally, small deletions and insertions, rather than single nucleotide polymorphisms, are not uncommon in the general population, and often do not alter the function of the protein.

Accordingly, it is an aspect of the present invention to provide nucleic acids not only identical in sequence to those described with particularity herein, but also to provide isolated nucleic acids at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention, as by random or directed mutagenesis.

For purposes herein, percent identity of two nucleic acid sequences is determined using the procedure of Tatiana *et al*., "Blast 2 sequences - a new tool for comparing protein and nucleotide sequences", *FEMS Microbiol Lett.* 174:247-250 (1999), which procedure is effectuated by the computer program BLAST 2 SEQUENCES, available online at
http://www.ncbi.nlm.nih.gov/blast/bl2seq/bl2.html.
To assess percent identity of nucleic acids, the BLASTN module of BLAST 2 SEQUENCES is used with default values of (i) reward for a match: 1; (ii) penalty for a mismatch: -2; (iii) open gap 5 and extension gap 2 penalties; (iv) gap X_dropoff 50 expect 10 word size 11 filter, and both sequences are entered in their entireties.

As is well known, the genetic code is degenerate, with each amino acid except methionine translated from a plurality of codons, thus permitting a plurality of nucleic acids of disparate sequence to encode the identical protein. As is also well known, codon choice for optimal expression varies from species to species. The isolated nucleic acids of the present invention being useful for expression of LCP proteins and protein fragments, it is, therefore, another aspect of the present invention to provide isolated nucleic acids that encode LCP proteins and portions thereof not only identical in sequence to those described with particularity herein, but degenerate variants thereof as well.

As is also well known, amino acid substitutions occur frequently among natural allelic variants, with conservative substitutions often occasioning only *de minimis* change in protein function.

Accordingly, it is an aspect of the present invention to provide nucleic acids not only identical in sequence to those described with particularity herein, but also to provide isolated nucleic acids that encode LCP, and portions thereof, having conservative amino acid substitutions, and also to provide isolated nucleic acids that encode LCP, and portions thereof, having moderately conservative amino acid substitutions.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below *(see* Gonnet *et al*., *Science* 256(5062):1443-5 (1992)):

| | **A** | **R** | **N** | **D** | **C** | **Q** | **E** | **G** | **H** | **I** | **L** | **K** | **M** | **F** | **P** | **S** | **T** | **W** | **Y** | **V** |
|---|---|---|---|---|---|---|---|---|---|---|---|---|---|---|---|---|---|---|---|---|
| **A** | 2 | -1 | 0 | 0 | 0 | 0 | 0 | 0 | -1 | -1 | -1 | 0 | -1 | -2 | 0 | 1 | 1 | -4 | -2 | 0 |
| **R** | -1 | 5 | 0 | 0 | -2 | 2 | 0 | -1 | 1 | -2 | -2 | 3 | -2 | -3 | -1 | 0 | 0 | -2 | -2 | -2 |
| **N** | 0 | 0 | 4 | 2 | -2 | 1 | 1 | 0 | 1 | -3 | -3 | 1 | -2 | -3 | -1 | 1 | 0 | -4 | -1 | -2 |
| **D** | 0 | 0 | 2 | 5 | -3 | 1 | 3 | 0 | 0 | -4 | -4 | 0 | -3 | -4 | -1 | 0 | 0 | -5 | -3 | -3 |
| **C** | 0 | -2 | -2 | -3 | 12 | -2 | -3 | -2 | -1 | -1 | -2 | -3 | -1 | -1 | -3 | 0 | 0 | -1 | 0 | 0 |
| **Q** | 0 | 2 | 1 | 1 | -2 | 3 | 2 | -1 | 1 | -2 | -2 | 2 | -1 | -3 | 0 | 0 | 0 | -3 | -2 | -2 |
| **E** | 0 | 0 | 1 | 3 | -3 | 2 | 4 | -1 | 0 | -3 | -3 | 1 | -2 | -4 | 0 | 0 | 0 | -4 | -3 | -2 |
| **G** | 0 | -1 | 0 | 0 | -2 | -1 | -1 | 7 | -1 | -4 | -4 | -1 | -4 | -5 | -2 | 0 | -1 | -4 | -4 | -3 |
| **H** | -1 | 1 | 1 | 0 | -1 | 1 | 0 | -1 | 6 | -2 | -2 | 1 | -1 | 0 | -1 | 0 | 0 | -1 | 2 | -2 |
| **I** | -1 | -2 | -3 | -4 | -1 | -2 | -3 | -4 | -2 | 4 | 3 | -2 | 2 | 1 | -3 | -2 | -1 | -2 | -1 | 3 |
| **L** | -1 | -2 | -3 | -4 | -2 | -2 | -3 | -4 | -2 | 3 | 4 | -2 | 3 | 2 | -2 | -2 | -1 | -1 | 0 | 2 |
| **K** | 0 | 3 | 1 | 0 | -3 | 2 | 1 | -1 | 1 | -2 | -2 | 3 | -1 | -3 | -1 | 0 | 0 | -4 | -2 | -2 |
| **M** | -1 | -2 | -2 | -3 | -1 | -1 | -2 | -4 | -1 | 2 | 3 | -1 | 4 | 2 | -2 | -1 | -1 | -1 | 0 | 2 |
| **F** | -2 | -3 | -3 | -4 | -1 | -3 | -4 | -5 | 0 | 1 | 2 | -3 | 2 | 7 | -4 | -3 | -2 | 4 | 5 | 0 |
| **P** | 0 | -1 | -1 | -1 | -3 | 0 | 0 | -2 | -1 | -3 | -2 | -1 | -2 | -4 | 8 | 0 | 0 | -5 | -3 | -2 |
| **S** | 1 | 0 | 1 | 0 | 0 | 0 | 0 | 0 | 0 | -2 | -2 | 0 | -1 | -3 | 0 | 2 | 2 | -3 | -2 | -1 |
| **T** | 1 | 0 | 0 | 0 | 0 | 0 | 0 | -1 | 0 | -1 | -1 | 0 | -1 | -2 | 0 | 2 | 2 | -4 | -2 | 0 |
| **W** | -4 | -2 | -4 | -5 | -1 | -3 | -4 | -4 | -1 | -2 | -1 | -4 | -1 | 4 | -5 | -3 | -4 | 14 | 4 | -3 |
| **Y** | -2 | -2 | -1 | -3 | 0 | -2 | -3 | -4 | 2 | -1 | 0 | -2 | 0 | 5 | -3 | -2 | -2 | 4 | 8 | -1 |
| **V** | 0 | -2 | -2 | -3 | 0 | -2 | -2 | -3 | -2 | 3 | 2 | -2 | 2 | 0 | -2 | -1 | 0 | -3 | -1 | 3 |

For purposes herein, a "moderately conservative" replacement is any change having a nonnegative value in the PAM250 log-likelihood matrix reproduced herein above.

As is also well known in the art, relatedness of nucleic acids can also be characterized using a functional test, the ability of the two nucleic acids to base-pair to one another at defined hybridization stringencies.

It is, therefore, another aspect of the invention to provide isolated nucleic acids not only identical in sequence to those described with particularity herein, but also to provide isolated nucleic acids ("cross-hybridizing nucleic acids") that hybridize under high stringency conditions (as defined herein below) to all or to a portion of various of the isolated LCP nucleic acids of the present invention ("reference nucleic acids"), as well as cross-hybridizing nucleic acids that hybridize under moderate stringency conditions to all or to a portion of various of the isolated LCP nucleic acids of the present invention.

Such cross-hybridizing nucleic acids are useful, *inter alia,* as probes for, and to drive expression of, proteins related to the proteins of the present invention as alternative isoforms, homologues, paralogues, and orthologues. Particularly useful orthologues are those from other primate species, such as chimpanzee, rhesus macaque, monkey, baboon, orangutan, and gorilla; from rodents, such as rats, mice, guinea pigs; from lagomorphs, such as rabbits; and from domestic livestock, such as cow, pig, sheep, horse, goat and chicken.

For purposes herein, high stringency conditions are defined as aqueous hybridization (*i.e.*, free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. For purposes herein, moderate stringency conditions are defined as aqueous hybridization *(i.e.,* free of formamide) in 6X SSC, 1% SDS at 65°C for at least 8 hours, followed by one or more washes in 2x SSC, 0.1% SDS at room temperature.

The hybridizing portion of the reference nucleic acid is typically at least 15 nucleotides in length, often at least 17 nucleotides in length. Often, however, the hybridizing portion of the reference nucleic acid is at least 20 nucleotides in length, 25 nucleotides in length, and even 30 nucleotides, 35 nucleotides, 40 nucleotides, and 50 nucleotides in length. Of course, cross-hybridizing nucleic acids that hybridize to a larger portion of the reference nucleic acid - for example, to a portion of at least 50 nt, at least 100 nt, at least 150 nt, 200 nt, 250 nt, 300 nt, 350 nt, 400 nt, 450 nt, or 500 nt or more - or even to the entire length of the reference nucleic acid, are also useful.

The hybridizing portion of the cross-hybridizing nucleic acid is at least 75% identical in sequence to at least a portion of the reference nucleic acid. Typically, the hybridizing portion of the cross-hybridizing nucleic acid is at least 80%, often at least 85%, 86%, 87%, 88%, 89% or even at least 90% identical in sequence to at least a portion of the reference nucleic acid. Often, the hybridizing portion of the cross-hybridizing nucleic acid will be at least 91%, 92%, 93%, 94%, 95%, 96%, 97%, 98%, or 99% identical in sequence to at least a portion of the reference nucleic acid sequence. At times, the hybridizing portion of the cross-hybridizing nucleic acid will be at least 99.5% identical in sequence to at least a portion of the reference nucleic acid.

The invention also provides fragments of various of the isolated nucleic acids of the present invention.

By "fragments" of a reference nucleic acid is here intended isolated nucleic acids, however obtained, that have a nucleotide sequence identical to a portion of the reference nucleic acid sequence, which portion is at least 17 nucleotides and less than the entirety of the reference nucleic acid. As so defined, "fragments" need not be obtained by physical fragmentation of the reference nucleic acid, although such provenance is not thereby precluded.

In theory, an oligonucleotide of 17 nucleotides is of sufficient length as to occur at random less frequently than once in the three gigabase human genome, and thus to provide a nucleic acid probe that can uniquely identify the reference sequence in a nucleic acid mixture of genomic complexity. As is well known, further specificity can be obtained by probing nucleic acid samples of subgenomic complexity, and/or by using plural fragments as short as 17 nucleotides in length collectively to prime amplification of nucleic acids, as, *e.g.*, by polymerase chain reaction (PCR).

As further described herein below, nucleic acid fragments that encode at least 6 contiguous amino acids (*i*.*e*., fragments of 18 nucleotides or more) are useful in directing the expression or the synthesis of peptides that have utility in mapping the epitopes of the protein encoded by the reference nucleic acid. *See, e.g.,* Geysen *et al*., "Use of peptide synthesis to probe viral antigens for epitopes to a resolution of a single amino acid," *Proc*. *Natl*. *Acad*. *Sci*. *USA* 81:3998-4002 (1984); and U.S. Pat. Nos. 4,708,871 and 5,595,915, the disclosures of which are incorporated herein by reference in their entireties.

As further described herein below, fragments that encode at least 8 contiguous amino acids *(i.e.,* fragments of 24 nucleotides or more) are useful in directing the expression or the synthesis of peptides that have utility as immunogens. *See, e.g.*, Lerner, "Tapping the immunological repertoire to produce antibodies of predetermined specificity," Nature 299:592-596 (1982); Shinnick *et al.,* "Synthetic peptide immunogens as vaccines," *Annu. Rev. Microbiol.* 37:425-46 (1983); Sutcliffe *et al*., "Antibodies that react with predetermined sites on proteins," *Science* 219:660-6 (1983), the disclosures of which are incorporated herein by reference in their entireties.

The nucleic acid fragment of the present invention is thus at least 17 nucleotides in length, typically at least 18 nucleotides in length, and often at least 24 nucleotides in length. Often, the nucleic acid of the present invention is at least 25 nucleotides in length, and even 30 nucleotides, 35 nucleotides, 40 nucleotides, or 45 nucleotides in length. Of course, larger fragments having at least 50 nt, at least 100 nt, at least 150 nt, 200 nt, 250 nt, 300 nt, 350 nt, 400 nt, 450 nt, or 500 nt or more are also useful, and at times preferred.

Having been based upon the mining of genomic sequence, rather than upon surveillance of expressed message, the present invention further provides isolated genome-derived nucleic acids that include portions of the LCP gene.

The invention particularly provides genome-derived single exon probes.

As further described in commonly owned and copending U.S. patent application serial nos. 09/864,761, filed May 23, 2001; 09/774,203, filed January 29, 2001; and 09/632,366, filed August 3, 2000, the disclosures of which are incorporated herein by reference in their entireties, "a single exon probe" comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and instead consist of one or more exons that are found adjacent to the reference exon in the genome.

Genome-derived single exon probes typically further comprise, contiguous to a first end of the exon portion, a first intronic and/or intergenic sequence that is identically contiguous to the exon in the genome. Often, the genome-derived single exon probe further comprises, contiguous to a second end of the exonic portion, a second intronic and/or intergenic sequence that is identically contiguous to the exon in the genome.

The minimum length of genome-derived single exon probes is defined by the requirement that the exonic portion be of sufficient length to hybridize under high stringency conditions to transcript-derived nucleic acids. Accordingly, the exon portion is at least 17 nucleotides, typically at least 18 nucleotides, 20 nucleotides, 24 nucleotides, 25 nucleotides or even 30, 35, 40, 45, or 50 nucleotides in length, and can usefully include the entirety of the exon, up to 100 nt, 150 nt, 200 nt, 250 nt, 300 nt, 350 nt, 400 nt or even 500 nt or more in length.

The maximum length of genome-derived single exon probes is defined by the requirement that the probes contain portions of no more than one exon, that is, be unable to hybridize detectably under high stringency conditions to nucleic acids that lack the reference exon but include one or more exons that are found adjacent to the reference exon the genome.

Given variable spacing of exons through eukaryotic genomes, the maximum length of single exon probes of the present invention is typically no more than 25 kb, often no more than 20 kb, 15 kb, 10 kb or 7.5 kb, or even no more than 5 kb, 4 kb, 3 kb, or even no more than about 2.5 kb in length.

The genome-derived single exon probes of the present invention can usefully include at least a first terminal priming sequence not found in contiguity with the rest of the probe sequence in the genome, and often will contain a second terminal priming sequence not found in contiguity with the rest of the probe sequence in the genome.

The present invention also provides isolated genome-derived nucleic acids that include nucleic acid sequence elements that control transcription of the LCP gene.

With a complete draft of the human genome now available, genomic sequences that are within the vicinity of the LCP coding region (and that are additional to those described with particularity herein) can readily be obtained by PCR amplification.

The isolated nucleic acids of the present invention can be composed of natural nucleotides in native 5'-3' phosphodiester internucleoside linkage - *e.g.,* DNA or RNA - or can contain any or all of nonnatural nucleotide analogues, nonnative internucleoside bonds, or post-synthesis modifications, either throughout the length of the nucleic acid or localized to one or more portions thereof.

As is well known in the art, when the isolated nucleic acid is used as a hybridization probe, the range of such nonnatural analogues, nonnative internucleoside bonds, or post-synthesis modifications will be limited to those that permit sequence-discriminating basepairing of the resulting nucleic acid. When used to direct expression or RNA or protein *in vitro* or *in vivo,* the range of such nonnatural analogues, nonnative internucleoside bonds, or post-synthesis modifications will be limited to those that permit the nucleic acid to function properly as a polymerization substrate. When the isolated nucleic acid is used as a therapeutic agent, the range of such changes will be limited to those that do not confer toxicity upon the isolated nucleic acid.

For example, when desired to be used as probes, the isolated nucleic acids of the present invention can usefully include nucleotide analogues that incorporate labels that are directly detectable, such as radiolabels or fluorophores, or nucleotide analogues that incorporate labels that can be visualized in a subsequent reaction, such as biotin or various haptens.

Common radiolabeled analogues include those labeled with ³³P, ³²P, and ³⁵S, such as α-³²P-dATP, α-³²P-dCTP, α-³²P-dGTP, α-³²P-dTTP, α-³²P-3'dATP, α-³²P-ATP, α-³²P-CTP, α-³²P-GTP, α-³²P-UTP, α-³⁵S-dATP, γ-³⁵S-GTP, γ-³³P-dATP, and the like.

Commercially available fluorescent nucleotide analogues readily incorporated into the nucleic acids of the present invention include Cy3-dCTP, Cy3-dUTP, Cy5-dCTP, Cy3-dUTP (Amersham Pharmacia Biotech, Piscataway, New Jersey, USA), fluorescein-12-dUTP, tetramethylrhodamine-6-dUTP, Texas Red®-5-dUTP, Cascade Blue®-7-dUTP, BODIPY® FL-14-dUTP, BODIPY® TMR-14-dUTP, BODIPY® TR-14-dUTP, Rhodamine Green™-5-dUTP, Oregon Green® 488-5-dUTP, Texas Red®-12-dUTP, BODIPY® 630/650-14-dUTP, BODIPY® 650/665-14-dUTP, Alexa Fluor® 488-5-dUTP, Alexa Fluor® 532-5-dUTP, Alexa Fluor® 568-5-dUTP, Alexa Fluor® 594-5-dUTP, Alexa Fluor® 546-14-dUTP, fluorescein-12-UTP, tetramethylrhodamine-6-UTP, Texas Red®-5-UTP, Cascade Blue®-7-UTP, BODIPY® FL-14-UTP, BODIPY® TMR-14-UTP, BODIPY® TR-14-UTP, Rhodamine Green™-5-UTP, Alexa Fluor® 488-5-UTP, Alexa Fluor® 546-14-UTP (Molecular Probes, Inc. Eugene, OR, USA).

Protocols are available for custom synthesis of nucleotides having other fluorophores. Henegariu *et al*., "Custom Fluorescent-Nucleotide Synthesis as an Alternative Method for Nucleic Acid Labeling," *Nature Biotechnol.* 18:345 - 348 (2000), the disclosure of which is incorporated herein by reference in its entirety.

Haptens that are commonly conjugated to nucleotides for subsequent labeling include biotin (biotin-11-dUTP, Molecular Probes, Inc., Eugene, OR, USA; biotin-21-UTP, biotin-21-dUTP, Clontech Laboratories, Inc., Palo Alto, CA, USA), digoxigenin (DIG-11-dUTP, alkali labile, DIG-11-UTP, Roche Diagnostics Corp., Indianapolis, IN, USA), and dinitrophenyl (dinitrophenyl-11-dUTP, Molecular Probes, Inc., Eugene, OR, USA).

As another example, when desired to be used for antisense inhibition of transcription or translation, the isolated nucleic acids of the present invention can usefully include altered, often nuclease-resistant, internucleoside bonds. *See* Hartmann *et al*. (eds.), Manual of Antisense Methodology (Perspectives in Antisense Science), Kluwer Law International (1999) (ISBN:079238539X); Stein *et al*. (eds.), Applied Antisense Oligonucleotide Technology, Wiley-Liss (cover (1998) (ISBN: 0471172790); Chadwick *et al*. (eds.), Oligonucleotides as Therapeutic Agents - Symposium No. 209, John Wiley & Son Ltd (1997) (ISBN: 0471972797), the disclosures of which are incorporated herein by reference in their entireties. Such altered internucloside bonds are often desired also when the isolated nucleic acid of the present invention is to be used for targeted gene correction, Gamper *et al., Nucl. Acids Res.* 28(21):4332-4339 (2000), the disclosures of which are incorporated herein by reference in its entirety.

Modified oligonucleotide backbones often preferred when the nucleic acid is to be used for antisense purposes are, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Representative U.S. patents that teach the preparation of the above phosphorus-containing linkages include, but are not limited to, U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, the disclosures of which are incorporated herein by reference in their entireties.

Preferred modified oligonucleotide backbones for antisense use that do not include a phosphorus atom have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Representative U.S. patents that teach the preparation of the above backbones include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, the disclosures of which are incorporated herein by reference in their entireties.

In other preferred oligonucleotide mimetics, both the sugar and the internucleoside linkage are replaced with novel groups, such as peptide nucleic acids (PNA).

In PNA compounds, the phosphodiester backbone of the nucleic acid is replaced with an amide-containing backbone, in particular by repeating N-(2-aminoethyl) glycine units linked by amide bonds. Nucleobases are bound directly or indirectly to aza nitrogen atoms of the amide portion of the backbone, typically by methylene carbonyl linkages.

The uncharged nature of the PNA backbone provides PNA/DNA and PNA/RNA duplexes with a higher thermal stability than is found in DNA/DNA and DNA/RNA duplexes, resulting from the lack of charge repulsion between the PNA and DNA or RNA strand. In general, the Tm of a PNA/DNA or PNA/RNA duplex is 1°C higher per base pair than the Tm of the corresponding DNA/DNA or DNA/RNA duplex (in 100 mM NaCl).

The neutral backbone also allows PNA to form stable DNA duplexes largely independent of salt concentration. At low ionic strength, PNA can be hybridized to a target sequence at temperatures that make DNA hybridization problematic or impossible. And unlike DNA/DNA duplex formation, PNA hybridization is possible in the absence of magnesium. Adjusting the ionic strength, therefore, is useful if competing DNA or RNA is present in the sample, or if the nucleic acid being probed contains a high level of secondary structure.

PNA also demonstrates greater specificity in binding to complementary DNA. A PNA/DNA mismatch is more destabilizing than DNA/DNA mismatch. A single mismatch in mixed a PNA/DNA 15-mer lowers the Tm by 8-20°C (15°C on average). In the corresponding DNA/DNA duplexes, a single mismatch lowers the Tm by 4-16°C (11°C on average). Because PNA probes can be significantly shorter than DNA probes, their specificity is greater.

Additionally, nucleases and proteases do not recognize the PNA polyamide backbone with nucleobase sidechains. As a result, PNA oligomers are resistant to degradation by enzymes, and the lifetime of these compounds is extended both *in vivo* and *in vitro.* In addition, PNA is stable over a wide pH range.

Because its backbone is formed from amide bonds, PNA can be synthesized using a modified peptide synthesis protocol. PNA oligomers can be synthesized by both Fmoc and tBoc methods. Representative U.S. patents that teach the preparation of PNA compounds include, but are not limited to, U.S. Pat. Nos. 5,539,082; 5,714,331; and 5,719,262, each of which is herein incorporated by reference; automated PNA synthesis is readily achievable on commercial synthesizers (*see, e.g.*, "PNA User's Guide," Rev. 2, February 1998, Perseptive Biosystems Part No. 60138, Applied Biosystems, Inc., Foster City, CA).

PNA chemistry and applications are reviewed, *inter alia,* in Ray *et al*., *FASEB J.* 14(9):1041-60 (2000); Nielsen *et al*., *Pharmacol Toxicol*. 86(1):3-7 (2000); Larsen *et al*., *Biochim* *Biophys Acta.* 1489(1):159-66 (1999); Nielsen, *Curr. Opin. Struct. Biol.* 9(3):353-7 (1999), and Nielsen, *Curr. Opin. Biotechnol.* 10(1):71-5 (1999), the disclosures of which are incorporated herein by reference in their entireties.

Differences from nucleic acid compositions found in nature - *e.g.,* nonnative bases, altered internucleoside linkages, post-synthesis modification - can be present throughout the length of the nucleic acid or can, instead, usefully be localized to discrete portions thereof. As an example of the latter, chimeric nucleic acids can be synthesized that have discrete DNA and RNA domains and demonstrated utility for targeted gene repair, as further described in U.S. Pat. Nos. 5,760,012 and 5,731,181, the disclosures of which are incorporated herein by reference in their entireties. As another example, chimeric nucleic acids comprising both DNA and PNA have been demonstrated to have utility in modified PCR reactions. *See* Misra *et al*., *Biochem.* 37: 1917-1925 (1998); *see also* Finn *et al*., *Nucl*. *Acids Res.* 24: 3357-3363 (1996), incorporated herein by reference.

Unless otherwise specified, nucleic acids of the present invention can include any topological conformation appropriate to the desired use; the term thus explicitly comprehends, among others, single-stranded, double-stranded, triplexed, quadruplexed, partially double-stranded, partially-triplexed, partially-quadruplexed, branched, hairpinned, circular, and padlocked conformations. Padlock conformations and their utilities are further described in Banér *et al., Curr. Opin. Biotechnol.* 12:11-15 (2001); Escude *et al.,* Proc. Natl. Acad. Sci. USA 14;96(19):10603-7 (1999); Nilsson *et al., Science* 265(5181):2085-8 (1994), the disclosures of which are incorporated herein by reference in their entireties. Triplex and quadruplex conformations, and their utilities, are reviewed in Praseuth *et al*., *Biochim*. *Biophys*. *Acta*. 1489(1):181-206 (1999); Fox, *Curr*. *Med*. *Chem.* 7(1):17-37 (2000); Kochetkova *et al*., *Methods Mol*. *Biol.* 130:189-201 (2000); Chan *et al*., *J*. *Mol. Med.* 75(4):267-82 (1997), the disclosures of which are incorporated herein by reference in their entireties.

The nucleic acids of the present invention can be detectably labeled.

Commonly-used labels include radionuclides, such as ³²P, ³³P, ³⁵S, ³H (and for NMR detection, ¹³C and ¹⁵N), haptens that can be detected by specific antibody or high affinity binding partner (such as avidin), and fluorophores.

As noted above, detectable labels can be incorporated by inclusion of labeled nucleotide analogues in the nucleic acid. Such analogues can be incorporated by enzymatic polymerization, such as by nick translation, random priming, polymerase chain reaction (PCR), terminal transferase tailing, and end-filling of overhangs, for DNA molecules, and *in vitro* transcription driven, *e.g.,* from phage promoters, such as T7, T3, and SP6, for RNA molecules. Commercial kits are readily available for each such labeling approach.

Analogues can also be incorporated during automated solid phase chemical synthesis.

As is well known, labels can also be incorporated after nucleic acid synthesis, with the 5' phosphate and 3' hydroxyl providing convenient sites for post-synthetic covalent attachment of detectable labels.

Various other post-synthetic approaches permit internal labeling of nucleic acids.

For example, fluorophores can be attached using a cisplatin reagent that reacts with the N7 of guanine residues (and, to a lesser extent, adenine bases) in DNA, RNA, and PNA to provide a stable coordination complex between the nucleic acid and fluorophore label (Universal Linkage System) (available from Molecular Probes, Inc., Eugene, OR, USA and Amersham Pharmacia Biotech, Piscataway, NJ, USA); *see* Alers *et al*., *Genes, Chromosomes & Cancer,* Vol. 25, pp. 301 - 305 (1999); Jelsma *et al*., *J*. *NIH Res.* 5:82 (1994); Van Belkum *et al*., *BioTechniques* 16:148-153 (1994), incorporated herein by reference. As another example, nucleic acids can be labeled using a disulfide-containing linker (FastTag™ Reagent, Vector Laboratories, Inc., Burlingame, CA, USA) that is photo- or thermally coupled to the target nucleic acid using aryl azide chemistry; after reduction, a free thiol is available for coupling to a hapten, fluorophore, sugar, affinity ligand, or other marker.

Multiple independent or interacting labels can be incorporated into the nucleic acids of the present invention.

For example, both a fluorophore and a moiety that in proximity thereto acts to quench fluorescence can be included to report specific hybridization through release of fluorescence quenching, Tyagi *et al*., *Nature Biotechnol*. 14: 303-308 (1996); Tyagi *et al*., *Nature Biotechnol*. 16, 49-53 (1998); Sokol *et al*., *Proc*. *Natl*. *Acad*. *Sci*. *USA* 95: 11538-11543 (1998); Kostrikis *et al*., *Science* 279:1228-1229 (1998); Marras *et al*., *Genet. Anal.* 14: 151-156 (1999); U.S. Pat. Nos. 5,846,726, 5,925,517, 5925517, or to report exonucleotidic excision, U.S. Pat. No. 5,538,848; Holland *et al., Proc. Natl. Acad. Sci. USA* 88:7276-7280 (1991); Heid *et al., Genome Res.* 6(10):986-94 (1996); Kuimelis *et al., Nucleic Acids Symp Ser.* (37):255-6 (1997); U.S. Patent No. 5,723,591, the disclosures of which are incorporated herein by reference in their entireties.

So labeled, the isolated nucleic acids of the present invention can be used as probes, as further described below.

Nucleic acids of the present invention can also usefully be bound to a substrate. The substrate can porous or solid, planar or non-planar, unitary or distributed; the bond can be covalent or noncovalent. Bound to a substrate, nucleic acids of the present invention can be used as probes in their unlabeled state.

For example, the nucleic acids of the present invention can usefully be bound to a porous substrate, commonly a membrane, typically comprising nitrocellulose, nylon, or positively-charged derivatized nylon; so attached, the nucleic acids of the present invention can be used to detect LCP nucleic acids present within a labeled nucleic acid sample, either a sample of genomic nucleic acids or a sample of transcript-derived nucleic acids, *e.g.* by reverse dot blot.

The nucleic acids of the present invention can also usefully be bound to a solid substrate, such as glass, although other solid materials, such as amorphous silicon, crystalline silicon, or plastics, can also be used. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof.

Typically, the solid substrate will be rectangular, although other shapes, particularly disks and even spheres, present certain advantages. Particularly advantageous alternatives to glass slides as support substrates for array of nucleic acids are optical discs, as described in Demers, "Spatially Addressable Combinatorial Chemical Arrays in CD-ROM Format," international patent publication WO 98/12559, incorporated herein by reference in its entirety.

The nucleic acids of the present invention can be attached covalently to a surface of the support substrate or applied to a derivatized surface in a chaotropic agent that facilitates denaturation and adherence by presumed noncovalent interactions, or some combination thereof.

The nucleic acids of the present invention can be bound to a substrate to which a plurality of other nucleic acids are concurrently bound, hybridization to each of the plurality of bound nucleic acids being separately detectable. At low density, *e.g.* on a porous membrane, these substrate-bound collections are typically denominated macroarrays; at higher density, typically on a solid support, such as glass, these substrate bound collections of plural nucleic acids are colloquially termed microarrays. As used herein, the term microarray includes arrays of all densities. It is, therefore, another aspect of the invention to provide microarrays that include the nucleic acids of the present invention.

The isolated nucleic acids of the present invention can be used as hybridization probes to detect, characterize, and quantify LCP nucleic acids in, and isolate LCP nucleic acids from, both genomic and transcript-derived nucleic acid samples. When free in solution, such probes are typically, but not invariably, detectably labeled; bound to a substrate, as in a microarray, such probes are typically, but not invariably unlabeled.

For example, the isolated nucleic acids of the present invention can be used as probes to detect and characterize gross alterations in the LCP genomic locus, such as deletions, insertions, translocations, and duplications of the LCP genomic locus through fluorescence *in situ* hybridization (FISH) to chromosome spreads. *See, e*.*g*., Andreeff *et al*. (eds.), Introduction to Fluorescence *In Situ* Hybridization: Principles and Clinical Applications, John Wiley & Sons (1999) (ISBN: 0471013455), the disclosure of which is incorporated herein by reference in its entirety. The isolated nucleic acids of the present invention can be used as probes to assess smaller genomic alterations using, *e.g.,* Southern blot detection of restriction fragment length polymorphisms. The isolated nucleic acids of the present invention can be used as probes to isolate genomic clones that include the nucleic acids of the present invention, which thereafter can be restriction mapped and sequenced to identify deletions, insertions, translocations, and substitutions (single nucleotide polymorphisms, SNPs) at the sequence level.

The isolated nucleic acids of the present invention can also be used as probes to detect, characterize, and quantify LCP nucleic acids in, and isolate LCP nucleic acids from, transcript-derived nucleic acid samples.

For example, the isolated nucleic acids of the present invention can be used as hybridization probes to detect, characterize by length, and quantify LCP mRNA by northern blot of total or poly-A⁺- selected RNA samples. For example, the isolated nucleic acids of the present invention can be used as hybridization probes to detect, characterize by location, and quantify LCP message by *in situ* hybridization to tissue sections (see, *e.g.,* Schwarchzacher *et al*., In Situ Hybridization, Springer-Verlag New York (2000) (ISBN: 0387915966), the disclosure of which is incorporated herein by reference in its entirety). For example, the isolated nucleic acids of the present invention can be used as hybridization probes to measure the representation of LCP clones in a cDNA library. For example, the isolated nucleic acids of the present invention can be used as hybridization probes to isolate LCP nucleic acids from cDNA libraries, permitting sequence level characterization of LCP messages, including identification of deletions, insertions, truncations ― including deletions, insertions, and truncations of exons in alternatively spliced forms - and single nucleotide polymorphisms.

All of the aforementioned probe techniques are well within the skill in the art, and are described at greater length in standard texts such as Sambrook *et al*., Molecular Cloning: A Laboratory Manual (3^{rd} ed.), Cold Spring Harbor Laboratory Press (2001) (ISBN: 0879695773); Ausubel *et al*. (eds.), Short Protocols in Molecular Biology: A Compendium of Methods from Current Protocols in Molecular Biology (4^{th} ed.), John Wiley & Sons, 1999 (ISBN: 047132938X); and Walker *et al*. (eds.), The Nucleic Acids Protocols Handbook, Humana Press (2000) (ISBN: 0896034593), the disclosures of which are incorporated herein by reference in their entirety.

As described in the Examples herein below, the nucleic acids of the present invention can also be used to detect and quantify LCP nucleic acids in transcript-derived samples - that is, to measure expression of the LCP gene - when included in a microarray. Measurement of LCP expression has particular utility as LCP proteins have potential therapeutic as well as diagnostic roles for neurological and developmental disorders, as well as diseases involving cell-cell adhesion process, as further described in the Examples herein below.

As would be readily apparent to one of skill in the art, each LCP nucleic acid probe - whether labeled, substrate-bound, or both - is thus currently available for use as a tool for measuring the level of LCP expression in each of the tissues in which expression has already been confirmed, notably adrenal, adult liver, bone marrow, brain, fetal liver, heart, kidney, lung, placenta, skeletal muscle, colon and prostate. The utility is specific to the probe: under high stringency conditions, the probe reports the level of expression of message specifically containing that portion of the LCP gene included within the probe.

Measuring tools are well known in many arts, not just in molecular biology, and are known to possess credible, specific, and substantial utility. For example, U.S. Patent No. 6,016,191 describes and claims a tool for measuring characteristics of fluid flow in a hydrocarbon well; U.S. Patent No. 6,042,549 describes and claims a device for measuring exercise intensity; U.S. Patent No. 5,889,351 describes and claims a device for measuring viscosity and for measuring characteristics of a fluid; U.S. Patent No. 5,570,694 describes and claims a device for measuring blood pressure; U.S. Patent No. 5,930,143 describes and claims a device for measuring the dimensions of machine tools; U.S. Patent No. 5,279,044 describes and claims a measuring device for determining an absolute position of a movable element; U.S. Patent No. 5,186,042 describes and claims a device for measuring action force of a wheel; and U.S. Patent No. 4,246,774 describes and claims a device for measuring the draft of smoking articles such as cigarettes.

As for tissues not yet demonstrated to express LCP, the LCP nucleic acid probes of the present invention are currently available as tools for surveying such tissues to detect the presence of LCP nucleic acids.

Survey tools - *i.e.,* tools for determining the presence and/or location of a desired object by search of an area - are well known in many arts, not just in molecular biology, and are known to possess credible, specific, and substantial utility. For example, U.S. Patent No. 6,046,800 describes and claims a device for surveying an area for objects that move; U.S. Patent No. 6,025,201 describes and claims an apparatus for locating and discriminating platelets from non-platelet particles or cells on a cell-by-cell basis in a whole blood sample; U.S. Patent No. 5,990,689 describes and claims a device for detecting and locating anomalies in the electromagnetic protection of a system; U.S. Patent No. 5,984,175 describes and claims a device for detecting and identifying wearable user identification units; U.S. Patent No. 3,980,986 ("Oil well survey tool"), describes and claims a tool for finding the position of a drill bit working at the bottom of a borehole.

As noted above, the nucleic acid probes of the present invention are useful in constructing microarrays; the microarrays, in turn, are products of manufacture that are useful for measuring and for surveying gene expression.

When included on a microarray, each LCP nucleic acid probe makes the microarray specifically useful for detecting that portion of the LCP gene included within the probe, thus imparting upon the microarray device the ability to detect a signal where, absent such probe, it would have reported no signal. This utility makes each individual probe on such microarray akin to an antenna, circuit, firmware or software element included in an electronic apparatus, where the antenna, circuit, firmware or software element imparts upon the apparatus the ability newly and additionally to detect signal in a portion of the radio-frequency spectrum where previously it could not; such devices are known to have specific, substantial, and credible utility.

Changes in the level of expression need not be observed for the measurement of expression to have utility.

For example, where gene expression analysis is used to assess toxicity of chemical agents on cells, the failure of the agent to change a gene's expression level is evidence that the drug likely does not affect the pathway of which the gene's expressed protein is a part. Analogously, where gene expression analysis is used to assess side effects of pharmacologic agents - whether in lead compound discovery or in subsequent screening of lead compound derivatives - the inability of the agent to alter a gene's expression level is evidence that the drug does not affect the pathway of which the gene's expressed protein is a part.

WO 99/58720, incorporated herein by reference in its entirety, provides methods for quantifying the relatedness of a first and second gene expression profile and for ordering the relatedness of a plurality of gene expression profiles, without regard to the identity or function of the genes whose expression is used in the calculation.

Gene expression analysis, including gene expression analysis by microarray hybridization, is, of course, principally a laboratory-based art. Devices and apparatus used principally in laboratories to facilitate laboratory research are well-established to possess specific, substantial, and credible utility. For example, U.S. Patent No. 6,001,233 describes and claims a gel electrophoresis apparatus having a cam-activated clamp; for example, U.S. Patent No. 6,051,831 describes and claims a high mass detector for use in time-of-flight mass spectrometers; for example, U.S. Patent NO. 5,824,269 describes and claims a flow cytometer-as is well known, few gel electrophoresis apparatuses, TOF-MS devices, or flow cytometers are sold for consumer use.

Indeed, and in particular, nucleic acid microarrays, as devices intended for laboratory use in measuring gene expression, are well-established to have specific, substantial and credible utility. Thus, the microarrays of the present invention have at least the specific, substantial and credible utilities of the microarrays claimed as devices and articles of manufacture in the following U.S. patents, the disclosures of each of which is incorporated herein by reference: U.S. Patent Nos. 5,445,934 ("Array of oligonucleotides on a solid substrate"); 5,744,305 ("Arrays of materials attached to a substrate"); and 6,004,752 ("Solid support with attached molecules").

Genome-derived single exon probes and genome-derived single exon probe microarrays have the additional utility, *inter alia,* of permitting high-throughput detection of splice variants of the nucleic acids of the present invention, as further described in copending and commonly owned U.S. Patent application no. 09/632,366, filed August 3, 2000, the disclosure of which is incorporated herein by reference in its entirety.

The isolated nucleic acids of the present invention can also be used to prime synthesis of nucleic acid, for purpose of either analysis or isolation, using mRNA, cDNA, or genomic DNA as template.

For use as primers, at least 17 contiguous nucleotides of the isolated nucleic acids of the present invention will be used. Often, at least 18, 19, or 20 contiguous nucleotides of the nucleic acids of the present invention will be used, and on occasion at least 20, 22, 24, or 25 contiguous nucleotides of the nucleic acids of the present invention will be used, and even 30 nucleotides or more of the nucleic acids of the present invention can be used to prime specific synthesis.

The nucleic acid primers of the present invention can be used, for example, to prime first strand cDNA synthesis on an mRNA template.

Such primer extension can be done directly to analyze the message. Alternatively, synthesis on an mRNA template can be done to produce first strand cDNA. The first strand cDNA can thereafter be used, *inter alia,* directly as a single-stranded probe, as above-described, as a template for sequencing - permitting identification of alterations, including deletions, insertions, and substitutions, both normal allelic variants and mutations associated with abnormal phenotypes- or as a template, either for second strand cDNA synthesis (*e.g.*, as an antecedent to insertion into a cloning or expression vector), or for amplification.

The nucleic acid primers of the present invention can also be used, for example, to prime single base extension (SBE) for SNP detection (*see, e.g.*, U.S. Pat. No. 6,004,744, the disclosure of which is incorporated herein by reference in its entirety).

As another example, the nucleic acid primers of the present invention can be used to prime amplification of LCP nucleic acids, using transcript-derived or genomic DNA as template.

Primer-directed amplification methods are now well-established in the art. Methods for performing the polymerase chain reaction (PCR) are compiled, *inter alia*, in McPherson, PCR (Basics: From Background to Bench), Springer Verlag (2000) (ISBN: 0387916008); Innis *et al*. (eds.), PCR Applications: Protocols for Functional Genomics, Academic Press (1999) (ISBN: 0123721857); Gelfand *et al*. (eds.), PCR Strategies, Academic Press (1998) (ISBN: 0123721822); Newton *et al*., PCR, Springer-Verlag New York (1997) (ISBN: 0387915060); Burke (ed.), PCR: Essential Techniques, John Wiley & Son Ltd (1996) (ISBN: 047195697X); White (ed.), PCR *Cloning* Protocols: From Molecular Cloning to Genetic Engineering, Vol. 67, Humana Press (1996) (ISBN: 0896033430); McPherson et al. (eds.), PCR 2: A Practical Approach, Oxford University Press, Inc. (1995) (ISBN: 0199634254), the disclosures of which are incorporated herein by reference in their entireties. Methods for performing RT-PCR are collected, *e.g.,* in Siebert *et al*. (eds.), Gene Cloning and Analysis by RT-PCR, Eaton Publishing Company/Bio Techniques Books Division, 1998 (ISBN: 1881299147); Siebert (ed.), PCR Technique:RT-PCR, Eaton Publishing Company/BioTechniques Books (1995) (ISBN:1881299139), the disclosure of which is incorporated herein by reference in its entirety.

Isothermal amplification approaches, such as rolling circle amplification, are also now well-described. *See, e.g.,* Schweitzer *et al., Curr. Opin. Biotechnol.* 12(1):21-7 (2001); U.S. Patent Nos. 6,235,502, 6,221,603, 6,210,884, 6,183,960, 5,854,033, 5,714,320, 5,648,245, and international patent publications WO 97/19193 and WO 00/15779, the disclosures of which are incorporated herein by reference in their entireties. Rolling circle amplification can be combined with other techniques to facilitate SNP detection. See, *e.g.,* Lizardi *et al., Nature Genet.* 19(3) :225-32 (1998).

As further described below, nucleic acids of the present invention, inserted into vectors that flank the nucleic acid insert with a phage promoter, such as T7, T3, or SP6 promoter, can be used to drive *in vitro* expression of RNA complementary to either strand of the nucleic acid of the present invention. The RNA can be used, *inter alia,* as a single-stranded probe, in cDNA-mRNA subtraction, or for *in vitro* translation.

As will be further discussed herein below, nucleic acids of the present invention that encode LCP protein or portions thereof can be used, *inter alia,* to express the LCP proteins or protein fragments, either alone, or as part of fusion proteins.

Expression can be from genomic nucleic acids of the present invention, or from transcript-derived nucleic acids of the present invention.

Where protein expression is effected from genomic DNA, expression will typically be effected in eukaryotic, typically mammalian, cells capable of splicing introns from the initial RNA transcript. Expression can be driven from episomal vectors, such as EBV-based vectors, or can be effected from genomic DNA integrated into a host cell chromosome. As will be more fully described below, where expression is from transcript-derived (or otherwise intron-less) nucleic acids of the present invention, expression can be effected in wide variety of prokaryotic or eukaryotic cells.

Expressed *in vitro,* the protein, protein fragment, or protein fusion can thereafter be isolated, to be used, *inter alia,* as a standard in immunoassays specific for the proteins, or protein isoforms, of the present invention; to be used as a therapeutic agent, *e.g.,* to be administered as passive replacement therapy in individuals deficient in the proteins of the present invention, or to be administered as a vaccine; to be used for *in vitro* production of specific antibody, the antibody thereafter to be used, *e.g.,* as an analytical reagent for detection and quantitation of the proteins of the present invention or to be used as an immunotherapeutic agent.

The isolated nucleic acids of the present invention can also be used to drive *in vivo* expression of the proteins of the present invention. *In vivo* expression can be driven from a vector - typically a viral vector, often a vector based upon a replication incompetent retrovirus, an adenovirus, or an adeno-associated virus (AAV) - for purpose of gene therapy. *In vivo* expression can also be driven from signals endogenous to the nucleic acid or from a vector, often a plasmid vector, such as pVAX1 (Invitrogen, Carlsbad CA, USA), for purpose of "naked" nucleic acid vaccination, as further described in U.S. Pat. Nos. 5,589,466; 5,679,647; 5,804,566; 5,830,877; 5,843,913; 5,880,104; 5,958,891; 5,985,847; 6,017,897; 6,110,898; 6,204,250, the disclosures of which are incorporated herein by reference in their entireties.

The nucleic acids of the present invention can also be used for antisense inhibition of transcription or translation. *See* Phillips (ed.), Antisense Technology, Part B, Methods in Enzymology Vol. 314, Academic Press, Inc. (1999) (ISBN: 012182215X); Phillips (ed.), Antisense Technology, Part A, Methods in Enzymology Vol. 313, Academic Press, Inc. (1999) (ISBN: 0121822141); Hartmann *et al.* (eds.), Manual of Antisense Methodology (Perspectives in Antisense Science), Kluwer Law International (1999) (ISBN:079238539X); Stein *et al.* (eds.), Applied Antisense Oligonucleotide Technology, Wiley-Liss (cover (1998) (ISBN: 0471172790); Agrawal *et al.* (eds.), Antisense Research and Application, Springer-Verlag New York, Inc. (1998) (ISBN: 3540638334); Lichtenstein *et al.* (eds.), Antisense Technology: A Practical Approach, Vol. 185, Oxford University Press, INC. (1998) (ISBN: 0199635838); Gibson (ed.), Antisense and Ribozyme Methodology: Laboratory Companion, Chapman & Hall (1997) (ISBN: 3826100794); Chadwick *et al.* (eds.), Oligonucleotides as Therapeutic Agents - Symposium No. 209, John Wiley & Son Ltd (1997) (ISBN: 0471972797), the disclosures of which are incorporated herein by reference in their entireties.

Nucleic acids of the present invention, particularly cDNAs of the present invention, that encode full-length human LCP protein isoforms, have additional, well-recognized, immediate, real world utility as commercial products of manufacture suitable for sale.

For example, Invitrogen Corp. (Carlsbad, CA, USA), through its Research Genetics subsidiary, sells full length human cDNAs cloned into one of a selection of expression vectors as GeneStorm® expression-ready clones; utility is specific for the gene, since each gene is capable of being ordered separately and has a distinct catalogue number, and utility is substantial, each clone selling for $650.00 US. Similarly, Incyte Genomics (Palo Alto, CA, USA) sells clones from public and proprietary sources in multi-well plates or individual tubes.

Nucleic acids of the present invention that include genomic regions encoding the human LCP protein, or portions thereof, have yet further utilities.

For example, genomic nucleic acids of the present invention can be used as amplification substrates, *e.g.* for preparation of genome-derived single exon probes of the present invention, as described above and in copending and commonly-owned U.S. patent application nos. 09/864,761, filed May 23, 2001, 09/774,203, filed January 29, 2001, and 09/632,366, filed August 3, 2000, the disclosures of which are incorporated herein by reference in their entireties.

As another example, genomic nucleic acids of the present invention can be integrated non-homologously into the genome of somatic cells, *e.g.* CHO cells, COS cells, or 293 cells, with or without amplification of the insertional locus, in order, *e.g.,* to create stable cell lines capable of producing the proteins of the present invention.

As another example, more fully described herein below, genomic nucleic acids of the present invention can be integrated nonhomologously into embryonic stem (ES) cells to create transgenic non-human animals capable of producing the proteins of the present invention.

Genomic nucleic acids of the present invention can also be used to target homologous recombination to the human LCP locus. *See, e.g.,* U.S. Patent Nos. 6,187,305; 6,204,061; 5,631,153; 5,627,059; 5,487,992; 5,464,764; 5,614,396; 5,527,695 and 6,063,630; and Kmiec *et al*. (eds.), Gene Targeting Protocols, Vol. 133, Humana Press (2000) (ISBN: 0896033600); Joyner (ed.), Gene Targeting: A Practical Approach, Oxford University Press, Inc. (2000) (ISBN: 0199637938); Sedivy *et al*., Gene Targeting, Oxford University Press (1998) (ISBN: 071677013X); Tymms *et al*. (eds.), Gene Knockout Protocols, Humana Press (2000) (ISBN: 0896035727); Mak *et al*. (eds.), The Gene Knockout FactsBook, Vol. 2, Academic Press, Inc. (1998) (ISBN: 0124660444); Torres *et al*., Laboratory Protocols for Conditional Gene Targeting, Oxford University Press (1997) (ISBN: 019963677X); Vega (ed.), Gene Targeting, CRC Press, LLC (1994) (ISBN: 084938950X), the disclosures of which are incorporated herein by reference in their entireties.

Where the genomic region includes transcription regulatory elements, homologous recombination can be used to alter the expression of LCP, both for purpose of *in vitro* production of LCP protein from human cells, and for purpose of gene therapy. *See, e.g.,* U.S. Pat. Nos. 5,981,214, 6,048,524; 5,272,071.

Fragments of the nucleic acids of the present invention smaller than those typically used for homologous recombination can also be used for targeted gene correction or alteration, possibly by cellular mechanisms different from those engaged during homologous recombination.

For example, partially duplexed RNA/DNA chimeras have been shown to have utility in targeted gene correction, U.S. Pat. Nos. 5,945,339, 5,888,983, 5,871,984, 5,795,972, 5,780,296, 5,760,012, 5,756,325, 5,731,181, the disclosures of which are incorporated herein by reference in their entireties. So too have small oligonucleotides fused to triplexing domains have been shown to have utility in targeted gene correction, Culver *et al*., "Correction of chromosomal point mutations in human cells with bifunctional oligonucleotides," *Nature Biotechnol.* 17(10):989-93 (1999), as have oligonucleotides having modified terminal bases or modified terminal internucleoside bonds, Gamper *et al., Nucl. Acids Res.* 28(21):4332-9 (2000), the disclosures of which are incorporated herein by reference.

The isolated nucleic acids of the present invention can also be used to provide the initial substrate for recombinant engineering of LCP protein variants having desired phenotypic improvements. Such engineering includes, for example, site-directed mutagenesis, random mutagenesis with subsequent functional screening, and more elegant schemes for recombinant evolution of proteins, as are described, *inter alia,* in U.S. Pat. Nos. 6,180,406; 6,165,793; 6,117,679; and 6,096,548, the disclosures of which are incorporated herein by reference in their entireties.

Nucleic acids of the present invention can be obtained by using the labeled probes of the present invention to probe nucleic acid samples, such as genomic libraries, cDNA libraries, and mRNA samples, by standard techniques. Nucleic acids of the present invention can also be obtained by amplification, using the nucleic acid primers of the present invention, as further demonstrated in Example 1, herein below. Nucleic acids of the present invention of fewer than about 100 nt can also be synthesized chemically, typically by solid phase synthesis using commercially available automated synthesizers.

### "Full Length" LCP Nucleic Acids

In a first series of nucleic acid embodiments, the invention provides isolated nucleic acids that encode the entirety of the LCP proteins. As discussed above, the "full-length" nucleic acids of the present invention can be used, *inter alia,* to express full length LCP proteins. The full-length nucleic acids can also be used as nucleic acid probes; used as probes, the isolated nucleic acids of these embodiments will hybridize to LCP.

In a first such embodiment, the invention provides an isolated nucleic acid comprising (i) the nucleotide sequence of SEQ ID NO: 1, or (ii) the complement of (i). SEQ ID NO: 1 presents the entire cDNA of LCP1, including the 5' untranslated (UT) region and 3' UT.

In a second embodiment, the invention provides an isolated nucleic acid comprising (i) the nucleotide sequence of SEQ ID NOs: 2 or 1113, (ii) a degenerate variant of the nucleotide sequence of SEQ ID NOs: 2 or 1113, or (iii) the complement of (i) or (ii). SEQ ID NO: 2 and 1113 present the open reading frame (ORF) of LCP1 and LCP2.

In a third embodiment, the invention provides an isolated nucleic acid comprising (i) a nucleotide sequence that encodes a polypeptide with the amino acid sequence of SEQ ID NOs: 3 or 1114 or (ii) the complement of a nucleotide sequence that encodes a polypeptide with the amino acid sequence of SEQ ID NOs: 3 or 1114. SEQ ID NO: 3 and 1114 provides the amino acid sequences of LCP1 and LCP2.

In a fourth embodiment, the invention provides an isolated nucleic acid having a nucleotide sequence that (i) encodes a polypeptide having the sequence of SEQ ID NOs: 3 or 1114, (ii) encodes a polypeptide having the sequence of SEQ ID NOs: 3 or 1114 with conservative amino acid substitutions, or (iii) that is the complement of (i) or (ii), where SEQ ID NO: 3 and 1114 provides the amino acid sequence of LCP1 and LCP2.

### Selected Partial Nucleic Acids

In a second series of nucleic acid embodiments, the invention provides isolated nucleic acids that encode select portions of LCP. As will be further discussed herein below, these "partial" nucleic acids can be used, *inter alia,* to express specific portions of the LCP. These "partial" nucleic acids can also be used, *inter alia,* as nucleic probes.

In a first such embodiment, the invention provides an isolated nucleic acid comprising (i) the nucleotide sequence of SEQ ID NO: 4, (ii) a degenerate variant of SEQ ID NO: 4, or (iii) the complement of (i) or (ii), wherein the isolated nucleic acid is no more than about 100 kb in length, typically no more than about 75 kb in length, more typically no more than about 50 kb length. SEQ ID NO: 4 encodes a novel portion of LCP. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 5 or (ii) the complement of a nucleotide sequence that encodes SEQ ID NO: 5, wherein the isolated nucleic acid is no more than about 100 kb in length, typically no more than about 75 kb in length, frequently no more than about 50 kb in length. SEQ ID NO: 5 is the amino acid sequence encoded by a portion of LCP not found in any EST fragments. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 5, (ii) a nucleotide sequence that encodes SEQ ID NO: 5 with conservative substititions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising (i) the nucleotide sequence of SEQ ID NO: 6, (ii) a degenerate variant of SEQ ID NO: 7, or (iii) the complement of (i) or (ii), wherein the isolated nucleic acid is no more than about 100 kb in length, typically no more than about 75 kb in length, more typically no more than about 50 kb length. SEQ ID NO: 7 encodes a novel portion of LCP. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 9 or (ii) the complement of a nucleotide sequence that encodes SEQ ID NO: 9, wherein the isolated nucleic acid is no more than about 100 kb in length, typically no more than about 75 kb in length, frequently no more than about 50 kb in length. SEQ ID NO: 9 is the amino acid sequence encoded by another portion of LCP not found in any EST fragments. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 9, (ii) a nucleotide sequence that encodes SEQ ID NO: 9 with conservative substititions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a first such embodiment, the invention provides an isolated nucleic acid comprising (i) the nucleotide sequence of SEQ ID NO: 1115, (ii) a degenerate variant of SEQ ID NO: 1115, or (iii) the complement of (i) or (ii), wherein the isolated nucleic acid is no more than about 100 kb in length, typically no more than about 75 kb in length, more typically no more than about 50 kb length. SEQ ID NO: 1115 represents the splice junction of exons 1 and 3 of LCP1 and encodes a novel portion of LCP2. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 1116 or (ii) the complement of a nucleotide sequence that encodes SEQ ID NO: 1116, wherein the isolated nucleic acid is no more than about 100 kb in length, typically no more than about 75 kb in length, frequently no more than about 50 kb in length. SEQ ID NO: 1116 is the amino acid sequence encoded by SEQ ID NO: 1115. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 1116, (ii) a nucleotide sequence that encodes SEQ ID NO: 1116 with conservative substititions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

### Cross-Hybridizing Nucleic Acids

In another series of nucleic acid embodiments, the invention provides isolated nucleic acids that hybridize to various of the LCP nucleic acids of the present invention. These cross-hybridizing nucleic acids can be used, *inter alia,* as probes for, and to drive expression of, proteins that are related to LCP of the present invention as further isoforms, homologues, paralogues, or orthologues.

In a first embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 4 or the complement of SEQ ID NO: 4, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under moderate stringency conditions to a probe the nucleotide sequence of which consists of atleast 17 nt, 18, 19, 20, 21, 22, 23, 24, 25, 30, 40, or 50 nt of SEQ ID NO: 4 or the complement of SEQ ID NO: 4, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 5, (ii) encodes a polypeptide having the sequence of SEQ ID NO: 5 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 6 or the complement of SEQ ID NO: 6, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under moderate stringency conditions to a probe the nucleotide sequence of which consists of atleast 17 nt, 18, 19, 20, 21, 22, 23, 24, 25, 30, 40, or 50 nt of SEQ ID NO: 6 or the complement of SEQ ID NO: 6, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 9, (ii) encodes a polypeptide having the sequence of SEQ ID NO: 9 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 1115 or the complement of SEQ ID NO: 1115, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under moderate stringency conditions to a probe the nucleotide sequence of which consists of atleast 17 nt, 18, 19, 20, 21, 22, 23, 24, 25, 30, 40, or 50 nt of SEQ ID NO: 1115 or the complement of SEQ ID NO: 1115, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 1116, (ii) encodes a polypeptide having the sequence of SEQ ID NO: 1116 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

### Particularly Useful Nucleic Acids

Particularly useful among the above-described nucleic acids are those that are expressed, or the complement of which are expressed, in adrenal, adult liver, bone marrow, brain, fetal liver, heart, kidney, lung, placenta, skeletal muscle, colon and prostate as well as a cell line hela.

Also particularly useful among the above-described nucleic acids are those that encode, or the complement of which encode, a polypeptide that plays a potential therapeutic as well as diagnostic role for neurological and developmental disorders, as well as diseases involving cell-cell adhesion process.

Other particularly useful embodiments of the nucleic acids above-described are those that encode, or the complement of which encode, a polypeptide having any or all of CUB, LCCL, and discoidin/FA8C domains.

### Nucleic Acid Fragments

In another series of nucleic acid embodiments, the invention provides fragments of various of the isolated nucleic acids of the present invention which prove useful, *inter alia,* as nucleic acid probes, as amplification primers, and to direct expression or synthesis of epitopic or immunogenic protein fragments.

In a first such embodiment, the invention provides an isolated nucleic acid comprising at least 17 nucleotides, 18 nucleotides, 20 nucleotides, 24 nucleotides, or 25 nucleotides of (i) SEQ ID NO: 4, (ii) a degenerate variant of SEQ ID NO: 4, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 5, (ii) a nucleotide sequence that encodes a peptide of at least 15 contiguous amino acids of SEQ ID NO: 5, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 5 with conservative amino acid substitutions, (ii) a polypeptide having the sequence of at least 15 contiguous amino acids of SEQ ID NO: 5 with conservative amino acid substitutions, (iii) a polypeptide having the sequence of at least 8 contiguous amino acids of SEQ ID NO: 5 with moderately conservative substitutions, (iv) a polypeptide having the sequence of at last 15 congiuous amino acids of SEQ ID NO: 5 with moderately conservative substitutions, or (v) the complement of any of (i) - (iv), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising at least 17 nucleotides, 18 nucleotides, 20 nucleotides, 24 nucleotides, or 25 nucleotides of (i) SEQ ID NO: 6, (ii) a degenerate variant of SEQ ID NO: 7, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 9, (ii) a nucleotide sequence that encodes a peptide of at least 15 contiguous amino acids of SEQ ID NO: 9, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 9 with conservative amino acid substitutions, (ii) a polypeptide having the sequence of at least 15 contiguous amino acids of SEQ ID NO: 9 with conservative amino acid substitutions, (iii) a polypeptide having the sequence of at least 8 contiguous amino acids of SEQ ID NO: 9 with moderately conservative substitutions, (iv) a polypeptide having the sequence of at last 15 congiuous amino acids of SEQ ID NO: 9 with moderately conservative substitutions, or (v) the complement of any of (i) - (iv), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising at least 17 nucleotides, 18 nucleotides, 20 nucleotides, 24 nucleotides, or 25 nucleotides of (i) SEQ ID NO: 1115, (ii) a degenerate variant of SEQ ID NO: 1115, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 1116, (ii) a nucleotide sequence that encodes a peptide of at least 15 contiguous amino acids of SEQ ID NO: 1116, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 1116 with conservative amino acid substitutions, (ii) a polypeptide having the sequence of at least 15 contiguous amino acids of SEQ ID NO: 1116 with conservative amino acid substitutions, (iii) a polypeptide having the sequence of at least 8 contiguous amino acids of SEQ ID NO: 1116 with moderately conservative substitutions, (iv) a polypeptide having the sequence of at last 15 congiuous amino acids of SEQ ID NO: 1116 with moderately conservative substitutions, or (v) the complement of any of (i) - (iv), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### Single Exon Probes

The invention further provides genome-derived single exon probes having portions of no more than one exon of the LCP gene. As further described in commonly owned and copending U.S. patent application serial no. 09/632,366, filed August 3, 2000 ("Methods and Apparatus for High Throughput Detection and Characterization of alternatively Spliced Genes"), the disclosure of which is incorporated herein by reference in its entirety, such single exon probes have particular utility in identifying and characterizing splice variants. In particular, such single exon probes are useful for identifying and discriminating the expression of distinct isoforms of LCP.

In a first embodiment, the invention provides an isolated nucleic acid comprising a nucleotide sequence of no more than one portion of SEQ ID NOs: 10 - 25 or the complement of SEQ ID NOs: 10 - 25, wherein the portion comprises at least 17 contiguous nucleotides, 18 contiguous nucleotides, 20 contiguous nucleotides, 24 contiguous nucleotides, 25 contiguous nucleotides, or 50 contiguous nucleotides of any one of SEQ ID NOs: 10 - 25, or their complement. In a further embodiment, the exonic portion comprises the entirety of the referenced SEQ ID NO: or its complement.

In other embodiments, the invention provides isolated single exon probes having the nucleotide sequence of any one of SEQ ID NOs: 26 - 41.

### Transcription Control Nucleic Acids

In another aspect, the present invention provides genome-derived isolated nucleic acids that include nucleic acid sequence elements that control transcription of the LCP gene. These nucleic acids can be used, *inter alia,* to drive expression of heterologous coding regions in recombinant constructs, thus conferring upon such heterologous coding regions the expression pattern of the native LCP gene. These nucleic acids can also be used, conversely, to target heterologous transcription control elements to the LCP genomic locus, altering the expression pattern of the LCP gene itself.

In a first such embodiment, the invention provides an isolated nucleic acid comprising the nucleotide sequence of SEQ ID NO: 42 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising at least 17, 18, 20, 24, or 25 nucleotides of the sequence of SEQ ID NO: 42 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### VECTORS AND HOST CELLS

In another aspect, the present invention provides vectors that comprise one or more of the isolated nucleic acids of the present invention, and host cells in which such vectors have been introduced.

The vectors can be used, *inter alia,* for propagating the nucleic acids of the present invention in host cells (cloning vectors), for shuttling the nucleic acids of the present invention between host cells derived from disparate organisms (shuttle vectors), for inserting the nucleic acids of the present invention into host cell chromosomes (insertion vectors), for expressing sense or antisense RNA transcripts of the nucleic acids of the present invention *in vitro* or within a host cell, and for expressing polypeptides encoded by the nucleic acids of the present invention, alone or as fusions to heterologous polypeptides. Vectors of the present invention will often be suitable for several such uses.

Vectors are by now well-known in the art, and are described, *inter alia,* in Jones *et al.* (eds.), Vectors: Cloning Applications : Essential Techniques (Essential Techniques Series), John Wiley & Son Ltd 1998 (ISBN: 047196266X); Jones *et al.* (eds.), Vectors: Expression Systems : Essential Techniques (Essential Techniques Series), John Wiley & Son Ltd, 1998 (ISBN:0471962678); Gacesa *et al.,* Vectors: Essential Data, John Wiley & Sons, 1995 (ISBN: 0471948411); Cid-Arregui (eds.), Viral Vectors: Basic Science and Gene Therapy, Eaton Publishing Co., 2000 (ISBN: 188129935X); Sambrook *et al.,* Molecular Cloning: A Laboratory Manual (3^{rd} ed.), Cold Spring Harbor Laboratory Press, 2001 (ISBN: 0879695773); Ausubel *et al.* (eds.), Short Protocols in Molecular Biology: A Compendium of Methods from Current Protocols in Molecular Biology (4^{th} ed.), John Wiley & Sons, 1999 (ISBN: 047132938X), the disclosures of which are incorporated herein by reference in their entireties. Furthermore, an enormous variety of vectors are available commercially. Use of existing vectors and modifications thereof being well within the skill in the art, only basic features need be described here.

Typically, vectors are derived from virus, plasmid, prokaryotic or eukaryotic chromosomal elements, or some combination thereof, and include at least one origin of replication, at least one site for insertion of heterologous nucleic acid, typically in the form of a polylinker with multiple, tightly clustered, single cutting restriction sites, and at least one selectable marker, although some integrative vectors will lack an origin that is functional in the host to be chromosomally modified, and some vectors will lack selectable markers. Vectors of the present invention will further include at least one nucleic acid of the present invention inserted into the vector in at least one location.

Where present, the origin of replication and selectable markers are chosen based upon the desired host cell or host cells; the host cells, in turn, are selected based upon the desired application.

For example, prokaryotic cells, typically *E*. *coli*, are typically chosen for cloning. In such case, vector replication is predicated on the replication strategies of coliform-infecting phage - such as phage lambda, M13, T7, T3 and P1 - or on the replication origin of autonomously replicating episomes, notably the ColE1 plasmid and later derivatives, including pBR322 and the pUC series plasmids. Where *E*. *coli* is used as host, selectable markers are, analogously, chosen for selectivity in gram negative bacteria: *e.g.,* typical markers confer resistance to antibiotics, such as ampicillin, tetracycline, chloramphenicol, kanamycin, streptomycin, zeocin; auxotrophic markers can also be used.

As another example, yeast cells, typically *S*. *cerevisiae*, are chosen, *inter alia*, for eukaryotic genetic studies, due to the ease of targeting genetic changes by homologous recombination and to the ready ability to complement genetic defects using recombinantly expressed proteins, for identification of interacting protein components, *e.g.* through use of a two-hybrid system, and for protein expression. Vectors of the present invention for use in yeast will typically, but not invariably, contain an origin of replication suitable for use in yeast and a selectable marker that is functional in yeast.

Integrative YIp vectors do not replicate autonomously, but integrate, typically in single copy, into the yeast genome at low frequencies and thus replicate as part of the host cell chromosome; these vectors lack an origin of replication that is functional in yeast, although they typically have at least one origin of replication suitable for propagation of the vector in bacterial cells. YEp vectors, in contrast, replicate episomally and autonomously due to presence of the yeast 2 micron plasmid origin (2 µm ori). The YCp yeast centromere plasmid vectors are autonomously replicating vectors containing centromere sequences, CEN, and autonomously replicating sequences, ARS; the ARS sequences are believed to correspond to the natural replication origins of yeast chromosomes. YACs are based on yeast linear plasmids, denoted YLp, containing homologous or heterologous DNA sequences that function as telomeres (TEL) *in vivo*, as well as containing yeast ARS (origins of replication) and CEN (centromeres) segments.

Selectable markers in yeast vectors include a variety of auxotrophic markers, the most common of which are (in *Saccharomyces cerevisiae*) URA3, HIS3, LEU2, TRP1 and LYS2, which complement specific auxotrophic mutations, such as ura3-52, his3-D1, leu2-D1, trp1-D1 and lys2-201. The URA3 and LYS2 yeast genes further permit negative selection based on specific inhibitors, 5-fluoro-orotic acid (FOA) and α-aminoadipic acid (αAA), respectively, that prevent growth of the prototrophic strains but allows growth of the ura3 and lys2 mutants, respectively. Other selectable markers confer resistance to, *e.g.*, zeocin.

As yet another example, insect cells are often chosen for high efficiency protein expression. Where the host cells are from *Spodoptera fruglperda - e.g.,* Sf9 and Sf21 cell lines, and expresSF™ cells (Protein Sciences Corp., Meriden, CT, USA) - the vector replicative strategy is typically based upon the baculovirus life cycle. Typically, baculovirus transfer vectors are used to replace the wild-type AcMNPV polyhedrin gene with a heterologous gene of interest. Sequences that flank the polyhedrin gene in the wild-type genome are positioned 5' and 3' of the expression cassette on the transfer vectors. Following cotransfection with AcMNPV DNA, a homologous recombination event occurs between these sequences resulting in a recombinant virus carrying the gene of interest and the polyhedrin or p10 promoter. Selection can be based upon visual screening for lacZ fusion activity.

As yet another example, mammalian cells are often chosen for expression of proteins intended as pharmaceutical agents, and are also chosen as host cells for screening of potential agonist and antagonists of a protein or a physiological pathway.

Where mammalian cells are chosen as host cells, vectors intended for autonomous extrachromosomal replication will typically include a viral origin, such as the SV40 origin (for replication in cell lines expressing the large T-antigen, such as COS1 and COS7 cells), the papillomavirus origin, or the EBV origin for long term episomal replication (for use, *e.g.*, in 293-EBNA cells, which constitutively express the EBV EBNA-1 gene product and adenovirus E1A). Vectors intended for integration, and thus replication as part of the mammalian chromosome, can, but need not, include an origin of replication functional in mammalian cells, such as the SV40 origin. Vectors based upon viruses, such as adenovirus, adeno-associated virus, vaccinia virus, and various mammalian retroviruses, will typically replicate according to the viral replicative strategy.

Selectable markers for use in mammalian cells include resistance to neomycin (G418), blasticidin, hygromycin and to zeocin, and selection based upon the purine salvage pathway using HAT medium.

Plant cells can also be used for expression, with the vector replicon typically derived from a plant virus *(e.g.,* cauliflower mosaic virus, CaMV; tobacco mosaic virus, TMV) and selectable markers chosen for suitability in plants.

For propagation of nucleic acids of the present invention that are larger than can readily be accomodated in vectors derived from plasmids or virus, the invention further provides artificial chromosomes - BACs, YACs, PACs, and HACs - that comprise LCP nucleic acids, often genomic nucleic acids.

The BAC system is based on the well-characterized *E*. *coli* F-factor, a low copy plasmid that exists in a supercoiled circular form in host cells. The structural features of the F-factor allow stable maintenance of individual human DNA clones as well as easy manipulation of the cloned DNA. *See* Shizuya *et al*., *Keio J*. *Med.* 50(1):26-30 (2001); Shizuya *et al*., *Proc*. *Natl*. *Acad*. *Sci*. *USA* 89(18):8794-7 (1992).

YACs are based on yeast linear plasmids, denoted YLp, containing homologous or heterologous DNA sequences that function as telomeres (TEL) *in vivo,* as well as containing yeast ARS (origins of replication) and CEN (centromeres) segments.

HACs are human artifical chromosomes. Kuroiwa *et al., Nature Biotechnol.* 18(10):1086-90 (2000); Henning *et al., Proc. Natl. Acad. Sci. USA* 96(2):592-7 (1999); Harrington *et al., Nature Genet.* 15(4):345-55 (1997). In one version, long synthetic arrays of alpha satellite DNA are combined with telomeric DNA and genomic DNA to generate linear microchromosomes that are mitotically and cytogenetically stable in the absence of selection.

PACs are P1-derived artificial chromosomes. Sternberg, *Proc. Natl. Acad. Sci. USA* 87(1):103-7 (1990); Sternberg *et al., New Biol.* 2(2):151-62 (1990); Pierce *et al., Proc. Natl Acad. Sci. USA* 89(6):2056-60 (1992).

Vectors of the present invention will also often include elements that permit *in vitro* transcription of RNA from the inserted heterologous nucleic acid. Such vectors typically include a phage promoter, such as that from T7, T3, or SP6, flanking the nucleic acid insert. Often two different such promoters flank the inserted nucleic acid, permitting separate *in vitro* production of both sense and antisense strands.

Expression vectors of the present invention - that is, those vectors that will drive expression of polypeptides from the inserted heterologous nucleic acid - will often include a variety of other genetic elements operatively linked to the protein-encoding heterologous nucleic acid insert, typically genetic elements that drive transcription, such as promoters and enhancer elements, those that facilitate RNA processing, such as transcription termination and/or polyadenylation signals, and those that facilitate translation, such as ribosomal consensus sequences.

For example, vectors for expressing proteins of the present invention in prokaryotic cells, typically *E. coli*, will include a promoter, often a phage promoter, such as phage lambda pL promoter, the trc promoter, a hybrid derived from the trp and lac promoters, the bacteriophage T7 promoter (in *E. coli* cells engineered to express the T7 polymerase), or the araBAD operon. Often, such prokaryotic expression vectors will further include transcription terminators, such as the aspA terminator, and elements that facilitate translation, such as a consensus ribosome binding site and translation termination codon, Schomer *et al., Proc. Natl. Acad. Sci. USA* 83:8506-8510 (1986).

As another example, vectors for expressing proteins of the present invention in yeast cells, typically *S. cerevisiae*, will include a yeast promoter, such as the CYC1 promoter, the GAL1 promoter, ADH1 promoter, or the GPD promoter, and will typically have elements that facilitate transcription termination, such as the transcription termination signals from the CYC1 or ADH1 gene.

As another example, vectors for expressing proteins of the present invention in mammalian cells will include a promoter active in mammalian cells. Such promoters are often drawn from mammalian viruses - such as the enhancer-promoter sequences from the immediate early gene of the human cytomegalovirus (CMV), the enhancer-promoter sequences from the Rous sarcoma virus long terminal repeat (RSV LTR), and the enhancer-promoter from SV40. Often, expression is enhanced by incorporation of polyadenylation sites, such as the late SV40 polyadenylation site and the polyadenylation signal and transcription termination sequences from the bovine growth hormone (BGH) gene, and ribosome binding sites. Furthermore, vectors can include introns, such as intron II of rabbit β-globin gene and the SV40 splice elements.

Vector-drive protein expression can be constitutive or inducible.

Inducible vectors include either naturally inducible promoters, such as the trc promoter, which is regulated by the lac operon, and the pL promoter, which is regulated by tryptophan, the MMTV-LTR promoter, which is inducible by dexamethasone, or can contain synthetic promoters and/or additional elements that confer inducible control on adjacent promoters. Examples of inducible synthetic promoters are the hybrid Plac/ara-1 promoter and the PLtetO-1 promoter. The PltetO-1 promoter takes advantage of the high expression levels from the PL promoter of phage lambda, but replaces the lambda repressor sites with two copies of operator 2 of the Tn10 tetracycline resistance operon, causing this promoter to be tightly repressed by the Tet repressor protein and induced in response to tetracycline (Tc) and Tc derivatives such as anhydrotetracycline.

As another example of inducible elements, hormone response elements, such as the glucocorticoid response element (GRE) and the estrogen response element (ERE), can confer hormone inducibility where vectors are used for expression in cells having the respective hormone receptors. To reduce background levels of expression, elements responsive to ecdysone, an insect hormone, can be used instead, with coexpression of the ecdysone receptor.

Expression vectors can be designed to fuse the expressed polypeptide to small protein tags that facilitate purification and/or visualization.

For example, proteins of the present invention can be expressed with a polyhistidine tag that facilitates purification of the fusion protein by immobilized metal affinity chromatography, for example using NiNTA resin (Qiagen Inc., Valencia, CA, USA) or TALON™ resin (cobalt immobilized affinity chromatography medium, Clontech Labs, Palo Alto, CA, USA). As another example, the fusion protein can include a chitin-binding tag and self-excising intein, permitting chitin-based purification with self-removal of the fused tag (IMPACT™ system, New England Biolabs, Inc., Beverley, MA, USA). Alternatively, the fusion protein can include a calmodulin-binding peptide tag, permitting purification by calmodulin affinity resin (Stratagene, La Jolla, CA, USA), or a specifically excisable fragment of the biotin carboxylase carrier protein, permitting purification of *in vivo* biotinylated protein using an avidin resin and subsequent tag removal (Promega, Madison, WI, USA). As another useful alternative, the proteins of the present invention can be expressed as a fusion to glutathione-S-transferase, the affinity and specificity of binding to glutathione permitting purification using glutathione affinity resins, such as Glutathione-Superflow Resin (Clontech Laboratories, Palo Alto, CA, USA), with subsequent elution with free glutathione.

Other tags include, for example, the Xpress epitope, detectable by anti-Xpress antibody (Invitrogen, Carlsbad, CA, USA), a myc tag, detectable by anti-myc tag antibody, the V5 epitope, detectable by anti-V5 antibody (Invitrogen, Carlsbad, CA, USA), FLAG® epitope, detectable by anti-FLAG® antibody (Stratagene, La Jolla, CA, USA), and the HA epitope.

For secretion of expressed proteins, vectors can include appropriate sequences that encode secretion signals, such as leader peptides. For example, the pSecTag2 vectors (Invitrogen, Carlsbad, CA, USA) are 5.2 kb mammalian expression vectors that carry the secretion signal from the V-J2-C region of the mouse Ig kappa-chain for efficient secretion of recombinant proteins from a variety of mammalian cell lines.

Expression vectors can also be designed to fuse proteins encoded by the heterologous nucleic acid insert to polypeptides larger than purification and/or identification tags. Useful protein fusions include those that permit display of the encoded protein on the surface of a phage or cell, fusions to intrinsically fluorescent proteins, such as those that have a green fluorescent protein (GFP)-like chromophore, fusions to the IgG Fc region, and fusions for use in two hybrid systems.

Vectors for phage display fuse the encoded polypeptide to, *e.g.,* the gene III protein (pIII) or gene VIII protein (pVIII) for display on the surface of filamentous phage, such as M13. *See* Barbas *et al.,* Phage Display: A Laboratory Manual, Cold Spring Harbor Laboratory Press (2001) (ISBN 0-87969-546-3); Kay *et al.* (eds.), Phage Display of Peptides and Proteins: A Laboratory Manual, San Diego: Academic Press, Inc., 1996; Abelson *et al.* (eds.), Combinatorial Chemistry, Methods in Enzymology vol. 267, Academic Press (May 1996).

Vectors for yeast display, *e.g.* the pYD1 yeast display vector (Invitrogen, Carlsbad, CA, USA), use the α-agglutinin yeast adhesion receptor to display recombinant protein on the surface of *S. cerevisiae*. Vectors for mammalian display, *e*.*g*., the pDisplay™ vector (Invitrogen, Carlsbad, CA, USA), target recombinant proteins using an N-terminal cell surface targeting signal and a C-terminal transmembrane anchoring domain of platelet derived growth factor receptor.

A wide variety of vectors now exist that fuse proteins encoded by heterologous nucleic acids to the chromophore of the substrate-independent, intrinsically fluorescent green fluorescent protein from *Aequorea victoria* ("GFP") and its variants. These proteins are intrinsically fluorescent: the GFP-like chromophore is entirely encoded by its amino acid sequence and can fluoresce without requirement for cofactor or substrate.

Structurally, the GFP-like chromophore comprises an 11-stranded β-barrel (β-can) with a central α-helix, the central α-helix having a conjugated π-resonance system that includes two aromatic ring systems and the bridge between them. The π-resonance system is created by autocatalytic cyclization among amino acids; cyclization proceeds through an imidazolinone intermediate, with subsequent dehydrogenation by molecular oxygen at the Cα-Cβ bond of a participating tyrosine.

The GFP-like chromophore can be selected from GFP-like chromophores found in naturally occurring proteins, such as *A. victoria* GFP (GenBank accession number AAA27721), *Renilla reniformis* GFP, FP583 (GenBank accession no. AF168419) (DsRed), FP593 (AF272711), FP483 (AF168420), FP484 (AF168424), FP595 (AF246709), FP486 (AF168421), FP538 (AF168423), and FP506 (AF168422), and need include only so much of the native protein as is needed to retain the chromophore's intrinsic fluorescence. Methods for determining the minimal domain required for fluorescence are known in the art. Li *et al*.,"Deletions of the *Aeguorea victoria* Green Fluorescent Protein Define the Minimal Domain Required for Fluorescence," *J. Biol. Chem.* 272:28545-28549 (1997).

Alternatively, the GFP-like chromophore can be selected from GFP-like chromophores modified from those found in nature. Typically, such modifications are made to improve recombinant production in heterologous expression systems (with or without change in protein sequence), to alter the excitation and/or emission spectra of the native protein, to facilitate purification, to facilitate or as a consequence of cloning, or are a fortuitous consequence of research investigation.

The methods for engineering such modified GFP-like chromophores and testing them for fluorescence activity, both alone and as part of protein fusions, are well-known in the art. Early results of these efforts are reviewed in Heim *et al., Curr. Biol.* 6:178-182 (1996), incorporated herein by reference in its entirety; a more recent review, with tabulation of useful mutations, is found in Palm *et al*., "Spectral Variants of Green Fluorescent Protein," in Green Fluorescent Proteins, Conn (ed.), *Methods Enzymol.* vol. 302, pp. 378 - 394 (1999), incorporated herein by reference in its entirety. A variety of such modified chromophores are now commercially available and can readily be used in the fusion proteins of the present invention.

For example, EGFP ("enhanced GFP"), Cormack *et al., Gene* 173:33-38 (1996); U.S. Pat. Nos. 6,090,919 and 5,804,387, is a red-shifted, human codon-optimized variant of GFP that has been engineered for brighter fluorescence, higher expression in mammalian cells, and for an excitation spectrum optimized for use in flow cytometers. EGFP can usefully contribute a GFP-like chromophore to the fusion proteins of the present invention. A variety of EGFP vectors, both plasmid and viral, are available commercially (Clontech Labs, Palo Alto, CA, USA), including vectors for bacterial expression, vectors for N-terminal protein fusion expression, vectors for expression of C-terminal protein fusions, and for bicistronic expression.

Toward the other end of the emission spectrum, EBFP ("enhanced blue fluorescent protein") and BFP2 contain four amino acid substitutions that shift the emission from green to blue, enhance the brightness of fluorescence and improve solubility of the protein, Heim *et al*., *Curr*. *Biol.* 6:178-182 (1996); Cormack *et al*., *Gene* 173:33-38 (1996). EBFP is optimized for expression in mammalian cells whereas BFP2, which retains the original jellyfish codons, can be expressed in bacteria; as is further discussed below, the host cell of production does not affect the utility of the resulting fusion protein. The GFP-like chromophores from EBFP and BFP2 can usefully be included in the fusion proteins of the present invention, and vectors containing these blue-shifted variants are available from Clontech Labs (Palo Alto, CA, USA).

Analogously, EYFP ("enhanced yellow fluorescent protein"), also available from Clontech Labs, contains four amino acid substitutions, different from EBFP, Ormö *et al., Science* 273:1392-1395 (1996), that shift the emission from green to yellowish-green. Citrine, an improved yellow fluorescent protein mutant, is described in Heikal *et al., Proc. Natl. Acad. Sci. USA* 97:11996-12001 (2000). ECFP ("enhanced cyan fluorescent protein") (Clontech Labs, Palo Alto, CA, USA) contains six amino acid substitutions, one of which shifts the emission spectrum from green to cyan. Heim *et al., Curr. Biol.* 6:178-182 (1996); Miyawaki *et al*., *Nature* 388:882-887 (1997). The GFP-like chromophore of each of these GFP variants can usefully be included in the fusion proteins of the present invention.

The GFP-like chromophore can also be drawn from other modified GFPs, including those described in U.S. Pat. Nos. 6,124,128; 6,096,865; 6,090,919; 6,066,476; 6,054,321; 6,027,881; 5,968,750; 5,874,304; 5,804,387; 5,777,079; 5,741,668; and 5,625,048, the disclosures of which are incorporated herein by reference in their entireties. See also Conn (ed.), Green Fluorescent Protein, *Methods in Enzymol.* Vol. 302, pp 378-394 (1999), incorporated herein by reference in its entirety. A variety of such modified chromophores are now commercially available and can readily be used in the fusion proteins of the present invention.

Fusions to the IgG Fc region increase serum half life of protein pharmaceutical products through interaction with the FcRn receptor (also denominated the FcRp receptor and the Brambell receptor, FcRb), further described in international patent application nos. WO 97/43316, WO 97/34631, WO 96/32478, WO 96/18412.

For long-term, high-yield recombinant production of the proteins, protein fusions, and protein fragments of the present invention, stable expression is particularly useful.

Stable expression is readily achieved by integration into the host cell genome of vectors having selectable markers, followed by selection for integrants.

For example, the pUB6/V5-His A, B, and C vectors (Invitrogen, Carlsbad, CA, USA) are designed for high-level stable expression of heterologous proteins in a wide range of mammalian tissue types and cell lines. pUB6/V5-His uses the promoter/enhancer sequence from the human ubiquitin C gene to drive expression of recombinant proteins: expression levels in 293, CHO, and NIH3T3 cells are comparable to levels from the CMV and human EF-la promoters. The bsd gene permits rapid selection of stably transfected mammalian cells with the potent antibiotic blasticidin.

Replication incompetent retroviral vectors, typically derived from Moloney murine leukemia virus, prove particularly useful for creating stable transfectants having integrated provirus. The highly efficient transduction machinery of retroviruses, coupled with the availability of a variety of packaging cell lines - such as RetroPack™ PT 67, EcoPack2™ -293, AmphoPack-293, GP2-293 cell lines (all available from Clontech Laboratories, Palo Alto, CA, USA) - allow a wide host range to be infected with high efficiency; varying the multiplicity of infection readily adjusts the copy number of the integrated provirus. Retroviral vectors are available with a variety of selectable markers, such as resistance to neomycin, hygromycin, and puromycin, permitting ready selection of stable integrants.

The present invention further includes host cells comprising the vectors of the present invention, either present episomally within the cell or integrated, in whole or in part, into the host cell chromosome.

Among other considerations, some of which are described above, a host cell strain may be chosen for its ability to process the expressed protein in the desired fashion. Such post-translational modifications of the polypeptide include, but are not limited to, acetylation, carboxylation, glycosylation, phosphorylation, lipidation, and acylation, and it is an aspect of the present invention to provide LCP proteins with such post-translational modifications.

As noted earlier, host cells can be prokaryotic or eukaryotic. Representative examples of appropriate host cells include, but are not limited to, bacterial cells, such as *E. coli, Caulobacter crescentus, Streptomyces* species, and *Salmonella typhimurium*; yeast cells, such as *Saccharomyces cerevisiae, Schizosaccharomyces pombe, Pichia pastoris, Pichia methanolica*; insect cell lines, such as those from *Spodoptera frugiperda - e.g.,* Sf9 and Sf21 cell lines, and expresSF™ cells (Protein Sciences Corp., Meriden, CT, USA) - *Drosophila* S2 cells, and *Trichoplusia ni* High Five® Cells (Invitrogen, Carlsbad, CA, USA); and mammalian cells. Typical mammalian cells include COS1 and COS7 cells, chinese hamster ovary (CHO) cells, NIH 3T3 cells, 293 cells, HEPG2 cells, HeLa cells, L cells, murine ES cell lines (*e.g*., from strains 129/SV, C57/BL6, DBA-1, 129/SVJ), K562, Jurkat cells, and BW5147. Other mammalian cell lines are well known and readily available from the American Type Culture Collection (ATCC) (Manassas, VA, USA) and the National Institute of General medical Sciences (NIGMS) Human Genetic Cell Repository at the Coriell Cell Repositories (Camden, NJ, USA).

Methods for introducing the vectors and nucleic acids of the present invention into the host cells are well known in the art; the choice of technique will depend primarily upon the specific vector to be introduced and the host cell chosen.

For example, phage lambda vectors will typically be packaged using a packaging extract *(e.g.,* Gigapack® packaging extract, Stratagene, La Jolla, CA, USA), and the packaged virus used to infect *E. coli.* Plasmid vectors will typically be introduced into chemically competent or electrocompetent bacterial cells.

*E. coli* cells can be rendered chemically competent by treatment, *e.g.,* with CaCl₂, or a solution of Mg²⁺, Mn²⁺, Ca²⁺, Rb⁺ or K⁺, dimethyl sulfoxide, dithiothreitol, and hexamine cobalt (III), Hanahan, *J. Mol. Biol.* 166(4):557-80 (1983), and vectors introduced by heat shock. A wide variety of chemically competent strains are also available commercially (*e.g.*, Epicurian Coli® XL10-Gold® Ultracompetent Cells (Stratagene, La Jolla, CA, USA); DH5α competent cells (Clontech Laboratories, Palo Alto, CA, USA); TOP10 Chemically Competent E. coli Kit (Invitrogen, Carlsbad, CA, USA)).

Bacterial cells can be rendered electrocompetent - that is, competent to take up exogenous DNA by electroporation - by various pre-pulse treatments; vectors are introduced by electroporation followed by subsequent outgrowth in selected media. An extensive series of protocols is provided online in Electroprotocols (BioRad, Richmond, CA, USA) (http://www.bio-rad.com/LifeScience/pdf/New_Gene_Pulser.pdf).

Vectors can be introduced into yeast cells by spheroplasting, treatment with lithium salts, electroporation, or protoplast fusion.

Spheroplasts are prepared by the action of hydrolytic enzymes - a snail-gut extract, usually denoted Glusulase, or Zymolyase, an enzyme from *Arthrobacter luteus -* to remove portions of the cell wall in the presence of osmotic stabilizers, typically 1 M sorbitol. DNA is added to the spheroplasts, and the mixture is co-precipitated with a solution of polyethylene glycol (PEG) and Ca²⁺. Subsequently, the cells are resuspended in a solution of sorbitol, mixed with molten agar and then layered on the surface of a selective plate containing sorbitol. For lithium-mediated transformation, yeast cells are treated with lithium acetate, which apparently permeabilizes the cell wall, DNA is added and the cells are co-precipitated with PEG. The cells are exposed to a brief heat shock, washed free of PEG and lithium acetate, and subsequently spread on plates containing ordinary selective medium. Increased frequencies of transformation are obtained by using specially-prepared single-stranded carrier DNA and certain organic solvents. Schiestl *et al*., *Curr. Genet*. 16(5-6):339-46 (1989). For electroporation, freshly-grown yeast cultures are typically washed, suspended in an osmotic protectant, such as sorbitol, mixed with DNA, and the cell suspension pulsed in an electroporation device. Subsequently, the cells are spread on the surface of plates containing selective media. Becker *et al*., *Methods Enzymol*. 194:182-7 (1991). The efficiency of transformation by electroporation can be increased over 100-fold by using PEG, single-stranded carrier DNA and cells that are in late log-phase of growth. Larger constructs, such as YACs, can be introduced by protoplast fusion.

Mammalian and insect cells can be directly infected by packaged viral vectors, or transfected by chemical or electrical means.

For chemical transfection, DNA can be coprecipitated with CaPO₄ or introduced using liposomal and nonliposomal lipid-based agents. Commercial kits are available for CaPO₄ transfection (CalPhos™ Mammalian Transfection Kit, Clontech Laboratories, Palo Alto, CA, USA), and lipid-mediated transfection can be practiced using commercial reagents, such as LIPOFECTAMINE™ 2000, LIPOFECTAMINE™ Reagent, CELLFECTIN® Reagent, and LIPOFECTIN® Reagent (Invitrogen, Carlsbad, CA, USA), DOTAP Liposomal Transfection Reagent, FuGENE 6, X-tremeGENE Q2, DOSPER, (Roche Molecular Biochemicals, Indianapolis, IN USA), Effectene™, PolyFect®, Superfect® (Qiagen, Inc., Valencia, CA, USA). Protocols for electroporating mammalian cells can be found online in Electroprotocols (Bio-Rad, Richmond, CA, USA) (http://www.bio-rad.com/LifeScience/pdf/New_Gene_Pulser.pdf). *See also,* Norton *et al.* (eds.), Gene Transfer Methods: Introducing DNA into Living Cells and Organisms, BioTechniques Books, Eaton Publishing Co. (2000) (ISBN 1-881299-34-1), incorporated herein by reference in its entirety.

Other transfection techniques include transfection by particle embardment. *See, e.g.,* Cheng *et al*., *Proc*. *Natl*. *Acad*. *Sci. USA* 90(10):4455-9 (1993); Yang *et al*., *Proc*. *Natl*. *Acad*. *Sci*. *USA* 87(24):9568-72 (1990).

### PROTEINS

In another aspect, the present invention provides LCP proteins, various fragments thereof suitable for use as antigens *(e.g.,* for epitope mapping) and for use as immunogens (e.g., for raising antibodies or as vaccines), fusions of LCP polypeptides and fragments to heterologous polypeptides, and conjugates of the proteins, fragments, and fusions of the present invention to other moieties *(e.g.,* to carrier proteins, to fluorophores).

FIG. 3 and 4 presents the predicted amino acid sequences encoded by the LCP1 and LCP2 cDNA clones. The amino acid sequence is further presented, respectively, in SEQ ID NOs: 3 and 1114.

Unless otherwise indicated, amino acid sequences of the proteins of the present invention were determined as a predicted translation from a nucleic acid sequence. Accordingly, any amino acid sequence presented herein may contain errors due to errors in the nucleic acid sequence, as described in detail above. Furthermore, single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Small deletions and insertions can often be found that do not alter the function of the protein.

Accordingly, it is an aspect of the present invention to provide proteins not only identical in sequence to those described with particularity herein, but also to provide isolated proteins at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention by way of random or directed mutagenesis.

For purposes herein, percent identity of two amino acid sequences is determined using the procedure of Tatiana *et al.,* "Blast 2 sequences - a new tool for comparing protein and nucleotide sequences", *FEMS Microblol Lett.* 174:247-250 (1999), which procedure is effectuated by the computer program BLAST 2 SEQUENCES, available online at
http://www.ncbi.nlm.nih.gov/blast/bl2seq/bl2.html,
To assess percent identity of amino acid sequences, the BLASTP module of BLAST 2 SEQUENCES is used with default values of (i) BLOSUM62 matrix, Henikoff *et al*., *Proc*. *Natl*. *Acad*. *Sci USA* 89(22):10915-9 (1992); (ii) open gap 11 and extension gap 1 penalties; and (iii) gap x_dropoff 50 expect 10 word size 3 filter, and both sequences are entered in their entireties.

As is well known, amino acid substitutions occur frequently among natural allelic variants, with conservative substitutions often occasioning only *de minimis* change in protein function.

Accordingly, it is an aspect of the present invention to provide proteins not only identical in sequence to those described with particularity herein, but also to provide isolated proteins having the sequence of LCP proteins, or portions thereof, with conservative amino acid substitutions. It is a further aspect to provide isolated proteins having the sequence of LCP proteins, and portions thereof, with moderately conservative amino acid substitutions. These conservatively-substituted and moderately conservatively-substituted variants can be naturally occurring or can result from human intervention.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below *(see* Gonnet *et al*., *Science* 256(5062):1443-5 (1992)):

| | **A** | **R** | **N** | **D** | **C** | **Q** | **E** | **G** | **H** | **I** | **L** | **K** | **M** | **F** | **P** | **S** | **T** | **W** | **Y** | **V** |
|---|---|---|---|---|---|---|---|---|---|---|---|---|---|---|---|---|---|---|---|---|
| **A** | 2 | -1 | 0 | 0 | 0 | 0 | 0 | 0 | -1 | -1 | -1 | 0 | -1 | -2 | 0 | 1 | 1 | -4 | -2 | 0 |
| **R** | -1 | 5 | 0 | 0 | -2 | 2 | 0 | -1 | 1 | -2 | -2 | 3 | -2 | -3 | -1 | 0 | 0 | -2 | -2 | -2 |
| **N** | 0 | 0 | 4 | 2 | -2 | 1 | 1 | 0 | 1 | -3 | -3 | 1 | -2 | -3 | -1 | 1 | 0 | -4 | -1 | -2 |
| **D** | 0 | 0 | 2 | 5 | -3 | 1 | 3 | 0 | 0 | -4 | -4 | 0 | -3 | -4 | -1 | 0 | 0 | -5 | -3 | -3 |
| **C** | 0 | -2 | -2 | -3 | 12 | -2 | -3 | -2 | -1 | -1 | -2 | -3 | -1 | -1 | -3 | 0 | 0 | -1 | 0 | 0 |
| **Q** | 0 | 2 | 1 | 1 | -2 | 3 | 2 | -1 | 1 | -2 | -2 | 2 | -1 | -3 | 0 | 0 | 0 | -3 | -2 | -2 |
| **E** | 0 | 0 | 1 | 3 | -3 | 2 | 4 | -1 | 0 | -3 | -3 | 1 | -2 | -4 | 0 | 0 | 0 | -4 | -3 | -2 |
| **G** | 0 | -1 | 0 | 0 | -2 | -1 | -1 | 7 | -1 | -4 | -4 | -1 | -4 | -5 | -2 | 0 | -1 | -4 | -4 | -3 |
| **H** | -1 | 1 | 1 | 0 | -1 | 1 | 0 | -1 | 6 | -2 | -2 | 1 | -1 | 0 | -1 | 0 | 0 | -1 | 2 | -2 |
| **I** | -1 | -2 | -3 | -4 | -1 | -2 | -3 | -4 | -2 | 4 | 3 | -2 | 2 | 1 | -3 | -2 | -1 | -2 | -1 | 3 |
| **L** | -1 | -2 | -3 | -4 | -2 | -2 | -3 | -4 | -2 | 3 | 4 | -2 | 3 | 2 | -2 | -2 | -1 | -1 | 0 | 2 |
| **K** | 0 | 3 | 1 | 0 | -3 | 2 | 1 | -1 | 1 | -2 | -2 | 3 | -1 | -3 | -1 | 0 | 0 | -4 | -2 | -2 |
| **M** | -1 | -2 | -2 | -3 | -1 | -1 | -2 | -4 | -1 | 2 | 3 | -1 | 4 | 2 | -2 | -1 | -1 | -1 | 0 | 2 |
| **F** | -2 | -3 | -3 | -4 | -1 | -3 | -4 | -5 | 0 | 1 | 2 | -3 | 2 | 7 | -4 | -3 | -2 | 4 | 5 | 0 |
| **P** | 0 | -1 | -1 | -1 | -3 | 0 | 0 | -2 | -1 | -3 | -2 | -1 | -2 | -4 | 8 | 0 | 0 | -5 | -3 | -2 |
| **S** | 1 | 0 | 1 | 0 | 0 | 0 | 0 | 0 | 0 | -2 | -2 | 0 | -1 | -3 | 0 | 2 | 2 | -3 | -2 | -1 |
| **T** | 1 | 0 | 0 | 0 | 0 | 0 | 0 | -1 | 0 | -1 | -1 | 0 | -1 | -2 | 0 | 2 | 2 | -4 | -2 | 0 |
| **W** | -4 | -2 | -4 | -5 | -1 | -3 | -4 | -4 | -1 | -2 | -1 | -4 | -1 | 4 | -5 | -3 | -4 | 14 | 4 | -3 |
| **Y** | -2 | -2 | -1 | -3 | 0 | -2 | -3 | -4 | 2 | -1 | 0 | -2 | 0 | 5 | -3 | -2 | -2 | 4 | 8 | -1 |
| **V** | 0 | -2 | -2 | -3 | 0 | -2 | -2 | -3 | -2 | 3 | 2 | -2 | 2 | 0 | -2 | -1 | 0 | -3 | -1 | 3 |

For purposes herein, a "moderately conservative" replacement is any change having a nonnegative value in the PAM250 log-likelihood matrix reproduced herein above.

As is also well known in the art, relatedness of proteins can also be characterized using a functional test, the ability of the encoding nucleic acids to base-pair to one another at defined hybridization stringencies.

It is, therefore, another aspect of the invention to provide isolated proteins not only identical in sequence to those described with particularity herein, but also to provide isolated proteins ("hybridization related proteins") that are encoded by nucleic acids that hybridize under high stringency conditions (as defined herein above) to all or to a portion of various of the isolated nucleic acids of the present invention ("reference nucleic acids"). It is a further aspect of the invention to provide isolated proteins ("hybridization related proteins") that are encoded by nucleic acids that hybridize under moderate stringency conditions (as defined herein above) to all or to a portion of various of the isolated nucleic acids of the present invention ("reference nucleic acids").

The hybridization related proteins can be alternative isoforms, homologues, paralogues, and orthologues of the LCP protein of the present invention. Particularly useful orthologues are those from other primate species, such as chimpanzee, rhesus macaque monkey, baboon, orangutan, and gorilla, from rodents, such as rats, mice, guinea pigs; from lagomorphs, such as rabbits, and from domestic livestock, such as cow, pig, sheep, horse, and goat.

Relatedness of proteins can also be characterized using a second functional test, the ability of a first protein competitively to inhibit the binding of a second protein to an antibody.

It is, therefore, another aspect of the present invention to provide isolated proteins not only identical in sequence to those described with particularity herein, but also to provide isolated proteins ("cross-reactive proteins") that competitively inhibit the binding of antibodies to all or to a portion of various of the isolated LCP proteins of the present invention ("reference proteins"). Such competitive inhibition can readily be determined using immunoassays well known in the art.

Among the proteins of the present invention that differ in amino acid sequence from those described with particularity herein - including those that have deletions and insertions causing up to 10% non-identity, those having conservative or moderately conservative substitutions, hybridization related proteins, and cross-reactive proteins - those that substantially retain one or more LCP activities are particularly useful. As described above, those activities include activities of CUB, LCCL or FA58C/DS domain.

Residues that are tolerant of change while retaining function can be identified by altering the protein at known residues using methods known in the art, such as alanine scanning mutagenesis, Cunningham *et al*., *Science* 244(4908):1081-5 (1989); transposon linker scanning mutagenesis, Chen *et al*., *Gene* 263(1-2):39-48 (2001); combinations of homolog- and alanine-scanning mutagenesis, Jin *et al*., J. Mol. Biol. 226(3):851-65 (1992); combinatorial alanine scanning, Weiss *et al*., Proc. Natl. Acad. Sci USA 97(16):8950-4 (2000), followed by functional assay. Transposon linker scanning kits are available commercially (New England Biolabs, Beverly, MA, USA, catalog. no. E7-102S; EZ::TN™ In-Frame Linker Insertion Kit, catalogue no. EZI04KN, Epicentre Technologies Corporation, Madison, WI, USA).

As further described below, the isolated proteins of the present invention can readily be used as specific immunogens to raise antibodies that specifically recognize LCP proteins, their isoforms, homologues, paralogues, and/or orthologues. The antibodies, in turn, can be used, *inter alia,* specifically to assay for the LCP proteins of the present invention - *e.g.* by ELISA for detection of protein fluid samples, such as serum, by immunohistochemistry or laser scanning cytometry, for detection of protein in tissue samples, or by flow cytometry, for detection of intracellular protein in cell suspensions - for specific antibody-mediated isolation and/or purification of LCP proteins, as for example by immunoprecipitation, and for use as specific agonists or antagonists of LCP action.

The isolated proteins of the present invention are also immediately available for use as specific standards in assays used to determine the concentration and/or amount specifically of the LCP proteins of the present invention. As is well known, ELISA kits for detection and quantitation of protein analytes typically include isolated and purified protein of known concentration for use as a measurement standard *(e.g.,* the human interferon-γ OptEIA kit, catalog no. 555142, Pharmingen, San Diego, CA, USA includes human recombinant gamma interferon, baculovirus produced).

The isolated proteins of the present invention are also immediately available for use as specific biomolecule capture probes for surface-enhanced laser desorption ionization (SELDI) detection of protein-protein interactions, WO 98/59362; WO 98/59360; WO 98/59361; and Merchant *et al., Electrophoresis* 21(6):1164-77 (2000), the disclosures of which are incorporated herein by reference in their entireties. Analogously, the isolated proteins of the present invention are also immediately available for use as specific biomolecule capture probes on BIACORE surface plasmon resonance probes. . *See* Weinberger *et al*., *Pharmacogenomics* 1(4):395-416 (2000); Malmqvist, *Biochem. Soc*. *Trans.* 27(2):335-40 (1999).

The isolated proteins of the present invention are also useful as a therapeutic supplement in patients having a specific deficiency in LCP production.

In another aspect, the invention also provides fragments of various of the proteins of the present invention. The protein fragments are useful, *inter alia,* as antigenic and immunogenic fragments of LCP.

By "fragments" of a protein is here intended isolated proteins (equally, polypeptides, peptides, oligopeptides), however obtained, that have an amino acid sequence identical to a portion of the reference amino acid sequence, which portion is at least 6 amino acids and less than the entirety of the reference nucleic acid. As so defined, "fragments" need not be obtained by physical fragmentation of the reference protein, although such provenance is not thereby precluded.

Fragments of at least 6 contiguous amino acids are useful in mapping B cell and T cell epitopes of the reference protein. *See, e.g.,* Geysen *et al.,* "Use of peptide synthesis to probe viral antigens for epitopes to a resolution of a single amino acid," *Proc. Natl. Acad. Sci. USA* 81:3998-4002 (1984) and U.S. Pat. Nos. 4,708,871 and 5,595,915, the disclosures of which are incorporated herein by reference in their entireties. Because the fragment need not itself be immunogenic, part of an immunodominant epitope, nor even recognized by native antibody, to be useful in such epitope mapping, all fragments of at least 6 amino acids of the proteins of the present invention have utility in such a study.

Fragments of at least 8 contiguous amino acids, often at least 15 contiguous amino acids, have utility as immunogens for raising antibodies that recognize the proteins of the present invention. *See, e.g*., Lerner, "Tapping the immunological repertoire to produce antibodies of predetermined specificity," Nature 299:592-596 (1982); Shinnick *et al.,* "Synthetic peptide immunogens as vaccines," *Annu. Rev. Microbiol.* 37:425-46 (1983); Sutcliffe *et al.,* "Antibodies that react with predetermined sites on proteins," *Science* 219:660-6 (1983), the disclosures of which are incorporated herein by reference in their entireties. As further described in the above-cited references, virtually all 8-mers, conjugated to a carrier, such as a protein, prove immunogenic - that is, prove capable of eliciting antibody for the conjugated peptide; accordingly, all fragments of at least 8 amino acids of the proteins of the present invention have utility as immunogens.

Fragments of at least 8, 9, 10 or 12 contiguous amino acids are also useful as competitive inhibitors of binding of the entire protein, or a portion thereof, to antibodies (as in epitope mapping), and to natural binding partners, such as subunits in a multimeric complex or to receptors or ligands of the subject protein; this competitive inhibition permits identification and separation of molecules that bind specifically to the protein of interest, U.S. Pat. Nos. 5,539,084 and 5,783,674, incorporated herein by reference in their entireties.

The protein, or protein fragment, of the present invention is thus at least 6 amino acids in length, typically at least 8, 9, 10 or 12 amino acids in length, and often at least 15 amino acids in length. Often, the protein or the present invention, or fragment thereof, is at least 20 amino acids in length, even 25 amino acids, 30 amino acids, 35 amino acids, or 50 amino acids or more in length. Of course, larger fragments having at least 75 amino acids, 100 amino acids, or even 150 amino acids are also useful, and at times preferred.

The present invention further provides fusions of each of the proteins and protein fragments of the present invention to heterologous polypeptides.

By fusion is here intended that the protein or protein fragment of the present invention is linearly contiguous to the heterologous polypeptide in a peptide-bonded polymer of amino acids or amino acid analogues; by "heterologous polypeptide" is here intended a polypeptide that does not naturally occur in contiguity with the protein or protein fragment of the present invention. As so defined, the fusion can consist entirely of a plurality of fragments of the LCP protein in altered arrangement; in such case, any of the LCP fragments can be considered heterologous to the other LCP fragments in the fusion protein. More typically, however, the heterologous polypeptide is not drawn from the LCP protein itself.

The fusion proteins of the present invention will include at least one fragment of the protein of the present invention, which fragment is at least 6, typically at least 8, often at least 15, and usefully at least 16, 17, 18, 19, or 20 amino acids long. The fragment of the protein of the present to be included in the fusion can usefully be at least 25 amino acids long, at least 50 amino acids long, and can be at least 75, 100, or even 150 amino acids long. Fusions that include the entirety of the proteins of the present invention have particular utility.

The heterologous polypeptide included within the fusion protein of the present invention is at least 6 amino acids in length, often at least 8 amino acids in length, and usefully at least 15, 20, and 25 amino acids in length. Fusions that include larger polypeptides, such as the IgG Fc region, and even entire proteins (such as GFP chromophore-containing proteins), have particular utility.

As described above in the description of vectors and expression vectors of the present invention, which discussion is incorporated herein by reference in its entirety, heterologous polypeptides to be included in the fusion proteins of the present invention can usefully include those designed to facilitate purification and/or visualization of recombinantly-expressed proteins. Although purification tags can also be incorporated into fusions that are chemically synthesized, chemical synthesis typically provides sufficient purity that further purification by HPLC suffices; however, visualization tags as above described retain their utility even when the protein is produced by chemical synthesis, and when so included render the fusion proteins of the present invention useful as directly detectable markers of LCP presence.

As also discussed above, heterologous polypeptides to be included in the fusion proteins of the present invention can usefully include those that facilitate secretion of recombinantly expressed proteins - into the periplasmic space or extracellular milieu for prokaryotic hosts, into the culture medium for eukaryotic cells - through incorporation of secretion signals and/or leader sequences.

Other useful protein fusions of the present invention include those that permit use of the protein of the present invention as bait in a yeast two-hybrid system. *See* Bartel *et al*. (eds.), The Yeast Two-Hybrid System, Oxford University Press (1997) (ISBN: 0195109384); Zhu *et al*., Yeast Hybrid Technologies, Eaton Publishing, (2000) (ISBN 1-881299-15-5); Fields *et al*., *Trends Genet.* 10(8):286-92 (1994); Mendelsohn *et al*., *Curr*. *Opin*. *Biotechnol*. 5(5):482-6 (1994); Luban *et al*., *Curr*. *Opin*. *Biotechnol.* 6(1):59-64 (1995); Allen *et al*., Trends Biochem. Sci. 20(12):511-6 (1995); Drees, *Curr*. *Opin. Chem*. *Biol.* 3(1):64-70 (1999); Topcu *et al*., *Pharm*. *Res.* 17(9):1049-55 (2000); Fashena et al., Gene 250(1-2):1-14 (2000), the disclosures of which are incorporated herein by reference in their entireties. Typically, such fusion is to either *E. coli* LexA or yeast GAL4 DNA binding domains. Related bait plasmids are available that express the bait fused to a nuclear localization signal.

Other useful protein fusions include those that permit display of the encoded protein on the surface of a phage or cell, fusions to intrinsically fluorescent proteins, such as green fluorescent protein (GFP), and fusions to the IgG Fc region, as described above, which discussion is incorporated here by reference in its entirety.

The proteins and protein fragments of the present invention can also usefully be fused to protein toxins, such as Pseudomonas exotoxin A, diphtheria toxin, shiga toxin A, anthrax toxin lethal factor, ricin, in order to effect ablation of cells that bind or take up the proteins of the present invention.

The isolated proteins, protein fragments, and protein fusions of the present invention can be composed of natural amino acids linked by native peptide bonds, or can contain any or all of nonnatural amino acid analogues, nonnative bonds, and post-synthetic (post translational) modifications, either throughout the length of the protein or localized to one or more portions thereof.

As is well known in the art, when the isolated protein is used, *e.g.,* for epitope mapping, the range of such nonnatural analogues, nonnative inter-residue bonds, or post-synthesis modifications will be limited to those that permit binding of the peptide to antibodies. When used as an immunogen for the preparation of antibodies in a non-human host, such as a mouse, the range of such nonnatural analogues, nonnative inter-residue bonds, or post-synthesis modifications will be limited to those that do not interfere with the immunogenicity of the protein. When the isolated protein is used as a therapeutic agent, such as a vaccine or for replacement therapy, the range of such changes will be limited to those that do not confer toxicity upon the isolated protein.

Non-natural amino acids can be incorporated during solid phase chemical synthesis or by recombinant techniques, although the former is typically more common.

Solid phase chemical synthesis of peptides is well established in the art. Procedures are described, inter alia, in Chan *et al*. (eds.), Fmoc Solid Phase Peptide Synthesis: A Practical Approach (Practical Approach Series), Oxford Univ. Press (March 2000) (ISBN: 0199637245); Jones, Amino Acid and Peptide Synthesis (Oxford Chemistry Primers, No 7), Oxford Univ. Press (August 1992) (ISBN: 0198556683); and Bodanszky, Principles of Peptide Synthesis (Springer Laboratory), Springer Verlag (December 1993) (ISBN: 0387564314), the disclosures of which are incorporated herein by reference in their entireties.

For example, D-enantiomers of natural amino acids can readily be incorporated during chemical peptide synthesis: peptides assembled from D-amino acids are more resistant to proteolytic attack; incorporation of D-enantiomers can also be used to confer specific three dimensional conformations on the peptide. Other amino acid analogues commonly added during chemical synthesis include ornithine, norleucine, phosphorylated amino acids (typically phosphoserine, phosphothreonine, phosphotyrosine), L-malonyltyrosine, a non-hydrolyzable analog of phosphotyrosine (Kole *et al., Biochem. Biophys. Res. Com.* 209:817-821 (1995)), and various halogenated phenylalanine derivatives.

Amino acid analogues having detectable labels are also usefully incorporated during synthesis to provide a labeled polypeptide.

Biotin, for example (indirectly detectable through interaction with avidin, streptavidin, neutravidin, captavidin, or anti-biotin antibody), can be added using biotinoyl--(9-fluorenylmethoxycarbonyl)-L-lysine (FMOC biocytin) (Molecular Probes, Eugene, OR, USA). (Biotin can also be added enzymatically by incorporation into a fusion protein of a *E. coli* BirA substrate peptide.)

The FMOC and *t*BOC derivatives of dabcyl-L-lysine (Molecular Probes, Inc., Eugene, OR, USA) can be used to incorporate the dabcyl chromophore at selected sites in the peptide sequence during synthesis. The aminonaphthalene derivative EDANS, the most common fluorophore for pairing with the dabcyl quencher in fluorescence resonance energy transfer (FRET) systems, can be introduced during automated synthesis of peptides by using EDANS--FMOC-L-glutamic acid or the corresponding *t*BOC derivative (both from Molecular Probes, Inc., Eugene, OR, USA). Tetramethylrhodamine fluorophores can be incorporated during automated FMOC synthesis of peptides using (FMOC)--TMR-L-lysine (Molecular Probes, Inc. Eugene, OR, USA).

Other useful amino acid analogues that can be incorporated during chemical synthesis include aspartic acid, glutamic acid, lysine, and tyrosine analogues having allyl side-chain protection (Applied Biosystems, Inc., Foster City, CA, USA); the allyl side chain permits synthesis of cyclic, branched-chain, sulfonated, glycosylated, and phosphorylated peptides.

A large number of other FMOC-protected non-natural amino acid analogues capable of incorporation during chemical synthesis are available commercially, including, *e.g.*, Fmoc-2-aminobicyclo[2.2.1]heptane-2-carboxylic acid, Fmoc-3-endo-aminobicyclo[2.2.1]heptane-2-endo-carboxylic acid, Fmoc-3-exo-aminobicyclo[2.2.1]heptane-2-exo-carboxylic acid, Fmoc-3-endo-amino-bicyclo[2.2.1]hept-5-ene-2-endo-carboxylic acid, Fmoc-3-exo-amino-bicyclo[2.2.1]hept-5-ene-2-exo-carboxylic acid, Fmoc-cis-2-amino-1-cyclohexanecarboxylic acid, Fmoc-trans-2-amino-1-cyclohexanecarboxylic acid, Fmoc-1-amino-1-cyclopentanecarboxylic acid, Fmoc-cis-2-amino-1-cyclopentanecarboxylic acid, Fmoc-1-amino-1-cyclopropanecarboxylic acid, Fmoc-D-2-amino-4-(ethylthio)butyric acid, Fmoc-L-2-amino-4-(ethylthio)butyric acid, Fmoc-L-buthionine, Fmoc-S-methyl-L-Cysteine, Fmoc-2-aminobenzoic acid (anthranillic acid), Fmoc-3-aminobenzoic acid, Fmoc-4-aminobenzoic acid, Fmoc-2-aminobenzophenone-2'-carboxylic acid, Fmoc-N-(4-aminobenzoyl)-b-alanine, Fmoc-2-amino-4,5-dimethoxybenzoic acid, Fmoc-4-aminohippuric acid, Fmoc-2-amino-3-hydroxybenzoic acid, Fmoc-2-amino-5-hydroxybenzoic acid, Fmoc-3-amino-4-hydroxybenzoic acid, Fmoc-4-amino-3-hydroxybenzoic acid, Fmoc-4-amino-2-hydroxybenzoic acid, Fmoc-5-amino-2-hydroxybenzoic acid, Fmoc-2-amino-3-methoxybenzoic acid, Fmoc-4-amino-3-methoxybenzoic acid, Fmoc-2-amino-3-methylbenzoic acid, Fmoc-2-amino-5-methylbenzoic acid, Fmoc-2-amino-6-methylbenzoic acid, Fmoc-3-amino-2-methylbenzoic acid, Fmoc-3-amino-4-methylbenzoic acid, Fmoc-4-amino-3-methylbenzoic acid, Fmoc-3-amino-2-naphtoic acid, Fmoc-D,L-3-amino-3-phenylpropionic acid, Fmoc-L-Methyldopa, Fmoc-2-amino-4,6-dimethyl-3-pyridinecarboxylic acid, Fmoc-D,L-?-amino-2-thiophenacetic acid, Fmoc-4-(carboxymethyl)piperazine, Fmoc-4-carboxypiperazine, Fmoc-4-(carboxymethyl)homopiperazine, Fmoc-4-phenyl-4-piperidinecarboxylic acid, Fmoc-L-1,2,3,4-tetrahydronorharman-3-carboxylic acid, Fmoc-L-thiazolidine-4-carboxylic acid, all available from The Peptide Laboratory (Richmond, CA, USA).

Non-natural residues can also be added biosynthetically by engineering a suppressor tRNA, typically one that recognizes the UAG stop codon, by chemical aminoacylation with the desired unnatural amino acid and. Conventional site-directed mutagenesis is used to introduce the chosen stop codon UAG at the site of interest in the protein gene. When the acylated suppressor tRNA and the mutant gene are combined in an *in vitro* transcription/translation system, the unnatural amino acid is incorporated in response to the UAG codon to give a protein containing that amino acid at the specified position. Liu *et al., Proc. Natl Acad. Sci. USA* 96(9):4780-5 (1999); Wang *et al., Science* 292(5516):498-500 (2001).

The isolated proteins, protein fragments and fusion proteins of the present invention can also include nonnative inter-residue bonds, including bonds that lead to circular and branched forms.

The isolated proteins and protein fragments of the present invention can also include post-translational and post-synthetic modifications, either throughout the length of the protein or localized to one or more portions thereof.

For example, when produced by recombinant expression in eukaryotic cells, the isolated proteins, fragments, and fusion proteins of the present invention will typically include N-linked and/or O-linked glycosylation, the pattern of which will reflect both the availability of glycosylation sites on the protein sequence and the identity of the host cell. Further modification of glycosylation pattern can be performed enzymatically.

As another example, recombinant polypeptides of the invention may also include an initial modified methionine residue, in some cases resulting from host-mediated processes.

When the proteins, protein fragments, and protein fusions of the present invention are produced by chemical synthesis, post-synthetic modification can be performed before deprotection and cleavage from the resin or after deprotection and cleavage. Modification before deprotection and cleavage of the synthesized protein often allows greater control, *e.g.* by allowing targeting of the modifying moiety to the N-terminus of a resin-bound synthetic peptide.

Useful post-synthetic (and post-translational) modifications include conjugation to detectable labels, such as fluorophores.

A wide variety of amine-reactive and thiol-reactive fluorophore derivatives have been synthesized that react under nondenaturing conditions with N-terminal amino groups and epsilon amino groups of lysine residues, on the one hand, and with free thiol groups of cysteine residues, on the other.

Kits are available commercially that permit conjugation of proteins to a variety of amine-reactive or thiol-reactive fluorophores: Molecular Probes, Inc. (Eugene, OR, USA), *e.g.*, offers kits for conjugating proteins to Alexa Fluor 350, Alexa Fluor 430, Fluorescein-EX, Alexa Fluor 488, Oregon Green 488, Alexa Fluor 532, Alexa Fluor 546, Alexa Fluor 546, Alexa Fluor 568, Alexa Fluor 594, and Texas Red-X.

A wide variety of other amine-reactive and thiol-reactive fluorophores are available commercially (Molecular Probes, Inc., Eugene, OR, USA), including Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA).

The polypeptides of the present invention can also be conjugated to fluorophores, other proteins, and other macromolecules, using bifunctional linking reagents.

Common homobifunctional reagents include, *e.g.,* APG, AEDP, BASED, BMB, BMDB, BMH, BMOE, BM[PEO]3, BM[PEO]4, BS3, BSOCOES, DFDNB, DMA, DMP, DMS, DPDPB, DSG, DSP (Lomant's Reagent), DSS, DST, DTBP, DTME, DTSSP, EGS, HBVS, Sulfo-BSOCOES, Sulfo-DST, Sulfo-EGS (all available from Pierce, Rockford, IL, USA); common heterobifunctional cross-linkers include ABH, AMAS, ANB-NOS, APDP, ASBA, BMPA, BMPH, BMPS, EDC, EMCA, EMCH, EMCS, KMUA, KMUH, GMBS, LC-SMCC, LC-SPDP, MBS, M2C2H, MPBH, MSA, NHS-ASA, PDPH, PMPI, SADP, SAED, SAND, SANPAH, SASD, SATP, SBAP, SFAD, SIA, SIAB, SMCC, SMPB, SMPH, SMPT, SPDP, Sulfo-EMCS, Sulfo-GMBS, Sulfo-HSAB, Sulfo-KMUS, Sulfo-LC-SPDP, Sulfo-MBS, Sulfo-NHS-LC-ASA, Sulfo-SADP, Sulfo-SANPAH, Sulfo-SIAB, Sulfo-SMCC, Sulfo-SMPB, Sulfo-LC-SMPT, SVSB, TFCS (all available Pierce, Rockford, IL, USA).

The proteins, protein fragments, and protein fusions of the present invention can be conjugated, using such cross-linking reagents, to fluorophores that are not amine- or thiol-reactive.

Other labels that usefully can be conjugated to the proteins, protein fragments, and fusion proteins of the present invention include radioactive labels, echosonographic contrast reagents, and MRI contrast agents.

The proteins, protein fragments, and protein fusions of the present invention can also usefully be conjugated using cross-linking agents to carrier proteins, such as KLH, bovine thyroglobulin, and even bovine serum albumin (BSA), to increase immunogenicity for raising anti-LCP antibodies.

The proteins, protein fragments, and protein fusions of the present invention can also usefully be conjugated to polyethylene glycol (PEG); PEGylation increases the serum half life of proteins administered intravenously for replacement therapy. Delgado et *al., Crit. Rev. Ther. Drug Carrier Syst.* 9(3-4):249-304 (1992); Scott *et al., Curr. Pharm. Des.* 4(6):423-38 (1998); DeSantis *et al., Curr. Opin. Biotechnol.* 10(4):324-30 (1999), incorporated herein by reference in their entireties. PEG monomers can be attached to the protein directly or through a linker, with PEGylation using PEG monomers activated with tresyl chloride (2,2,2-trifluoroethanesulphonyl chloride) permitting direct attachment under mild conditions.

The isolated proteins of the present invention, including fusions thereof, can be produced by recombinant expression, typically using the expression vectors of the present invention as above-described or, if fewer than about 100 amino acids, by chemical synthesis (typically, solid phase synthesis), and, on occasion, by *in vitro* translation.

Production of the isolated proteins of the present invention can optionally be followed by purification. Purification of recombinantly expressed proteins is now well within the skill in the art. *See, e.g.,* Thorner *et al.* (eds.), Applications of Chimeric Genes and Hybrid Proteins, Part A: Gene Expression and Protein Purification (Methods in Enzymology, Volume 326), Academic Press (2000), (ISBN: 0121822273); Harbin (ed.), Cloning, Gene Expression and Protein Purification : Experimental Procedures and Process Rationale, Oxford Univ. Press (2001) (ISBN: 0195132947); Marshak *et al*., Strategies for Protein Purification and Characterization: A Laboratory Course Manual, Cold Spring Harbor Laboratory Press (1996) (ISBN: 0-87969-385-1); and Roe (ed.), Protein Purification Applications, Oxford University Press (2001), the disclosures of which are incorporated herein by reference in their entireties, and thus need not be detailed here.

Briefly, however, if purification tags have been fused through use of an expression vector that appends such tag, purification can be effected, at least in part, by means appropriate to the tag, such as use of immobilized metal affinity chromatography for polyhistidine tags. Other techniques common in the art include ammonium sulfate fractionation, immunoprecipitation, fast protein liquid chromatography (FPLC), high performance liquid chromatography (HPLC), and preparative gel electrophoresis.

Purification of chemically-synthesized peptides can readily be effected, *e.g.*, by HPLC.

Accordingly, it is an aspect of the present invention to provide the isolated proteins of the present invention in pure or substantially pure form.

A purified protein of the present invention is an isolated protein, as above described, that is present at a concentration of at least 95%, as measured on a weight basis (w/w) with respect to total protein in a composition. Such purities can often be obtained during chemical synthesis without further purification, as, *e.g.,* by HPLC. Purified proteins of the present invention can be present at a concentration (measured on a weight basis with respect to total protein in a composition) of 96%, 97%, 98%, and even 99%. The proteins of the present invention can even be present at levels of 99.5%, 99.6%, and even 99.7%, 99.8%, or even 99.9% following purification, as by HPLC.

Although high levels of purity are particularly useful when the isolated proteins of the present invention are used as therapeutic agents - such as vaccines, or for replacement therapy - the isolated proteins of the present invention are also useful at lower purity. For example, partially purified proteins of the present invention can be used as immunogens to raise antibodies in laboratory animals.

Thus, in another aspect, the present invention provides the isolated proteins of the present invention in substantially purified form. A "substantially purified protein" of the present invention is an isolated protein, as above described, present at a concentration of at least 70%, measured on a weight basis with respect to total protein in a composition. Usefully, the substantially purified protein is present at a concentration, measured on a weight basis with respect to total protein in a composition, of at least 75%, 80%, or even at least 85%, 90%, 91%, 92%, 93%, 94%, 94.5% or even at least 94.9%.

In preferred embodiments, the purified and substantially purified proteins of the present invention are in compositions that lack detectable ampholytes, acrylamide monomers, bis-acrylamide monomers, and polyacrylamide.

The proteins, fragments, and fusions of the present invention can usefully be attached to a substrate. The substrate can porous or solid, planar or non-planar; the bond can be covalent or noncovalent.

For example, the proteins, fragments, and fusions of the present invention can usefully be bound to a porous substrate, commonly a membrane, typically comprising nitrocellulose, polyvinylidene fluoride (PVDF), or cationically derivatized, hydrophilic PVDF; so bound, the proteins, fragments, and fusions of the present invention can be used to detect and quantify antibodies, *e.g.* in serum, that bind specifically to the immobilized protein of the present invention.

As another example, the proteins, fragments, and fusions of the present invention can usefully be bound to a substantially nonporous substrate, such as plastic, to detect and quantify antibodies, *e.g.* in serum, that bind specifically to the immobilized protein of the present invention. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof; when the assay is performed in standard microtiter dish, the plastic is typically polystyrene.

The proteins, fragments, and fusions of the present invention can also be attached to a substrate suitable for use as a surface enhanced laser desorption ionization source; so attached, the protein, fragment, or fusion of the present invention is useful for binding and then detecting secondary proteins that bind with sufficient affinity or avidity to the surface-bound protein to indicate biologic interaction therebetween. The proteins, fragments, and fusions of the present invention can also be attached to a substrate suitable for use in surface plasmon resonance detection; so attached, the protein, fragment, or fusion of the present invention is useful for binding and then detecting secondary proteins that bind with sufficient affinity or avidity to the surface-bound protein to indicate biological interaction therebetween.

### LCP Proteins

In a first series of protein embodiments, the invention provides isolated LCP polypeptides having amino acid sequences in SEQ ID NO: 3 or 1114, which are full length LCP1 and LCP2 proteins. When used as immunogens, the full length proteins of the present invention can be used, *inter alia,* to elicit antibodies that bind to a variety of epitopes of the LCP proteins.

The invention further provides fragments of the above-described polypeptides, particularly fragments having at least 6 amino acids, typically at least 8 amino acids, often at least 15 amino acids, and even the entirety of the sequence given in SEQ ID NOs: 3 or 1114.

The invention further provides fragments of at least 6 amino acids, typically at least 8 amino acids, often at least 15 amino acids, and even the entirety of the sequence given in SEQ ID NO: 5.

The invention also provides fragments of at least 6 amino acids, typically at least 8 amino acids, often at least 15 amino acids, and even the entirety of the sequence given in SEQ ID NO: 9.

The invention also provides fragments of at least 6 amino acids, typically at least 8 amino acids, often at least 15 amino acids, and even the entirety of the sequence given in SEQ ID NO: 1116.

As described above, the invention further provides proteins that differ in sequence from those described with particularity in the above-referenced SEQ ID NOs., whether by way of insertion or deletion, by way of conservative or moderately conservative substitutions, as hybridization related proteins, or as cross-hybridizing proteins, with those that substantially retain an LCP activity particularly useful.

The invention further provides fusions of the proteins and protein fragments herein described to heterologous polypeptides.

### ANTIBODIES AND ANTIBODY-PRODUCING CELLS

In another aspect, the invention provides antibodies, including fragments and derivatives thereof, that bind specifically to LCP proteins and protein fragments of the present invention or to one or more of the proteins and protein fragments encoded by the isolated LCP nucleic acids of the present invention. The antibodies of the present invention can be specific for all of linear epitopes, discontinuous epitopes, or conformational epitopes of such proteins or protein fragments, either as present on the protein in its native conformation or, in some cases, as present on the proteins as denatured, as, *e.g.,* by solubilization in SDS.

In other embodiments, the invention provides antibodies, including fragments and derivatives thereof, the binding of which can be competitively inhibited by one or more of the LCP proteins and protein fragments of the present invention, or by one or more of the proteins and protein fragments encoded by the isolated LCP nucleic acids of the present invention.

As used herein, the term "antibody" refers to a polypeptide, at least a portion of which is encoded by at least one immunoglobulin gene, which can bind specifically to a first molecular species, and to fragments or derivatives thereof that remain capable of such specific binding.

By "bind specifically" and "specific binding" is here intended the ability of the antibody to bind to a first molecular species in preference to binding to other molecular species with which the antibody and first molecular species are admixed. An antibody is said specifically to "recognize" a first molecular species when it can bind specifically to that first molecular species.

As is well known in the art, the degree to which an antibody can discriminate as among molecular species in a mixture will depend, in part, upon the conformational relatedness of the species in the mixture; typically, the antibodies of the present invention will discriminate over adventitious binding to non-**LCP** proteins by at least two-fold, more typically by at least 5-fold, typically by more than 10-fold, 25-fold, 50-fold, 75-fold, and often by more than 100-fold, and on occasion by more than 500-fold or 1000-fold. When used to detect the proteins or protein fragments of the present invention, the antibody of the present invention is sufficiently specific when it can be used to determine the presence of the protein of the present invention in samples derived from human adrenal, adult liver, bone marrow, brain, fetal liver, heart, kidney, lung, placenta, skeletal muscle, colon and prostate, as well as a cell line, hela.

Typically, the affinity or avidity of an antibody (or antibody multimer, as in the case of an IgM pentamer) of the present invention for a protein or protein fragment of the present invention will be at least about 1 x 10⁻⁶ molar (M), typically at least about 5 x 10⁻⁷ M, usefully at least about 1 x 10⁻⁷ M, with affinities and avidities of at least 1 x 10⁻⁸ M, 5 x 10⁻⁹ M, and 1 x 10⁻¹⁰ M proving especially useful.

The antibodies of the present invention can be naturally-occurring forms, such as IgG, IgM, IgD, IgE, and IgA, from any mammalian species.

Human antibodies can, but will infrequently, be drawn directly from human donors or human cells. In such case, antibodies to the proteins of the present invention will typically have resulted from fortuitous immunization, such as autoimmune immunization, with the protein or protein fragments of the present invention. Such antibodies will typically, but will not invariably, be polyclonal.

Human antibodies are more frequently obtained using transgenic animals that express human immunoglobulin genes, which transgenic animals can be affirmatively immunized with the protein immunogen of the present invention. Human Ig-transgenic mice capable of producing human antibodies and methods of producing human antibodies therefrom upon specific immunization are described, *inter alia,* in U.S. Patent Nos. 6,162,963; 6,150,584; 6,114,598; 6,075,181; 5,939,598; 5,877,397; 5,874,299; 5,814,318; 5,789,650; 5,770,429; 5,661,016; 5,633,425; 5,625,126; 5,569,825; 5,545,807; 5,545,806, and 5,591,669, the disclosures of which are incorporated herein by reference in their entireties. Such antibodies are typically monoclonal, and are typically produced using techniques developed for production of murine antibodies.

Human antibodies are particularly useful, and often preferred, when the antibodies of the present invention are to be administered to human beings as *in vivo* diagnostic or therapeutic agents, since recipient immune response to the administered antibody will often be substantially less than that occasioned by administration of an antibody derived from another species, such as mouse.

IgG, IgM, IgD, IgE and IgA antibodies of the present invention are also usefully obtained from other mammalian species, including rodents - typically mouse, but also rat, guinea pig, and hamster - lagomorphs, typically rabbits, and also larger mammals, such as sheep, goats, cows, and horses. In such cases, as with the transgenic human-antibody-producing non-human mammals, fortuitous immunization is not required, and the non-human mammal is typically affirmatively immunized, according to standard immunization protocols, with the protein or protein fragment of the present invention.

As discussed above, virtually all fragments of 8 or more contiguous amino acids of the proteins of the present invention can be used effectively as immunogens when conjugated to a carrier, typically a protein such as bovine thyroglobulin, keyhole limpet hemocyanin, or bovine serum albumin, conveniently using a bifunctional linker such as those described elsewhere above, which discussion is incorporated by reference here.

Immunogenicity can also be conferred by fusion of the proteins and protein fragments of the present invention to other moieties.

For example, peptides of the present invention can be produced by solid phase synthesis on a branched polylysine core matrix; these multiple antigenic peptides (MAPs) provide high purity, increased avidity, accurate chemical definition and improved safety in vaccine development. Tam *et al.,* Proc. Natl. Acad. Sci. USA 85:5409-5413 (1988); Posnett et al., *J. Biol. Chem.* 263, 1719-1725 (1988).

Protocols for immunizing non-human mammals are well-established in the art, Harlow *et al.* (eds.), Antibodies: A Laboratory Manual, Cold Spring Harbor Laboratory (1998) (ISBN: 0879693142); Coligan *et al.* (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907), the disclosures of which are incorporated herein by reference, and often include multiple immunizations, either with or without adjuvants such as Freund's complete adjuvant and Freund's incomplete adjuvant.

Antibodies from nonhuman mammals can be polyclonal or monoclonal, with polyclonal antibodies having certain advantages in immunohistochemical detection of the proteins of the present invention and monoclonal antibodies having advantages in identifying and distinguishing particular epitopes of the proteins of the present invention.

Following immunization, the antibodies of the present invention can be produced using any art-accepted technique. Such techniques are well known in the art, Coligan *et al.* (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907); Howard *et al.* (eds.), Basic Methods in Antibody Production and Characterization, CRC Press (2000) (ISBN: 0849394457); Harlow *et al.* (eds.), Antibodies: A Laboratory Manual, Cold Spring Harbor Laboratory (1998) (ISBN: 0879693142); Davis (ed.), Monoclonal Antibody Protocols, Vol. 45, Humana Press (1995) (ISBN: 0896033082); Delves (ed.), Antibody Production: Essential Techniques, John Wiley & Son Ltd (1997) (ISBN: 0471970107); Kenney, Antibody Solution: An Antibody Methods Manual, Chapman & Hall (1997) (ISBN: 0412141914), incorporated herein by reference in their entireties, and thus need not be detailed here.

Briefly, however, such techniques include, *inter alia*, production of monoclonal antibodies by hybridomas and expression of antibodies or fragments or derivatives thereof from host cells engineered to express immunoglobulin genes or fragments thereof. These two methods of production are not mutually exclusive: genes encoding antibodies specific for the proteins or protein fragments of the present invention can be cloned from hybridomas and thereafter expressed in other host cells. Nor need the two necessarily be performed together: *e.g.*, genes encoding antibodies specific for the proteins and protein fragments of the present invention can be cloned directly from B cells known to be specific for the desired protein, as further described in U.S. Pat. No. 5,627,052, the disclosure of which is incorporated herein by reference in its entirety, or from antibody-displaying phage.

Recombinant expression in host cells is particularly useful when fragments or derivatives of the antibodies of the present invention are desired.

Host cells for recombinant antibody production - either whole antibodies, antibody fragments, or antibody derivatives - can be prokaryotic or eukaryotic.

Prokaryotic hosts are particularly useful for producing phage displayed antibodies of the present invention.

The technology of phage-displayed antibodies, in which antibody variable region fragments are fused, for example, to the gene III protein (pIII) or gene VIII protein (pVIII) for display on the surface of filamentous phage, such as M13, is by now well-established, Sidhu, *Curr. Opin. Biotechnol.* 11(6):610-6 (2000); Griffiths *et al., Curr. Opin. Biotechnol.* 9(1):102-8 (1998); Hoogenboom et *al., Immunotechnology,* 4(1):1-20 (1998); Rader *et al., Current Opinion in Biotechnology* 8:503-508 (1997); Aujame *et al., Human Antibodies* 8:155-168 (1997); Hoogenboom, *Trends in Biotechnol.* 15:62-70 (1997); de Kruif *et al.,* 17:453-455 (1996); Barbas *et al., Trends in Biotechnol.* 14:230-234 (1996); Winter *et al., Ann. Rev. Immunol.* 433-455 (1994), and techniques and protocols required to generate, propagate, screen (pan), and use the antibody fragments from such libraries have recently been compiled, Barbas *et al*., Phage Display: A Laboratory Manual, Cold Spring Harbor Laboratory Press (2001) (ISBN 0-87969-546-3); Kay *et al*. (eds.), Phage Display of Peptides and Proteins: A Laboratory Manual, Academic Press, Inc. (1996); Abelson *et al*. (eds.), Combinatorial Chemistry, Methods in Enzymology vol. 267, Academic Press (May 1996), the disclosures of which are incorporated herein by reference in their entireties.

Typically, phage-displayed antibody fragments are scFv fragments or Fab fragments; when desired, full length antibodies can be produced by cloning the variable regions from the displaying phage into a complete antibody and expressing the full length antibody in a further prokaryotic or a eukaryotic host cell.

Eukaryotic cells are also useful for expression of the antibodies, antibody fragments, and antibody derivatives of the present invention.

For example, antibody fragments of the present invention can be produced in *Pichia pastoris,* Takahashi *et al., Biosci. Biotechnol. Biochem.* 64(10):2138-44 (2000); Freyre *et al.,* J. Biotechnol. 76(2-3):157-63 (2000); Fischer *et al., Biotechnol. Appl. Biochem.* 30 (Pt 2):117-20 (1999); Pennell *et al., Res. Immunol.* 149(6):599-603 (1998); Eldin *et al., J. Immunol. Methods.* 201(1):67-75 (1997); and in *Saccharomyces cerevisiae,* Frenken *et al., Res. Immunol.* 149(6):589-99 (1998); Shusta et al., *Nature Biotechnol.* 16(8):773-7 (1998), the disclosures of which are incorporated herein by reference in their entireties.

Antibodies, including antibody fragments and derivatives, of the present invention can also be produced in insect cells, Li *et al., Protein Expr. Purif.* 21(1):121-8 (2001); Ailor *et al*., *Biotechnol*. *Bioeng*. 58(2-3):196-203 (1998); Hsu *et al*., *Biotechnol*. *Prog.* 13(1):96-104 (1997); Edelman *et al*., *Immunology* 91(1):13-9 (1997); and Nesbit *et al*., *J*. *Immunol*. *Methods.* 151(1-2):201-8 (1992), the disclosures of which are incorporated herein by reference in their entireties.

Antibodies and fragments and derivatives thereof of the present invention can also be produced in plant cells, Giddings *et al., Nature Biotechnol.* 18(11):1151-5 (2000); Gavilondo *et al., Biotechniques* 29(1):128-38 (2000); Fischer *et al., J. Biol. Regul. Homeost. Agents* 14(2):83-92 (2000); Fischer *et al., Biotechnol. Appl. Biochem.* 30 (Pt 2):113-6 (1999); Fischer *et al., Biol. Chem.* 380(7-8):825-39 (1999); Russell, *Curr. Top. Microbiol. Immunol.* 240:119-38 (1999); and Ma *et al., Plant Physiol.* 109(2):341-6 (1995), the disclosures of which are incorporated herein by reference in their entireties.

Mammalian cells useful for recombinant expression of antibodies, antibody fragments, and antibody derivatives of the present invention include CHO cells, COS cells, 293 cells, and myeloma cells.

Verma *et al*., *J*. *Immunol*. *Methods* 216(1-2):165-81 (1998), review and compare bacterial, yeast, insect and mammalian expression systems for expression of antibodies.

Antibodies of the present invention can also be prepared by cell free translation, as further described in Merk *et al.,* J. Biochem. (Tokyo). 125(2):328-33 (1999) and Ryabova *et al., Nature Biotechnol.* 15(1):79-84 (1997), and in the milk of transgenic animals, as further described in Pollock *et al., J. Immunol. Methods* 231(1-2):147-57 (1999), the disclosures of which are incorporated herein by reference in their entireties.

The invention further provides antibody fragments that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful fragments are Fab, Fab', Fv, F(ab)'₂, and single chain Fv (scFv) fragments. Other useful fragments are described in Hudson, *Curr. Opin. Biotechnol*. 9(4):395-402 (1998).

It is also an aspect of the present invention to provide antibody derivatives that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful derivatives are chimeric, primatized, and humanized antibodies; such derivatives are less immunogenic in human beings, and thus more suitable for in vivo administration, than are unmodified antibodies from non-human mammalian species.

Chimeric antibodies typically include heavy and/or light chain variable regions (including both CDR and framework residues) of immunoglobulins of one species, typically mouse, fused to constant regions of another species, typically human. *See, e.g.,* U.S. Pat. No. 5,807,715; Morrison *et al., Proc. Natl. Acad. Sci USA*. 81(21):6851-5 (1984); Sharon *et al., Nature* 309(5966):364-7 (1984); Takeda *et al*., *Nature* 314 (6010):452-4 (1985), the disclosures of which are incorporated herein by reference in their entireties. Primatized and humanized antibodies typically include heavy and/or light chain CDRs from a murine antibody grafted into a non-human primate or human antibody V region framework, usually further comprising a human constant region, Riechmann *et al*., *Nature* 332(6162):323-7 (1988); Co *et al*., *Nature* 351(6326):501-2 (1991); U.S. Pat. Nos. 6,054,297; 5,821,337; 5,770,196; 5,766,886; 5,821,123; 5,869,619; 6,180,377; 6,013,256; 5,693,761; and 6,180,370, the disclosures of which are incorporated herein by reference in their entireties.

Other useful antibody derivatives of the invention include heteromeric antibody complexes and antibody fusions, such as diabodies (bispecific antibodies), single-chain diabodies, and intrabodies.

The antibodies of the present invention, including fragments and derivatives thereof, can usefully be labeled. It is, therefore, another aspect of the present invention to provide labeled antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

The choice of label depends, in part, upon the desired use.

For example, when the antibodies of the present invention are used for immunohistochemical staining of tissue samples, the label can usefully be an enzyme that catalyzes production and local deposition of a detectable product.

Enzymes typically conjugated to antibodies to permit their immunohistochemical visualization are well known, and include alkaline phosphatase, β-galactosidase, glucose oxidase, horseradish peroxidase (HRP), and urease. Typical substrates for production and deposition of visually detectable products include o-nitrophenyl-beta-D-galactopyranoside (ONPG); o-phenylenediamine dihydrochloride (OPD); p-nitrophenyl phosphate (PNPP); p-nitrophenyl-beta-D-galactopryanoside (PNPG); 3',3'-diaminobenzidine (DAB); 3-amino-9-ethylcarbazole (AEC); 4-chloro-1-naphthol (CN); 5-bromo-4-chloro-3-indolyl-phosphate (BCIP); ABTS®; BluoGal; iodonitrotetrazolium (INT); nitroblue tetrazolium chloride (NBT); phenazine methosulfate (PMS); phenolphthalein monophosphate (PMP); tetramethyl benzidine (TMB); tetranitroblue tetrazolium (TNBT); X-Gal; X-Gluc; and X-Glucoside.

Other substrates can be used to produce products for local deposition that are luminescent. For example, in the presence of hydrogen peroxide (H₂O₂), horseradish peroxidase (HRP) can catalyze the oxidation of cyclic diacylhydrazides, such as luminol. Immediately following the oxidation, the luminol is in an excited state (intermediate reaction product), which decays to the ground state by emitting light. Strong enhancement of the light emission is produced by enhancers, such as phenolic compounds. Advantages include high sensitivity, high resolution, and rapid detection without radioactivity and requiring only small amounts of antibody. *See, e.g.,* Thorpe *et al*., *Methods Enzymol*. 133:331-53 (1986); Kricka *et al*., *J*. *Immunoassay* 17(1):67-83 (1996); and Lundqvist *et al*., *J*. *Biolumin*. *Chemilumin*. 10(6):353-9 (1995), the disclosures of which are incorporated herein by reference in their entireties. Kits for such enhanced chemiluminescent detection (ECL) are available commercially.

The antibodies can also be labeled using colloidal gold.

As another example, when the antibodies of the present invention are used, *e.g.,* for flow cytometric detection, for scanning laser cytometric detection, or for fluorescent immunoassay, they can usefully be labeled with fluorophores.

There are a wide variety of fluorophore labels that can usefully be attached to the antibodies of the present invention.

For flow cytometric applications, both for extracellular detection and for intracellular detection, common useful fluorophores can be fluorescein isothiocyanate (FITC), allophycocyanin (APC), R-phycoerythrin (PE), peridinin chlorophyll protein (PerCP), Texas Red, Cy3, Cy5, fluorescence resonance energy tandem fluorophores such as PerCP-Cy5.5, PE-Cy5, PE-Cy5.5, PE-Cy7, PE-Texas Red, and APC-Cy7.

Other fluorophores include, *inter alia,* Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA), and Cy2, Cy3, Cy3.5, Cy5, Cy5.5, Cy7, all of which are also useful for fluorescently labeling the antibodies of the present invention.

For secondary detection using labeled avidin, streptavidin, captavidin or neutravidin, the antibodies of the present invention can usefully be labeled with biotin.

When the antibodies of the present invention are used, *e*.*g*., for western blotting applications, they can usefully be labeled with radioisotopes, such as ³³P, ³²P, ³⁵S, ³H, and ¹²⁵I.

As another example, when the antibodies of the present invention are used for radioimmunotherapy, the label can usefully be ²²⁸Th, ²²⁷Ac, ²²⁵Ac, ²²³Ra, ²¹³Bi, ²¹²Pb, ²¹²Bi, ²¹¹At, ²⁰³Pb, ¹⁹⁴Os, ¹⁸⁸Re, ¹⁸⁶Re, ¹⁵³Sm, ¹⁴⁹Tb, ¹³¹I, ¹²⁵I, ¹¹¹In, ¹⁰⁵Rh, ^{99m}Tc, ⁹⁷Ru, ⁹⁰Y, ⁹⁰Sr, ⁸⁸Y, ⁷²Se, ⁶⁷Cu, or ⁴⁷Sc.

As another example, when the antibodies of the present invention are to be used for *in vivo* diagnostic use, they can be rendered detectable by conjugation to MRI contrast agents, such as gadolinium diethylenetriaminepentaacetic acid (DTPA), Lauffer *et al., Radiology* 207(2):529-38 (1998), or by radioisotopic labeling

As would be understood, use of the labels described above is not restricted to the application as for which they were mentioned.

The antibodies of the present invention, including fragments and derivatives thereof, can also be conjugated to toxins, in order to target the toxin's ablative action to cells that display and/or express the proteins of the present invention. Commonly, the antibody in such immunotoxins is conjugated to Pseudomonas exotoxin A, diphtheria toxin, shiga toxin A, anthrax toxin lethal factor, or ricin. *See* Hall (ed.), Immunotoxin Methods and Protocols (Methods in Molecular Biology, Vol 166), Humana Press (2000) (ISBN:0896037754); and Frankel *et* *al*. (eds.), Clinical Applications of Immunotoxins, Springer-Verlag New York, Incorporated (1998) (ISBN:3540640975), the disclosures of which are incorporated herein by reference in their entireties, for review.

The antibodies of the present invention can usefully be attached to a substrate, and it is, therefore, another aspect of the invention to provide antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention, attached to a substrate.

Substrates can be porous or nonporous, planar or nonplanar.

For example, the antibodies of the present invention can usefully be conjugated to filtration media, such as NHS-activated Sepharose or CNBr-activated Sepharose for purposes of immunoaffinity chromatography.

For example, the antibodies of the present invention can usefully be attached to paramagnetic microspheres, typically by biotin-streptavidin interaction, which microsphere can then be used for isolation of cells that express or display the proteins of the present invention. As another example, the antibodies of the present invention can usefully be attached to the surface of a microtiter plate for ELISA.

As noted above, the antibodies of the present invention can be produced in prokaryotic and eukaryotic cells. It is, therefore, another aspect of the present invention to provide cells that express the antibodies of the present invention, including hybridoma cells, B cells, plasma cells, and host cells recombinantly modified to express the antibodies of the present invention.

In yet a further aspect, the present invention provides aptamers evolved to bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

### LCP Antibodies

In a first series of antibody embodiments, the invention provides antibodies, both polyclonal and monoclonal, and fragments and derivatives thereof, that bind specifically to a polypeptide having an amino acid sequence in SEQ ID NOs: 3 or 1114, which are full length LCP1 and LCP2 proteins.

Such antibodies are useful in *in vitro* immunoassays, such as ELISA, western blot or immunohistochemical assay of disease tissue or cells. Such antibodies are also useful in isolating and purifying LCP proteins, including related cross-reactive proteins, by immunoprecipitation, immunoaffinity chromatography, or magnetic bead-mediated purification.

In a second series of antibody embodiments, the invention provides antibodies, both polyclonal and monoclonal, and fragments and derivative thereof, that bind specifically to polypeptides comprising an amino acid sequence as provided in SEQ ID NO: 1116 - a 20 amino acid region of LCP2 centered about the splice junction of exons 1 and 3 of LCP1 - and binding of which can be competitively inhibited by a polypeptide the sequence of which is given in SEQ ID NO: 1116 and cannot be competitively inhibited by a polypeptide having the amino acid sequence of SEQ ID NO: 3 (the full length LCP1 protein).

Such antibodies can be used to discriminate LCP2 from the LCP1 isoform and are useful in *in vitro* immunoassays, such as ELISA, western blot or immunohistochemical assay of disease tissue or cells. Such antibodies are also useful in isolating and purifying LCP2 proteins, including related cross-reactive proteins, by immunoprecipitation, immunoaffinity chromatography, or magnetic bead-mediated purification.

In another series of antibody embodiments, the invention provides antibodies, both polyclonal and monoclonal, and fragments and derivatives thereof, the specific binding of which can be competitively inhibited by the isolated proteins and polypeptides of the present invention.

In other embodiments, the invention further provides the above-described antibodies detectably labeled, and in yet other embodiments, provides the above-described antibodies attached to a substrate.

### PHARMACEUTICAL COMPOSITIONS

LCP is important for neurological and developmental disorders, as well as diseases involving cell-cell adhesion process; defects in LCP expression, activity, distribution, localization, and/or solubility are a cause of human disease, which disease can manifest as a disorder of adrenal, adult liver, bone marrow, brain, fetal liver, heart, kidney, lung, placenta, skeletal muscle, colon or prostate function.

Accordingly, pharmaceutical compositions comprising nucleic acids, proteins, and antibodies of the present invention, as well as mimetics, agonists, antagonists, or inhibitors of LCP activity, can be administered as therapeutics for treatment of LCP defects.

Thus, in another aspect, the invention provides pharmaceutical compositions comprising the nucleic acids, nucleic acid fragments, proteins, protein fusions, protein fragments, antibodies, antibody derivatives, antibody fragments, mimetics, agonists, antagonists, and inhibitors of the present invention.

Such a composition typically contains from about 0.1 to 90% by weight of a therapeutic agent of the invention formulated in and/or with a pharmaceutically acceptable carrier or excipient.

Pharmaceutical formulation is a well-established art, and is further described in Gennaro (ed.), Remington: The Science and Practice of Pharmacy, 20^{th} ed., Lippincott, Williams & Wilkins (2000) (ISBN: 0683306472); Ansel *et al*., Pharmaceutical Dosage Forms and Drug Delivery Systems, 7^{th} ed., Lippincott Williams & Wilkins Publishers (1999) (ISBN: 0683305727); and Kibbe (ed.), Handbook of Pharmaceutical Excipients American Pharmaceutical Association, 3^{rd} ed. (2000) (ISBN: 091733096X), the disclosures of which are incorporated herein by reference in their entireties, and thus need not be described in detail herein.

Briefly, however, formulation of the pharmaceutical compositions of the present invention will depend upon the route chosen for administration. The pharmaceutical compositions utilized in this invention can be administered by various routes including both enteral and parenteral routes, including oral, intravenous, intramuscular, subcutaneous, inhalation, topical, sublingual, rectal, intra-arterial, intramedullary, intrathecal, intraventricular, transmucosal, transdermal, intranasal, intraperitoneal, intrapulmonary, and intrauterine.

Oral dosage forms can be formulated as tablets, pills, dragees, capsules, liquids, gels, syrups, slurries, suspensions, and the like, for ingestion by the patient.

Solid formulations of the compositions for oral administration can contain suitable carriers or excipients, such as carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, sodium carboxymethylcellulose, or microcrystalline cellulose; gums including arabic and tragacanth; proteins such as gelatin and collagen; inorganics, such as kaolin, calcium carbonate, dicalcium phosphate, sodium chloride; and other agents such as acacia and alginic acid.

Agents that facilitate disintegration and/or solubilization can be added, such as the cross-linked polyvinyl pyrrolidone, agar, alginic acid, or a salt thereof, such as sodium alginate, microcrystalline cellulose, corn starch, sodium starch glycolate, and alginic acid.

Tablet binders that can be used include acacia, methylcellulose, sodium carboxymethylcellulose, polyvinylpyrrolidone (Povidone™), hydroxypropyl methylcellulose, sucrose, starch and ethylcellulose.

Lubricants that can be used include magnesium stearates, stearic acid, silicone fluid, talc, waxes, oils, and colloidal silica.

Fillers, agents that facilitate disintegration and/or solubilization, tablet binders and lubricants, including the aforementioned, can be used singly or in combination.

Solid oral dosage forms need not be uniform throughout.

For example, dragee cores can be used in conjunction with suitable coatings, such as concentrated sugar solutions, which can also contain gum arabic, talc, polyvinylpyrrolidone, carbopol gel, polyethylene glycol, and/or titanium dioxide, lacquer solutions, and suitable organic solvents or solvent mixtures.

Oral dosage forms of the present invention include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Additionally, dyestuffs or pigments can be added to the tablets or dragee coatings for product identification or to characterize the quantity of active compound, *i.e.,* dosage.

Liquid formulations of the pharmaceutical compositions for oral (enteral) administration are prepared in water or other aqueous vehicles and can contain various suspending agents such as methylcellulose, alginates, tragacanth, pectin, kelgin, carrageenan, acacia, polyvinylpyrrolidone, and polyvinyl alcohol. The liquid formulations can also include solutions, emulsions, syrups and elixirs containing, together with the active compound(s), wetting agents, sweeteners, and coloring and flavoring agents.

The pharmaceutical compositions of the present invention can also be formulated for parenteral administration.

For intravenous injection, water soluble versions of the compounds of the present invention are formulated in, or if provided as a lyophilate, mixed with, a physiologically acceptable fluid vehicle, such as 5% dextrose ("D5"), physiologically buffered saline, 0.9% saline, Hanks' solution, or Ringer's solution.

Intramuscular preparations, *e.g.* a sterile formulation of a suitable soluble salt form of the compounds of the present invention, can be dissolved and administered in a pharmaceutical excipient such as Water-for-Injection, 0.9% saline, or 5% glucose solution. Alternatively, a suitable insoluble form of the compound can be prepared and administered as a suspension in an aqueous base or a pharmaceutically acceptable oil base, such as an ester of a long chain fatty acid (*e*.*g*., ethyl oleate), fatty oils such as sesame oil, triglycerides, or liposomes.

Parenteral formulations of the compositions can contain various carriers such as vegetable oils, dimethylacetamide, dimethylformamide, ethyl lactate, ethyl carbonate, isopropyl myristate, ethanol, polyols (glycerol, propylene glycol, liquid polyethylene glycol, and the like).

Aqueous injection suspensions can also contain substances that increase the viscosity of the suspension, such as sodium carboxymethyl cellulose, sorbitol, or dextran. Non-lipid polycationic amino polymers can also be used for delivery. Optionally, the suspension can also contain suitable stabilizers or agents that increase the solubility of the compounds to allow for the preparation of highly concentrated solutions.

Pharmaceutical compositions of the present invention can also be formulated to permit injectable, long-term, deposition.

The pharmaceutical compositions of the present invention can be administered topically.

A topical semi-solid ointment formulation typically contains a concentration of the active ingredient from about 1 to 20%, *e.g.,* 5 to 10%, in a carrier such as a pharmaceutical cream base. Various formulations for topical use include drops, tinctures, lotions, creams, solutions, and ointments containing the active ingredient and various supports and vehicles. In other transdermal formulations, typically in patch-delivered formulations, the pharmaceutically active compound is formulated with one or more skin penetrants, such as 2-N-methyl-pyrrolidone (NMP) or Azone.

Inhalation formulations can also readily be formulated. For inhalation, various powder and liquid formulations can be prepared.

The pharmaceutically active compound in the pharmaceutical compositions of the present inention can be provided as the salt of a variety of acids, including but not limited to hydrochloric, sulfuric, acetic, lactic, tartaric, malic, and succinic acid. Salts tend to be more soluble in aqueous or other protonic solvents than are the corresponding free base forms.

After pharmaceutical compositions have been prepared, they are packaged in an appropriate container and labeled for treatment of an indicated condition.

The active compound will be present in an amount effective to achieve the intended purpose. The determination of an effective dose is well within the capability of those skilled in the art.

A "therapeutically effective dose" refers to that amount of active ingredient - for example LCP protein, fusion protein, or fragments thereof, antibodies specific for LCP, agonists, antagonists or inhibitors of LCP - which ameliorates the signs or symptoms of the disease or prevents progression thereof; as would be understood in the medical arts, cure, although desired, is not required.

The therapeutically effective dose of the pharmaceutical agents of the present invention can be estimated initially by *in vitro* tests, such as cell culture assays, followed by assay in model animals, usually mice, rats, rabbits, dogs, or pigs. The animal model can also be used to determine an initial useful concentration range and route of administration.

For example, the ED50 (the dose therapeutically effective in 50% of the population) and LD50 (the dose lethal to 50% of the population) can be determined in one or more cell culture of animal model systems. The dose ratio of toxic to therapeutic effects is the therapeutic index, which can be expressed as LD50/ED50. Pharmaceutical compositions that exhibit large therapeutic indices are particularly useful.

The data obtained from cell culture assays and animal studies is used in formulating an initial dosage range for human use, and preferably provides a range of circulating concentrations that includes the ED50 with little or no toxicity. After administration, or between successive administrations, the circulating concentration of active agent varies within this range depending upon pharmacokinetic factors well known in the art, such as the dosage form employed, sensitivity of the patient, and the route of administration.

The exact dosage will be determined by the practitioner, in light of factors specific to the subject requiring treatment. Factors that can be taken into account by the practitioner include the severity of the disease state, general health of the subject, age, weight, gender of the subject, diet, time and frequency of administration, drug combination(s), reaction sensitivities, and tolerance/response to therapy. Long-acting pharmaceutical compositions can be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Where the therapeutic agent is a protein or antibody of the present invention, the therapeutic protein or antibody agent typically is administered at a daily dosage of 0.01 mg to 30 mg/kg of body weight of the patient (e.g., 1mg/kg to 5 mg/kg). The pharmaceutical formulation can be administered in multiple doses per day, if desired, to achieve the total desired daily dose.

Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

Conventional methods, known to those of ordinary skill in the art of medicine, can be used to administer the pharmaceutical formulation(s) of the present invention to the patient. The pharmaceutical compositions of the present invention can be administered alone, or in combination with other therapeutic agents or interventions.

### THERAPEUTIC METHODS

The present invention further provides methods of treating subjects having defects in LCP - *e*.*g*., in expression, activity, distribution, localization, and/or solubility of LCP - which can manifest as a disorder of adrenal, adult liver, bone marrow, brain, fetal liver, heart, kidney, lung, placenta, skeletal muscle, colon or prostate function. As used herein, "treating" includes all medically-acceptable types of therapeutic intervention, including palliation and prophylaxis (prevention) of disease.

In one embodiment of the therapeutic methods of the present invention, a therapeutically effective amount of a pharmaceutical composition comprising LCP protein, fusion, fragment or derivative thereof is administered to a subject with a clinically-significant LCP defect.

Protein compositions are administered, for example, to complement a deficiency in native LCP. In other embodiments, protein compositions are administered as a vaccine to elicit a humoral and/or cellular immune response to LCP. The immune response can be used to modulate activity of LCP or, depending on the immunogen, to immunize against aberrant or aberrantly expressed forms, such as mutant or inappropriately expressed isoforms. In yet other embodiments, protein fusions having a toxic moiety are administered to ablate cells that aberrantly accumulate LCP.

In another embodiment of the therapeutic methods of the present invention, a therapeutically effective amount of a pharmaceutical composition comprising nucleic acid of the present invention is administered. The nucleic acid can be delivered in a vector that drives expression of LCP protein, fusion, or fragment thereof, or without such vector.

Nucleic acid compositions that can drive expression of LCP are administered, for example, to complement a deficiency in native LCP, or as DNA vaccines. Expression vectors derived from virus, replication deficient retroviruses, adenovirus, adeno-associated (AAV) virus, herpes virus, or vaccinia virus can be used - *see, e.g.,* Cid-Arregui (ed.), Viral Vectors: Basic Science and Gene Therapy, Eaton Publishing Co., 2000 (ISBN: 188129935X) - as can plasmids .

Antisense nucleic acid compositions, or vectors that drive expression of LCP antisense nucleic acids, are administered to downregulate transcription and/or translation of LCP in circumstances in which excessive production, or production of aberrant protein, is the pathophysiologic basis of disease.

Antisense compositions useful in therapy can have sequence that is complementary to coding or to noncoding regions of the LCP gene. For example, oligonucleotides derived from the transcription initiation site, *e.g.,* between positions -10 and +10 from the start site, are particularly useful.

Catalytic antisense compositions, such as ribozymes, that are capable of sequence-specific hybridization to LCP transcripts, are also useful in therapy. *See, e.g.,* Phylactou, *Adv*. *Drug Deliv*. *Rev.* 44(2-3):97-108 (2000); Phylactou *et al*., *Hum*. *Mol*. *Genet*. 7(10):1649-53 (1998); Rossi, *Ciba Found*. *Symp.* 209:195-204 (1997); and Sigurdsson *et al*., *Trends Biotechnol*. 13(8):286-9 (1995), the disclosures of which are incorporated herein by reference in their entireties.

Other nucleic acids useful in the therapeutic methods of the present invention are those that are capable of triplex helix formation in or near the LCP genomic locus. Such triplexing oligonucleotides are able to inhibit transcription, Intody *et al.,* Nucleic Acids Res. 28(21):4283-90 (2000); McGuffie *et al., Cancer Res.* 60(14):3790-9 (2000), the disclosures of which are incorporated herein by reference, and pharmaceutical compositions comprising such triplex forming oligos (TFOs) are administered in circumstances in which excessive production, or production of aberrant protein, is a pathophysiologic basis of disease.

In another embodiment of the therapeutic methods of the present invention, a therapeutically effective amount of a pharmaceutical composition comprising an antibody (including fragment or derivative thereof) of the present invention is administered. As is well known, antibody compositions are administered, for example, to antagonize activity of LCP, or to target therapeutic agents to sites of LCP presence and/or accumulation.

In another embodiment of the therapeutic methods of the present invention, a pharmaceutical composition comprising a non-antibody antagonist of LCP is administered. Antagonists of LCP can be produced using methods generally known in the art. In particular, purified LCP can be used to screen libraries of pharmaceutical agents, often combinatorial libraries of small molecules, to identify those that specifically bind and antagonize at least one activity of LCP.

In other embodiments a pharmaceutical composition comprising an agonist of LCP is administered. Agonists can be identified using methods analogous to those used to identify antagonists.

In still other therapeutic methods of the present invention, pharmaceutical compositions comprising host cells that express LCP, fusions, or fragments thereof can be administered. In such cases, the cells are typically autologous, so as to circumvent xenogeneic or allotypic rejection, and are administered to complement defects in LCP production or activity.

In other embodiments, pharmaceutical compositions comprising the LCP proteins, nucleic acids, antibodies, antagonists, and agonists of the present invention can be administered in combination with other appropriate therapeutic agents. Selection of the appropriate agents for use in combination therapy can be made by one of ordinary skill in the art according to conventional pharmaceutical principles. The combination of therapeutic agents or approaches can act additively or synergistically to effect the treatment or prevention of the various disorders described above, providing greater therapeutic efficacy and/or permitting use of the pharmaceutical compositions of the present invention using lower dosages, reducing the potential for adverse side effects.

### TRANSGENIC ANIMALS AND CELLS

In another aspect, the invention provides transgenic cells and non-human organisms comprising LCP isoform nucleic acids, and transgenic cells and non-human organisms with targeted disruption of the endogenous orthologue of the human LCP gene.

The cells can be embryonic stem cells or somatic cells. The transgenic non-human organisms can be chimeric, nonchimeric heterozygotes, and nonchimeric homozygotes.

### DIAGNOSTIC METHODS

The nucleic acids of the present invention can be used as nucleic acid probes to assess the levels of LCP mRNA in adrenal, adult liver, bone marrow, brain, fetal liver, heart, kidney, lung, placenta, skeletal muscle, colon and prostate, and antibodies of the present invention can be used to assess the expression levels of LCP proteins in adrenal, adult liver, bone marrow, brain, fetal liver, heart, kidney, lung, placenta, skeletal muscle, colon and prostate to diagnose neurological and developmental disorders, as well as diseases involving cell-cell adhesion process.

The following examples are offered for purpose of illustration, not limitation.

### EXAMPLE 1

### Identification and Characterization of cDNAs Encoding LCP Proteins

Predicating our gene discovery efforts on use of genome-derived single exon probes and hybridization to genome-derived single exon microarrays - an approach that we have previously demonstrated will readily identify novel genes that have proven refractory to mRNA-based identification efforts - we identified an exon in raw human genomic sequence that is particularly expressed in human adrenal, adult liver, bone marrow, brain, fetal liver, heart, kidney, lung, placenta and prostate, as well as a cell line, hela.

Briefly, bioinformatic algorithms were applied to human genomic sequence data to identify putative exons. Each of the predicted exons was amplified from genomic DNA, typically centering the putative coding sequence within a larger amplicon that included flanking noncoding sequence. These genome-derived single exon probes were arrayed on a support and expression of the bioinformatically predicted exons assessed through a series of simultaneous two-color hybridizations to the genome-derived single exon microarrays.

The approach and procedures are further described in detail in Penn *et al*., "Mining the Human Genome using Microarrays of Open Reading Frames," *Nature Genetics* 26:315-318 (2000); commonly owned and copending U.S. patent application nos. 09/864,761, filed May 23, 2001, 09/774,203, filed January 29, 2001, and 09/632,366, filed August 3, 2000, the disclosures of which are incorporated herein by reference in their entireties.

Using a graphical display particularly designed to facilitate computerized query of the resulting exon-specific expression data, as further described in commonly owned and copending U.S. patent application no. 09/864,761, filed May 23, 2001, 09/774,203, filed January 29, 2001 and 09/632,366, filed August 3, 2000, the disclosures of which are incorporated herein by reference in their entireties, two exons were identified that are expressed in all the human tissues tested; subsequent analysis revealed that the two exons belong to the same gene.

Table 1 summarizes the microarray expression data obtained using genome-derived single exon probes corresponding to exons two and sixteen of LCP1. Each probe was completely sequenced on both strands prior to its use on a genome-derived single exon microarray; sequencing confirmed the exact chemical structure of each probe. An added benefit of sequencing is that it placed us in possession of a set of single base-incremented fragments of the sequenced nucleic acid, starting from the sequencing primer's 3' OH. (Since the single exon probes were first obtained by PCR amplification from genomic DNA, we were of course additionally in possession of an even larger set of single base incremented fragments of each of the single exon probes, each fragment corresponding to an extension product from one of the two amplification primers.)

Signals and expression ratios are normalized values measured and calculated as further described in commonly owned and copending U.S. patent application nos. 9/864,761, filed May 23, 2001, 09/774,203, filed January 29, 2001, 09/632,366, filed August 3, 2000, and U.S. provisional patent application no. 60/207,456, filed May 26, 2000, the disclosures of which are incorporated herein by reference in their entireties.

**Table 1**

| Expression Analysis Genome-Derived Single Exon Microarray | | | | |
|---|---|---|---|---|
| | Amplicon 24980 (exon 2 of LCP1) | | Amplicon 24976 (exon 16 of LCP1) | |
| | Signal | Expression ratio | Signal | Expression ratio |
| ADRENAL | 1.23 | 1.31 | | |
| ADULT LIVER | 1.16 | -1.07 | | |
| BONE MARROW | 0.93 | -1.34 | 0.46 | -1.72 |
| BRAIN | 0.96 | -1.17 | 0.47 | -1.03 |
| FETAL LIVER | 1.05 | -1.09 | 0.60 | -1.24 |
| HEART | 0.99 | 1.04 | | |
| HELA | 1.43 | 1.07 | | |
| KIDNEY | 1.18 | 1.06 | 0.53 | -1.08 |
| LUNG | 1.13 | -1.11 | | |
| PLACENTA | 1.06 | -1.02 | 0.61 | 1.53 |
| PROSTATE | 1.05 | -1.01 | | |

As shown in Table 1, significant expression of exons two and sixteen of LCP1 were seen only in adrenal, adult liver, bone marrow, brain, fetal liver, heart, kidney, lung, placenta and prostate, as well as a cell line hela. Adrenal, adult liver, bone marrow, brain, fetal liver, heart, kidney, lung, placenta and prostate as well as a cell line hela-specific expression was further confirmed by RT_PCR analysis (see below).

Marathon-Ready™ liver cDNA (Clontech Laboratories, Palo Alto, CA) was used as a substrate for standard RACE (rapid amplification of cDNA ends) to obtain the full length cDNA clones. Oligonucleotides OL683(5'-ATGGTCCTCACTACTCTCATTCTCATATTAGTGTG-3') and OL684(5'-TCAAAGGATTTCTTTAAAAACATCACATTCCCC-3') were used to PCR out a 0.7 kb fragment of the open reading frame (ORF) using manufacture's protocols (Clontech). Oligonucleotides OL714(5'-CTGTACTAGGCCCTGAGAGTGGAACCCTTACATCC-3') and OL687(5'-GGGGTTTCCTCATGGTCCACTGCTTTTGCAG-3') were used to PCR out another 1.6 kb fragment of the ORF. Based on the sequences of these two fragments, a series of RACE reaction were performed to find out the 5' and 3' end of the ORF. Finally, OL759(5'-CCACCATGCCTCTGTTCCTCCTGCTCTTACTTGTCCTGC-3') and OL760(5'-TCAAAGGATTTCTTTAAAAACATCACATTCCCCATC-3') were used to PCR out the full length ORF. The RACE products were sequenced using MegaBACE™ instruments (Molecular Dynamics, Sunnyvale, CA).

To subclone LCP into cloning vector, the RACE product generated with oligonucleotides OL759 and OL760 was ligated and T/A cloned into pGem-Teasy vector (Promega Corp.). Individual clones were picked and inserts sequenced using MegaBACE™ instruments. Two splice variants were identified following the sequence analysis of this gene. One of the cDNA clones spans 2.3 kilobases, the other one is ∼200 bp shorter. Both appear to contain long open reading frames. For reasons described below, we termed the cDNAs LCP1 and LCP2. Cloning and sequencing provided us with the exact chemical structure of the cDNA, which is shown in FIGs. 3 and 4 and further presented in the SEQUENCE LISTING as SEQ ID NO: 1 and 1113, and placed us in actual physical possession of the entire set of single-base incremented fragments of the sequenced clone, starting at the 5' and 3' termini.

As shown in FIG. 3, the LCP1 cDNA spans 2280 nucleotides and contains an open reading frame from nucleotide 76 through and including nt 2265 (inclusive of termination codon), predicting a protein of 729 amino acids with a (posttranslationally unmodified) molecular weight of 80.3 kD. The clone appears full length, with the reading frame opening starting with a methionine and terminating with a stop codon.

As shown in FIG. 4, the LCP2 cDNA contains an open reading frame spanning 1962 nucleotides and encodes a protein of 653 amino acids. LCP2 has a predicted molecular weight, prior to any post-translational modification, of 80.3 kD.

BLAST query of genomic sequence identified two BACS, spanning over 70 kb, that constitute the minimum set of clones encompassing the cDNA sequence. Based upon the known origin of the BACs (GenBank accession numbers AC091213.8 and AC016962.27), the LCP gene can be mapped to human chromosome 3q12.1.

Comparison of the LCP1 cDNA and genomic sequences identified 16 exons. Exon organization is listed in Table 2.

**Table 2**

| LCP1 Exon Structure | | | |
|---|---|---|---|
| Exon no. | cDNA range | genomic range | BAC accession |
| 1 | 1-142 | 95410-95269 | AC091213.8 |
| 2 | 143-370 | 134826-135053 | |
| 3 | 371-508 | 9275-9138 | |
| 4 | 509-560 | 10658-10709 | AC016962.27 |
| 5 | 561-633 | 13291-13363 | |
| 6 | 634-767 | 13671-13804 | |
| 7 | 768-808 | 15742-15782 | |
| 8 | 809-1024 | 16613-16828 | |
| 9 | 1025-1149 | 18137-18261 | |
| 10 | 1150-1300 | 23549-23699 | |
| 11 | 1301-1387 | 24477-24563 | |
| 12 | 1388-1513 | 24712-24837 | |
| 13 | 1514-1607 | 27857-27950 | |
| 14 | 1608-1657 | 34382-34431 | |
| 15 | 1658-1795 | 35315-35452 | |
| 16 | 1796-2280 | 36190-36674 | |

FIG. 2 schematizes the exon organization of the **LCP** clones.

At the top is shown the two bacterial artificial chromosomes (BACs), with GenBank accession numbers, that span the LCP locus. The genome-derived single-exon probes first used to demonstrate expression from this locus are shown below the BACs and labeled "500".

As shown in FIG. 2, LCP1 encodes a protein of 729 amino acids and is comprised of exons 1 - 16. LCP1 has a predicted molecular weight, prior to any post-translational modification of 80.3 kD. LCP2 encodes a protein of 653 amino acids and is lacking exon 2 of LCP1. LCP2 has a predicted molecular weight, prior to any post-translational modification, of 80.3 kD.

As further discussed in the examples herein, expression of LCP was assessed using hybridization to genome-derived single exon microarrays and RT-PCR. Microarray analysis of the exons two and sixteen showed expression in adrenal, adult liver, bone marrow, brain, fetal liver, heart, kidney, lung, placenta and prostate, as well as a cell line, hela. RT-PCR confirmed microarray data, and further provided expression data for skeletal muscle and colon.

The sequence of the LCP cDNAs was used as a BLAST query into the GenBank nr and dbEst databases. The nr database includes all non-redundant GenBank coding sequence translations, sequences derived from the 3-dimensional structures in the Brookhaven Protein Data Bank (PDB), sequences from SwissProt, sequences from the protein information resource (PIR), and sequences from protein research foundation (PRF). The dbEst (database of expressed sequence tags) includes ESTs, short, single pass read cDNA (mRNA) sequences, and cDNA sequences from differential display experiments and RACE experiments.

BLAST search identified multiple human and mouse ESTs, four from cow, two ESTs from pig (AW315748.1 and AW437031.1), two from chicken (AJ396784.1 and AL586285.1) and two from rat (BE098908.1 and BF543094.1) as having sequence closely related to LCP.

Globally, the N_terminal half of the human LCP1 protein resembles Neurophilin-1 precursor (NRP1), with 27 % amino acid identity and 41 % amino acid similarity over 443 amino acids.

Motif searches using Pfam (http://pfam.wustl.edu), SMART (http://smart.embl- heidelberg.de), and PROSITE pattern and profile databases (http://www.expasy.ch/prosite), identified several known domains.

FIG. 1 shows the domain structure of LCP1 and alignment of the identified domains with that of other proteins.

The newly isolated membrane protein LCP contains three distinct protein domains, including a CUB, an LCCL and a DSD/FA58C domain, respectively. The following four paragraphs describe the protein structure of LCP using LCP1 as an example. However, such description is also true for LCP2 except that, in comparison to LCP1, the LCP2 protein product lacks amino acid sequence 23 - 98 of LCP1, and therefore has a partial CUB domain the N-terminal of which is truncated. The structural features of LCP1 are schematized in FIG. 1.

LCP1 contains a CUB domain at residues 26 - 138 (http://smart.embl-heidelberg.de/) or alternatively at residues 26 - 141 (http://pfam.wustl.edu/). CUB is a protein domain with a predicted beta-barrel structure similar to that of immunoglobulins. It is an extracellular domain found in functionally diverse, mostly developmentally regulated proteins.

LCP1 has an LCCL domain at residues 147 - 230 (http://smart.embl-heidelberg.de/). First identified in Limulus factor C, Coch-5b2 and Lg11, the LCCL domain is hypothesized to have an antimicrobial function. Mutations in the LCCL domain have been shown to cause the deafness disorder DFNA9 in humans.

LCP1 also has a discoidin domain, also known as a F5/8 type C domain or an FA58C domain. In LCP1, the discoidin/FA58C domain occurs at residues 250 - 394 (discoidin domain, http://smart.embl-heidelberg.de/) or alternatively at residues 250 - 400 (F5/8 type C domain, http://pfam.wustl.edu/) or at residues 248-403 (FA58C, http://smart.embl-heidelberg.de/). The discoidin domain is a protein domain with a predicted amphipathic, membrane binding alpha helical structure at the C-terminal. This domain is found in a number of coagulation factors and has been shown to be responsible for phosphatidylserine-binding and essential for phosphatidylserine activity. The discoidin domain is also present in a subset of the tyrosine kinase receptor family known as discoidin domain receptors that are putatively involved in tumor progression.

The LCP1 protein contains a signal peptide consisting of the first 20 amino acid sequence of the protein. It also contains a transmembrane domain between amino acids 487 and 506 (http://www.ch.embnet.org/software/TMPRED_form.html). Other signatures of the newly isolated LCP1 proteins were identified by searching the PROSITE database (http://www.expasy.ch/tools/scnpsitl.html). These include six *N*-glycosylation sites (49-52, 109-112, 226-229, 428-431, 470-473, and 476-479), two cAMP- and cGMP-dependent protein kinase phosphorylation sites (313-316 and 512-515), six protein kinase C phosphorylation sites (130-132, 240-242, 279-281, 560-562, 592-594, and 654-656), thirteen casein kinase II phosphorylation sites, a single tyrosine kinase phosphorylation site (512-519), and twenty *N*-myristoylation sites.

Possession of the genomic sequence permitted search for promoter and other control sequences for the LCP gene.
A putative transcriptional control region, inclusive of promoter and downstream elements, was defined as 1 kb around the transcription start site, itself defined as the first nucleotide of the LCP1 cDNA clone. The region, drawn from sequence of BAC AC091213.8 , has the sequence given in SEQ ID NO: 42, which lists 1000 nucleotides before the transcription start site.

Transcription factor binding sites were identified using a web based program (http://motif.genome.ad.jp/), including binding sites for MZF1 (739 - 746 and 713 - 706 bp) and for GATA-1 (934 - 943 bp, with numbering according to SEQ ID NO: 29), amongst others.

We have thus identified a human transmembrane protein, LCP, which contains a CUB domain, a LCCL domain, and a FA58C/DS domain. The structural features strongly imply that the LCP protein plays potential therapeutic as well as diagnostic roles for neurological and developmental disorders, as well as diseases involving the cell-cell adhesion process.

### EXAMPLE 2

### Preparation and Labeling of Useful Fragments of LCP

Useful fragments of LCP are produced by PCR, using standard techniques, or solid phase chemical synthesis using an automated nucleic acid synthesizer. Each fragment is sequenced, confirming the exact chemical structure thereof.

The exact chemical structure of preferred fragments is provided in the attached SEQUENCE LISTING, the disclosure of which is incorporated herein by reference in its entirety. The following summary identifies the fragments whose structures are more fully described in the SEQUENCE LISTING:
SEQ ID NO: 1 nt, full length LCP1 cDNA
SEQ ID NO: 2 nt, cDNA ORF of LCP1
SEQ ID NO: 3 aa, full length LCP1 protein
SEQ ID NO: 4 nt, (nt 1602 - 1901) portion of LCP1
SEQ ID NO: 5 aa, (aa 510 - 608) CDS entirely within SEQ ID NO: 4
SEQ ID NO: 6 nt, (nt 2006 - 2280) portion of LCP1
SEQ ID NO: 7 nt, coding portion of SEQ ID NO: 6
SEQ ID NO: 8 nt, 3' UTR portion of SEQ ID NO: 6
SEQ ID NO: 9 aa, (aa 645 - 729) CDS entirely within SEQ ID NO: 7
SEQ ID NO: 10 - 25 nt, exons 1 - 16 of LCP1 (from genomic sequence)
SEQ ID NO: 26 - 41 nt, 500 bp genomic amplicons centered about exons 1 - 16 of LCP1
SEQ ID NO: 42 nt, 1000 bp putative promoter of LCP
SEQ ID NOs: 43 - 326 nt, 17-mers scanning (nt 1602 - 1901) portion of LCP1
SEQ ID NOs: 327 - 602 nt, 25-mers scanning (nt 1602 - 1901) portion of LCP1
SEQ ID NOs: 603 - 861 nt, 17-mers scanning (nt 2006 - 2280) portion of LCP1
SEQ ID NOs: 862 - 1112 nt, 25-mers scanning (nt 2006 - 2280) portion of LCP1
SEQ ID NO: 1113 nt, cDNA ORF of LCP2
SEQ ID NO: 1114 aa, full length LCP2 protein
SEQ ID NO: 1115 nt, splice junction of exons 1 and 3 of LCP1 (novel junction of LCP2)
SEQ ID NO: 1116 aa, CDS within SEQ ID NO: 1115
SEQ ID NO: 1117 nt, RACE primer OL683
SEQ ID NO: 1118 nt, RACE primer OL684
SEQ ID NO: 1119 nt, RACE primer OL714
SEQ ID NO: 1120 nt, RACE primer OL687
SEQ ID NO: 1121 nt, RACE and RT_PCR primer OL759
SEQ ID NO: 1122 nt, RACE primer OL760
SEQ ID NO: 1123 nt, RT_PCR primer OL688

Upon confirmation of the exact structure, each of the above-described nucleic acids of confirmed structure is recognized to be immediately useful as an LCP-specific probe.

For use as labeled nucleic acid probes, the above-described LCP nucleic acids are separately labeled by random priming. As is well known in the art of molecular biology, random priming places the investigator in possession of a near-complete set of labeled fragments of the template of varying length and varying starting nucleotide.

The labeled probes are used to identify the LCP gene on a Southern blot, and are used to measure expression of LCP mRNA on a northern blot and by RT-PCR, using standard techniques.

### EXAMPLE 3

### LCP expression analysis by RT_PCR

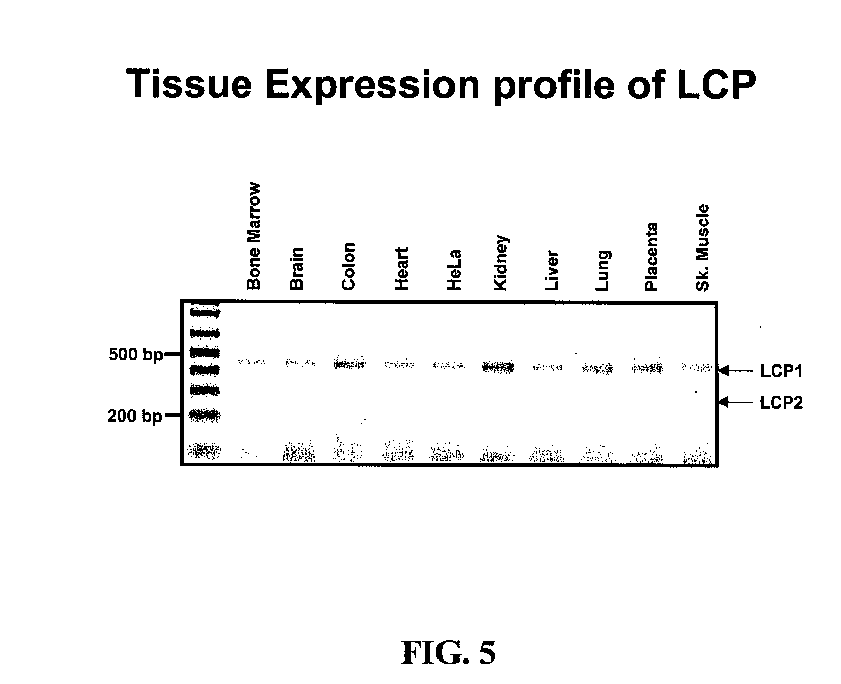

To explore the potential function of LCP, the expression of LCP in human tissues was examined by PCR using marathon-ready cDNAs. Oligonucleotides OL759 and OL688(5'-CTGCCCGGTCCCAGTAAGGTAAGTCATAGGTGC-3') were used to amplify a 5' fragment from LCP from human cDNAs of bone marrow, brain, colon, heart, kidney, liver, lung, placenta, skeletal muscle and Hela cells. The PCR conditions were according to a touchdown PCR procedure. The tubes containing the oligonucleotides, cDNA and Taq polymerase were first incubated at 94°C for 15 seconds followed by 70°C for 2 minutes, cycle 5 times. The tubes were then incubated at 94°C for 15 seconds followed by 68°C for 2 minutes, cycle 5 times. Finally the tubes were incubated at 94°C for 15 seconds followed by 66°C for 2 minutes, cycle 25 times. To distinguish the two splice variants, the PCR fragments were cut by restriction enzyme BglII and the rsulting mixture was run on an agarose gel. The sizes of the fragment expected from LCP1 is 435 bp and from LCP2 is 207 bp. The result of the expression profile is shown in FIG. 5. The abundance of PCR product indicates that LCP1 is expressed in all tissues examined with the highest expression in kidney. LCP2 is not detectable in all tissues except a very slight expression in kidney. Therefore, LCP1 is the dominant form of LCP expression and may play the major role for LCP function.

### EXAMPLE 4

### Production of LCP Protein

The full length LCP1 or LCP2 cDNA clone is cloned into the mammalian expression vector pcDNA3.1/HISA (Invitrogen, Carlsbad, CA, USA), transfected into COS7 cells, transfectants selected with G418, and protein expression in transfectants confirmed by detection of the anti-Xpress™ epitope according to manufacturer's instructions. Protein is purified using immobilized metal affinity chromatography and vector-encoded protein sequence is then removed with enterokinase, per manufacturer's instructions, followed by gel filtration and/or HPLC.

Following epitope tag removal, LCP protein is present at a concentration of at least 70%, measured on a weight basis with respect to total protein (i.e., w/w), and is free of acrylamide monomers, bis acrylamide monomers, polyacrylamide and ampholytes. Further HPLC purification provides LCP protein at a concentration of at least 95%, measured on a weight basis with respect to total protein (i.e., w/w).

### EXAMPLE 5

### Production of Anti- LCP Antibody

Purified proteins prepared as in Example 4 are conjugated to carrier proteins and used to prepare murine monoclonal antibodies by standard techniques. Initial screening with the unconjugated purified proteins, followed by competitive inhibition screening using peptide fragments of the LCP, identifies monoclonal antibodies with specificity for LCP.

### EXAMPLE 6

### Use of LCP Probes and Antibodies for Diagnosis

After informed consent is obtained, samples are drawn from disease tissue or cells and tested for LCP mRNA levels by standard techniques and tested additionally for LCP protein levels using anti- LCP antibodies in a standard ELISA.

### EXAMPLE 7

### Use of LCP Nucleic Acids, Proteins, and Antibodies in Therapy

Once over-expression of LCP is detected in patients, LCP specific antisense RNA or LCP-specific antibody is introduced into disease cells to reduce the amount of the protein.

Once mutations of LCP have been detected in patients, normal LCP is reintroduced into the patient's disease cells by introduction of expression vectors that drive LCP expression or by introducing LCP proteins into cells. Antibodies for the mutated forms of LCP are used to block the function of the abnormal forms of the protein.

### EXAMPLE 8

### Human LCP Disease Associations

Diseases that map to the human LCP chromosomal region are shown in Table 3:

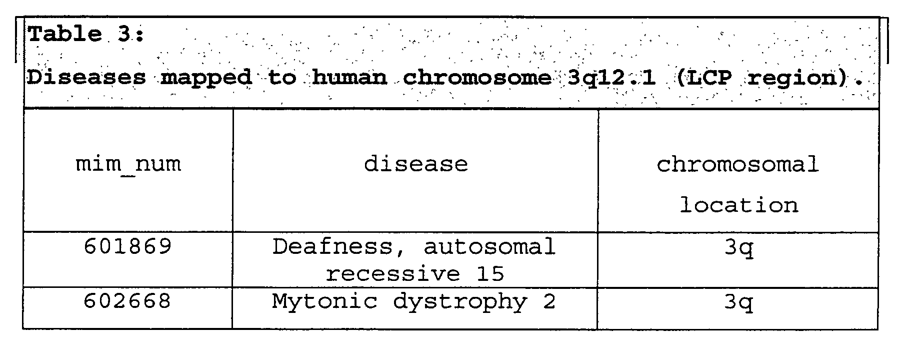

At least one of these diseases has physiological characteristics consistent with alteration of an LCP-like gene. For example, a number of recent studies have shown that mutations in the LCCL domain of a gene named COCH cause a autosomal recessive deafness disorder, DFNA9 (Robertson N.G. *et al*, *Nature Genetics* 20:299-302 (1998)). LCP is therefore a candidate gene for the deafness syndrome, autosomal recessive 15.

All patents, patent publications, and other published references mentioned herein are hereby incorporated by reference in their entireties as if each had been individually and specifically incorporated by reference herein. While preferred illustrative embodiments of the present invention are described, one skilled in the art will appreciate that the present invention can be practiced by other than the described embodiments, which are presented for purposes of illustration only and not by way of limitation. The present invention is limited only by the claims that follow.

## Claims

1. An isolated nucleic acid that encodes a protein involved in neurological and developmental disorders, as well as diseases involving cell-cell adhesion process, comprising:
(a) a nucleotide sequence selected from the group consisting of:
(i) SEQ ID NO: 1;
(ii) the complement of the sequences set forth in (i);
(iii) the nucleotide sequence of SEQ ID NO: 2, 1113;
(iv) a degenerate variant of the sequences set forth in (iii); and
(v) the complement of the sequences set forth in (iii) and (iv); or
(b) a nucleotide sequence selected from the group consisting of:
(i) a nucleotide sequence that encodes a polypeptide having the sequence of SEQ ID NO: 3, 1114;
(ii) a nucleotide sequence that encodes a polypeptide having the sequence of SEQ ID NO: 3, 1114, with conservative amino acid substitutions; and
(iii) the complement of the sequences set forth in (i) and (ii),
wherein said isolated nucleic acid comprising a nucleotide sequence selected from group (b) is no more than about 100 kb in length.

2. The isolated nucleic acid of claim 1 wherein said nucleic acid, or the complement of said nucleic acid, encodes a polypeptide involved in neurological and developmental disorders, as well as diseases involving cell-cell adhesion process.

3. The isolated nucleic acid of either of claims 1 or 2, wherein said nucleic acid, or the complement of said nucleic acid, is expressed in adrenal, adult liver, bone marrow, brain, fetal liver, heart, kidney, lung, placenta, skeletal muscle, colon, prostate, and/or a cell line, hela.

4. A nucleic acid probe, comprising:
(a) the nucleic acid of claim 1; or
(b) at least 17 contiguous nucleotides of SEQ ID Nos: 4, 6 or 1115,
wherein said probe according to (b) is no longer than about 100 kb in length.

5. The probe of claim 4, wherein said probe is detectably labeled.

6. The probe of either of claims 4 or 5, attached to a substrate.

7. A microarray, wherein at least one probe of said array is a probe according to claim 4.

8. The isolated nucleic acid molecule of any of claims 1 - 3, wherein said nucleic acid molecule is operably linked to one or more expression control elements.

9. A replicable vector comprising a nucleic acid molecule of any of claims 1 - 3 or 8.

10. A host cell transformed to contain the nucleic acid molecule of any one of claims 1 - 3 or 8 or 9, or the progeny thereof.

11. A method for producing a polypeptide, the method comprising: culturing the host cell of claim 10 under conditions in which the protein encoded by said nucleic acid molecule is expressed.

12. An isolated polypeptide produced by the method of claim 11.

13. An isolated polypeptide, comprising:
(a) an amino acid sequence selected from the group consisting of SEQ ID NO: 3 and 1114;
(b) an amino acid sequence having at least 65% amino acid sequence identity to that of (a) (i) or (a) (ii);
(c) an amino acid sequence according to (a) (i) or (a) (ii) in which at least 95% of deviations from the sequence of (a) (i) or (a) (ii) are conservative substitutions; or
(d) a fragment of at least 8 contiguous amino acids of any of (a) - (c).

14. A fusion protein, said fusion protein comprising a polypeptide of claim 13 fused to a heterologous amino acid sequence.

15. An isolated antibody, or antigen-binding fragment or derivative thereof, the binding of which can be competitively inhibited by a polypeptide of claim 13.

16. A transgenic non-human animal modified to contain the nucleic acid molecule of any one of claims 1 - 3 or 8 or 9.

17. A transgenic non-human animal unable to express the endogenous orthologue of the nucleic acid molecule of any of claims 1 - 3 or 8 or 9.

18. A method of identifying agents that modulate the expression of human LCP, the method comprising:
contacting a cell or tissue sample believed to express human LCP with a chemical or biological agent, and then
comparing the amount of human LCP expression in said cell or tissue sample with that of a control,
changes in the amount relative to control identifying an agent that modulates expression of human LCP.

19. A method of identifying agonists and antagonists of human LCP, the method comprising:
contacting a cell or tissue sample believed to express human LCP with a chemical or biological agent, and then
comparing the activity of human LCP with that of a control,
increased activity relative to a control identifying an agonist, decreased activity relative to a control identifying an antagonist.

20. A purified agonist of the polypeptide of claim 13.

21. A purified antagonist of the polypeptide of claim 13.

22. A method of identifying a specific binding partner for a polypeptide according to claim 13, the method comprising:
contacting said polypeptide to a potential binding partner; and
determining if the potential binding partner binds to said polypeptide.

23. The method of claim 22, wherein said contacting is performed *in vivo.*

24. A purified binding partner of the polypeptide of claim 13.

25. A method for detecting a target nucleic acid in a sample, said target being a nucleic acid according to any one of claims 1 - 3 or 8 or 9, the method comprising:
a) hybridizing the sample with a probe comprising at least 17 contiguous nucleotides of a sequence complementary to said target nucleic acid in said sample under high stringency hybridization conditions, and
b) detecting the presence or absence, and optionally the amount, of said binding.

26. A method of diagnosing a disease caused by mutation in human LCP, comprising:
detecting said mutation in a sample of nucleic acids that derives from a subject suspected to have said disease.

27. A method of diagnosing or monitoring a disease caused by altered expression of human LCP, comprising:
determining the level of expression of human LCP in a sample of nucleic acids or proteins that derives from a subject suspected to have said disease,
alterations from a normal level of expression providing diagnostic and/or monitoring information.

28. A diagnostic composition comprising the nucleic acid of any of claims 1 - 3, said nucleic acid being detectably labeled.

29. The diagnostic composition of claim 28, wherein said composition is further suitable for *in vivo* administration.

30. A diagnostic composition comprising the polypeptide of claim 13, said polypeptide being detectably labeled.

31. The diagnostic composition of claim 30, wherein said composition is further suitable for *in vivo* administration.

32. A diagnostic composition comprising the antibody, or antigen-binding fragment or derivative thereof, of claim 15.

33. The diagnostic composition of claim 32, wherein said antibody or antigen-binding fragment or derivative thereof is detectably labeled.

34. The diagnostic composition of claim 33, wherein said composition is further suitable for *in vivo* administration.

35. A pharmaceutical composition comprising the nucleic acid of any one of claims 1 - 3 or 8 or 9 and a pharmaceutically acceptable excipient.

36. A pharmaceutical composition comprising the polypeptide of claim 13 and a pharmaceutically acceptable excipient.

37. A pharmaceutical composition comprising the antibody or antigen-binding fragment or derivative thereof of claim 15 and a pharmaceutically acceptable excipient.

38. A pharmaceutical composition comprising the agonist of claim 20 and a pharmaceutically acceptable excipient.

39. A pharmaceutical composition comprising the antagonist of claim 21 and a pharmaceutically acceptable excipient.

40. Nucleic acid of any of claims 1 - 3 or 8 or 9 for use in therapy.

41. Use of the nucleic acid of any of claims 1 - 3 or 8 or 9 for the manufacture of a medicament for the treatment or prevention of a disorder associated with decreased expression or activity of human LCP.

42. Polypeptide of claim 13 for use in therapy.

43. Use of the polypeptide of claim 13 for the manufacture of a medicament for the treatment or prevention of a disorder associated with decreased expression or activity of human LCP.

44. Use of the agonist of claim 20 for the manufacture of a medicament for the treatment or prevention of a disorder associated with decreased expression or activity of human LCP.

45. Antibody or antigen-binding fragment or derivative thereof of claim 15 for use in therapy.

46. Use of the antibody or antigen-binding fragment or derivative thereof of claim 15 for the manufacture of a medicament for the treatment or prevention of a disorder associated with increased expression or activity of human LCP.

47. Use of the antagonist of claim 21 for the manufacture of a medicament for the treatment or prevention of a disorder associated with increased expression or activity of human LCP.

48. A method of modulating the expression of a nucleic acid according to any one of claims 1 - 3 or 8 or 9, the method comprising:
administering an effective amount of an agent which modulates the expression of a nucleic acid according to any one of claims 1 - 3 or 8 or 9.

49. A method of modulating at least one activity of a polypeptide according to claim 13, the method comprising:
administering an effective amount of an agent which modulates at least one activity of a polypeptide according to claim 13.


PB0169 sequence listing

<110> Gu, Yizhong
      Nguyen, Cung-Tuong

<120> HUMAN LCCL_DOMAN CONTAINING PROTEIN

<130> AEOMICA-22

<150> PCT/US01/00667
<151> 2001-01-30

<150> PCT/US01/00664
<151> 2001-01-30

<150> PCT/US01/00669
<151> 2001-01-30

<150> PCT/US01/00665
<151> 2001-01-30

<150> PCT/US01/00668
<151> 2001-01-30

<150> PCT/US01/00663
<151> 2001-01-30

<150> US 09/864,761
<151> 2001-05-23

<160> 1123

<170> Aeomica Sequence Listing Engine

<210> 1
<211> 2280
<212> DNA
<213> Homo sapiens

<400> 1
gccgccgccc ccgcctgggc cgcgctcccc ctctcccgct ccctccctcc ctgctccaac 60
tcctcctcct tctccatgcc tctgttcctc ctgctcttac ttgtcctgct cctgctgctc 120
gaggacgctg gagcccagca aggtgatgga tgtggacaca ctgtactagg ccctgagagt 180
ggaaccctta catccataaa ctacccacag acctatccca acagcactgt ttgtgaatgg 240
gagatccgtg taaagatggg agagagagtt cgcatcaaat ttggtgactt tgacattgaa 300
gattctgatt cttgtcactt taattacttg agaatttata atggaattgg agtcagcaga 360
actgaaatag gcaaatactg tggtctgggg ttgcaaatga accattcaat tgaatcaaaa 420
ggcaatgaaa tcacattgct gttcatgagt ggaatccatg tttctggacg cggatttttg 480
gcctcatact ctgttataga taaacaagat ctaattactt gtttggacac tgcatccaat 540
tttttggaac ctgagttcag taagtactgc ccagctggtt gtctgcttcc ttttgctgag 600
atatctggaa caattcctca tggatataga gattcctcgc cattgtgcat ggctggtgtg 660
catgcaggag tagtgtcaaa cacgttgggc ggccaaatca gtgttgtaat tagtaaaggt 720
attccctatt atgaaagttc tttggctaac aacgtcacat ctgtggtggg acacttatct 780
acaagtcttt ttacatttaa gacaagtgga tgttatggaa cactggggat ggagtctggt 840
gtgatcgcgg atcctcaaat aacagcatca tctgtgctgg agtggactga ccacacaggg 900
caagagaaca gttggaaacc caaaaaagcc aggctgaaaa aacctggacc gccttgggct 960
gcttttgcca ctgatgaata ccagtggtta caaatagatt tgaataagga aaagaaaata 1020
acaggcatta taaccactgg atccaccatg gtggagcaca attactatgt gtctgcctac 1080
agaatcctgt acagtgatga tgggcagaaa tggactgtgt acagagagcc tggtgtggag 1140
caagataaga tatttcaagg aaacaaagat tatcaccagg atgtgcgtaa taactttttg 1200
ccaccaatta ttgcacgttt tattagagtg aatcctaccc aatggcagca gaaaattgcc 1260
atgaaaatgg agctgctcgg atgtcagttt attcctaaag gtcgtcctcc aaaacttact 1320
caacctccac ctcctcggaa cagcaatgac ctcaaaaaca ctacagcccc tccaaaaata 1380

gccaaaggtc gtgccccaaa atttacgcaa ccactacaac ctcgcagtag caatgaattt 1440
cctgcacaga cagaacaaac aactgccagt cctgatatca gaaatactac cgtaactcca 1500
aatgtaacca aagatgtagc gctggctgca gttcttgtcc ctgtgctggt catggtcctc 1560
actactctca ttctcatatt agtgtgtgct tggcactgga gaaacagaaa gaaaaaaact 1620
gaaggcacct atgacttacc ttactgggac cgggcaggtt ggtggaaagg aatgaagcag 1680
tttcttcctg caaaagcagt ggaccatgag gaaaccccag ttcgctatag cagcagcgaa 1740
gttaatcacc tgagtccaag agaagtcacc acagtgctgc aggctgactc tgcagagtat 1800
gctcagccac tggtaggagg aattgttggt acacttcatc aaagatctac ctttaaacca 1860
gaagaaggaa aagaagcagg ctatgcagac ctagatcctt acaactcacc agggcaggaa 1920
gtttatcatg cctatgctga accactccca attacggggc ctgagtatgc aaccccaatc 1980
atcatggaca tgtcagggca ccccacaact tcagttggtc agccctccac atccactttc 2040
aaggctacgg ggaaccaacc tcccccacta gtgggaactt acaatacact tctctccagg 2100
actgacagct gctcctcagc ccaggcccag tatgataccc cgaaagctgg gaagccaggt 2160
ctacctgccc cagacgaatt ggtgtaccag gtgccacaga gcacacaaga agtatcagga 2220
gcaggaaggg atggggaatg tgatgttttt aaagaaatcc tttgaagatg atgctgcttt 2280

<210> 2
<211> 2190
<212> DNA
<213> Homo sapiens

<400> 2
atgcctctgt tcctcctgct cttacttgtc ctgctcctgc tgctcgagga cgctggagcc 60
cagcaaggtg atggatgtgg acacactgta ctaggccctg agagtggaac ccttacatcc 120
ataaactacc cacagaccta tcccaacagc actgtttgtg aatgggagat ccgtgtaaag 180
atgggagaga gagttcgcat caaatttggt gactttgaca ttgaagattc tgattcttgt 240
cactttaatt acttgagaat ttataatgga attggagtca gcagaactga aataggcaaa 300
tactgtggtc tggggttgca aatgaaccat tcaattgaat caaaaggcaa tgaaatcaca 360
ttgctgttca tgagtggaat ccatgtttct ggacgcggat ttttggcctc atactctgtt 420
atagataaac aagatctaat tacttgtttg gacactgcat ccaatttttt ggaacctgag 480
ttcagtaagt actgcccagc tggttgtctg cttccttttg ctgagatatc tggaacaatt 540
cctcatggat atagagattc ctcgccattg tgcatggctg gtgtgcatgc aggagtagtg 600
tcaaacacgt tgggcggcca aatcagtgtt gtaattagta aaggtattcc ctattatgaa 660
agttctttgg ctaacaacgt cacatctgtg gtgggacact tatctacaag tctttttaca 720
tttaagacaa gtggatgtta tggaacactg gggatggagt ctggtgtgat cgcggatcct 780
caaataacag catcatctgt gctggagtgg actgaccaca cagggcaaga gaacagttgg 840
aaacccaaaa aagccaggct gaaaaaacct ggaccgcctt gggctgcttt tgccactgat 900
gaataccagt ggttacaaat agatttgaat aaggaaaaga aaataacagg cattataacc 960
actggatcca ccatggtgga gcacaattac tatgtgtctg cctacagaat cctgtacagt 1020
gatgatgggc agaaatggac tgtgtacaga gagcctggtg tggagcaaga taagatattt 1080
caaggaaaca aagattatca ccaggatgtg cgtaataact ttttgccacc aattattgca 1140
cgttttatta gagtgaatcc tacccaatgg cagcagaaaa ttgccatgaa aatggagctg 1200
ctcggatgtc agtttattcc taaaggtcgt cctccaaaac ttactcaacc tccacctcct 1260
cggaacagca atgacctcaa aaacactaca gcccctccaa aaatagccaa aggtcgtgcc 1320
ccaaaattta cgcaaccact acaacctcgc agtagcaatg aatttcctgc acagacagaa 1380
caaacaactg ccagtcctga tatcagaaat actaccgtaa ctccaaatgt aaccaaagat 1440
gtagcgctgg ctgcagttct tgtccctgtg ctggtcatgg tcctcactac tctcattctc 1500
atattagtgt gtgcttggca ctggagaaac agaaagaaaa aaactgaagg cacctatgac 1560
ttaccttact gggaccgggc aggttggtgg aaaggaatga agcagtttct tcctgcaaaa 1620
gcagtggacc atgaggaaac cccagttcgc tatagcagca gcgaagttaa tcacctgagt 1680
ccaagagaag tcaccacagt gctgcaggct gactctgcag agtatgctca gccactggta 1740
ggaggaattg ttggtacact tcatcaaaga tctaccttta aaccagaaga aggaaaagaa 1800
gcaggctatg cagacctaga tccttacaac tcaccagggc aggaagttta tcatgcctat 1860
gctgaaccac tcccaattac ggggcctgag tatgcaaccc caatcatcat ggacatgtca 1920
gggcacccca caacttcagt tggtcagccc tccacatcca ctttcaaggc tacggggaac 1980
caacctcccc cactagtggg aacttacaat acacttctct ccaggactga cagctgctcc 2040
tcagcccagg cccagtatga taccccgaaa gctgggaagc caggtctacc tgccccagac 2100
gaattggtgt accaggtgcc acagagcaca caagaagtat caggagcagg aagggatggg 2160
gaatgtgatg tttttaaaga aatcctttga 2190

<210> 3

<211> 729
<212> PRT
<213> Homo sapiens

<400> 3

<210> 4
<211> 300
<212> DNA
<213> Homo sapiens

<400> 4
aaacagaaag aaaaaaactg aaggcaccta tgacttacct tactgggacc gggcaggttg 60
gtggaaagga atgaagcagt ttcttcctgc aaaagcagtg gaccatgagg aaaccccagt 120
tcgctatagc agcagcgaag ttaatcacct gagtccaaga gaagtcacca cagtgctgca 180
ggctgactct gcagagtatg ctcagccact ggtaggagga attgttggta cacttcatca 240
aagatctacc tttaaaccag aagaaggaaa agaagcaggc tatgcagacc tagatcctta 300

<210> 5
<211> 99
<212> PRT
<213> Homo sapiens

<400> 5
Asn Arg Lys Lys Lys Thr Glu Gly Thr Tyr Asp Leu Pro Tyr Trp Asp
1               5                   10                  15

Arg Ala Gly Trp Trp Lys Gly Met Lys Gln Phe Leu Pro Ala Lys Ala
            20                  25                  30

Val Asp His Glu Glu Thr Pro Val Arg Tyr Ser Ser Ser Glu Val Asn
        35                  40                  45

His Leu Ser Pro Arg Glu Val Thr Thr Val Leu Gln Ala Asp Ser Ala
    50                  55                  60

Glu Tyr Ala Gln Pro Leu Val Gly Gly Ile Val Gly Thr Leu His Gln
65                  70                  75                  80

Arg Ser Thr Phe Lys Pro Glu Glu Gly Lys Glu Ala Gly Tyr Ala Asp
                85                  90                  95

Leu Asp Pro


<210> 6

<211> 275
<212> DNA
<213> Homo sapiens

<400> 6
caacttcagt tggtcagccc tccacatcca ctttcaaggc tacggggaac caacctcccc 60
cactagtggg aacttacaat acacttctct ccaggactga cagctgctcc tcagcccagg 120
cccagtatga taccccgaaa gctgggaagc caggtctacc tgccccagac gaattggtgt 180
accaggtgcc acagagcaca caagaagtat caggagcagg aagggatggg gaatgtgatg 240
tttttaaaga aatcctttga agatgatgct gcttt 275

<210> 7
<211> 260
<212> DNA
<213> Homo sapiens

<400> 7
caacttcagt tggtcagccc tccacatcca ctttcaaggc tacggggaac caacctcccc 60
cactagtggg aacttacaat acacttctct ccaggactga cagctgctcc tcagcccagg 120
cccagtatga taccccgaaa gctgggaagc caggtctacc tgccccagac gaattggtgt 180
accaggtgcc acagagcaca caagaagtat caggagcagg aagggatggg gaatgtgatg 240
tttttaaaga aatcctttga 260

<210> 8
<211> 15
<212> DNA
<213> Homo sapiens

<400> 8
agatgatgct gcttt 15

<210> 9
<211> 85
<212> PRT
<213> Homo sapiens

<400> 9
Thr Ser Val Gly Gln Pro Ser Thr Ser Thr Phe Lys Ala Thr Gly Asn
1               5                   10                  15

Gln Pro Pro Pro Leu Val Gly Thr Tyr Asn Thr Leu Leu Ser Arg Thr
            20                  25                  30

Asp Ser Cys Ser Ser Ala Gln Ala Gln Tyr Asp Thr Pro Lys Ala Gly
        35                  40                  45

Lys Pro Gly Leu Pro Ala Pro Asp Glu Leu Val Tyr Gln Val Pro Gln
    50                  55                  60

Ser Thr Gln Glu Val Ser Gly Ala Gly Arg Asp Gly Glu Cys Asp Val
65                  70                  75                  80

Phe Lys Glu Ile Leu
                85

<210> 10
<211> 142
<212> DNA
<213> Homo sapiens

<400> 10

gccgccgccc ccgcctgggc cgcgctcccc ctctcccgct ccctccctcc ctgctccaac 60
tcctcctcct tctccatgcc tctgttcctc ctgctcttac ttgtcctgct cctgctgctc 120
gaggacgctg gagcccagca ag 142

<210> 11
<211> 228
<212> DNA
<213> Homo sapiens

<400> 11
gtgatggatg tggacacact gtactaggcc ctgagagtgg aacccttaca tccataaact 60
acccacagac ctatcccaac agcactgttt gtgaatggga gatccgtgta aagatgggag 120
agagagttcg catcaaattt ggtgactttg acattgaaga ttctgattct tgtcacttta 180
attacttgag aatttataat ggaattggag tcagcagaac tgaaatag 228

<210> 12
<211> 138
<212> DNA
<213> Homo sapiens

<400> 12
gcaaatactg tggtctgggg ttgcaaatga accattcaat tgaatcaaaa ggcaatgaaa 60
tcacattgct gttcatgagt ggaatccatg tttctggacg cggatttttg gcctcatact 120
ctgttataga taaacaag 138

<210> 13
<211> 52
<212> DNA
<213> Homo sapiens

<400> 13
atctaattac ttgtttggac actgcatcca attttttgga acctgagttc ag 52

<210> 14
<211> 73
<212> DNA
<213> Homo sapiens

<400> 14
taagtactgc ccagctggtt gtctgcttcc ttttgctgag atatctggaa caattcctca 60
tggatataga gat 73

<210> 15
<211> 134
<212> DNA
<213> Homo sapiens

<400> 15
tcctcgccat tgtgcatggc tggtgtgcat gcaggagtag tgtcaaacac gttgggcggc 60
caaatcagtg ttgtaattag taaaggtatt ccctattatg aaagttcttt ggctaacaac 120
gtcacatctg tggt 134

<210> 16
<211> 41
<212> DNA
<213> Homo sapiens

<400> 16
gggacactta tctacaagtc tttttacatt taagacaagt g 41

<210> 17

<211> 216
<212> DNA
<213> Homo sapiens

<400> 17
gatgttatgg aacactgggg atggagtctg gtgtgatcgc ggatcctcaa ataacagcat 60
catctgtgct ggagtggact gaccacacag ggcaagagaa cagttggaaa cccaaaaaag 120
ccaggctgaa aaaacctgga ccgccttggg ctgcttttgc cactgatgaa taccagtggt 180
tacaaataga tttgaataag gaaaagaaaa taacag 216

<210> 18
<211> 125
<212> DNA
<213> Homo sapiens

<400> 18
gcattataac cactggatcc accatggtgg agcacaatta ctatgtgtct gcctacagaa 60
tcctgtacag tgatgatggg cagaaatgga ctgtgtacag agagcctggt gtggagcaag 120
ataag 125

<210> 19
<211> 151
<212> DNA
<213> Homo sapiens

<400> 19
atatttcaag gaaacaaaga ttatcaccag gatgtgcgta ataacttttt gccaccaatt 60
attgcacgtt ttattagagt gaatcctacc caatggcagc agaaaattgc catgaaaatg 120
gagctgctcg gatgtcagtt tattcctaaa g 151

<210> 20
<211> 87
<212> DNA
<213> Homo sapiens

<400> 20
gtcgtcctcc aaaacttact caacctccac ctcctcggaa cagcaatgac ctcaaaaaca 60
ctacagcccc tccaaaaata gccaaag 87

<210> 21
<211> 126
<212> DNA
<213> Homo sapiens

<400> 21
gtcgtgcccc aaaatttacg caaccactac aacctcgcag tagcaatgaa tttcctgcac 60
agacagaaca aacaactgcc agtcctgata tcagaaatac taccgtaact ccaaatgtaa 120
ccaaag 126

<210> 22
<211> 94
<212> DNA
<213> Homo sapiens

<400> 22
atgtagcgct ggctgcagtt cttgtccctg tgctggtcat ggtcctcact actctcattc 60
tcatattagt gtgtgcttgg cactggagaa acag 94

<210> 23
<211> 50
<212> DNA

<213> Homo sapiens

<400> 23
aaagaaaaaa actgaaggca cctatgactt accttactgg gaccgggcag          50

<210> 24
<211> 138
<212> DNA
<213> Homo sapiens

<400> 24
gttggtggaa aggaatgaag cagtttcttc ctgcaaaagc agtggaccat gaggaaaccc 60
cagttcgcta tagcagcagc gaagttaatc acctgagtcc aagagaagtc accacagtgc 120
tgcaggctga ctctgcag                                               138

<210> 25
<211> 485
<212> DNA
<213> Homo sapiens

<400> 25
agtatgctca gccactggta ggaggaattg ttggtacact tcatcaaaga tctaccttta 60
aaccagaaga aggaaaagaa gcaggctatg cagacctaga tccttacaac tcaccagggc 120
aggaagttta tcatgcctat gctgaaccac tcccaattac ggggcctgag tatgcaaccc 180
caatcatcat ggacatgtca gggcacccca caacttcagt tggtcagccc tccacatcca 240
ctttcaaggc tacggggaac caacctcccc cactagtggg aacttacaat acacttctct 300
ccaggactga cagctgctcc tcagcccagg cccagtatga taccccgaaa gctgggaagc 360
caggtctacc tgccccagac gaattggtgt accaggtgcc acagagcaca caagaagtat 420
caggagcagg aagggatggg gaatgtgatg tttttaaaga aatcctttga agatgatgct 480
gcttt                                                             485

<210> 26
<211> 500
<212> DNA
<213> Homo sapiens

<400> 26
gggctgcctc ttgttctccc gccgctgccg ccgtctcctg gtcgggtgcc gcggccagag 60
gcgcgcgggg ctgccgaggc acccgcacta tgcaggcaga ctgccggccg ccgcgatggc 120
gagccgggcg gtggtgagag ccaggcgctg cccgcagtgt ccccaagtcc gggccgcggc 180
cgccgccccc gcctgggccg cgctccccct ctcccgctcc ctccctccct gctccaactc 240
ctcctccttc tccatgcctc tgttcctcct gctcttactt gtcctgctcc tgctgctcga 300
ggacgctgga gcccagcaag gtgagtggtc ccaggggagt gagcggcggg ggaacctccg 360
tcggggctgc gggtctcttg gcccctgaac aaatctgcag acgctgcagg gaggcgcggt 420
gcgctttcgt gcgtggccgg acctggctgg gtactcacgg ggttgagttg caaaggcaag 480
gtcttgcccg tctgtccccc                                             500

<210> 27
<211> 500
<212> DNA
<213> Homo sapiens

<400> 27
aaaaaaaata aaatagaaag tatgggtggt ataaaatatt tgatgtcgtg catattttta 60
aatcttcaca aaaaaatcat tatagaggaa gtaaaatatg tgagaataat ttatcattct 120
tttcactttt tttaaaggtg atggatgtgg acacactgta ctaggccctg agagtggaac 180
ccttacatcc ataaactacc cacagaccta tcccaacagc actgtttgtg aatgggagat 240
ccgtgtaaag atgggagaga gagttcgcat caaatttggt gactttgaca ttgaagattc 300
tgattcttgt cactttaatt acttgagaat ttataatgga attggagtca gcagaactga 360
aataggtagg actttttttt gtaaatgtac atgtaaatac atcattgtct caatgatgta 420
tttttttgat attaacatta aataaccatg cagtgtaagg tgctatgtgg gaaattattg 480

aatatgtgta aaaattttga 500

<210> 28
<211> 500
<212> DNA
<213> Homo sapiens

<400> 28
ttacttgtca tttttttcct ccagagcaat atgattggta atcattaaac attttagtca 60
ttattccata ctaatttaac agccagcttg gtatattatt ctgcttctgt tatctacatt 120
gcaaatatta ttgacttcat gagagtcatc ccaaataatc tgcttttctt tcattcctag 180
gcaaatactg tggtctgggg ttgcaaatga accattcaat tgaatcaaaa ggcaatgaaa 240
tcacattgct gttcatgagt ggaatccatg tttctggacg cggatttttg gcctcatact 300
ctgttataga taaacaaggt aattttcacc ttttgcaatg gttcctacag tttgtaattt 360
ctaacaacaa aaataaaatg ctttttttaa gtacctgttt tctgaaattg gaatagagca 420
gaaagactga acaattaata gcacatatat aaatttgaca aataaggtga gttcagtttc 480
aaagttccaa aaataaaagt 500

<210> 29
<211> 500
<212> DNA
<213> Homo sapiens

<400> 29
gtagtaaaca gtattgaggg gacattgctc caaaaatcaa gaaacttaat atcagaaaga 60
aattgtgatt aacaaaagtt ggtaaagttt ttagtaccac tttgacagtg ccattgagat 120
ttttgaaaat tagagtagta cagtttggga ttctgagaca cttggagttt cagcaaggta 180
atcctgtata ttaatggaat cagtttttta tttttgtttt tctagatcta attacttgtt 240
tggacactgc atccaatttt ttggaacctg agttcaggta tgattatatt tgtgactttt 300
tttttttttt tgcttaaatg ttacttttca ttcacttctt cattttcatt ctgtatactc 360
ttagggttaa tgtatattgt gtgatacata tcattgttcc gagacttcaa taaattagag 420
cttacttaaa gcataaagcc atttagttgc atgtttacat tttgctttta ttgtagcatt 480
tagatctgca aaatgaaagt 500

<210> 30
<211> 500
<212> DNA
<213> Homo sapiens

<400> 30
   ggttgcagtg agccaagaag gtgccattgc actccagcct gggcgacaag agtgaaactc 60
   cgtgtcaaaa aaaagaagtg gtgggtcttt gagggtagtg aaattatggt attcaccttg 120
   aactgaaaaa tgtgtctgtg atattttggg ggggttgctt ttttccttgt aaaatacagc 180
   attatgtaac cttttgcttg ttttcatttc tcagtaagta ctgcccagct ggttgtctgc 240
   ttccttttgc tgagatatct ggaacaattc ctcatggata tagagatgta agccagatat 300
   aaacttattt gaaaattgtg atataactct atttttttag aactccagag tagttagagg 360
   tgtgttaatc atatatcatt atcttttgcc tttttaagac attttttaat catatgtgtt 420
   ttattctttt tcttctgcta aaactgttgt gttacagtac caataatcta tttttgttta 480
   tgaagtttat gaagaaactg 500

<210> 31
<211> 500
<212> DNA
<213> Homo sapiens

<400> 31
catatgtgtt ttattctttt tcttctgcta aaactgttgt gttacagtac caataatcta 60
tttttgttta tgaagtttat gaagaaactg cctagattaa ctgatttctc aggaagtaga 120
gtgtcttgtt tatgaattat taagagaaaa ttaaaaatga atcttttctt ttcccttcct 180
taagtcctcg ccattgtgca tggctggtgt gcatgcagga gtagtgtcaa acacgttggg 240
cggccaaatc agtgttgtaa ttagtaaagg tattccctat tatgaaagtt ctttggctaa 300

caacgtcaca tctgtggtgt aagtatatat gcaatttaaa aaatatatgc tatatataat 360
tagcattctg ggtagcattg ggctaagaac tagctagatc tcagcagcat cactgttaat 420
ggtgttcatg aaagaacatt taggcagtaa gcactcacat tagtgtgctc agtgtctgtt 480
tttttgtttt gttttgtttg 500

<210> 32
<211> 500
<212> DNA
<213> Homo sapiens

<400> 32
gggtgctaga tacttgtaga gaaagctgtt tatgctgatt tcagaagttg tcctgcttag 60
aaatgagact tttttcctag gaacaatatt agttgtctaa tttttagcca cacagtcttc 120
ttggttcttt tattgggtgg ggggaaccaa atcaaaagaa aatgtttcac taaatatggg 180
gcatcatgtg ttatgtgagt atcaccaaaa aaaacccttc tctctttcag gggacactta 240
tctacaagtc tttttacatt taagacaagt ggtaagcctt ttatggactt tataattttt 300
tatttatata atattttttg aacactgtta ggcactgtgt cagttgctga tttattgtac 360
tttctcacgt agttgtctta ataacactat gaggtaagat agttatctaa taatataatg 420
aatagagcaa attgaaagtt ttatatttat agagaaatgg tcatgtaagc aaggaaggtt 480
ccttaagaaa gatgactttt 500

<210> 33
<211> 500
<212> DNA
<213> Homo sapiens

<400> 33
tagttttgaa atttctctgt tacagtgtat tattagtgta gtgtatgtta attaaagatg 60
gaatagaaag ttttcagtga gcaggtctac atgcattttg aaattatacc tggaaaccaa 120
atttagtctt ttctttgttt caggatgtta tggaacactg gggatggagt ctggtgtgat 180
cgcggatcct caaataacag catcatctgt gctggagtgg actgaccaca cagggcaaga 240
gaacagttgg aaacccaaaa aagccaggct gaaaaaacct ggaccgcctt gggctgcttt 300
tgccactgat gaataccagt ggttacaaat agatttgaat aaggaaaaga aaataacagg 360
ttaagagaca aaaattttct aaagcaattt gtaacactta ttttatttga aaaactagta 420
acatctaaga ttttcagaga aaattaaggc attaaaattg taattatatg gttttatatt 480
ttcttcactg atgttaatat 500

<210> 34
<211> 500
<212> DNA
<213> Homo sapiens

<400> 34
cctctctctc caagggaata aggccaagga tttagtaatt gttatgacaa aaacgtgcac 60
ttggatatgg aaatgagcca taattagaaa tgttcgagct gtgcatttca agtgtttata 120
ctttgatgct gaacaaactt agggaatcag accatgtgca ttaatgaaag caactcttca 180
ttccccaggc attataacca ctggatccac catggtggag cacaattact atgtgtctgc 240
ctacagaatc ctgtacagtg atgatgggca gaaatggact gtgtacagag agcctggtgt 300
ggagcaagat aaggtaaaat gattaccaag gtatttttaa aaaacatttt ttaaatgggt 360
ggttgccaag tcacagagct ctcctgtata aaagagatgt agaagtttag aatgtcttat 420
actaccctct tgtggttatt ttaggtagtg ttattggcct tactctactg actttagaaa 480
attctcagta gttctaggtt 500

<210> 35
<211> 500
<212> DNA
<213> Homo sapiens

<400> 35
agcatcttaa tatttttcca aattatgatt attttcttaa aaattgatcc ttattgtcat 60
actttccaga tgtaatttaa ggcctagagc aaagaacaaa aacatgttta agtggagaaa 120

tttagccctg acctctattg atttgaagta ccaatttttt gttgtttttt taaagatatt 180
tcaaggaaac aaagattatc accaggatgt gcgtaataac tttttgccac caattattgc 240
acgttttatt agagtgaatc ctacccaatg gcagcagaaa attgccatga aaatggagct 300
gctcggatgt cagtttattc ctaaaggtaa agagacagtt cgtttaagat atgtttttac 360
tggattgcca atttaaaatg gttttgctgt tctcagagag agtaattaat aatgtgagtg 420
tctttctggt taaactctaa ttacaatatc ttcttaaagg gatgttaaaa aaaatggtta 480
ccctattaaa aagttagtta 500

<210> 36
<211> 500
<212> DNA
<213> Homo sapiens

<400> 36
aattatttaa aaactgagaa atcctggcat atttgtgcct ttattgcaga gtataatatt 60
tatccttcca atatatttct ctctgagagt taaaattatt ctgcagaatt aaatacttta 120
ggaaatattg aagacattta gcattactat ttttaattat gatttttaaa aacaaaatgt 180
ttaaattttt ctaaaccatt tttcaaggtc gtcctccaaa acttactcaa cctccacctc 240
ctcggaacag caatgacctc aaaaacacta cagcccctcc aaaaatagcc aaaggtatgc 300
ctgtttttaa tgtgagaatt tgatattcct tggatttatg gtgcaacttt ccaaaaatat 360
attaaaagca ttatcaaata agtttatgtt aatcaagttg gtacagatta aaaagtaaag 420
agcttttgaa acttttttaa aggtcgtgcc ccaaaattta cgcaaccact acaacctcgc 480
agtagcaatg aatttcctgc 500

<210> 37
<211> 500
<212> DNA
<213> Homo sapiens

<400> 37
gacctcaaaa acactacagc ccctccaaaa atagccaaag gtatgcctgt ttttaatgtg 60
agaatttgat attccttgga tttatggtgc aactttccaa aaatatatta aaagcattat 120
caaataagtt tatgttaatc aagttggtac agattaaaaa gtaaagagct tttgaaactt 180
ttttaaaggt cgtgccccaa aatttacgca accactacaa cctcgcagta gcaatgaatt 240
tcctgcacag acagaacaaa caactgccag tcctgatatc agaaatacta ccgtaactcc 300
aaatgtaacc aaaggtatgc aaacatggaa atgaacgcct agtacatagt attgttttgt 360
tcttaactga agttttcacg ggcattttta aaccttcagt ttattttctt ccaactatag 420
tttttaagta tagcaaaaaa aaaaatttat tttctttcat aggaaaagac tgcattgatc 480
taagttgtag tctctgctgc 500

<210> 38
<211> 500
<212> DNA
<213> Homo sapiens

<400> 38
ggaatgaata gtgtaacaag cttttgtgta tctgcaaaag cttctacagt taccaccaca 60
tgcccagtct tgacaaataa tgtttatggt atttccgtga ggtagaaaaa tgttgaaaga 120
tgatgtggtt aattttccct agtgatttct gcctttggtg ttaatatggc ttttgtttgt 180
ctgtttttgt ctttgtaact tcagatgtag cgctggctgc agttcttgtc cctgtgctgg 240
tcatggtcct cactactctc attctcatat tagtgtgtgc ttggcactgg agaaacaggt 300
tagtacataa ctagttcacc tgagtccaaa actaccaaat gtgaagtaga agctaaatat 360
agaagatgaa aatgtttacc tgtttgagag tgagagttaa ggtaattatt aaaatgaaaa 420
tttcatgctt ctcctttatt cccattaaaa ataaataagt tcaattccac aatcagttac 480
taagtacctt ttttgtatca 500

<210> 39
<211> 500
<212> DNA
<213> Homo sapiens

<400> 39
ctttgtagtg acatggatga agctggaaac catcattctc agcaaactgt cccaaggaca 60
aaaaaccaaa caccgcatgt tctcactcat aggtgggaat tgaacaatga gaacacttgg 120
acacaggaag gggaatatca cataccaagg cctgtcatgg ggtaggggga gcaggcttac 180
cagtgaattt actctgtttg ctaacttctc ttctgtattt ttccagaaag aaaaaaactg 240
aaggcaccta tgacttacct tactgggacc gggcaggtaa ctcacgtggt ctttgcatct 300
catttctatc agagggatgt cgctccccta cagggggcag tagtgaaaaa agagtcattc 360
tctggcccag gtgaactccc cgacactgtt agaacaatgg cattactctt cagttctcac 420
catttttacc cttctgcaaa gtctcttgta attcctaagt aatgaaatga aaagtacaaa 480
tttcttaaaa caagctctgt 500

<210> 40
<211> 500
<212> DNA
<213> Homo sapiens

<400> 40
ctgcttggtg ctggatagtc tatcacagca aaaaaaaaaa aaaaagttgg attataaagg 60
caagtttgta gagatatttg ttatagaaat ggcttaaatg caacaggatt tttctcttga 120
tactactgtt tgagatacag gtttttattt aatggtctct ttggcttgcc gtcacaatga 180
aggttggtgg aaaggaatga agcagtttct tcctgcaaaa gcagtggacc atgaggaaac 240
cccagttcgc tatagcagca gcgaagttaa tcacctgagt ccaagagaag tcaccacagt 300
gctgcaggct gactctgcag gtaactatgt tgcagccttc tggtaccagg cagagggaga 360
aactgcttag gcttgctata aagtgctttg ggatttacag ttctttgatc cctttcatgt 420
ttaagaaatg aattgtttcc taaaggtaga accacttttt taaaaggtga ctcttcagag 480
agtcatttct gtatcttgga 500

<210> 41
<211> 500
<212> DNA
<213> Homo sapiens

<400> 41
atccacagag tatgctcagc cactggtagg aggaattgtt ggtacacttc atcaaagatc 60
tacctttaaa ccagaagaag gaaaagaagc aggctatgca gacctagatc cttacaactc 120
accagggcag gaagtttatc atgcctatgc tgaaccactc ccaattacgg ggcctgagta 180
tgcaacccca atcatcatgg acatgtcagg gcaccccaca acttcagttg gtcagccctc 240
cacatccact ttcaaggcta cggggaacca acctccccca ctagtgggaa cttacaatac 300
acttctctcc aggactgaca gctgctcctc agcccaggcc cagtatgata ccccgaaagc 360
tgggaagcca ggtctacctg ccccagacga attggtgtac caggtgccac agagcacaca 420
agaagtatca ggagcaggaa gggatgggga atgtgatgtt tttaaagaaa tcctttgaag 480
atgatgctgc tttttacaaa 500

<210> 42
<211> 1000
<212> DNA
<213> Homo sapiens

<400> 42
tgttgcaaca actggcggca gttttaacaa aacaagccac gctctgccat taaaaaaaaa 60
aaaaaagtcc cttagactca gctcccagct aaaacaaccc aggatccagg tttcccaccc 120
ccttccagtg gtctgatgtg cgtcggagga gcgaggagaa aggagcaagg agcaagctgg 180
gcctgaggag cagggagcgc gggatggggg acgtcccgga agtcactgac gagctggctg 240
ccgcctctgc gggatcagtt tgggctgagg gcccaacaat aacaggcgcc cgggccgggc 300
gggggccgcg gggatccgaa gctggagggc gggacctgga ttggaggagg cgggtgggac 360
gcctccaaag gccgaagggg attggaagag ggaggtaagg ggcggtggag ctcgaggaat 420
gggccggaga actagcgggt cgagcgagga gcgagcatgc tgattgggtt tcgcggaagg 480
agacggacca gagctggccg aggattggcc gcggtgccta gggggcgtgg ccggcgcgcg 540
cgagagggag cgcgagggag ctggctgcgg gcctgcctgc cagctagccg gagccgcggg 600
tgagcgcggc gagcggcgac cctggtgagg agcgcggcgc gggaggcacg ttccttagct 660
ccgccgcggc cgtcctccgc ggctcgagga ctccgcttcc ttccctcccc tcccctgcgc 720

tccggcctgg ggtctcggcg cggggagcgg agggaaggga cgaaggagga gtaggtgaaa 780
gcggggtgag gggcggaagg gtcccggcgc ggggtgaggc gagggctgcc tcttgttctc 840
ccgccgctgc cgccgtctcc tggtcgggtg ccgcggccag aggcgcgcgg ggctgccgag 900
gcacccgcac tatgcaggca gactgccggc cgccgcgatg gcgagccggg cggtggtgag 960
agccaggcgc tgcccgcagt gtccccaagt ccgggccgcg 1000

<210> 43
<211> 17
<212> DNA
<213> Homo sapiens

<400> 43
aaacagaaag aaaaaaa 17

<210> 44
<211> 17
<212> DNA
<213> Homo sapiens

<400> 44
aacagaaaga aaaaaac 17

<210> 45
<211> 17
<212> DNA
<213> Homo sapiens

<400> 45
acagaaagaa aaaaact 17

<210> 46
<211> 17
<212> DNA
<213> Homo sapiens

<400> 46
cagaaagaaa aaaactg 17

<210> 47
<211> 17
<212> DNA
<213> Homo sapiens

<400> 47
agaaagaaaa aaactga 17

<210> 48
<211> 17
<212> DNA
<213> Homo sapiens

<400> 48
gaaagaaaaa aactgaa 17

<210> 49
<211> 17
<212> DNA
<213> Homo sapiens

<400> 49
aaagaaaaaa actgaag 17

<210> 50
<211> 17
<212> DNA
<213> Homo sapiens

<400> 50
aagaaaaaaa ctgaagg                                                    17

<210> 51
<211> 17
<212> DNA
<213> Homo sapiens

<400> 51
agaaaaaaac tgaaggc                                                    17

<210> 52
<211> 17
<212> DNA
<213> Homo sapiens

<400> 52
gaaaaaaact gaaggca                                                    17

<210> 53
<211> 17
<212> DNA
<213> Homo sapiens

<400> 53
aaaaaaactg aaggcac                                                    17

<210> 54
<211> 17
<212> DNA
<213> Homo sapiens

<400> 54
aaaaaactga aggcacc                                                    17

<210> 55
<211> 17
<212> DNA
<213> Homo sapiens

<400> 55
aaaaactgaa ggcacct                                                    17

<210> 56
<211> 17
<212> DNA
<213> Homo sapiens

<400> 56
aaaactgaag gcaccta                                                    17

<210> 57
<211> 17
<212> DNA
<213> Homo sapiens

<400> 57
aaactgaagg cacctat                                                    17

<210> 58
<211> 17
<212> DNA
<213> Homo sapiens

<400> 58
aactgaaggc acctatg                                                    17

<210> 59
<211> 17
<212> DNA
<213> Homo sapiens

<400> 59
actgaaggca cctatga                                                    17

<210> 60
<211> 17
<212> DNA
<213> Homo sapiens

<400> 60
ctgaaggcac ctatgac                                                    17

<210> 61
<211> 17
<212> DNA
<213> Homo sapiens

<400> 61
tgaaggcacc tatgact                                                    17

<210> 62
<211> 17
<212> DNA
<213> Homo sapiens

<400> 62
gaaggcacct atgactt                                                    17

<210> 63
<211> 17
<212> DNA
<213> Homo sapiens

<400> 63
aaggcaccta tgactta                                                    17

<210> 64
<211> 17
<212> DNA
<213> Homo sapiens

<400> 64
aggcacctat gacttac                                                    17

<210> 65

<211> 17
<212> DNA
<213> Homo sapiens

<400> 65
ggcacctatg acttacc                                                    17

<210> 66
<211> 17
<212> DNA
<213> Homo sapiens

<400> 66
gcacctatga cttacct                                                    17

<210> 67
<211> 17
<212> DNA
<213> Homo sapiens

<400> 67
cacctatgac ttacctt                                                    17

<210> 68
<211> 17
<212> DNA
<213> Homo sapiens

<400> 68
acctatgact tacctta                                                    17

<210> 69
<211> 17
<212> DNA
<213> Homo sapiens

<400> 69
cctatgactt accttac                                                    17

<210> 70
<211> 17
<212> DNA
<213> Homo sapiens

<400> 70
ctatgactta ccttact                                                    17

<210> 71
<211> 17
<212> DNA
<213> Homo sapiens

<400> 71
tatgacttac cttactg                                                    17

<210> 72
<211> 17
<212> DNA
<213> Homo sapiens

<400> 72

atgacttacc ttactgg 17

<210> 73
<211> 17
<212> DNA
<213> Homo sapiens

<400> 73
tgacttacct tactggg 17

<210> 74
<211> 17
<212> DNA
<213> Homo sapiens

<400> 74
gacttacctt actggga 17

<210> 75
<211> 17
<212> DNA
<213> Homo sapiens

<400> 75
acttacctta ctgggac 17

<210> 76
<211> 17
<212> DNA
<213> Homo sapiens

<400> 76
cttaccttac tgggacc 17

<210> 77
<211> 17
<212> DNA
<213> Homo sapiens

<400> 77
ttaccttact gggaccg 17

<210> 78
<211> 17
<212> DNA
<213> Homo sapiens

<400> 78
taccttactg ggaccgg 17

<210> 79
<211> 17
<212> DNA
<213> Homo sapiens

<400> 79
accttactgg gaccggg 17

<210> 80
<211> 17
<212> DNA

<213> Homo sapiens

<400> 80
ccttactggg accgggc                                                    17

<210> 81
<211> 17
<212> DNA
<213> Homo sapiens

<400> 81
cttactggga ccgggca                                                    17

<210> 82
<211> 17
<212> DNA
<213> Homo sapiens

<400> 82
ttactgggac cgggcag                                                    17

<210> 83
<211> 17
<212> DNA
<213> Homo sapiens

<400> 83
tactgggacc gggcagg                                                    17

<210> 84
<211> 17
<212> DNA
<213> Homo sapiens

<400> 84
actgggaccg ggcaggt                                                    17

<210> 85
<211> 17
<212> DNA
<213> Homo sapiens

<400> 85
ctgggaccgg gcaggtt                                                    17

<210> 86
<211> 17
<212> DNA
<213> Homo sapiens

<400> 86
tgggaccggg caggttg                                                    17

<210> 87
<211> 17
<212> DNA
<213> Homo sapiens

<400> 87
gggaccgggc aggttgg                                                    17

<210> 88
<211> 17
<212> DNA
<213> Homo sapiens

<400> 88
ggaccgggca ggttggt                                                    17

<210> 89
<211> 17
<212> DNA
<213> Homo sapiens

<400> 89
gaccgggcag gttggtg                                                    17

<210> 90
<211> 17
<212> DNA
<213> Homo sapiens

<400> 90
accgggcagg ttggtgg                                                    17

<210> 91
<211> 17
<212> DNA
<213> Homo sapiens

<400> 91
ccgggcaggt tggtgga                                                    17

<210> 92
<211> 17
<212> DNA
<213> Homo sapiens

<400> 92
cgggcaggtt ggtggaa                                                    17

<210> 93
<211> 17
<212> DNA
<213> Homo sapiens

<400> 93
gggcaggttg gtggaaa                                                    17

<210> 94
<211> 17
<212> DNA
<213> Homo sapiens

<400> 94
ggcaggttgg tggaaag                                                    17

<210> 95
<211> 17
<212> DNA
<213> Homo sapiens

<400> 95
gcaggttggt ggaaagg                                                    17

<210> 96
<211> 17
<212> DNA
<213> Homo sapiens

<400> 96
caggttggtg gaaagga                                                    17

<210> 97
<211> 17
<212> DNA
<213> Homo sapiens

<400> 97
aggttggtgg aaaggaa                                                    17

<210> 98
<211> 17
<212> DNA
<213> Homo sapiens

<400> 98
ggttggtgga aaggaat                                                    17

<210> 99
<211> 17
<212> DNA
<213> Homo sapiens

<400> 99
gttggtggaa aggaatg                                                    17

<210> 100
<211> 17
<212> DNA
<213> Homo sapiens

<400> 100
ttggtggaaa ggaatga                                                    17

<210> 101
<211> 17
<212> DNA
<213> Homo sapiens

<400> 101
tggtggaaag gaatgaa                                                    17

<210> 102
<211> 17
<212> DNA
<213> Homo sapiens

<400> 102
ggtggaaagg aatgaag                                                    17

<210> 103
<211> 17

<212> DNA
<213> Homo sapiens

<400> 103
gtggaaagga atgaagc                                                    17

<210> 104
<211> 17
<212> DNA
<213> Homo sapiens

<400> 104
tggaaaggaa tgaagca                                                    17

<210> 105
<211> 17
<212> DNA
<213> Homo sapiens

<400> 105
ggaaaggaat gaagcag                                                    17

<210> 106
<211> 17
<212> DNA
<213> Homo sapiens

<400> 106
gaaaggaatg aagcagt                                                    17

<210> 107
<211> 17
<212> DNA
<213> Homo sapiens

<400> 107
aaaggaatga agcagtt                                                    17

<210> 108
<211> 17
<212> DNA
<213> Homo sapiens

<400> 108
aaggaatgaa gcagttt                                                    17

<210> 109
<211> 17
<212> DNA
<213> Homo sapiens

<400> 109
aggaatgaag cagtttc                                                    17

<210> 110
<211> 17
<212> DNA
<213> Homo sapiens

<400> 110
ggaatgaagc agtttct                                                    17

<210> 111
<211> 17
<212> DNA
<213> Homo sapiens

<400> 111
gaatgaagca gtttctt                                                    17

<210> 112
<211> 17
<212> DNA
<213> Homo sapiens

<400> 112
aatgaagcag tttcttc                                                    17

<210> 113
<211> 17
<212> DNA
<213> Homo sapiens

<400> 113
atgaagcagt ttcttcc                                                    17

<210> 114
<211> 17
<212> DNA
<213> Homo sapiens

<400> 114
tgaagcagtt tcttcct                                                    17

<210> 115
<211> 17
<212> DNA
<213> Homo sapiens

<400> 115
gaagcagttt cttcctg                                                    17

<210> 116
<211> 17
<212> DNA
<213> Homo sapiens

<400> 116
aagcagtttc ttcctgc                                                    17

<210> 117
<211> 17
<212> DNA
<213> Homo sapiens

<400> 117
agcagtttct tcctgca                                                    17

<210> 118
<211> 17
<212> DNA
<213> Homo sapiens

<400> 118
gcagtttctt cctgcaa                                                    17

<210> 119
<211> 17
<212> DNA
<213> Homo sapiens

<400> 119
cagtttcttc ctgcaaa                                                    17

<210> 120
<211> 17
<212> DNA
<213> Homo sapiens

<400> 120
agtttcttcc tgcaaaa                                                    17

<210> 121
<211> 17
<212> DNA
<213> Homo sapiens

<400> 121
gtttcttcct gcaaaag                                                    17

<210> 122
<211> 17
<212> DNA
<213> Homo sapiens

<400> 122
tttcttcctg caaaagc                                                    17

<210> 123
<211> 17
<212> DNA
<213> Homo sapiens

<400> 123
ttcttcctgc aaaagca                                                    17

<210> 124
<211> 17
<212> DNA
<213> Homo sapiens

<400> 124
tcttcctgca aaagcag                                                    17

<210> 125
<211> 17
<212> DNA
<213> Homo sapiens

<400> 125
cttcctgcaa aagcagt                                                    17

<210> 126

<211> 17
<212> DNA
<213> Homo sapiens

<400> 126
ttcctgcaaa agcagtg                                                    17

<210> 127
<211> 17
<212> DNA
<213> Homo sapiens

<400> 127
tcctgcaaaa gcagtgg                                                    17

<210> 128
<211> 17
<212> DNA
<213> Homo sapiens

<400> 128
cctgcaaaag cagtgga                                                    17

<210> 129
<211> 17
<212> DNA
<213> Homo sapiens

<400> 129
ctgcaaaagc agtggac                                                    17

<210> 130
<211> 17
<212> DNA
<213> Homo sapiens

<400> 130
tgcaaaagca gtggacc                                                    17

<210> 131
<211> 17
<212> DNA
<213> Homo sapiens

<400> 131
gcaaaagcag tggacca                                                    17

<210> 132
<211> 17
<212> DNA
<213> Homo sapiens

<400> 132
caaaagcagt ggaccat                                                    17

<210> 133
<211> 17
<212> DNA
<213> Homo sapiens

<400> 133

aaaagcagtg gaccatg                                                    17

<210> 134
<211> 17
<212> DNA
<213> Homo sapiens

<400> 134
aaagcagtgg accatga                                                    17

<210> 135
<211> 17
<212> DNA
<213> Homo sapiens

<400> 135
aagcagtgga ccatgag                                                    17

<210> 136
<211> 17
<212> DNA
<213> Homo sapiens

<400> 136
agcagtggac catgagg                                                    17

<210> 137
<211> 17
<212> DNA
<213> Homo sapiens

<400> 137
gcagtggacc atgagga                                                    17

<210> 138
<211> 17
<212> DNA
<213> Homo sapiens

<400> 138
cagtggacca tgaggaa                                                    17

<210> 139
<211> 17
<212> DNA
<213> Homo sapiens

<400> 139
agtggaccat gaggaaa                                                    17

<210> 140
<211> 17
<212> DNA
<213> Homo sapiens

<400> 140
gtggaccatg aggaaac                                                    17

<210> 141
<211> 17
<212> DNA

<213> Homo sapiens

<400> 141
tggaccatga ggaaacc                                                    17

<210> 142
<211> 17
<212> DNA
<213> Homo sapiens

<400> 142
ggaccatgag gaaaccc                                                    17

<210> 143
<211> 17
<212> DNA
<213> Homo sapiens

<400> 143
gaccatgagg aaacccc                                                    17

<210> 144
<211> 17
<212> DNA
<213> Homo sapiens

<400> 144
accatgagga aacccca                                                    17

<210> 145
<211> 17
<212> DNA
<213> Homo sapiens

<400> 145
ccatgaggaa accccag                                                    17

<210> 146
<211> 17
<212> DNA
<213> Homo sapiens

<400> 146
catgaggaaa ccccagt                                                    17

<210> 147
<211> 17
<212> DNA
<213> Homo sapiens

<400> 147
atgaggaaac cccagtt                                                    17

<210> 148
<211> 17
<212> DNA
<213> Homo sapiens

<400> 148
tgaggaaacc ccagttc                                                    17

<210> 149
<211> 17
<212> DNA
<213> Homo sapiens

<400> 149
gaggaaaccc cagttcg                                                    17

<210> 150
<211> 17
<212> DNA
<213> Homo sapiens

<400> 150
aggaaacccc agttcgc                                                    17

<210> 151
<211> 17
<212> DNA
<213> Homo sapiens

<400> 151
ggaaacccca gttcgct                                                    17

<210> 152
<211> 17
<212> DNA
<213> Homo sapiens

<400> 152
gaaaccccag ttcgcta                                                    17

<210> 153
<211> 17
<212> DNA
<213> Homo sapiens

<400> 153
aaaccccagt tcgctat                                                    17

<210> 154
<211> 17
<212> DNA
<213> Homo sapiens

<400> 154
aaccccagtt cgctata                                                    17

<210> 155
<211> 17
<212> DNA
<213> Homo sapiens

<400> 155
accccagttc gctatag                                                    17

<210> 156
<211> 17
<212> DNA
<213> Homo sapiens

<400> 156
ccccagttcg ctatagc                                                    17

<210> 157
<211> 17
<212> DNA
<213> Homo sapiens

<400> 157
cccagttcgc tatagca                                                    17

<210> 158
<211> 17
<212> DNA
<213> Homo sapiens

<400> 158
ccagttcgct atagcag                                                    17

<210> 159
<211> 17
<212> DNA
<213> Homo sapiens

<400> 159
cagttcgcta tagcagc                                                    17

<210> 160
<211> 17
<212> DNA
<213> Homo sapiens

<400> 160
agttcgctat agcagca                                                    17

<210> 161
<211> 17
<212> DNA
<213> Homo sapiens

<400> 161
gttcgctata gcagcag                                                    17

<210> 162
<211> 17
<212> DNA
<213> Homo sapiens

<400> 162
ttcgctatag cagcagc                                                    17

<210> 163
<211> 17
<212> DNA
<213> Homo sapiens

<400> 163
tcgctatagc agcagcg                                                    17

<210> 164
<211> 17

<212> DNA
<213> Homo sapiens

<400> 164
cgctatagca gcagcga                                                    17

<210> 165
<211> 17
<212> DNA
<213> Homo sapiens

<400> 165
gctatagcag cagcgaa                                                    17

<210> 166
<211> 17
<212> DNA
<213> Homo sapiens

<400> 166
ctatagcagc agcgaag                                                    17

<210> 167
<211> 17
<212> DNA
<213> Homo sapiens

<400> 167
tatagcagca gcgaagt                                                    17

<210> 168
<211> 17
<212> DNA
<213> Homo sapiens

<400> 168
atagcagcag cgaagtt                                                    17

<210> 169
<211> 17
<212> DNA
<213> Homo sapiens

<400> 169
tagcagcagc gaagtta                                                    17

<210> 170
<211> 17
<212> DNA
<213> Homo sapiens

<400> 170
agcagcagcg aagttaa                                                    17

<210> 171
<211> 17
<212> DNA
<213> Homo sapiens

<400> 171
gcagcagcga agttaat                                                    17

<210> 172
<211> 17
<212> DNA
<213> Homo sapiens

<400> 172
cagcagcgaa gttaatc                                                    17

<210> 173
<211> 17
<212> DNA
<213> Homo sapiens

<400> 173
agcagcgaag ttaatca                                                    17

<210> 174
<211> 17
<212> DNA
<213> Homo sapiens

<400> 174
gcagcgaagt taatcac                                                    17

<210> 175
<211> 17
<212> DNA
<213> Homo sapiens

<400> 175
cagcgaagtt aatcacc                                                    17

<210> 176
<211> 17
<212> DNA
<213> Homo sapiens

<400> 176
agcgaagtta atcacct                                                    17

<210> 177
<211> 17
<212> DNA
<213> Homo sapiens

<400> 177
gcgaagttaa tcacctg                                                    17

<210> 178
<211> 17
<212> DNA
<213> Homo sapiens

<400> 178
cgaagttaat cacctga                                                    17

<210> 179
<211> 17
<212> DNA
<213> Homo sapiens

<400> 179
gaagttaatc acctgag                                                    17

<210> 180
<211> 17
<212> DNA
<213> Homo sapiens

<400> 180
aagttaatca cctgagt                                                    17

<210> 181
<211> 17
<212> DNA
<213> Homo sapiens

<400> 181
agttaatcac ctgagtc                                                    17

<210> 182
<211> 17
<212> DNA
<213> Homo sapiens

<400> 182
gttaatcacc tgagtcc                                                    17

<210> 183
<211> 17
<212> DNA
<213> Homo sapiens

<400> 183
ttaatcacct gagtcca                                                    17

<210> 184
<211> 17
<212> DNA
<213> Homo sapiens

<400> 184
taatcacctg agtccaa                                                    17

<210> 185
<211> 17
<212> DNA
<213> Homo sapiens

<400> 185
aatcacctga gtccaag                                                    17

<210> 186
<211> 17
<212> DNA
<213> Homo sapiens

<400> 186
atcacctgag tccaaga                                                    17

<210> 187

<211> 17
<212> DNA
<213> Homo sapiens

<400> 187
tcacctgagt ccaagag                                                    17

<210> 188
<211> 17
<212> DNA
<213> Homo sapiens

<400> 188
cacctgagtc caagaga                                                    17

<210> 189
<211> 17
<212> DNA
<213> Homo sapiens

<400> 189
acctgagtcc aagagaa                                                    17

<210> 190
<211> 17
<212> DNA
<213> Homo sapiens

<400> 190
cctgagtcca agagaag                                                    17

<210> 191
<211> 17
<212> DNA
<213> Homo sapiens

<400> 191
ctgagtccaa gagaagt                                                    17

<210> 192
<211> 17
<212> DNA
<213> Homo sapiens

<400> 192
tgagtccaag agaagtc                                                    17

<210> 193
<211> 17
<212> DNA
<213> Homo sapiens

<400> 193
gagtccaaga gaagtca                                                    17

<210> 194
<211> 17
<212> DNA
<213> Homo sapiens

<400> 194

agtccaagag aagtcac                                                    17

<210> 195
<211> 17
<212> DNA
<213> Homo sapiens

<400> 195
gtccaagaga agtcacc                                                    17

<210> 196
<211> 17
<212> DNA
<213> Homo sapiens

<400> 196
tccaagagaa gtcacca                                                    17

<210> 197
<211> 17
<212> DNA
<213> Homo sapiens

<400> 197
ccaagagaag tcaccac                                                    17

<210> 198
<211> 17
<212> DNA
<213> Homo sapiens

<400> 198
caagagaagt caccaca                                                    17

<210> 199
<211> 17
<212> DNA
<213> Homo sapiens

<400> 199
aagagaagtc accacag                                                    17

<210> 200
<211> 17
<212> DNA
<213> Homo sapiens

<400> 200
agagaagtca ccacagt                                                    17

<210> 201
<211> 17
<212> DNA
<213> Homo sapiens

<400> 201
gagaagtcac cacagtg                                                    17

<210> 202
<211> 17
<212> DNA

<213> Homo sapiens

<400> 202
agaagtcacc acagtgc                                                    17

<210> 203
<211> 17
<212> DNA
<213> Homo sapiens

<400> 203
gaagtcacca cagtgct                                                    17

<210> 204
<211> 17
<212> DNA
<213> Homo sapiens

<400> 204
aagtcaccac agtgctg                                                    17

<210> 205
<211> 17
<212> DNA
<213> Homo sapiens

<400> 205
agtcaccaca gtgctgc                                                    17

<210> 206
<211> 17
<212> DNA
<213> Homo sapiens

<400> 206
gtcaccacag tgctgca                                                    17

<210> 207
<211> 17
<212> DNA
<213> Homo sapiens

<400> 207
tcaccacagt gctgcag                                                    17

<210> 208
<211> 17
<212> DNA
<213> Homo sapiens

<400> 208
caccacagtg ctgcagg                                                    17

<210> 209
<211> 17
<212> DNA
<213> Homo sapiens

<400> 209
accacagtgc tgcaggc                                                    17

<210> 210
<211> 17
<212> DNA
<213> Homo sapiens

<400> 210
ccacagtgct gcaggct                                                    17

<210> 211
<211> 17
<212> DNA
<213> Homo sapiens

<400> 211
cacagtgctg caggctg                                                    17

<210> 212
<211> 17
<212> DNA
<213> Homo sapiens

<400> 212
acagtgctgc aggctga                                                    17

<210> 213
<211> 17
<212> DNA
<213> Homo sapiens

<400> 213
cagtgctgca ggctgac                                                    17

<210> 214
<211> 17
<212> DNA
<213> Homo sapiens

<400> 214
agtgctgcag gctgact                                                    17

<210> 215
<211> 17
<212> DNA
<213> Homo sapiens

<400> 215
gtgctgcagg ctgactc                                                    17

<210> 216
<211> 17
<212> DNA
<213> Homo sapiens

<400> 216
tgctgcaggc tgactct                                                    17

<210> 217
<211> 17
<212> DNA
<213> Homo sapiens

<400> 217
gctgcaggct gactctg                                                    17

<210> 218
<211> 17
<212> DNA
<213> Homo sapiens

<400> 218
ctgcaggctg actctgc                                                    17

<210> 219
<211> 17
<212> DNA
<213> Homo sapiens

<400> 219
tgcaggctga ctctgca                                                    17

<210> 220
<211> 17
<212> DNA
<213> Homo sapiens

<400> 220
gcaggctgac tctgcag                                                    17

<210> 221
<211> 17
<212> DNA
<213> Homo sapiens

<400> 221
caggctgact ctgcaga                                                    17

<210> 222
<211> 17
<212> DNA
<213> Homo sapiens

<400> 222
aggctgactc tgcagag                                                    17

<210> 223
<211> 17
<212> DNA
<213> Homo sapiens

<400> 223
ggctgactct gcagagt                                                    17

<210> 224
<211> 17
<212> DNA
<213> Homo sapiens

<400> 224
gctgactctg cagagta                                                    17

<210> 225
<211> 17

<212> DNA
<213> Homo sapiens

<400> 225
ctgactctgc agagtat                                                    17

<210> 226
<211> 17
<212> DNA
<213> Homo sapiens

<400> 226
tgactctgca gagtatg                                                    17

<210> 227
<211> 17
<212> DNA
<213> Homo sapiens

<400> 227
gactctgcag agtatgc                                                    17

<210> 228
<211> 17
<212> DNA
<213> Homo sapiens

<400> 228
actctgcaga gtatgct                                                    17

<210> 229
<211> 17
<212> DNA
<213> Homo sapiens

<400> 229
ctctgcagag tatgctc                                                    17

<210> 230
<211> 17
<212> DNA
<213> Homo sapiens

<400> 230
tctgcagagt atgctca                                                    17

<210> 231
<211> 17
<212> DNA
<213> Homo sapiens

<400> 231
ctgcagagta tgctcag                                                    17

<210> 232
<211> 17
<212> DNA
<213> Homo sapiens

<400> 232
tgcagagtat gctcagc                                                    17

<210> 233
<211> 17
<212> DNA
<213> Homo sapiens

<400> 233
gcagagtatg ctcagcc                                                    17

<210> 234
<211> 17
<212> DNA
<213> Homo sapiens

<400> 234
cagagtatgc tcagcca                                                    17

<210> 235
<211> 17
<212> DNA
<213> Homo sapiens

<400> 235
agagtatgct cagccac                                                    17

<210> 236
<211> 17
<212> DNA
<213> Homo sapiens

<400> 236
gagtatgctc agccact                                                    17

<210> 237
<211> 17
<212> DNA
<213> Homo sapiens

<400> 237
agtatgctca gccactg                                                    17

<210> 238
<211> 17
<212> DNA
<213> Homo sapiens

<400> 238
gtatgctcag ccactgg                                                    17

<210> 239
<211> 17
<212> DNA
<213> Homo sapiens

<400> 239
tatgctcagc cactggt                                                    17

<210> 240
<211> 17
<212> DNA
<213> Homo sapiens

<400> 240
atgctcagcc actggta                                                    17

<210> 241
<211> 17
<212> DNA
<213> Homo sapiens

<400> 241
tgctcagcca ctggtag                                                    17

<210> 242
<211> 17
<212> DNA
<213> Homo sapiens

<400> 242
gctcagccac tggtagg                                                    17

<210> 243
<211> 17
<212> DNA
<213> Homo sapiens

<400> 243
ctcagccact ggtagga                                                    17

<210> 244
<211> 17
<212> DNA
<213> Homo sapiens

<400> 244
tcagccactg gtaggag                                                    17

<210> 245
<211> 17
<212> DNA
<213> Homo sapiens

<400> 245
cagccactgg taggagg                                                    17

<210> 246
<211> 17
<212> DNA
<213> Homo sapiens

<400> 246
agccactggt aggagga                                                    17

<210> 247
<211> 17
<212> DNA
<213> Homo sapiens

<400> 247
gccactggta ggaggaa                                                    17

<210> 248

<211> 17
<212> DNA
<213> Homo sapiens

<400> 248
ccactggtag gaggaat                                                    17

<210> 249
<211> 17
<212> DNA
<213> Homo sapiens

<400> 249
cactggtagg aggaatt                                                    17

<210> 250
<211> 17
<212> DNA
<213> Homo sapiens

<400> 250
actggtagga ggaattg                                                    17

<210> 251
<211> 17
<212> DNA
<213> Homo sapiens

<400> 251
ctggtaggag gaattgt                                                    17

<210> 252
<211> 17
<212> DNA
<213> Homo sapiens

<400> 252
tggtaggagg aattgtt                                                    17

<210> 253
<211> 17
<212> DNA
<213> Homo sapiens

<400> 253
ggtaggagga attgttg                                                    17

<210> 254
<211> 17
<212> DNA
<213> Homo sapiens

<400> 254
gtaggaggaa ttgttgg                                                    17

<210> 255
<211> 17
<212> DNA
<213> Homo sapiens

<400> 255

taggaggaat tgttggt                                                    17

<210> 256
<211> 17
<212> DNA
<213> Homo sapiens

<400> 256
aggaggaatt gttggta                                                    17

<210> 257
<211> 17
<212> DNA
<213> Homo sapiens

<400> 257
ggaggaattg ttggtac                                                    17

<210> 258
<211> 17
<212> DNA
<213> Homo sapiens

<400> 258
gaggaattgt tggtaca                                                    17

<210> 259
<211> 17
<212> DNA
<213> Homo sapiens

<400> 259
aggaattgtt ggtacac                                                    17

<210> 260
<211> 17
<212> DNA
<213> Homo sapiens

<400> 260
ggaattgttg gtacact                                                    17

<210> 261
<211> 17
<212> DNA
<213> Homo sapiens

<400> 261
gaattgttgg tacactt                                                    17

<210> 262
<211> 17
<212> DNA
<213> Homo sapiens

<400> 262
aattgttggt acacttc                                                    17

<210> 263
<211> 17
<212> DNA

<213> Homo sapiens

<400> 263
attgttggta cacttca                                                    17

<210> 264
<211> 17
<212> DNA
<213> Homo sapiens

<400> 264
ttgttggtac acttcat                                                    17

<210> 265
<211> 17
<212> DNA
<213> Homo sapiens

<400> 265
tgttggtaca cttcatc                                                    17

<210> 266
<211> 17
<212> DNA
<213> Homo sapiens

<400> 266
gttggtacac ttcatca                                                    17

<210> 267
<211> 17
<212> DNA
<213> Homo sapiens

<400> 267
ttggtacact tcatcaa                                                    17

<210> 268
<211> 17
<212> DNA
<213> Homo sapiens

<400> 268
tggtacactt catcaaa                                                    17

<210> 269
<211> 17
<212> DNA
<213> Homo sapiens

<400> 269
ggtacacttc atcaaag                                                    17

<210> 270
<211> 17
<212> DNA
<213> Homo sapiens

<400> 270
gtacacttca tcaaaga                                                    17

<210> 271
<211> 17
<212> DNA
<213> Homo sapiens

<400> 271
tacacttcat caaagat                                                    17

<210> 272
<211> 17
<212> DNA
<213> Homo sapiens

<400> 272
acacttcatc aaagatc                                                    17

<210> 273
<211> 17
<212> DNA
<213> Homo sapiens

<400> 273
cacttcatca aagatct                                                    17

<210> 274
<211> 17
<212> DNA
<213> Homo sapiens

<400> 274
acttcatcaa agatcta                                                    17

<210> 275
<211> 17
<212> DNA
<213> Homo sapiens

<400> 275
cttcatcaaa gatctac                                                    17

<210> 276
<211> 17
<212> DNA
<213> Homo sapiens

<400> 276
ttcatcaaag atctacc                                                    17

<210> 277
<211> 17
<212> DNA
<213> Homo sapiens

<400> 277
tcatcaaaga tctacct                                                    17

<210> 278
<211> 17
<212> DNA
<213> Homo sapiens

<400> 278
catcaaagat ctacctt                                                    17

<210> 279
<211> 17
<212> DNA
<213> Homo sapiens

<400> 279
atcaaagatc taccttt                                                    17

<210> 280
<211> 17
<212> DNA
<213> Homo sapiens

<400> 280
tcaaagatct accttta                                                    17

<210> 281
<211> 17
<212> DNA
<213> Homo sapiens

<400> 281
caaagatcta cctttaa                                                    17

<210> 282
<211> 17
<212> DNA
<213> Homo sapiens

<400> 282
aaagatctac ctttaaa                                                    17

<210> 283
<211> 17
<212> DNA
<213> Homo sapiens

<400> 283
aagatctacc tttaaac                                                    17

<210> 284
<211> 17
<212> DNA
<213> Homo sapiens

<400> 284
agatctacct ttaaacc                                                    17

<210> 285
<211> 17
<212> DNA
<213> Homo sapiens

<400> 285
gatctacctt taaacca                                                    17

<210> 286
<211> 17

<212> DNA
<213> Homo sapiens

<400> 286
atctaccttt aaaccag                                                    17

<210> 287
<211> 17
<212> DNA
<213> Homo sapiens

<400> 287
tctaccttta aaccaga                                                    17

<210> 288
<211> 17
<212> DNA
<213> Homo sapiens

<400> 288
ctacctttaa accagaa                                                    17

<210> 289
<211> 17
<212> DNA
<213> Homo sapiens

<400> 289
tacctttaaa ccagaag                                                    17

<210> 290
<211> 17
<212> DNA
<213> Homo sapiens

<400> 290
acctttaaac cagaaga                                                    17

<210> 291
<211> 17
<212> DNA
<213> Homo sapiens

<400> 291
cctttaaacc agaagaa                                                    17

<210> 292
<211> 17
<212> DNA
<213> Homo sapiens

<400> 292
ctttaaacca gaagaag                                                    17

<210> 293
<211> 17
<212> DNA
<213> Homo sapiens

<400> 293
tttaaaccag aagaagg                                                    17

<210> 294
<211> 17
<212> DNA
<213> Homo sapiens

<400> 294
ttaaaccaga agaagga                                                    17

<210> 295
<211> 17
<212> DNA
<213> Homo sapiens

<400> 295
taaaccagaa gaaggaa                                                    17

<210> 296
<211> 17
<212> DNA
<213> Homo sapiens

<400> 296
aaaccagaag aaggaaa                                                    17

<210> 297
<211> 17
<212> DNA
<213> Homo sapiens

<400> 297
aaccagaaga aggaaaa                                                    17

<210> 298
<211> 17
<212> DNA
<213> Homo sapiens

<400> 298
accagaagaa ggaaaag                                                    17

<210> 299
<211> 17
<212> DNA
<213> Homo sapiens

<400> 299
ccagaagaag gaaaaga                                                    17

<210> 300
<211> 17
<212> DNA
<213> Homo sapiens

<400> 300
cagaagaagg aaaagaa                                                    17

<210> 301
<211> 17
<212> DNA
<213> Homo sapiens

<400> 301
agaagaagga aaagaag                                                    17

<210> 302
<211> 17
<212> DNA
<213> Homo sapiens

<400> 302
gaagaaggaa aagaagc                                                    17

<210> 303
<211> 17
<212> DNA
<213> Homo sapiens

<400> 303
aagaaggaaa agaagca                                                    17

<210> 304
<211> 17
<212> DNA
<213> Homo sapiens

<400> 304
agaaggaaaa gaagcag                                                    17

<210> 305
<211> 17
<212> DNA
<213> Homo sapiens

<400> 305
gaaggaaaag aagcagg                                                    17

<210> 306
<211> 17
<212> DNA
<213> Homo sapiens

<400> 306
aaggaaaaga agcaggc                                                    17

<210> 307
<211> 17
<212> DNA
<213> Homo sapiens

<400> 307
aggaaaagaa gcaggct                                                    17

<210> 308
<211> 17
<212> DNA
<213> Homo sapiens

<400> 308
ggaaaagaag caggcta                                                    17

<210> 309

<211> 17
<212> DNA
<213> Homo sapiens

<400> 309
gaaaagaagc aggctat                                                    17

<210> 310
<211> 17
<212> DNA
<213> Homo sapiens

<400> 310
aaaagaagca ggctatg                                                    17

<210> 311
<211> 17
<212> DNA
<213> Homo sapiens

<400> 311
aaagaagcag gctatgc                                                    17

<210> 312
<211> 17
<212> DNA
<213> Homo sapiens

<400> 312
aagaagcagg ctatgca                                                    17

<210> 313
<211> 17
<212> DNA
<213> Homo sapiens

<400> 313
agaagcaggc tatgcag                                                    17

<210> 314
<211> 17
<212> DNA
<213> Homo sapiens

<400> 314
gaagcaggct atgcaga                                                    17

<210> 315
<211> 17
<212> DNA
<213> Homo sapiens

<400> 315
aagcaggcta tgcagac                                                    17

<210> 316
<211> 17
<212> DNA
<213> Homo sapiens

<400> 316

agcaggctat gcagacc                                                    17

<210> 317
<211> 17
<212> DNA
<213> Homo sapiens

<400> 317
gcaggctatg cagacct                                                    17

<210> 318
<211> 17
<212> DNA
<213> Homo sapiens

<400> 318
caggctatgc agaccta                                                    17

<210> 319
<211> 17
<212> DNA
<213> Homo sapiens

<400> 319
aggctatgca gacctag                                                    17

<210> 320
<211> 17
<212> DNA
<213> Homo sapiens

<400> 320
ggctatgcag acctaga                                                    17

<210> 321
<211> 17
<212> DNA
<213> Homo sapiens

<400> 321
gctatgcaga cctagat                                                    17

<210> 322
<211> 17
<212> DNA
<213> Homo sapiens

<400> 322
ctatgcagac ctagatc                                                    17

<210> 323
<211> 17
<212> DNA
<213> Homo sapiens

<400> 323
tatgcagacc tagatcc                                                    17

<210> 324
<211> 17
<212> DNA

<213> Homo sapiens

<400> 324
atgcagacct agatcct                                                    17

<210> 325
<211> 17
<212> DNA
<213> Homo sapiens

<400> 325
tgcagaccta gatcctt                                                    17

<210> 326
<211> 17
<212> DNA
<213> Homo sapiens

<400> 326
gcagacctag atcctta                                                    17

<210> 327
<211> 25
<212> DNA
<213> Homo sapiens

<400> 327
aaacagaaag aaaaaaactg aaggc                                           25

<210> 328
<211> 25
<212> DNA
<213> Homo sapiens

<400> 328
aacagaaaga aaaaaactga aggca                                           25

<210> 329
<211> 25
<212> DNA
<213> Homo sapiens

<400> 329
acagaaagaa aaaaactgaa ggcac                                           25

<210> 330
<211> 25
<212> DNA
<213> Homo sapiens

<400> 330
cagaaagaaa aaaactgaag gcacc                                           25

<210> 331
<211> 25
<212> DNA
<213> Homo sapiens

<400> 331
agaaagaaaa aaactgaagg cacct                                           25

<210> 332
<211> 25
<212> DNA
<213> Homo sapiens

<400> 332
gaaagaaaaa aactgaaggc accta                                            25

<210> 333
<211> 25
<212> DNA
<213> Homo sapiens

<400> 333
aaagaaaaaa actgaaggca cctat                                            25

<210> 334
<211> 25
<212> DNA
<213> Homo sapiens

<400> 334
aagaaaaaaa ctgaaggcac ctatg                                            25

<210> 335
<211> 25
<212> DNA
<213> Homo sapiens

<400> 335
agaaaaaaac tgaaggcacc tatga                                            25

<210> 336
<211> 25
<212> DNA
<213> Homo sapiens

<400> 336
gaaaaaaact gaaggcacct atgac                                            25

<210> 337
<211> 25
<212> DNA
<213> Homo sapiens

<400> 337
aaaaaaactg aaggcaccta tgact                                            25

<210> 338
<211> 25
<212> DNA
<213> Homo sapiens

<400> 338
aaaaaactga aggcacctat gactt                                            25

<210> 339
<211> 25
<212> DNA
<213> Homo sapiens

<400> 339
aaaaactgaa ggcacctatg actta                                          25

<210> 340
<211> 25
<212> DNA
<213> Homo sapiens

<400> 340
aaaactgaag gcacctatga cttac                                          25

<210> 341
<211> 25
<212> DNA
<213> Homo sapiens

<400> 341
aaactgaagg cacctatgac ttacc                                          25

<210> 342
<211> 25
<212> DNA
<213> Homo sapiens

<400> 342
aactgaaggc acctatgact tacct                                          25

<210> 343
<211> 25
<212> DNA
<213> Homo sapiens

<400> 343
actgaaggca cctatgactt acctt                                          25

<210> 344
<211> 25
<212> DNA
<213> Homo sapiens

<400> 344
ctgaaggcac ctatgactta cctta                                          25

<210> 345
<211> 25
<212> DNA
<213> Homo sapiens

<400> 345
tgaaggcacc tatgacttac cttac                                          25

<210> 346
<211> 25
<212> DNA
<213> Homo sapiens

<400> 346
gaaggcacct atgacttacc ttact                                          25

<210> 347
<211> 25

<212> DNA
<213> Homo sapiens

<400> 347
aaggcaccta tgacttacct tactg                                            25

<210> 348
<211> 25
<212> DNA
<213> Homo sapiens

<400> 348
aggcacctat gacttacctt actgg                                            25

<210> 349
<211> 25
<212> DNA
<213> Homo sapiens

<400> 349
ggcacctatg acttacctta ctggg                                            25

<210> 350
<211> 25
<212> DNA
<213> Homo sapiens

<400> 350
gcacctatga cttaccttac tggga                                            25

<210> 351
<211> 25
<212> DNA
<213> Homo sapiens

<400> 351
cacctatgac ttaccttact gggac                                            25

<210> 352
<211> 25
<212> DNA
<213> Homo sapiens

<400> 352
acctatgact taccttactg ggacc                                            25

<210> 353
<211> 25
<212> DNA
<213> Homo sapiens

<400> 353
cctatgactt accttactgg gaccg                                            25

<210> 354
<211> 25
<212> DNA
<213> Homo sapiens

<400> 354
ctatgactta ccttactggg accgg                                            25

<210> 355
<211> 25
<212> DNA
<213> Homo sapiens

<400> 355
tatgacttac cttactggga ccggg                                          25

<210> 356
<211> 25
<212> DNA
<213> Homo sapiens

<400> 356
atgacttacc ttactgggac cgggc                                          25

<210> 357
<211> 25
<212> DNA
<213> Homo sapiens

<400> 357
tgacttacct tactgggacc gggca                                          25

<210> 358
<211> 25
<212> DNA
<213> Homo sapiens

<400> 358
gacttacctt actgggaccg ggcag                                          25

<210> 359
<211> 25
<212> DNA
<213> Homo sapiens

<400> 359
acttacctta ctgggaccgg gcagg                                          25

<210> 360
<211> 25
<212> DNA
<213> Homo sapiens

<400> 360
cttaccttac tgggaccggg caggt                                          25

<210> 361
<211> 25
<212> DNA
<213> Homo sapiens

<400> 361
ttaccttact gggaccgggc aggtt                                          25

<210> 362
<211> 25
<212> DNA
<213> Homo sapiens

<400> 362
taccttactg ggaccgggca ggttg                                          25

<210> 363
<211> 25
<212> DNA
<213> Homo sapiens

<400> 363
accttactgg gaccgggcag gttgg                                          25

<210> 364
<211> 25
<212> DNA
<213> Homo sapiens

<400> 364
ccttactggg accgggcagg ttggt                                          25

<210> 365
<211> 25
<212> DNA
<213> Homo sapiens

<400> 365
cttactggga ccgggcaggt tggtg                                          25

<210> 366
<211> 25
<212> DNA
<213> Homo sapiens

<400> 366
ttactgggac cgggcaggtt ggtgg                                          25

<210> 367
<211> 25
<212> DNA
<213> Homo sapiens

<400> 367
tactgggacc gggcaggttg gtgga                                          25

<210> 368
<211> 25
<212> DNA
<213> Homo sapiens

<400> 368
actgggaccg ggcaggttgg tggaa                                          25

<210> 369
<211> 25
<212> DNA
<213> Homo sapiens

<400> 369
ctgggaccgg gcaggttggt ggaaa                                          25

<210> 370

<211> 25
<212> DNA
<213> Homo sapiens

<400> 370
tgggaccggg caggttggtg gaaag                                            25

<210> 371
<211> 25
<212> DNA
<213> Homo sapiens

<400> 371
gggaccgggc aggttggtgg aaagg                                            25

<210> 372
<211> 25
<212> DNA
<213> Homo sapiens

<400> 372
ggaccgggca ggttggtgga aagga                                            25

<210> 373
<211> 25
<212> DNA
<213> Homo sapiens

<400> 373
gaccgggcag gttggtggaa aggaa                                            25

<210> 374
<211> 25
<212> DNA
<213> Homo sapiens

<400> 374
accgggcagg ttggtggaaa ggaat                                            25

<210> 375
<211> 25
<212> DNA
<213> Homo sapiens

<400> 375
ccgggcaggt tggtggaaag gaatg                                            25

<210> 376
<211> 25
<212> DNA
<213> Homo sapiens

<400> 376
cgggcaggtt ggtggaaagg aatga                                            25

<210> 377
<211> 25
<212> DNA
<213> Homo sapiens

<400> 377

gggcaggttg gtggaaagga atgaa 25

<210> 378
<211> 25
<212> DNA
<213> Homo sapiens

<400> 378
ggcaggttgg tggaaaggaa tgaag 25

<210> 379
<211> 25
<212> DNA
<213> Homo sapiens

<400> 379
gcaggttggt ggaaaggaat gaagc 25

<210> 380
<211> 25
<212> DNA
<213> Homo sapiens

<400> 380
caggttggtg gaaaggaatg aagca 25

<210> 381
<211> 25
<212> DNA
<213> Homo sapiens

<400> 381
aggttggtgg aaaggaatga agcag 25

<210> 382
<211> 25
<212> DNA
<213> Homo sapiens

<400> 382
ggttggtgga aaggaatgaa gcagt 25

<210> 383
<211> 25
<212> DNA
<213> Homo sapiens

<400> 383
gttggtggaa aggaatgaag cagtt 25

<210> 384
<211> 25
<212> DNA
<213> Homo sapiens

<400> 384
ttggtggaaa ggaatgaagc agttt 25

<210> 385
<211> 25
<212> DNA

<213> Homo sapiens

<400> 385
tggtggaaag gaatgaagca gtttc                                          25

<210> 386
<211> 25
<212> DNA
<213> Homo sapiens

<400> 386
ggtggaaagg aatgaagcag tttct                                          25

<210> 387
<211> 25
<212> DNA
<213> Homo sapiens

<400> 387
gtggaaagga atgaagcagt ttctt                                          25

<210> 388
<211> 25
<212> DNA
<213> Homo sapiens

<400> 388
tggaaaggaa tgaagcagtt tcttc                                          25

<210> 389
<211> 25
<212> DNA
<213> Homo sapiens

<400> 389
ggaaaggaat gaagcagttt cttcc                                          25

<210> 390
<211> 25
<212> DNA
<213> Homo sapiens

<400> 390
gaaaggaatg aagcagtttc ttcct                                          25

<210> 391
<211> 25
<212> DNA
<213> Homo sapiens

<400> 391
aaaggaatga agcagtttct tcctg                                          25

<210> 392
<211> 25
<212> DNA
<213> Homo sapiens

<400> 392
aaggaatgaa gcagtttctt cctgc                                          25

<210> 393
<211> 25
<212> DNA
<213> Homo sapiens

<400> 393
aggaatgaag cagtttcttc ctgca                                            25

<210> 394
<211> 25
<212> DNA
<213> Homo sapiens

<400> 394
ggaatgaagc agtttcttcc tgcaa                                            25

<210> 395
<211> 25
<212> DNA
<213> Homo sapiens

<400> 395
gaatgaagca gtttcttcct gcaaa                                            25

<210> 396
<211> 25
<212> DNA
<213> Homo sapiens

<400> 396
aatgaagcag tttcttcctg caaaa                                            25

<210> 397
<211> 25
<212> DNA
<213> Homo sapiens

<400> 397
atgaagcagt ttcttcctgc aaaag                                            25

<210> 398
<211> 25
<212> DNA
<213> Homo sapiens

<400> 398
tgaagcagtt tcttcctgca aaagc                                            25

<210> 399
<211> 25
<212> DNA
<213> Homo sapiens

<400> 399
gaagcagttt cttcctgcaa aagca                                            25

<210> 400
<211> 25
<212> DNA
<213> Homo sapiens

<400> 400
aagcagtttc ttcctgcaaa agcag                                          25

<210> 401
<211> 25
<212> DNA
<213> Homo sapiens

<400> 401
agcagtttct tcctgcaaaa gcagt                                          25

<210> 402
<211> 25
<212> DNA
<213> Homo sapiens

<400> 402
gcagtttctt cctgcaaaag cagtg                                          25

<210> 403
<211> 25
<212> DNA
<213> Homo sapiens

<400> 403
cagtttcttc ctgcaaaagc agtgg                                          25

<210> 404
<211> 25
<212> DNA
<213> Homo sapiens

<400> 404
agtttcttcc tgcaaaagca gtgga                                          25

<210> 405
<211> 25
<212> DNA
<213> Homo sapiens

<400> 405
gtttcttcct gcaaaagcag tggac                                          25

<210> 406
<211> 25
<212> DNA
<213> Homo sapiens

<400> 406
tttcttcctg caaaagcagt ggacc                                          25

<210> 407
<211> 25
<212> DNA
<213> Homo sapiens

<400> 407
ttcttcctgc aaaagcagtg gacca                                          25

<210> 408
<211> 25

<212> DNA
<213> Homo sapiens

<400> 408
tcttcctgca aaagcagtgg accat                                            25

<210> 409
<211> 25
<212> DNA
<213> Homo sapiens

<400> 409
cttcctgcaa aagcagtgga ccatg                                            25

<210> 410
<211> 25
<212> DNA
<213> Homo sapiens

<400> 410
ttcctgcaaa agcagtggac catga                                            25

<210> 411
<211> 25
<212> DNA
<213> Homo sapiens

<400> 411
tcctgcaaaa gcagtggacc atgag                                            25

<210> 412
<211> 25
<212> DNA
<213> Homo sapiens

<400> 412
cctgcaaaag cagtggacca tgagg                                            25

<210> 413
<211> 25
<212> DNA
<213> Homo sapiens

<400> 413
ctgcaaaagc agtggaccat gagga                                            25

<210> 414
<211> 25
<212> DNA
<213> Homo sapiens

<400> 414
tgcaaaagca gtggaccatg aggaa                                            25

<210> 415
<211> 25
<212> DNA
<213> Homo sapiens

<400> 415
gcaaaagcag tggaccatga ggaaa                                            25

<210> 416
<211> 25
<212> DNA
<213> Homo sapiens

<400> 416
caaaagcagt ggaccatgag gaaac                                          25

<210> 417
<211> 25
<212> DNA
<213> Homo sapiens

<400> 417
aaaagcagtg gaccatgagg aaacc                                          25

<210> 418
<211> 25
<212> DNA
<213> Homo sapiens

<400> 418
aaagcagtgg accatgagga aaccc                                          25

<210> 419
<211> 25
<212> DNA
<213> Homo sapiens

<400> 419
aagcagtgga ccatgaggaa acccc                                          25

<210> 420
<211> 25
<212> DNA
<213> Homo sapiens

<400> 420
agcagtggac catgaggaaa cccca                                          25

<210> 421
<211> 25
<212> DNA
<213> Homo sapiens

<400> 421
gcagtggacc atgaggaaac cccag                                          25

<210> 422
<211> 25
<212> DNA
<213> Homo sapiens

<400> 422
cagtggacca tgaggaaacc ccagt                                          25

<210> 423
<211> 25
<212> DNA
<213> Homo sapiens

<400> 423
agtggaccat gaggaaaccc cagtt                                          25

<210> 424
<211> 25
<212> DNA
<213> Homo sapiens

<400> 424
gtggaccatg aggaaacccc agttc                                          25

<210> 425
<211> 25
<212> DNA
<213> Homo sapiens

<400> 425
tggaccatga ggaaacccca gttcg                                          25

<210> 426
<211> 25
<212> DNA
<213> Homo sapiens

<400> 426
ggaccatgag gaaaccccag ttcgc                                          25

<210> 427
<211> 25
<212> DNA
<213> Homo sapiens

<400> 427
gaccatgagg aaaccccagt tcgct                                          25

<210> 428
<211> 25
<212> DNA
<213> Homo sapiens

<400> 428
accatgagga aaccccagtt cgcta                                          25

<210> 429
<211> 25
<212> DNA
<213> Homo sapiens

<400> 429
ccatgaggaa accccagttc gctat                                          25

<210> 430
<211> 25
<212> DNA
<213> Homo sapiens

<400> 430
catgaggaaa ccccagttcg ctata                                          25

<210> 431

<211> 25
<212> DNA
<213> Homo sapiens

<400> 431
atgaggaaac cccagttcgc tatag                                             25

<210> 432
<211> 25
<212> DNA
<213> Homo sapiens

<400> 432
tgaggaaacc ccagttcgct atagc                                             25

<210> 433
<211> 25
<212> DNA
<213> Homo sapiens

<400> 433
gaggaaaccc cagttcgcta tagca                                             25

<210> 434
<211> 25
<212> DNA
<213> Homo sapiens

<400> 434
aggaaacccc agttcgctat agcag                                             25

<210> 435
<211> 25
<212> DNA
<213> Homo sapiens

<400> 435
ggaaacccca gttcgctata gcagc                                             25

<210> 436
<211> 25
<212> DNA
<213> Homo sapiens

<400> 436
gaaaccccag ttcgctatag cagca                                             25

<210> 437
<211> 25
<212> DNA
<213> Homo sapiens

<400> 437
aaaccccagt tcgctatagc agcag                                             25

<210> 438
<211> 25
<212> DNA
<213> Homo sapiens

<400> 438

aaccccagtt cgctatagca gcagc 25

<210> 439
<211> 25
<212> DNA
<213> Homo sapiens

<400> 439
accccagttc gctatagcag cagcg 25

<210> 440
<211> 25
<212> DNA
<213> Homo sapiens

<400> 440
ccccagttcg ctatagcagc agcga 25

<210> 441
<211> 25
<212> DNA
<213> Homo sapiens

<400> 441
cccagttcgc tatagcagca gcgaa 25

<210> 442
<211> 25
<212> DNA
<213> Homo sapiens

<400> 442
ccagttcgct atagcagcag cgaag 25

<210> 443
<211> 25
<212> DNA
<213> Homo sapiens

<400> 443
cagttcgcta tagcagcagc gaagt 25

<210> 444
<211> 25
<212> DNA
<213> Homo sapiens

<400> 444
agttcgctat agcagcagcg aagtt 25

<210> 445
<211> 25
<212> DNA
<213> Homo sapiens

<400> 445
gttcgctata gcagcagcga agtta 25

<210> 446
<211> 25
<212> DNA

<213> Homo sapiens

<400> 446
ttcgctatag cagcagcgaa gttaa 25

<210> 447
<211> 25
<212> DNA
<213> Homo sapiens

<400> 447
tcgctatagc agcagcgaag ttaat 25

<210> 448
<211> 25
<212> DNA
<213> Homo sapiens

<400> 448
cgctatagca gcagcgaagt taatc 25

<210> 449
<211> 25
<212> DNA
<213> Homo sapiens

<400> 449
gctatagcag cagcgaagtt aatca 25

<210> 450
<211> 25
<212> DNA
<213> Homo sapiens

<400> 450
ctatagcagc agcgaagtta atcac 25

<210> 451
<211> 25
<212> DNA
<213> Homo sapiens

<400> 451
tatagcagca gcgaagttaa tcacc 25

<210> 452
<211> 25
<212> DNA
<213> Homo sapiens

<400> 452
atagcagcag cgaagttaat cacct 25

<210> 453
<211> 25
<212> DNA
<213> Homo sapiens

<400> 453
tagcagcagc gaagttaatc acctg 25

<210> 454
<211> 25
<212> DNA
<213> Homo sapiens

<400> 454
agcagcagcg aagttaatca cctga                                            25

<210> 455
<211> 25
<212> DNA
<213> Homo sapiens

<400> 455
gcagcagcga agttaatcac ctgag                                            25

<210> 456
<211> 25
<212> DNA
<213> Homo sapiens

<400> 456
cagcagcgaa gttaatcacc tgagt                                            25

<210> 457
<211> 25
<212> DNA
<213> Homo sapiens

<400> 457
agcagcgaag ttaatcacct gagtc                                            25

<210> 458
<211> 25
<212> DNA
<213> Homo sapiens

<400> 458
gcagcgaagt taatcacctg agtcc                                            25

<210> 459
<211> 25
<212> DNA
<213> Homo sapiens

<400> 459
cagcgaagtt aatcacctga gtcca                                            25

<210> 460
<211> 25
<212> DNA
<213> Homo sapiens

<400> 460
agcgaagtta atcacctgag tccaa                                            25

<210> 461
<211> 25
<212> DNA
<213> Homo sapiens

<400> 461
gcgaagttaa tcacctgagt ccaag                                          25

<210> 462
<211> 25
<212> DNA
<213> Homo sapiens

<400> 462
cgaagttaat cacctgagtc caaga                                          25

<210> 463
<211> 25
<212> DNA
<213> Homo sapiens

<400> 463
gaagttaatc acctgagtcc aagag                                          25

<210> 464
<211> 25
<212> DNA
<213> Homo sapiens

<400> 464
aagttaatca cctgagtcca agaga                                          25

<210> 465
<211> 25
<212> DNA
<213> Homo sapiens

<400> 465
agttaatcac ctgagtccaa gagaa                                          25

<210> 466
<211> 25
<212> DNA
<213> Homo sapiens

<400> 466
gttaatcacc tgagtccaag agaag                                          25

<210> 467
<211> 25
<212> DNA
<213> Homo sapiens

<400> 467
ttaatcacct gagtccaaga gaagt                                          25

<210> 468
<211> 25
<212> DNA
<213> Homo sapiens

<400> 468
taatcacctg agtccaagag aagtc                                          25

<210> 469
<211> 25

<212> DNA
<213> Homo sapiens

<400> 469
aatcacctga gtccaagaga agtca                                          25

<210> 470
<211> 25
<212> DNA
<213> Homo sapiens

<400> 470
atcacctgag tccaagagaa gtcac                                          25

<210> 471
<211> 25
<212> DNA
<213> Homo sapiens

<400> 471
tcacctgagt ccaagagaag tcacc                                          25

<210> 472
<211> 25
<212> DNA
<213> Homo sapiens

<400> 472
cacctgagtc caagagaagt cacca                                          25

<210> 473
<211> 25
<212> DNA
<213> Homo sapiens

<400> 473
acctgagtcc aagagaagtc accac                                          25

<210> 474
<211> 25
<212> DNA
<213> Homo sapiens

<400> 474
cctgagtcca agagaagtca ccaca                                          25

<210> 475
<211> 25
<212> DNA
<213> Homo sapiens

<400> 475
ctgagtccaa gagaagtcac cacag                                          25

<210> 476
<211> 25
<212> DNA
<213> Homo sapiens

<400> 476
tgagtccaag agaagtcacc acagt                                          25

<210> 477
<211> 25
<212> DNA
<213> Homo sapiens

<400> 477
gagtccaaga gaagtcacca cagtg                                          25

<210> 478
<211> 25
<212> DNA
<213> Homo sapiens

<400> 478
agtccaagag aagtcaccac agtgc                                          25

<210> 479
<211> 25
<212> DNA
<213> Homo sapiens

<400> 479
gtccaagaga agtcaccaca gtgct                                          25

<210> 480
<211> 25
<212> DNA
<213> Homo sapiens

<400> 480
tccaagagaa gtcaccacag tgctg                                          25

<210> 481
<211> 25
<212> DNA
<213> Homo sapiens

<400> 481
ccaagagaag tcaccacagt gctgc                                          25

<210> 482
<211> 25
<212> DNA
<213> Homo sapiens

<400> 482
caagagaagt caccacagtg ctgca                                          25

<210> 483
<211> 25
<212> DNA
<213> Homo sapiens

<400> 483
aagagaagtc accacagtgc tgcag                                          25

<210> 484
<211> 25
<212> DNA
<213> Homo sapiens

<400> 484
agagaagtca ccacagtgct gcagg                                          25

<210> 485
<211> 25
<212> DNA
<213> Homo sapiens

<400> 485
gagaagtcac cacagtgctg caggc                                          25

<210> 486
<211> 25
<212> DNA
<213> Homo sapiens

<400> 486
agaagtcacc acagtgctgc aggct                                          25

<210> 487
<211> 25
<212> DNA
<213> Homo sapiens

<400> 487
gaagtcacca cagtgctgca ggctg                                          25

<210> 488
<211> 25
<212> DNA
<213> Homo sapiens

<400> 488
aagtcaccac agtgctgcag gctga                                          25

<210> 489
<211> 25
<212> DNA
<213> Homo sapiens

<400> 489
agtcaccaca gtgctgcagg ctgac                                          25

<210> 490
<211> 25
<212> DNA
<213> Homo sapiens

<400> 490
gtcaccacag tgctgcaggc tgact                                          25

<210> 491
<211> 25
<212> DNA
<213> Homo sapiens

<400> 491
tcaccacagt gctgcaggct gactc                                          25

<210> 492

<211> 25
<212> DNA
<213> Homo sapiens

<400> 492
caccacagtg ctgcaggctg actct                                          25

<210> 493
<211> 25
<212> DNA
<213> Homo sapiens

<400> 493
accacagtgc tgcaggctga ctctg                                          25

<210> 494
<211> 25
<212> DNA
<213> Homo sapiens

<400> 494
ccacagtgct gcaggctgac tctgc                                          25

<210> 495
<211> 25
<212> DNA
<213> Homo sapiens

<400> 495
cacagtgctg caggctgact ctgca                                          25

<210> 496
<211> 25
<212> DNA
<213> Homo sapiens

<400> 496
acagtgctgc aggctgactc tgcag                                          25

<210> 497
<211> 25
<212> DNA
<213> Homo sapiens

<400> 497
cagtgctgca ggctgactct gcaga                                          25

<210> 498
<211> 25
<212> DNA
<213> Homo sapiens

<400> 498
agtgctgcag gctgactctg cagag                                          25

<210> 499
<211> 25
<212> DNA
<213> Homo sapiens

<400> 499

gtgctgcagg ctgactctgc agagt                                                25

<210> 500
<211> 25
<212> DNA
<213> Homo sapiens

<400> 500
tgctgcaggc tgactctgca gagta                                                25

<210> 501
<211> 25
<212> DNA
<213> Homo sapiens

<400> 501
gctgcaggct gactctgcag agtat                                                25

<210> 502
<211> 25
<212> DNA
<213> Homo sapiens

<400> 502
ctgcaggctg actctgcaga gtatg                                                25

<210> 503
<211> 25
<212> DNA
<213> Homo sapiens

<400> 503
tgcaggctga ctctgcagag tatgc                                                25

<210> 504
<211> 25
<212> DNA
<213> Homo sapiens

<400> 504
gcaggctgac tctgcagagt atgct                                                25

<210> 505
<211> 25
<212> DNA
<213> Homo sapiens

<400> 505
caggctgact ctgcagagta tgctc                                                25

<210> 506
<211> 25
<212> DNA
<213> Homo sapiens

<400> 506
aggctgactc tgcagagtat gctca                                                25

<210> 507
<211> 25
<212> DNA

<213> Homo sapiens

<400> 507
ggctgactct gcagagtatg ctcag                                          25

<210> 508
<211> 25
<212> DNA
<213> Homo sapiens

<400> 508
gctgactctg cagagtatgc tcagc                                          25

<210> 509
<211> 25
<212> DNA
<213> Homo sapiens

<400> 509
ctgactctgc agagtatgct cagcc                                          25

<210> 510
<211> 25
<212> DNA
<213> Homo sapiens

<400> 510
tgactctgca gagtatgctc agcca                                          25

<210> 511
<211> 25
<212> DNA
<213> Homo sapiens

<400> 511
gactctgcag agtatgctca gccac                                          25

<210> 512
<211> 25
<212> DNA
<213> Homo sapiens

<400> 512
actctgcaga gtatgctcag ccact                                          25

<210> 513
<211> 25
<212> DNA
<213> Homo sapiens

<400> 513
ctctgcagag tatgctcagc cactg                                          25

<210> 514
<211> 25
<212> DNA
<213> Homo sapiens

<400> 514
tctgcagagt atgctcagcc actgg                                          25

<210> 515
<211> 25
<212> DNA
<213> Homo sapiens

<400> 515
ctgcagagta tgctcagcca ctggt                                            25

<210> 516
<211> 25
<212> DNA
<213> Homo sapiens

<400> 516
tgcagagtat gctcagccac tggta                                            25

<210> 517
<211> 25
<212> DNA
<213> Homo sapiens

<400> 517
gcagagtatg ctcagccact ggtag                                            25

<210> 518
<211> 25
<212> DNA
<213> Homo sapiens

<400> 518
cagagtatgc tcagccactg gtagg                                            25

<210> 519
<211> 25
<212> DNA
<213> Homo sapiens

<400> 519
agagtatgct cagccactgg tagga                                            25

<210> 520
<211> 25
<212> DNA
<213> Homo sapiens

<400> 520
gagtatgctc agccactggt aggag                                            25

<210> 521
<211> 25
<212> DNA
<213> Homo sapiens

<400> 521
agtatgctca gccactggta ggagg                                            25

<210> 522
<211> 25
<212> DNA
<213> Homo sapiens

<400> 522
gtatgctcag ccactggtag gagga                                          25

<210> 523
<211> 25
<212> DNA
<213> Homo sapiens

<400> 523
tatgctcagc cactggtagg aggaa                                          25

<210> 524
<211> 25
<212> DNA
<213> Homo sapiens

<400> 524
atgctcagcc actggtagga ggaat                                          25

<210> 525
<211> 25
<212> DNA
<213> Homo sapiens

<400> 525
tgctcagcca ctggtaggag gaatt                                          25

<210> 526
<211> 25
<212> DNA
<213> Homo sapiens

<400> 526
gctcagccac tggtaggagg aattg                                          25

<210> 527
<211> 25
<212> DNA
<213> Homo sapiens

<400> 527
ctcagccact ggtaggagga attgt                                          25

<210> 528
<211> 25
<212> DNA
<213> Homo sapiens

<400> 528
tcagccactg gtaggaggaa ttgtt                                          25

<210> 529
<211> 25
<212> DNA
<213> Homo sapiens

<400> 529
cagccactgg taggaggaat tgttg                                          25

<210> 530
<211> 25

<212> DNA
<213> Homo sapiens

<400> 530
agccactggt aggaggaatt gttgg                                            25

<210> 531
<211> 25
<212> DNA
<213> Homo sapiens

<400> 531
gccactggta ggaggaattg ttggt                                            25

<210> 532
<211> 25
<212> DNA
<213> Homo sapiens

<400> 532
ccactggtag gaggaattgt tggta                                            25

<210> 533
<211> 25
<212> DNA
<213> Homo sapiens

<400> 533
cactggtagg aggaattgtt ggtac                                            25

<210> 534
<211> 25
<212> DNA
<213> Homo sapiens

<400> 534
actggtagga ggaattgttg gtaca                                            25

<210> 535
<211> 25
<212> DNA
<213> Homo sapiens

<400> 535
ctggtaggag gaattgttgg tacac                                            25

<210> 536
<211> 25
<212> DNA
<213> Homo sapiens

<400> 536
tggtaggagg aattgttggt acact                                            25

<210> 537
<211> 25
<212> DNA
<213> Homo sapiens

<400> 537
ggtaggagga attgttggta cactt                                            25

<210> 538
<211> 25
<212> DNA
<213> Homo sapiens

<400> 538
gtaggaggaa ttgttggtac acttc                                          25

<210> 539
<211> 25
<212> DNA
<213> Homo sapiens

<400> 539
taggaggaat tgttggtaca cttca                                          25

<210> 540
<211> 25
<212> DNA
<213> Homo sapiens

<400> 540
aggaggaatt gttggtacac ttcat                                          25

<210> 541
<211> 25
<212> DNA
<213> Homo sapiens

<400> 541
ggaggaattg ttggtacact tcatc                                          25

<210> 542
<211> 25
<212> DNA
<213> Homo sapiens

<400> 542
gaggaattgt tggtacactt catca                                          25

<210> 543
<211> 25
<212> DNA
<213> Homo sapiens

<400> 543
aggaattgtt ggtacacttc atcaa                                          25

<210> 544
<211> 25
<212> DNA
<213> Homo sapiens

<400> 544
ggaattgttg gtacacttca tcaaa                                          25

<210> 545
<211> 25
<212> DNA
<213> Homo sapiens

<400> 545
gaattgttgg tacacttcat caaag                                          25

<210> 546
<211> 25
<212> DNA
<213> Homo sapiens

<400> 546
aattgttggt acacttcatc aaaga                                          25

<210> 547
<211> 25
<212> DNA
<213> Homo sapiens

<400> 547
attgttggta cacttcatca aagat                                          25

<210> 548
<211> 25
<212> DNA
<213> Homo sapiens

<400> 548
ttgttggtac acttcatcaa agatc                                          25

<210> 549
<211> 25
<212> DNA
<213> Homo sapiens

<400> 549
tgttggtaca cttcatcaaa gatct                                          25

<210> 550
<211> 25
<212> DNA
<213> Homo sapiens

<400> 550
gttggtacac ttcatcaaag atcta                                          25

<210> 551
<211> 25
<212> DNA
<213> Homo sapiens

<400> 551
ttggtacact tcatcaaaga tctac                                          25

<210> 552
<211> 25
<212> DNA
<213> Homo sapiens

<400> 552
tggtacactt catcaaagat ctacc                                          25

<210> 553

<211> 25
<212> DNA
<213> Homo sapiens

<400> 553
ggtacacttc atcaaagatc tacct                                          25

<210> 554
<211> 25
<212> DNA
<213> Homo sapiens

<400> 554
gtacacttca tcaaagatct acctt                                          25

<210> 555
<211> 25
<212> DNA
<213> Homo sapiens

<400> 555
tacacttcat caaagatcta ccttt                                          25

<210> 556
<211> 25
<212> DNA
<213> Homo sapiens

<400> 556
acacttcatc aaagatctac cttta                                          25

<210> 557
<211> 25
<212> DNA
<213> Homo sapiens

<400> 557
cacttcatca aagatctacc tttaa                                          25

<210> 558
<211> 25
<212> DNA
<213> Homo sapiens

<400> 558
acttcatcaa agatctacct ttaaa                                          25

<210> 559
<211> 25
<212> DNA
<213> Homo sapiens

<400> 559
cttcatcaaa gatctacctt taaac                                          25

<210> 560
<211> 25
<212> DNA
<213> Homo sapiens

<400> 560

ttcatcaaag atctaccttt aaacc                                                25

<210> 561
<211> 25
<212> DNA
<213> Homo sapiens

<400> 561
tcatcaaaga tctaccttta aacca                                                25

<210> 562
<211> 25
<212> DNA
<213> Homo sapiens

<400> 562
catcaaagat ctacctttaa accag                                                25

<210> 563
<211> 25
<212> DNA
<213> Homo sapiens

<400> 563
atcaaagatc tacctttaaa ccaga                                                25

<210> 564
<211> 25
<212> DNA
<213> Homo sapiens

<400> 564
tcaaagatct acctttaaac cagaa                                                25

<210> 565
<211> 25
<212> DNA
<213> Homo sapiens

<400> 565
caaagatcta cctttaaacc agaag                                                25

<210> 566
<211> 25
<212> DNA
<213> Homo sapiens

<400> 566
aaagatctac ctttaaacca gaaga                                                25

<210> 567
<211> 25
<212> DNA
<213> Homo sapiens

<400> 567
aagatctacc tttaaaccag aagaa                                                25

<210> 568
<211> 25
<212> DNA

<213> Homo sapiens

<400> 568
agatctacct ttaaaccaga agaag                                          25

<210> 569
<211> 25
<212> DNA
<213> Homo sapiens

<400> 569
gatctacctt taaaccagaa gaagg                                          25

<210> 570
<211> 25
<212> DNA
<213> Homo sapiens

<400> 570
atctaccttt aaaccagaag aagga                                          25

<210> 571
<211> 25
<212> DNA
<213> Homo sapiens

<400> 571
tctaccttta aaccagaaga aggaa                                          25

<210> 572
<211> 25
<212> DNA
<213> Homo sapiens

<400> 572
ctacctttaa accagaagaa ggaaa                                          25

<210> 573
<211> 25
<212> DNA
<213> Homo sapiens

<400> 573
tacctttaaa ccagaagaag gaaaa                                          25

<210> 574
<211> 25
<212> DNA
<213> Homo sapiens

<400> 574
acctttaaac cagaagaagg aaaag                                          25

<210> 575
<211> 25
<212> DNA
<213> Homo sapiens

<400> 575
cctttaaacc agaagaagga aaaga                                          25

<210> 576
<211> 25
<212> DNA
<213> Homo sapiens

<400> 576
ctttaaacca gaagaaggaa aagaa                                          25

<210> 577
<211> 25
<212> DNA
<213> Homo sapiens

<400> 577
tttaaaccag aagaaggaaa agaag                                          25

<210> 578
<211> 25
<212> DNA
<213> Homo sapiens

<400> 578
ttaaaccaga agaaggaaaa gaagc                                          25

<210> 579
<211> 25
<212> DNA
<213> Homo sapiens

<400> 579
taaaccagaa gaaggaaaag aagca                                          25

<210> 580
<211> 25
<212> DNA
<213> Homo sapiens

<400> 580
aaaccagaag aaggaaaaga agcag                                          25

<210> 581
<211> 25
<212> DNA
<213> Homo sapiens

<400> 581
aaccagaaga aggaaaagaa gcagg                                          25

<210> 582
<211> 25
<212> DNA
<213> Homo sapiens

<400> 582
accagaagaa ggaaaagaag caggc                                          25

<210> 583
<211> 25
<212> DNA
<213> Homo sapiens

<400> 583
ccagaagaag gaaaagaagc aggct                                          25

<210> 584
<211> 25
<212> DNA
<213> Homo sapiens

<400> 584
cagaagaagg aaaagaagca ggcta                                          25

<210> 585
<211> 25
<212> DNA
<213> Homo sapiens

<400> 585
agaagaagga aaagaagcag gctat                                          25

<210> 586
<211> 25
<212> DNA
<213> Homo sapiens

<400> 586
gaagaaggaa aagaagcagg ctatg                                          25

<210> 587
<211> 25
<212> DNA
<213> Homo sapiens

<400> 587
aagaaggaaa agaagcaggc tatgc                                          25

<210> 588
<211> 25
<212> DNA
<213> Homo sapiens

<400> 588
agaaggaaaa gaagcaggct atgca                                          25

<210> 589
<211> 25
<212> DNA
<213> Homo sapiens

<400> 589
gaaggaaaag aagcaggcta tgcag                                          25

<210> 590
<211> 25
<212> DNA
<213> Homo sapiens

<400> 590
aaggaaaaga agcaggctat gcaga                                          25

<210> 591
<211> 25

<212> DNA
<213> Homo sapiens

<400> 591
aggaaaagaa gcaggctatg cagac                                            25

<210> 592
<211> 25
<212> DNA
<213> Homo sapiens

<400> 592
ggaaaagaag caggctatgc agacc                                            25

<210> 593
<211> 25
<212> DNA
<213> Homo sapiens

<400> 593
gaaaagaagc aggctatgca gacct                                            25

<210> 594
<211> 25
<212> DNA
<213> Homo sapiens

<400> 594
aaaagaagca ggctatgcag accta                                            25

<210> 595
<211> 25
<212> DNA
<213> Homo sapiens

<400> 595
aaagaagcag gctatgcaga cctag                                            25

<210> 596
<211> 25
<212> DNA
<213> Homo sapiens

<400> 596
aagaagcagg ctatgcagac ctaga                                            25

<210> 597
<211> 25
<212> DNA
<213> Homo sapiens

<400> 597
agaagcaggc tatgcagacc tagat                                            25

<210> 598
<211> 25
<212> DNA
<213> Homo sapiens

<400> 598
gaagcaggct atgcagacct agatc                                            25

<210> 599
<211> 25
<212> DNA
<213> Homo sapiens

<400> 599
aagcaggcta tgcagaccta gatcc                                          25

<210> 600
<211> 25
<212> DNA
<213> Homo sapiens

<400> 600
agcaggctat gcagacctag atcct                                          25

<210> 601
<211> 25
<212> DNA
<213> Homo sapiens

<400> 601
gcaggctatg cagacctaga tcctt                                          25

<210> 602
<211> 25
<212> DNA
<213> Homo sapiens

<400> 602
caggctatgc agacctagat cctta                                          25

<210> 603
<211> 17
<212> DNA
<213> Homo sapiens

<400> 603
caacttcagt tggtcag                                                   17

<210> 604
<211> 17
<212> DNA
<213> Homo sapiens

<400> 604
aacttcagtt ggtcagc                                                   17

<210> 605
<211> 17
<212> DNA
<213> Homo sapiens

<400> 605
acttcagttg gtcagcc                                                   17

<210> 606
<211> 17
<212> DNA
<213> Homo sapiens

<400> 606
cttcagttgg tcagccc                                                    17

<210> 607
<211> 17
<212> DNA
<213> Homo sapiens

<400> 607
ttcagttggt cagccct                                                    17

<210> 608
<211> 17
<212> DNA
<213> Homo sapiens

<400> 608
tcagttggtc agccctc                                                    17

<210> 609
<211> 17
<212> DNA
<213> Homo sapiens

<400> 609
cagttggtca gccctcc                                                    17

<210> 610
<211> 17
<212> DNA
<213> Homo sapiens

<400> 610
agttggtcag ccctcca                                                    17

<210> 611
<211> 17
<212> DNA
<213> Homo sapiens

<400> 611
gttggtcagc cctccac                                                    17

<210> 612
<211> 17
<212> DNA
<213> Homo sapiens

<400> 612
ttggtcagcc ctccaca                                                    17

<210> 613
<211> 17
<212> DNA
<213> Homo sapiens

<400> 613
tggtcagccc tccacat                                                    17

<210> 614

<211> 17
<212> DNA
<213> Homo sapiens

<400> 614
ggtcagccct ccacatc                                                    17

<210> 615
<211> 17
<212> DNA
<213> Homo sapiens

<400> 615
gtcagccctc cacatcc                                                    17

<210> 616
<211> 17
<212> DNA
<213> Homo sapiens

<400> 616
tcagccctcc acatcca                                                    17

<210> 617
<211> 17
<212> DNA
<213> Homo sapiens

<400> 617
cagccctcca catccac                                                    17

<210> 618
<211> 17
<212> DNA
<213> Homo sapiens

<400> 618
agccctccac atccact                                                    17

<210> 619
<211> 17
<212> DNA
<213> Homo sapiens

<400> 619
gccctccaca tccactt                                                    17

<210> 620
<211> 17
<212> DNA
<213> Homo sapiens

<400> 620
ccctccacat ccacttt                                                    17

<210> 621
<211> 17
<212> DNA
<213> Homo sapiens

<400> 621

cctccacatc cactttc                                                    17

<210> 622
<211> 17
<212> DNA
<213> Homo sapiens

<400> 622
ctccacatcc actttca                                                    17

<210> 623
<211> 17
<212> DNA
<213> Homo sapiens

<400> 623
tccacatcca ctttcaa                                                    17

<210> 624
<211> 17
<212> DNA
<213> Homo sapiens

<400> 624
ccacatccac tttcaag                                                    17

<210> 625
<211> 17
<212> DNA
<213> Homo sapiens

<400> 625
cacatccact ttcaagg                                                    17

<210> 626
<211> 17
<212> DNA
<213> Homo sapiens

<400> 626
acatccactt tcaaggc                                                    17

<210> 627
<211> 17
<212> DNA
<213> Homo sapiens

<400> 627
catccacttt caaggct                                                    17

<210> 628
<211> 17
<212> DNA
<213> Homo sapiens

<400> 628
atccactttc aaggcta                                                    17

<210> 629
<211> 17
<212> DNA

<213> Homo sapiens

<400> 629
tccactttca aggctac                                                    17

<210> 630
<211> 17
<212> DNA
<213> Homo sapiens

<400> 630
ccactttcaa ggctacg                                                    17

<210> 631
<211> 17
<212> DNA
<213> Homo sapiens

<400> 631
cactttcaag gctacgg                                                    17

<210> 632
<211> 17
<212> DNA
<213> Homo sapiens

<400> 632
actttcaagg ctacggg                                                    17

<210> 633
<211> 17
<212> DNA
<213> Homo sapiens

<400> 633
ctttcaaggc tacgggg                                                    17

<210> 634
<211> 17
<212> DNA
<213> Homo sapiens

<400> 634
tttcaaggct acgggga                                                    17

<210> 635
<211> 17
<212> DNA
<213> Homo sapiens

<400> 635
ttcaaggcta cggggaa                                                    17

<210> 636
<211> 17
<212> DNA
<213> Homo sapiens

<400> 636
tcaaggctac ggggaac                                                    17

<210> 637
<211> 17
<212> DNA
<213> Homo sapiens

<400> 637
caaggctacg gggaacc                                                    17

<210> 638
<211> 17
<212> DNA
<213> Homo sapiens

<400> 638
aaggctacgg ggaacca                                                    17

<210> 639
<211> 17
<212> DNA
<213> Homo sapiens

<400> 639
aggctacggg gaaccaa                                                    17

<210> 640
<211> 17
<212> DNA
<213> Homo sapiens

<400> 640
ggctacgggg aaccaac                                                    17

<210> 641
<211> 17
<212> DNA
<213> Homo sapiens

<400> 641
gctacgggga accaacc                                                    17

<210> 642
<211> 17
<212> DNA
<213> Homo sapiens

<400> 642
ctacggggaa ccaacct                                                    17

<210> 643
<211> 17
<212> DNA
<213> Homo sapiens

<400> 643
tacggggaac caacctc                                                    17

<210> 644
<211> 17
<212> DNA
<213> Homo sapiens

<400> 644
acggggaacc aacctcc                                                    17

<210> 645
<211> 17
<212> DNA
<213> Homo sapiens

<400> 645
cggggaacca acctccc                                                    17

<210> 646
<211> 17
<212> DNA
<213> Homo sapiens

<400> 646
ggggaaccaa cctcccc                                                    17

<210> 647
<211> 17
<212> DNA
<213> Homo sapiens

<400> 647
gggaaccaac ctccccc                                                    17

<210> 648
<211> 17
<212> DNA
<213> Homo sapiens

<400> 648
ggaaccaacc tccccca                                                    17

<210> 649
<211> 17
<212> DNA
<213> Homo sapiens

<400> 649
gaaccaacct cccccac                                                    17

<210> 650
<211> 17
<212> DNA
<213> Homo sapiens

<400> 650
aaccaacctc ccccact                                                    17

<210> 651
<211> 17
<212> DNA
<213> Homo sapiens

<400> 651
accaacctcc cccacta                                                    17

<210> 652
<211> 17

<212> DNA
<213> Homo sapiens

<400> 652
ccaacctccc ccactag                                                    17

<210> 653
<211> 17
<212> DNA
<213> Homo sapiens

<400> 653
caacctcccc cactagt                                                    17

<210> 654
<211> 17
<212> DNA
<213> Homo sapiens

<400> 654
aacctccccc actagtg                                                    17

<210> 655
<211> 17
<212> DNA
<213> Homo sapiens

<400> 655
acctccccca ctagtgg                                                    17

<210> 656
<211> 17
<212> DNA
<213> Homo sapiens

<400> 656
cctcccccac tagtggg                                                    17

<210> 657
<211> 17
<212> DNA
<213> Homo sapiens

<400> 657
ctcccccact agtggga                                                    17

<210> 658
<211> 17
<212> DNA
<213> Homo sapiens

<400> 658
tcccccacta gtgggaa                                                    17

<210> 659
<211> 17
<212> DNA
<213> Homo sapiens

<400> 659
cccccactag tgggaac                                                    17

<210> 660
<211> 17
<212> DNA
<213> Homo sapiens

<400> 660
ccccactagt gggaact                                                    17

<210> 661
<211> 17
<212> DNA
<213> Homo sapiens

<400> 661
cccactagtg ggaactt                                                    17

<210> 662
<211> 17
<212> DNA
<213> Homo sapiens

<400> 662
ccactagtgg gaactta                                                    17

<210> 663
<211> 17
<212> DNA
<213> Homo sapiens

<400> 663
cactagtggg aacttac                                                    17

<210> 664
<211> 17
<212> DNA
<213> Homo sapiens

<400> 664
actagtggga acttaca                                                    17

<210> 665
<211> 17
<212> DNA
<213> Homo sapiens

<400> 665
ctagtgggaa cttacaa                                                    17

<210> 666
<211> 17
<212> DNA
<213> Homo sapiens

<400> 666
tagtgggaac ttacaat                                                    17

<210> 667
<211> 17
<212> DNA
<213> Homo sapiens

<400> 667
agtgggaact tacaata                                                    17

<210> 668
<211> 17
<212> DNA
<213> Homo sapiens

<400> 668
gtgggaactt acaatac                                                    17

<210> 669
<211> 17
<212> DNA
<213> Homo sapiens

<400> 669
tgggaactta caataca                                                    17

<210> 670
<211> 17
<212> DNA
<213> Homo sapiens

<400> 670
gggaacttac aatacac                                                    17

<210> 671
<211> 17
<212> DNA
<213> Homo sapiens

<400> 671
ggaacttaca atacact                                                    17

<210> 672
<211> 17
<212> DNA
<213> Homo sapiens

<400> 672
gaacttacaa tacactt                                                    17

<210> 673
<211> 17
<212> DNA
<213> Homo sapiens

<400> 673
aacttacaat acacttc                                                    17

<210> 674
<211> 17
<212> DNA
<213> Homo sapiens

<400> 674
acttacaata cacttct                                                    17

<210> 675

<211> 17
<212> DNA
<213> Homo sapiens

<400> 675
cttacaatac acttctc                                                    17

<210> 676
<211> 17
<212> DNA
<213> Homo sapiens

<400> 676
ttacaataca cttctct                                                    17

<210> 677
<211> 17
<212> DNA
<213> Homo sapiens

<400> 677
tacaatacac ttctctc                                                    17

<210> 678
<211> 17
<212> DNA
<213> Homo sapiens

<400> 678
acaatacact tctctcc                                                    17

<210> 679
<211> 17
<212> DNA
<213> Homo sapiens

<400> 679
caatacactt ctctcca                                                    17

<210> 680
<211> 17
<212> DNA
<213> Homo sapiens

<400> 680
aatacacttc tctccag                                                    17

<210> 681
<211> 17
<212> DNA
<213> Homo sapiens

<400> 681
atacacttct ctccagg                                                    17

<210> 682
<211> 17
<212> DNA
<213> Homo sapiens

<400> 682

tacacttctc tccagga                                                    17

<210> 683
<211> 17
<212> DNA
<213> Homo sapiens

<400> 683
acacttctct ccaggac                                                    17

<210> 684
<211> 17
<212> DNA
<213> Homo sapiens

<400> 684
cacttctctc caggact                                                    17

<210> 685
<211> 17
<212> DNA
<213> Homo sapiens

<400> 685
acttctctcc aggactg                                                    17

<210> 686
<211> 17
<212> DNA
<213> Homo sapiens

<400> 686
cttctctcca ggactga                                                    17

<210> 687
<211> 17
<212> DNA
<213> Homo sapiens

<400> 687
ttctctccag gactgac                                                    17

<210> 688
<211> 17
<212> DNA
<213> Homo sapiens

<400> 688
tctctccagg actgaca                                                    17

<210> 689
<211> 17
<212> DNA
<213> Homo sapiens

<400> 689
ctctccagga ctgacag                                                    17

<210> 690
<211> 17
<212> DNA

<213> Homo sapiens

<400> 690
tctccaggac tgacagc                                                    17

<210> 691
<211> 17
<212> DNA
<213> Homo sapiens

<400> 691
ctccaggact gacagct                                                    17

<210> 692
<211> 17
<212> DNA
<213> Homo sapiens

<400> 692
tccaggactg acagctg                                                    17

<210> 693
<211> 17
<212> DNA
<213> Homo sapiens

<400> 693
ccaggactga cagctgc                                                    17

<210> 694
<211> 17
<212> DNA
<213> Homo sapiens

<400> 694
caggactgac agctgct                                                    17

<210> 695
<211> 17
<212> DNA
<213> Homo sapiens

<400> 695
aggactgaca gctgctc                                                    17

<210> 696
<211> 17
<212> DNA
<213> Homo sapiens

<400> 696
ggactgacag ctgctcc                                                    17

<210> 697
<211> 17
<212> DNA
<213> Homo sapiens

<400> 697
gactgacagc tgctcct                                                    17

<210> 698
<211> 17
<212> DNA
<213> Homo sapiens

<400> 698
actgacagct gctcctc                                                    17

<210> 699
<211> 17
<212> DNA
<213> Homo sapiens

<400> 699
ctgacagctg ctcctca                                                    17

<210> 700
<211> 17
<212> DNA
<213> Homo sapiens

<400> 700
tgacagctgc tcctcag                                                    17

<210> 701
<211> 17
<212> DNA
<213> Homo sapiens

<400> 701
gacagctgct cctcagc                                                    17

<210> 702
<211> 17
<212> DNA
<213> Homo sapiens

<400> 702
acagctgctc ctcagcc                                                    17

<210> 703
<211> 17
<212> DNA
<213> Homo sapiens

<400> 703
cagctgctcc tcagccc                                                    17

<210> 704
<211> 17
<212> DNA
<213> Homo sapiens

<400> 704
agctgctcct cagccca                                                    17

<210> 705
<211> 17
<212> DNA
<213> Homo sapiens

<400> 705
gctgctcctc agcccag                                                    17

<210> 706
<211> 17
<212> DNA
<213> Homo sapiens

<400> 706
ctgctcctca gcccagg                                                    17

<210> 707
<211> 17
<212> DNA
<213> Homo sapiens

<400> 707
tgctcctcag cccaggc                                                    17

<210> 708
<211> 17
<212> DNA
<213> Homo sapiens

<400> 708
gctcctcagc ccaggcc                                                    17

<210> 709
<211> 17
<212> DNA
<213> Homo sapiens

<400> 709
ctcctcagcc caggccc                                                    17

<210> 710
<211> 17
<212> DNA
<213> Homo sapiens

<400> 710
tcctcagccc aggccca                                                    17

<210> 711
<211> 17
<212> DNA
<213> Homo sapiens

<400> 711
cctcagccca ggcccag                                                    17

<210> 712
<211> 17
<212> DNA
<213> Homo sapiens

<400> 712
ctcagcccag gcccagt                                                    17

<210> 713
<211> 17

<212> DNA
<213> Homo sapiens

<400> 713
tcagcccagg cccagta                                                    17

<210> 714
<211> 17
<212> DNA
<213> Homo sapiens

<400> 714
cagcccaggc ccagtat                                                    17

<210> 715
<211> 17
<212> DNA
<213> Homo sapiens

<400> 715
agcccaggcc cagtatg                                                    17

<210> 716
<211> 17
<212> DNA
<213> Homo sapiens

<400> 716
gcccaggccc agtatga                                                    17

<210> 717
<211> 17
<212> DNA
<213> Homo sapiens

<400> 717
cccaggccca gtatgat                                                    17

<210> 718
<211> 17
<212> DNA
<213> Homo sapiens

<400> 718
ccaggcccag tatgata                                                    17

<210> 719
<211> 17
<212> DNA
<213> Homo sapiens

<400> 719
caggcccagt atgatac                                                    17

<210> 720
<211> 17
<212> DNA
<213> Homo sapiens

<400> 720
aggcccagta tgatacc                                                    17

<210> 721
<211> 17
<212> DNA
<213> Homo sapiens

<400> 721
ggcccagtat gataccc                                                      17

<210> 722
<211> 17
<212> DNA
<213> Homo sapiens

<400> 722
gcccagtatg atacccc                                                      17

<210> 723
<211> 17
<212> DNA
<213> Homo sapiens

<400> 723
cccagtatga taccccg                                                      17

<210> 724
<211> 17
<212> DNA
<213> Homo sapiens

<400> 724
ccagtatgat accccga                                                      17

<210> 725
<211> 17
<212> DNA
<213> Homo sapiens

<400> 725
cagtatgata ccccgaa                                                      17

<210> 726
<211> 17
<212> DNA
<213> Homo sapiens

<400> 726
agtatgatac cccgaaa                                                      17

<210> 727
<211> 17
<212> DNA
<213> Homo sapiens

<400> 727
gtatgatacc ccgaaag                                                      17

<210> 728
<211> 17
<212> DNA
<213> Homo sapiens

<400> 728
tatgataccc cgaaagc                                                    17

<210> 729
<211> 17
<212> DNA
<213> Homo sapiens

<400> 729
atgatacccc gaaagct                                                    17

<210> 730
<211> 17
<212> DNA
<213> Homo sapiens

<400> 730
tgataccccg aaagctg                                                    17

<210> 731
<211> 17
<212> DNA
<213> Homo sapiens

<400> 731
gataccccga aagctgg                                                    17

<210> 732
<211> 17
<212> DNA
<213> Homo sapiens

<400> 732
ataccccgaa agctggg                                                    17

<210> 733
<211> 17
<212> DNA
<213> Homo sapiens

<400> 733
taccccgaaa gctggga                                                    17

<210> 734
<211> 17
<212> DNA
<213> Homo sapiens

<400> 734
accccgaaag ctgggaa                                                    17

<210> 735
<211> 17
<212> DNA
<213> Homo sapiens

<400> 735
ccccgaaagc tgggaag                                                    17

<210> 736

<211> 17
<212> DNA
<213> Homo sapiens

<400> 736
cccgaaagct gggaagc                                                    17

<210> 737
<211> 17
<212> DNA
<213> Homo sapiens

<400> 737
ccgaaagctg ggaagcc                                                    17

<210> 738
<211> 17
<212> DNA
<213> Homo sapiens

<400> 738
cgaaagctgg gaagcca                                                    17

<210> 739
<211> 17
<212> DNA
<213> Homo sapiens

<400> 739
gaaagctggg aagccag                                                    17

<210> 740
<211> 17
<212> DNA
<213> Homo sapiens

<400> 740
aaagctggga agccagg                                                    17

<210> 741
<211> 17
<212> DNA
<213> Homo sapiens

<400> 741
aagctgggaa gccaggt                                                    17

<210> 742
<211> 17
<212> DNA
<213> Homo sapiens

<400> 742
agctgggaag ccaggtc                                                    17

<210> 743
<211> 17
<212> DNA
<213> Homo sapiens

<400> 743

gctgggaagc caggtct                                                    17

<210> 744
<211> 17
<212> DNA
<213> Homo sapiens

<400> 744
ctgggaagcc aggtcta                                                    17

<210> 745
<211> 17
<212> DNA
<213> Homo sapiens

<400> 745
tgggaagcca ggtctac                                                    17

<210> 746
<211> 17
<212> DNA
<213> Homo sapiens

<400> 746
gggaagccag gtctacc                                                    17

<210> 747
<211> 17
<212> DNA
<213> Homo sapiens

<400> 747
ggaagccagg tctacct                                                    17

<210> 748
<211> 17
<212> DNA
<213> Homo sapiens

<400> 748
gaagccaggt ctacctg                                                    17

<210> 749
<211> 17
<212> DNA
<213> Homo sapiens

<400> 749
aagccaggtc tacctgc                                                    17

<210> 750
<211> 17
<212> DNA
<213> Homo sapiens

<400> 750
agccaggtct acctgcc                                                    17

<210> 751
<211> 17
<212> DNA

<213> Homo sapiens

<400> 751
gccaggtcta cctgccc                                                    17

<210> 752
<211> 17
<212> DNA
<213> Homo sapiens

<400> 752
ccaggtctac ctgcccc                                                    17

<210> 753
<211> 17
<212> DNA
<213> Homo sapiens

<400> 753
caggtctacc tgcccca                                                    17

<210> 754
<211> 17
<212> DNA
<213> Homo sapiens

<400> 754
aggtctacct gccccag                                                    17

<210> 755
<211> 17
<212> DNA
<213> Homo sapiens

<400> 755
ggtctacctg ccccaga                                                    17

<210> 756
<211> 17
<212> DNA
<213> Homo sapiens

<400> 756
gtctacctgc cccagac                                                    17

<210> 757
<211> 17
<212> DNA
<213> Homo sapiens

<400> 757
tctacctgcc ccagacg                                                    17

<210> 758
<211> 17
<212> DNA
<213> Homo sapiens

<400> 758
ctacctgccc cagacga                                                    17

<210> 759
<211> 17
<212> DNA
<213> Homo sapiens

<400> 759
tacctgcccc agacgaa                                                    17

<210> 760
<211> 17
<212> DNA
<213> Homo sapiens

<400> 760
acctgcccca gacgaat                                                    17

<210> 761
<211> 17
<212> DNA
<213> Homo sapiens

<400> 761
cctgccccag acgaatt                                                    17

<210> 762
<211> 17
<212> DNA
<213> Homo sapiens

<400> 762
ctgccccaga cgaattg                                                    17

<210> 763
<211> 17
<212> DNA
<213> Homo sapiens

<400> 763
tgccccagac gaattgg                                                    17

<210> 764
<211> 17
<212> DNA
<213> Homo sapiens

<400> 764
gccccagacg aattggt                                                    17

<210> 765
<211> 17
<212> DNA
<213> Homo sapiens

<400> 765
ccccagacga attggtg                                                    17

<210> 766
<211> 17
<212> DNA
<213> Homo sapiens

<400> 766
cccagacgaa ttggtgt                                                    17

<210> 767
<211> 17
<212> DNA
<213> Homo sapiens

<400> 767
ccagacgaat tggtgta                                                    17

<210> 768
<211> 17
<212> DNA
<213> Homo sapiens

<400> 768
cagacgaatt ggtgtac                                                    17

<210> 769
<211> 17
<212> DNA
<213> Homo sapiens

<400> 769
agacgaattg gtgtacc                                                    17

<210> 770
<211> 17
<212> DNA
<213> Homo sapiens

<400> 770
gacgaattgg tgtacca                                                    17

<210> 771
<211> 17
<212> DNA
<213> Homo sapiens

<400> 771
acgaattggt gtaccag                                                    17

<210> 772
<211> 17
<212> DNA
<213> Homo sapiens

<400> 772
cgaattggtg taccagg                                                    17

<210> 773
<211> 17
<212> DNA
<213> Homo sapiens

<400> 773
gaattggtgt accaggt                                                    17

<210> 774
<211> 17

<212> DNA
<213> Homo sapiens

<400> 774
aattggtgta ccaggtg                                                    17

<210> 775
<211> 17
<212> DNA
<213> Homo sapiens

<400> 775
attggtgtac caggtgc                                                    17

<210> 776
<211> 17
<212> DNA
<213> Homo sapiens

<400> 776
ttggtgtacc aggtgcc                                                    17

<210> 777
<211> 17
<212> DNA
<213> Homo sapiens

<400> 777
tggtgtacca ggtgcca                                                    17

<210> 778
<211> 17
<212> DNA
<213> Homo sapiens

<400> 778
ggtgtaccag gtgccac                                                    17

<210> 779
<211> 17
<212> DNA
<213> Homo sapiens

<400> 779
gtgtaccagg tgccaca                                                    17

<210> 780
<211> 17
<212> DNA
<213> Homo sapiens

<400> 780
tgtaccaggt gccacag                                                    17

<210> 781
<211> 17
<212> DNA
<213> Homo sapiens

<400> 781
gtaccaggtg ccacaga                                                    17

<210> 782
<211> 17
<212> DNA
<213> Homo sapiens

<400> 782
taccaggtgc cacagag                                                    17

<210> 783
<211> 17
<212> DNA
<213> Homo sapiens

<400> 783
accaggtgcc acagagc                                                    17

<210> 784
<211> 17
<212> DNA
<213> Homo sapiens

<400> 784
ccaggtgcca cagagca                                                    17

<210> 785
<211> 17
<212> DNA
<213> Homo sapiens

<400> 785
caggtgccac agagcac                                                    17

<210> 786
<211> 17
<212> DNA
<213> Homo sapiens

<400> 786
aggtgccaca gagcaca                                                    17

<210> 787
<211> 17
<212> DNA
<213> Homo sapiens

<400> 787
ggtgccacag agcacac                                                    17

<210> 788
<211> 17
<212> DNA
<213> Homo sapiens

<400> 788
gtgccacaga gcacaca                                                    17

<210> 789
<211> 17
<212> DNA
<213> Homo sapiens

<400> 789
tgccacagag cacacaa                                                    17

<210> 790
<211> 17
<212> DNA
<213> Homo sapiens

<400> 790
gccacagagc acacaag                                                    17

<210> 791
<211> 17
<212> DNA
<213> Homo sapiens

<400> 791
ccacagagca cacaaga                                                    17

<210> 792
<211> 17
<212> DNA
<213> Homo sapiens

<400> 792
cacagagcac acaagaa                                                    17

<210> 793
<211> 17
<212> DNA
<213> Homo sapiens

<400> 793
acagagcaca caagaag                                                    17

<210> 794
<211> 17
<212> DNA
<213> Homo sapiens

<400> 794
cagagcacac aagaagt                                                    17

<210> 795
<211> 17
<212> DNA
<213> Homo sapiens

<400> 795
agagcacaca agaagta                                                    17

<210> 796
<211> 17
<212> DNA
<213> Homo sapiens

<400> 796
gagcacacaa gaagtat                                                    17

<210> 797

<211> 17
<212> DNA
<213> Homo sapiens

<400> 797
agcacacaag aagtatc                                                    17

<210> 798
<211> 17
<212> DNA
<213> Homo sapiens

<400> 798
gcacacaaga agtatca                                                    17

<210> 799
<211> 17
<212> DNA
<213> Homo sapiens

<400> 799
cacacaagaa gtatcag                                                    17

<210> 800
<211> 17
<212> DNA
<213> Homo sapiens

<400> 800
acacaagaag tatcagg                                                    17

<210> 801
<211> 17
<212> DNA
<213> Homo sapiens

<400> 801
cacaagaagt atcagga                                                    17

<210> 802
<211> 17
<212> DNA
<213> Homo sapiens

<400> 802
acaagaagta tcaggag                                                    17

<210> 803
<211> 17
<212> DNA
<213> Homo sapiens

<400> 803
caagaagtat caggagc                                                    17

<210> 804
<211> 17
<212> DNA
<213> Homo sapiens

<400> 804

aagaagtatc aggagca                                                    17

<210> 805
<211> 17
<212> DNA
<213> Homo sapiens

<400> 805
agaagtatca ggagcag                                                    17

<210> 806
<211> 17
<212> DNA
<213> Homo sapiens

<400> 806
gaagtatcag gagcagg                                                    17

<210> 807
<211> 17
<212> DNA
<213> Homo sapiens

<400> 807
aagtatcagg agcagga                                                    17

<210> 808
<211> 17
<212> DNA
<213> Homo sapiens

<400> 808
agtatcagga gcaggaa                                                    17

<210> 809
<211> 17
<212> DNA
<213> Homo sapiens

<400> 809
gtatcaggag caggaag                                                    17

<210> 810
<211> 17
<212> DNA
<213> Homo sapiens

<400> 810
tatcaggagc aggaagg                                                    17

<210> 811
<211> 17
<212> DNA
<213> Homo sapiens

<400> 811
atcaggagca ggaaggg                                                    17

<210> 812
<211> 17
<212> DNA

<213> Homo sapiens

<400> 812
tcaggagcag gaaggga                                                    17

<210> 813
<211> 17
<212> DNA
<213> Homo sapiens

<400> 813
caggagcagg aagggat                                                    17

<210> 814
<211> 17
<212> DNA
<213> Homo sapiens

<400> 814
aggagcagga agggatg                                                    17

<210> 815
<211> 17
<212> DNA
<213> Homo sapiens

<400> 815
ggagcaggaa gggatgg                                                    17

<210> 816
<211> 17
<212> DNA
<213> Homo sapiens

<400> 816
gagcaggaag ggatggg                                                    17

<210> 817
<211> 17
<212> DNA
<213> Homo sapiens

<400> 817
agcaggaagg gatgggg                                                    17

<210> 818
<211> 17
<212> DNA
<213> Homo sapiens

<400> 818
gcaggaaggg atgggga                                                    17

<210> 819
<211> 17
<212> DNA
<213> Homo sapiens

<400> 819
caggaaggga tggggaa                                                    17

<210> 820
<211> 17
<212> DNA
<213> Homo sapiens

<400> 820
aggaagggat ggggaat                                                    17

<210> 821
<211> 17
<212> DNA
<213> Homo sapiens

<400> 821
ggaagggatg gggaatg                                                    17

<210> 822
<211> 17
<212> DNA
<213> Homo sapiens

<400> 822
gaagggatgg ggaatgt                                                    17

<210> 823
<211> 17
<212> DNA
<213> Homo sapiens

<400> 823
aagggatggg gaatgtg                                                    17

<210> 824
<211> 17
<212> DNA
<213> Homo sapiens

<400> 824
agggatgggg aatgtga                                                    17

<210> 825
<211> 17
<212> DNA
<213> Homo sapiens

<400> 825
gggatgggga atgtgat                                                    17

<210> 826
<211> 17
<212> DNA
<213> Homo sapiens

<400> 826
ggatggggaa tgtgatg                                                    17

<210> 827
<211> 17
<212> DNA
<213> Homo sapiens

<400> 827
gatggggaat gtgatgt                                                    17

<210> 828
<211> 17
<212> DNA
<213> Homo sapiens

<400> 828
atggggaatg tgatgtt                                                    17

<210> 829
<211> 17
<212> DNA
<213> Homo sapiens

<400> 829
tggggaatgt gatgttt                                                    17

<210> 830
<211> 17
<212> DNA
<213> Homo sapiens

<400> 830
ggggaatgtg atgtttt                                                    17

<210> 831
<211> 17
<212> DNA
<213> Homo sapiens

<400> 831
gggaatgtga tgttttt                                                    17

<210> 832
<211> 17
<212> DNA
<213> Homo sapiens

<400> 832
   ggaatgtgat gttttta                                                   17

<210> 833
<211> 17
<212> DNA
<213> Homo sapiens

<400> 833
gaatgtgatg tttttaa                                                    17

<210> 834
<211> 17
<212> DNA
<213> Homo sapiens

<400> 834
aatgtgatgt ttttaaa                                                    17

<210> 835
<211> 17

<212> DNA
<213> Homo sapiens

<400> 835
atgtgatgtt tttaaag                                                    17

<210> 836
<211> 17
<212> DNA
<213> Homo sapiens

<400> 836
tgtgatgttt ttaaaga                                                    17

<210> 837
<211> 17
<212> DNA
<213> Homo sapiens

<400> 837
gtgatgtttt taaagaa                                                    17

<210> 838
<211> 17
<212> DNA
<213> Homo sapiens

<400> 838
tgatgttttt aaagaaa                                                    17

<210> 839
<211> 17
<212> DNA
<213> Homo sapiens

<400> 839
gatgttttta aagaaat                                                    17

<210> 840
<211> 17
<212> DNA
<213> Homo sapiens

<400> 840
atgtttttaa agaaatc                                                    17

<210> 841
<211> 17
<212> DNA
<213> Homo sapiens

<400> 841
tgtttttaaa gaaatcc                                                    17

<210> 842
<211> 17
<212> DNA
<213> Homo sapiens

<400> 842
gtttttaaag aaatcct                                                    17

<210> 843
<211> 17
<212> DNA
<213> Homo sapiens

<400> 843
tttttaaaga aatcctt                                                    17

<210> 844
<211> 17
<212> DNA
<213> Homo sapiens

<400> 844
ttttaaagaa atccttt                                                    17

<210> 845
<211> 17
<212> DNA
<213> Homo sapiens

<400> 845
tttaaagaaa tcctttg                                                    17

<210> 846
<211> 17
<212> DNA
<213> Homo sapiens

<400> 846
ttaaagaaat cctttga                                                    17

<210> 847
<211> 17
<212> DNA
<213> Homo sapiens

<400> 847
taaagaaatc ctttgaa                                                    17

<210> 848
<211> 17
<212> DNA
<213> Homo sapiens

<400> 848
aaagaaatcc tttgaag                                                    17

<210> 849
<211> 17
<212> DNA
<213> Homo sapiens

<400> 849
aagaaatcct ttgaaga                                                    17

<210> 850
<211> 17
<212> DNA
<213> Homo sapiens

<400> 850
agaaatcctt tgaagat                                                    17

<210> 851
<211> 17
<212> DNA
<213> Homo sapiens

<400> 851
gaaatccttt gaagatg                                                    17

<210> 852
<211> 17
<212> DNA
<213> Homo sapiens

<400> 852
aaatcctttg aagatga                                                    17

<210> 853
<211> 17
<212> DNA
<213> Homo sapiens

<400> 853
aatcctttga agatgat                                                    17

<210> 854
<211> 17
<212> DNA
<213> Homo sapiens

<400> 854
atcctttgaa gatgatg                                                    17

<210> 855
<211> 17
<212> DNA
<213> Homo sapiens

<400> 855
tcctttgaag atgatgc                                                    17

<210> 856
<211> 17
<212> DNA
<213> Homo sapiens

<400> 856
cctttgaaga tgatgct                                                    17

<210> 857
<211> 17
<212> DNA
<213> Homo sapiens

<400> 857
ctttgaagat gatgctg                                                    17

<210> 858

<211> 17
<212> DNA
<213> Homo sapiens

<400> 858
tttgaagatg atgctgc                                                    17

<210> 859
<211> 17
<212> DNA
<213> Homo sapiens

<400> 859
ttgaagatga tgctgct                                                    17

<210> 860
<211> 17
<212> DNA
<213> Homo sapiens

<400> 860
tgaagatgat gctgctt                                                    17

<210> 861
<211> 17
<212> DNA
<213> Homo sapiens

<400> 861
gaagatgatg ctgcttt                                                    17

<210> 862
<211> 25
<212> DNA
<213> Homo sapiens

<400> 862
caacttcagt tggtcagccc tccac                                           25

<210> 863
<211> 25
<212> DNA
<213> Homo sapiens

<400> 863
aacttcagtt ggtcagccct ccaca                                           25

<210> 864
<211> 25
<212> DNA
<213> Homo sapiens

<400> 864
acttcagttg gtcagccctc cacat                                           25

<210> 865
<211> 25
<212> DNA
<213> Homo sapiens

<400> 865

cttcagttgg tcagccctcc acatc                                          25

<210> 866
<211> 25
<212> DNA
<213> Homo sapiens

<400> 866
ttcagttggt cagccctcca catcc                                          25

<210> 867
<211> 25
<212> DNA
<213> Homo sapiens

<400> 867
tcagttggtc agccctccac atcca                                          25

<210> 868
<211> 25
<212> DNA
<213> Homo sapiens

<400> 868
cagttggtca gccctccaca tccac                                          25

<210> 869
<211> 25
<212> DNA
<213> Homo sapiens

<400> 869
agttggtcag ccctccacat ccact                                          25

<210> 870
<211> 25
<212> DNA
<213> Homo sapiens

<400> 870
gttggtcagc cctccacatc cactt                                          25

<210> 871
<211> 25
<212> DNA
<213> Homo sapiens

<400> 871
ttggtcagcc ctccacatcc acttt                                          25

<210> 872
<211> 25
<212> DNA
<213> Homo sapiens

<400> 872
tggtcagccc tccacatcca ctttc                                          25

<210> 873
<211> 25
<212> DNA

<213> Homo sapiens

<400> 873
ggtcagccct ccacatccac tttca                                          25

<210> 874
<211> 25
<212> DNA
<213> Homo sapiens

<400> 874
gtcagccctc cacatccact ttcaa                                          25

<210> 875
<211> 25
<212> DNA
<213> Homo sapiens

<400> 875
tcagccctcc acatccactt tcaag                                          25

<210> 876
<211> 25
<212> DNA
<213> Homo sapiens

<400> 876
cagccctcca catccacttt caagg                                          25

<210> 877
<211> 25
<212> DNA
<213> Homo sapiens

<400> 877
agccctccac atccactttc aaggc                                          25

<210> 878
<211> 25
<212> DNA
<213> Homo sapiens

<400> 878
gccctccaca tccactttca aggct                                          25

<210> 879
<211> 25
<212> DNA
<213> Homo sapiens

<400> 879
ccctccacat ccactttcaa ggcta                                          25

<210> 880
<211> 25
<212> DNA
<213> Homo sapiens

<400> 880
cctccacatc cactttcaag gctac                                          25

<210> 881
<211> 25
<212> DNA
<213> Homo sapiens

<400> 881
ctccacatcc actttcaagg ctacg                                          25

<210> 882
<211> 25
<212> DNA
<213> Homo sapiens

<400> 882
tccacatcca ctttcaaggc tacgg                                          25

<210> 883
<211> 25
<212> DNA
<213> Homo sapiens

<400> 883
ccacatccac tttcaaggct acggg                                          25

<210> 884
<211> 25
<212> DNA
<213> Homo sapiens

<400> 884
cacatccact ttcaaggcta cgggg                                          25

<210> 885
<211> 25
<212> DNA
<213> Homo sapiens

<400> 885
acatccactt tcaaggctac gggga                                          25

<210> 886
<211> 25
<212> DNA
<213> Homo sapiens

<400> 886
catccacttt caaggctacg gggaa                                          25

<210> 887
<211> 25
<212> DNA
<213> Homo sapiens

<400> 887
atccactttc aaggctacgg ggaac                                          25

<210> 888
<211> 25
<212> DNA
<213> Homo sapiens

<400> 888
tccactttca aggctacggg gaacc                                          25

<210> 889
<211> 25
<212> DNA
<213> Homo sapiens

<400> 889
ccactttcaa ggctacgggg aacca                                          25

<210> 890
<211> 25
<212> DNA
<213> Homo sapiens

<400> 890
cactttcaag gctacgggga accaa                                          25

<210> 891
<211> 25
<212> DNA
<213> Homo sapiens

<400> 891
actttcaagg ctacggggaa ccaac                                          25

<210> 892
<211> 25
<212> DNA
<213> Homo sapiens

<400> 892
ctttcaaggc tacggggaac caacc                                          25

<210> 893
<211> 25
<212> DNA
<213> Homo sapiens

<400> 893
tttcaaggct acggggaacc aacct                                          25

<210> 894
<211> 25
<212> DNA
<213> Homo sapiens

<400> 894
ttcaaggcta cggggaacca acctc                                          25

<210> 895
<211> 25
<212> DNA
<213> Homo sapiens

<400> 895
tcaaggctac ggggaaccaa cctcc                                          25

<210> 896
<211> 25

<212> DNA
<213> Homo sapiens

<400> 896
caaggctacg gggaaccaac ctccc                                            25

<210> 897
<211> 25
<212> DNA
<213> Homo sapiens

<400> 897
aaggctacgg ggaaccaacc tcccc                                            25

<210> 898
<211> 25
<212> DNA
<213> Homo sapiens

<400> 898
aggctacggg gaaccaacct ccccc                                            25

<210> 899
<211> 25
<212> DNA
<213> Homo sapiens

<400> 899
ggctacgggg aaccaacctc cccca                                            25

<210> 900
<211> 25
<212> DNA
<213> Homo sapiens

<400> 900
gctacgggga accaacctcc cccac                                            25

<210> 901
<211> 25
<212> DNA
<213> Homo sapiens

<400> 901
ctacggggaa ccaacctccc ccact                                            25

<210> 902
<211> 25
<212> DNA
<213> Homo sapiens

<400> 902
tacggggaac caacctcccc cacta                                            25

<210> 903
<211> 25
<212> DNA
<213> Homo sapiens

<400> 903
acggggaacc aacctccccc actag                                            25

<210> 904
<211> 25
<212> DNA
<213> Homo sapiens

<400> 904
cggggaacca acctccccca ctagt                                            25

<210> 905
<211> 25
<212> DNA
<213> Homo sapiens

<400> 905
ggggaaccaa cctcccccac tagtg                                            25

<210> 906
<211> 25
<212> DNA
<213> Homo sapiens

<400> 906
gggaaccaac ctcccccact agtgg                                            25

<210> 907
<211> 25
<212> DNA
<213> Homo sapiens

<400> 907
ggaaccaacc tcccccacta gtggg                                            25

<210> 908
<211> 25
<212> DNA
<213> Homo sapiens

<400> 908
gaaccaacct cccccactag tggga                                            25

<210> 909
<211> 25
<212> DNA
<213> Homo sapiens

<400> 909
aaccaacctc ccccactagt gggaa                                            25

<210> 910
<211> 25
<212> DNA
<213> Homo sapiens

<400> 910
accaacctcc cccactagtg ggaac                                            25

<210> 911
<211> 25
<212> DNA
<213> Homo sapiens

<400> 911
ccaacctccc ccactagtgg gaact                                          25

<210> 912
<211> 25
<212> DNA
<213> Homo sapiens

<400> 912
caacctcccc cactagtggg aactt                                          25

<210> 913
<211> 25
<212> DNA
<213> Homo sapiens

<400> 913
aacctccccc actagtggga actta                                          25

<210> 914
<211> 25
<212> DNA
<213> Homo sapiens

<400> 914
acctccccca ctagtgggaa cttac                                          25

<210> 915
<211> 25
<212> DNA
<213> Homo sapiens

<400> 915
cctcccccac tagtgggaac ttaca                                          25

<210> 916
<211> 25
<212> DNA
<213> Homo sapiens

<400> 916
ctcccccact agtgggaact tacaa                                          25

<210> 917
<211> 25
<212> DNA
<213> Homo sapiens

<400> 917
tcccccacta gtgggaactt acaat                                          25

<210> 918
<211> 25
<212> DNA
<213> Homo sapiens

<400> 918
cccccactag tgggaactta caata                                          25

<210> 919

<211> 25
<212> DNA
<213> Homo sapiens

<400> 919
ccccactagt gggaacttac aatac                                          25

<210> 920
<211> 25
<212> DNA
<213> Homo sapiens

<400> 920
cccactagtg ggaacttaca ataca                                          25

<210> 921
<211> 25
<212> DNA
<213> Homo sapiens

<400> 921
ccactagtgg gaacttacaa tacac                                          25

<210> 922
<211> 25
<212> DNA
<213> Homo sapiens

<400> 922
cactagtggg aacttacaat acact                                          25

<210> 923
<211> 25
<212> DNA
<213> Homo sapiens

<400> 923
actagtggga acttacaata cactt                                          25

<210> 924
<211> 25
<212> DNA
<213> Homo sapiens

<400> 924
ctagtgggaa cttacaatac acttc                                          25

<210> 925
<211> 25
<212> DNA
<213> Homo sapiens

<400> 925
tagtgggaac ttacaataca cttct                                          25

<210> 926
<211> 25
<212> DNA
<213> Homo sapiens

<400> 926

agtgggaact tacaatacac ttctc 25

<210> 927
<211> 25
<212> DNA
<213> Homo sapiens

<400> 927
gtgggaactt acaatacact tctct 25

<210> 928
<211> 25
<212> DNA
<213> Homo sapiens

<400> 928
tgggaactta caatacactt ctctc 25

<210> 929
<211> 25
<212> DNA
<213> Homo sapiens

<400> 929
gggaacttac aatacacttc tctcc 25

<210> 930
<211> 25
<212> DNA
<213> Homo sapiens

<400> 930
ggaacttaca atacacttct ctcca 25

<210> 931
<211> 25
<212> DNA
<213> Homo sapiens

<400> 931
gaacttacaa tacacttctc tccag 25

<210> 932
<211> 25
<212> DNA
<213> Homo sapiens

<400> 932
aacttacaat acacttctct ccagg 25

<210> 933
<211> 25
<212> DNA
<213> Homo sapiens

<400> 933
acttacaata cacttctctc cagga 25

<210> 934
<211> 25
<212> DNA

<213> Homo sapiens

<400> 934
cttacaatac acttctctcc aggac                                          25

<210> 935
<211> 25
<212> DNA
<213> Homo sapiens

<400> 935
ttacaataca cttctctcca ggact                                          25

<210> 936
<211> 25
<212> DNA
<213> Homo sapiens

<400> 936
tacaatacac ttctctccag gactg                                          25

<210> 937
<211> 25
<212> DNA
<213> Homo sapiens

<400> 937
acaatacact tctctccagg actga                                          25

<210> 938
<211> 25
<212> DNA
<213> Homo sapiens

<400> 938
caatacactt ctctccagga ctgac                                          25

<210> 939
<211> 25
<212> DNA
<213> Homo sapiens

<400> 939
aatacacttc tctccaggac tgaca                                          25

<210> 940
<211> 25
<212> DNA
<213> Homo sapiens

<400> 940
atacacttct ctccaggact gacag                                          25

<210> 941
<211> 25
<212> DNA
<213> Homo sapiens

<400> 941
tacacttctc tccaggactg acagc                                          25

<210> 942
<211> 25
<212> DNA
<213> Homo sapiens

<400> 942
acacttctct ccaggactga cagct                                          25

<210> 943
<211> 25
<212> DNA
<213> Homo sapiens

<400> 943
cacttctctc caggactgac agctg                                          25

<210> 944
<211> 25
<212> DNA
<213> Homo sapiens

<400> 944
acttctctcc aggactgaca gctgc                                          25

<210> 945
<211> 25
<212> DNA
<213> Homo sapiens

<400> 945
cttctctcca ggactgacag ctgct                                          25

<210> 946
<211> 25
<212> DNA
<213> Homo sapiens

<400> 946
ttctctccag gactgacagc tgctc                                          25

<210> 947
<211> 25
<212> DNA
<213> Homo sapiens

<400> 947
tctctccagg actgacagct gctcc                                          25

<210> 948
<211> 25
<212> DNA
<213> Homo sapiens

<400> 948
ctctccagga ctgacagctg ctcct                                          25

<210> 949
<211> 25
<212> DNA
<213> Homo sapiens

<400> 949
tctccaggac tgacagctgc tcctc                                          25

<210> 950
<211> 25
<212> DNA
<213> Homo sapiens

<400> 950
ctccaggact gacagctgct cctca                                          25

<210> 951
<211> 25
<212> DNA
<213> Homo sapiens

<400> 951
tccaggactg acagctgctc ctcag                                          25

<210> 952
<211> 25
<212> DNA
<213> Homo sapiens

<400> 952
ccaggactga cagctgctcc tcagc                                          25

<210> 953
<211> 25
<212> DNA
<213> Homo sapiens

<400> 953
caggactgac agctgctcct cagcc                                          25

<210> 954
<211> 25
<212> DNA
<213> Homo sapiens

<400> 954
aggactgaca gctgctcctc agccc                                          25

<210> 955
<211> 25
<212> DNA
<213> Homo sapiens

<400> 955
ggactgacag ctgctcctca gccca                                          25

<210> 956
<211> 25
<212> DNA
<213> Homo sapiens

<400> 956
gactgacagc tgctcctcag cccag                                          25

<210> 957
<211> 25

<212> DNA
<213> Homo sapiens

<400> 957
actgacagct gctcctcagc ccagg                                          25

<210> 958
<211> 25
<212> DNA
<213> Homo sapiens

<400> 958
ctgacagctg ctcctcagcc caggc                                          25

<210> 959
<211> 25
<212> DNA
<213> Homo sapiens

<400> 959
tgacagctgc tcctcagccc aggcc                                          25

<210> 960
<211> 25
<212> DNA
<213> Homo sapiens

<400> 960
gacagctgct cctcagccca ggccc                                          25

<210> 961
<211> 25
<212> DNA
<213> Homo sapiens

<400> 961
acagctgctc ctcagcccag gccca                                          25

<210> 962
<211> 25
<212> DNA
<213> Homo sapiens

<400> 962
cagctgctcc tcagcccagg cccag                                          25

<210> 963
<211> 25
<212> DNA
<213> Homo sapiens

<400> 963
agctgctcct cagcccaggc ccagt                                          25

<210> 964
<211> 25
<212> DNA
<213> Homo sapiens

<400> 964
gctgctcctc agcccaggcc cagta                                          25

<210> 965
<211> 25
<212> DNA
<213> Homo sapiens

<400> 965
ctgctcctca gcccaggccc agtat                                          25

<210> 966
<211> 25
<212> DNA
<213> Homo sapiens

<400> 966
tgctcctcag cccaggccca gtatg                                          25

<210> 967
<211> 25
<212> DNA
<213> Homo sapiens

<400> 967
gctcctcagc ccaggcccag tatga                                          25

<210> 968
<211> 25
<212> DNA
<213> Homo sapiens

<400> 968
ctcctcagcc caggcccagt atgat                                          25

<210> 969
<211> 25
<212> DNA
<213> Homo sapiens

<400> 969
tcctcagccc aggcccagta tgata                                          25

<210> 970
<211> 25
<212> DNA
<213> Homo sapiens

<400> 970
cctcagccca ggcccagtat gatac                                          25

<210> 971
<211> 25
<212> DNA
<213> Homo sapiens

<400> 971
ctcagcccag gcccagtatg atacc                                          25

<210> 972
<211> 25
<212> DNA
<213> Homo sapiens

<400> 972
tcagcccagg cccagtatga taccc                                          25

<210> 973
<211> 25
<212> DNA
<213> Homo sapiens

<400> 973
cagcccaggc ccagtatgat acccc                                          25

<210> 974
<211> 25
<212> DNA
<213> Homo sapiens

<400> 974
agcccaggcc cagtatgata ccccg                                          25

<210> 975
<211> 25
<212> DNA
<213> Homo sapiens

<400> 975
gcccaggccc agtatgatac cccga                                          25

<210> 976
<211> 25
<212> DNA
<213> Homo sapiens

<400> 976
cccaggccca gtatgatacc ccgaa                                          25

<210> 977
<211> 25
<212> DNA
<213> Homo sapiens

<400> 977
ccaggcccag tatgataccc cgaaa                                          25

<210> 978
<211> 25
<212> DNA
<213> Homo sapiens

<400> 978
caggcccagt atgatacccc gaaag                                          25

<210> 979
<211> 25
<212> DNA
<213> Homo sapiens

<400> 979
aggcccagta tgataccccg aaagc                                          25

<210> 980

<211> 25
<212> DNA
<213> Homo sapiens

<400> 980
ggcccagtat gataccccga aagct                                          25

<210> 981
<211> 25
<212> DNA
<213> Homo sapiens

<400> 981
gcccagtatg ataccccgaa agctg                                          25

<210> 982
<211> 25
<212> DNA
<213> Homo sapiens

<400> 982
cccagtatga taccccgaaa gctgg                                          25

<210> 983
<211> 25
<212> DNA
<213> Homo sapiens

<400> 983
ccagtatgat accccgaaag ctggg                                          25

<210> 984
<211> 25
<212> DNA
<213> Homo sapiens

<400> 984
cagtatgata ccccgaaagc tggga                                          25

<210> 985
<211> 25
<212> DNA
<213> Homo sapiens

<400> 985
agtatgatac cccgaaagct gggaa                                          25

<210> 986
<211> 25
<212> DNA
<213> Homo sapiens

<400> 986
gtatgatacc ccgaaagctg ggaag                                          25

<210> 987
<211> 25
<212> DNA
<213> Homo sapiens

<400> 987

tatgataccc cgaaagctgg gaagc                                          25

<210> 988
<211> 25
<212> DNA
<213> Homo sapiens

<400> 988
atgatacccc gaaagctggg aagcc                                          25

<210> 989
<211> 25
<212> DNA
<213> Homo sapiens

<400> 989
tgataccccg aaagctggga agcca                                          25

<210> 990
<211> 25
<212> DNA
<213> Homo sapiens

<400> 990
gataccccga aagctgggaa gccag                                          25

<210> 991
<211> 25
<212> DNA
<213> Homo sapiens

<400> 991
ataccccgaa agctgggaag ccagg                                          25

<210> 992
<211> 25
<212> DNA
<213> Homo sapiens

<400> 992
taccccgaaa gctgggaagc caggt                                          25

<210> 993
<211> 25
<212> DNA
<213> Homo sapiens

<400> 993
accccgaaag ctgggaagcc aggtc                                          25

<210> 994
<211> 25
<212> DNA
<213> Homo sapiens

<400> 994
ccccgaaagc tgggaagcca ggtct                                          25

<210> 995
<211> 25
<212> DNA

<213> Homo sapiens

<400> 995
cccgaaagct gggaagccag gtcta                                            25

<210> 996
<211> 25
<212> DNA
<213> Homo sapiens

<400> 996
ccgaaagctg ggaagccagg tctac                                            25

<210> 997
<211> 25
<212> DNA
<213> Homo sapiens

<400> 997
cgaaagctgg gaagccaggt ctacc                                            25

<210> 998
<211> 25
<212> DNA
<213> Homo sapiens

<400> 998
gaaagctggg aagccaggtc tacct                                            25

<210> 999
<211> 25
<212> DNA
<213> Homo sapiens

<400> 999
aaagctggga agccaggtct acctg                                            25

<210> 1000
<211> 25
<212> DNA
<213> Homo sapiens

<400> 1000
aagctgggaa gccaggtcta cctgc                                            25

<210> 1001
<211> 25
<212> DNA
<213> Homo sapiens

<400> 1001
agctgggaag ccaggtctac ctgcc                                            25

<210> 1002
<211> 25
<212> DNA
<213> Homo sapiens

<400> 1002
gctgggaagc caggtctacc tgccc                                            25

<210> 1003
<211> 25
<212> DNA
<213> Homo sapiens

<400> 1003
ctgggaagcc aggtctacct gcccc                                          25

<210> 1004
<211> 25
<212> DNA
<213> Homo sapiens

<400> 1004
tgggaagcca ggtctacctg cccca                                          25

<210> 1005
<211> 25
<212> DNA
<213> Homo sapiens

<400> 1005
gggaagccag gtctacctgc cccag                                          25

<210> 1006
<211> 25
<212> DNA
<213> Homo sapiens

<400> 1006
ggaagccagg tctacctgcc ccaga                                          25

<210> 1007
<211> 25
<212> DNA
<213> Homo sapiens

<400> 1007
gaagccaggt ctacctgccc cagac                                          25

<210> 1008
<211> 25
<212> DNA
<213> Homo sapiens

<400> 1008
aagccaggtc tacctgcccc agacg                                          25

<210> 1009
<211> 25
<212> DNA
<213> Homo sapiens

<400> 1009
agccaggtct acctgcccca gacga                                          25

<210> 1010
<211> 25
<212> DNA
<213> Homo sapiens

<400> 1010
gccaggtcta cctgccccag acgaa                                          25

<210> 1011
<211> 25
<212> DNA
<213> Homo sapiens

<400> 1011
ccaggtctac ctgccccaga cgaat                                          25

<210> 1012
<211> 25
<212> DNA
<213> Homo sapiens

<400> 1012
caggtctacc tgccccagac gaatt                                          25

<210> 1013
<211> 25
<212> DNA
<213> Homo sapiens

<400> 1013
aggtctacct gccccagacg aattg                                          25

<210> 1014
<211> 25
<212> DNA
<213> Homo sapiens

<400> 1014
ggtctacctg ccccagacga attgg                                          25

<210> 1015
<211> 25
<212> DNA
<213> Homo sapiens

<400> 1015
gtctacctgc cccagacgaa ttggt                                          25

<210> 1016
<211> 25
<212> DNA
<213> Homo sapiens

<400> 1016
tctacctgcc ccagacgaat tggtg                                          25

<210> 1017
<211> 25
<212> DNA
<213> Homo sapiens

<400> 1017
ctacctgccc cagacgaatt ggtgt                                          25

<210> 1018
<211> 25

<212> DNA
<213> Homo sapiens

<400> 1018
tacctgcccc agacgaattg gtgta                                            25

<210> 1019
<211> 25
<212> DNA
<213> Homo sapiens

<400> 1019
acctgcccca gacgaattgg tgtac                                            25

<210> 1020
<211> 25
<212> DNA
<213> Homo sapiens

<400> 1020
cctgccccag acgaattggt gtacc                                            25

<210> 1021
<211> 25
<212> DNA
<213> Homo sapiens

<400> 1021
ctgccccaga cgaattggtg tacca                                            25

<210> 1022
<211> 25
<212> DNA
<213> Homo sapiens

<400> 1022
tgccccagac gaattggtgt accag                                            25

<210> 1023
<211> 25
<212> DNA
<213> Homo sapiens

<400> 1023
gccccagacg aattggtgta ccagg                                            25

<210> 1024
<211> 25
<212> DNA
<213> Homo sapiens

<400> 1024
ccccagacga attggtgtac caggt                                            25

<210> 1025
<211> 25
<212> DNA
<213> Homo sapiens

<400> 1025
cccagacgaa ttggtgtacc aggtg                                            25

<210> 1026
<211> 25
<212> DNA
<213> Homo sapiens

<400> 1026
ccagacgaat tggtgtacca ggtgc                                          25

<210> 1027
<211> 25
<212> DNA
<213> Homo sapiens

<400> 1027
cagacgaatt ggtgtaccag gtgcc                                          25

<210> 1028
<211> 25
<212> DNA
<213> Homo sapiens

<400> 1028
agacgaattg gtgtaccagg tgcca                                          25

<210> 1029
<211> 25
<212> DNA
<213> Homo sapiens

<400> 1029
gacgaattgg tgtaccaggt gccac                                          25

<210> 1030
<211> 25
<212> DNA
<213> Homo sapiens

<400> 1030
acgaattggt gtaccaggtg ccaca                                          25

<210> 1031
<211> 25
<212> DNA
<213> Homo sapiens

<400> 1031
cgaattggtg taccaggtgc cacag                                          25

<210> 1032
<211> 25
<212> DNA
<213> Homo sapiens

<400> 1032
gaattggtgt accaggtgcc acaga                                          25

<210> 1033
<211> 25
<212> DNA
<213> Homo sapiens

<400> 1033
aattggtgta ccaggtgcca cagag                                          25

<210> 1034
<211> 25
<212> DNA
<213> Homo sapiens

<400> 1034
attggtgtac caggtgccac agagc                                          25

<210> 1035
<211> 25
<212> DNA
<213> Homo sapiens

<400> 1035
ttggtgtacc aggtgccaca gagca                                          25

<210> 1036
<211> 25
<212> DNA
<213> Homo sapiens

<400> 1036
tggtgtacca ggtgccacag agcac                                          25

<210> 1037
<211> 25
<212> DNA
<213> Homo sapiens

<400> 1037
ggtgtaccag gtgccacaga gcaca                                          25

<210> 1038
<211> 25
<212> DNA
<213> Homo sapiens

<400> 1038
gtgtaccagg tgccacagag cacac                                          25

<210> 1039
<211> 25
<212> DNA
<213> Homo sapiens

<400> 1039
tgtaccaggt gccacagagc acaca                                          25

<210> 1040
<211> 25
<212> DNA
<213> Homo sapiens

<400> 1040
gtaccaggtg ccacagagca cacaa                                          25

<210> 1041

<211> 25
<212> DNA
<213> Homo sapiens

<400> 1041
taccaggtgc cacagagcac acaag                                            25

<210> 1042
<211> 25
<212> DNA
<213> Homo sapiens

<400> 1042
accaggtgcc acagagcaca caaga                                            25

<210> 1043
<211> 25
<212> DNA
<213> Homo sapiens

<400> 1043
ccaggtgcca cagagcacac aagaa                                            25

<210> 1044
<211> 25
<212> DNA
<213> Homo sapiens

<400> 1044
caggtgccac agagcacaca agaag                                            25

<210> 1045
<211> 25
<212> DNA
<213> Homo sapiens

<400> 1045
aggtgccaca gagcacacaa gaagt                                            25

<210> 1046
<211> 25
<212> DNA
<213> Homo sapiens

<400> 1046
ggtgccacag agcacacaag aagta                                            25

<210> 1047
<211> 25
<212> DNA
<213> Homo sapiens

<400> 1047
gtgccacaga gcacacaaga agtat                                            25

<210> 1048
<211> 25
<212> DNA
<213> Homo sapiens

<400> 1048

tgccacagag cacacaagaa gtatc                                          25

<210> 1049
<211> 25
<212> DNA
<213> Homo sapiens

<400> 1049
gccacagagc acacaagaag tatca                                          25

<210> 1050
<211> 25
<212> DNA
<213> Homo sapiens

<400> 1050
ccacagagca cacaagaagt atcag                                          25

<210> 1051
<211> 25
<212> DNA
<213> Homo sapiens

<400> 1051
cacagagcac acaagaagta tcagg                                          25

<210> 1052
<211> 25
<212> DNA
<213> Homo sapiens

<400> 1052
acagagcaca caagaagtat cagga                                          25

<210> 1053
<211> 25
<212> DNA
<213> Homo sapiens

<400> 1053
cagagcacac aagaagtatc aggag                                          25

<210> 1054
<211> 25
<212> DNA
<213> Homo sapiens

<400> 1054
agagcacaca agaagtatca ggagc                                          25

<210> 1055
<211> 25
<212> DNA
<213> Homo sapiens

<400> 1055
gagcacacaa gaagtatcag gagca                                          25

<210> 1056
<211> 25
<212> DNA

<213> Homo sapiens

<400> 1056
agcacacaag aagtatcagg agcag                                              25

<210> 1057
<211> 25
<212> DNA
<213> Homo sapiens

<400> 1057
gcacacaaga agtatcagga gcagg                                              25

<210> 1058
<211> 25
<212> DNA
<213> Homo sapiens

<400> 1058
cacacaagaa gtatcaggag cagga                                              25

<210> 1059
<211> 25
<212> DNA
<213> Homo sapiens

<400> 1059
acacaagaag tatcaggagc aggaa                                              25

<210> 1060
<211> 25
<212> DNA
<213> Homo sapiens

<400> 1060
cacaagaagt atcaggagca ggaag                                              25

<210> 1061
<211> 25
<212> DNA
<213> Homo sapiens

<400> 1061
acaagaagta tcaggagcag gaagg                                              25

<210> 1062
<211> 25
<212> DNA
<213> Homo sapiens

<400> 1062
caagaagtat caggagcagg aaggg                                              25

<210> 1063
<211> 25
<212> DNA
<213> Homo sapiens

<400> 1063
aagaagtatc aggagcagga aggga                                              25

<210> 1064
<211> 25
<212> DNA
<213> Homo sapiens

<400> 1064
agaagtatca ggagcaggaa gggat                                          25

<210> 1065
<211> 25
<212> DNA
<213> Homo sapiens

<400> 1065
gaagtatcag gagcaggaag ggatg                                          25

<210> 1066
<211> 25
<212> DNA
<213> Homo sapiens

<400> 1066
aagtatcagg agcaggaagg gatgg                                          25

<210> 1067
<211> 25
<212> DNA
<213> Homo sapiens

<400> 1067
agtatcagga gcaggaaggg atggg                                          25

<210> 1068
<211> 25
<212> DNA
<213> Homo sapiens

<400> 1068
gtatcaggag caggaaggga tgggg                                          25

<210> 1069
<211> 25
<212> DNA
<213> Homo sapiens

<400> 1069
tatcaggagc aggaagggat gggga                                          25

<210> 1070
<211> 25
<212> DNA
<213> Homo sapiens

<400> 1070
atcaggagca ggaagggatg gggaa                                          25

<210> 1071
<211> 25
<212> DNA
<213> Homo sapiens

<400> 1071
tcaggagcag gaagggatgg ggaat                                          25

<210> 1072
<211> 25
<212> DNA
<213> Homo sapiens

<400> 1072
caggagcagg aagggatggg gaatg                                          25

<210> 1073
<211> 25
<212> DNA
<213> Homo sapiens

<400> 1073
aggagcagga agggatgggg aatgt                                          25

<210> 1074
<211> 25
<212> DNA
<213> Homo sapiens

<400> 1074
ggagcaggaa gggatgggga atgtg                                          25

<210> 1075
<211> 25
<212> DNA
<213> Homo sapiens

<400> 1075
gagcaggaag ggatggggaa tgtga                                          25

<210> 1076
<211> 25
<212> DNA
<213> Homo sapiens

<400> 1076
agcaggaagg gatggggaat gtgat                                          25

<210> 1077
<211> 25
<212> DNA
<213> Homo sapiens

<400> 1077
gcaggaaggg atggggaatg tgatg                                          25

<210> 1078
<211> 25
<212> DNA
<213> Homo sapiens

<400> 1078
caggaaggga tggggaatgt gatgt                                          25

<210> 1079
<211> 25

<212> DNA
<213> Homo sapiens

<400> 1079
aggaagggat ggggaatgtg atgtt                                            25

<210> 1080
<211> 25
<212> DNA
<213> Homo sapiens

<400> 1080
ggaagggatg gggaatgtga tgttt                                            25

<210> 1081
<211> 25
<212> DNA
<213> Homo sapiens

<400> 1081
gaagggatgg ggaatgtgat gtttt                                            25

<210> 1082
<211> 25
<212> DNA
<213> Homo sapiens

<400> 1082
aagggatggg gaatgtgatg ttttt                                            25

<210> 1083
<211> 25
<212> DNA
<213> Homo sapiens

<400> 1083
agggatgggg aatgtgatgt tttta                                            25

<210> 1084
<211> 25
<212> DNA
<213> Homo sapiens

<400> 1084
gggatgggga atgtgatgtt tttaa                                            25

<210> 1085
<211> 25
<212> DNA
<213> Homo sapiens

<400> 1085
ggatggggaa tgtgatgttt ttaaa                                            25

<210> 1086
<211> 25
<212> DNA
<213> Homo sapiens

<400> 1086
gatggggaat gtgatgtttt taaag                                            25

<210> 1087
<211> 25
<212> DNA
<213> Homo sapiens

<400> 1087
atggggaatg tgatgttttt aaaga                                          25

<210> 1088
<211> 25
<212> DNA
<213> Homo sapiens

<400> 1088
tggggaatgt gatgttttta aagaa                                          25

<210> 1089
<211> 25
<212> DNA
<213> Homo sapiens

<400> 1089
ggggaatgtg atgtttttaa agaaa                                          25

<210> 1090
<211> 25
<212> DNA
<213> Homo sapiens

<400> 1090
gggaatgtga tgtttttaaa gaaat                                          25

<210> 1091
<211> 25
<212> DNA
<213> Homo sapiens

<400> 1091
ggaatgtgat gtttttaaag aaatc                                          25

<210> 1092
<211> 25
<212> DNA
<213> Homo sapiens

<400> 1092
gaatgtgatg tttttaaaga aatcc                                          25

<210> 1093
<211> 25
<212> DNA
<213> Homo sapiens

<400> 1093
aatgtgatgt ttttaaagaa atcct                                          25

<210> 1094
<211> 25
<212> DNA
<213> Homo sapiens

<400> 1094
atgtgatgtt tttaaagaaa tcctt                                          25

<210> 1095
<211> 25
<212> DNA
<213> Homo sapiens

<400> 1095
tgtgatgttt ttaaagaaat ccttt                                          25

<210> 1096
<211> 25
<212> DNA
<213> Homo sapiens

<400> 1096
gtgatgtttt taaagaaatc ctttg                                          25

<210> 1097
<211> 25
<212> DNA
<213> Homo sapiens

<400> 1097
tgatgttttt aaagaaatcc tttga                                          25

<210> 1098
<211> 25
<212> DNA
<213> Homo sapiens

<400> 1098
gatgttttta aagaaatcct ttgaa                                          25

<210> 1099
<211> 25
<212> DNA
<213> Homo sapiens

<400> 1099
atgtttttaa agaaatcctt tgaag                                          25

<210> 1100
<211> 25
<212> DNA
<213> Homo sapiens

<400> 1100
tgtttttaaa gaaatccttt gaaga                                          25

<210> 1101
<211> 25
<212> DNA
<213> Homo sapiens

<400> 1101
gtttttaaag aaatcctttg aagat                                          25

<210> 1102

<211> 25
<212> DNA
<213> Homo sapiens

<400> 1102
tttttaaaga aatcctttga agatg                                          25

<210> 1103
<211> 25
<212> DNA
<213> Homo sapiens

<400> 1103
ttttaaagaa atcctttgaa gatga                                          25

<210> 1104
<211> 25
<212> DNA
<213> Homo sapiens

<400> 1104
tttaaagaaa tcctttgaag atgat                                          25

<210> 1105
<211> 25
<212> DNA
<213> Homo sapiens

<400> 1105
ttaaagaaat cctttgaaga tgatg                                          25

<210> 1106
<211> 25
<212> DNA
<213> Homo sapiens

<400> 1106
taaagaaatc ctttgaagat gatgc                                          25

<210> 1107
<211> 25
<212> DNA
<213> Homo sapiens

<400> 1107
aaagaaatcc tttgaagatg atgct                                          25

<210> 1108
<211> 25
<212> DNA
<213> Homo sapiens

<400> 1108
aagaaatcct ttgaagatga tgctg                                          25

<210> 1109
<211> 25
<212> DNA
<213> Homo sapiens

<400> 1109

agaaatcctt tgaagatgat gctgc                                          25

<210> 1110
<211> 25
<212> DNA
<213> Homo sapiens

<400> 1110
gaaatccttt gaagatgatg ctgct                                          25

<210> 1111
<211> 25
<212> DNA
<213> Homo sapiens

<400> 1111
aaatcctttg aagatgatgc tgctt                                          25

<210> 1112
<211> 25
<212> DNA
<213> Homo sapiens

<400> 1112
aatcctttga agatgatgct gcttt                                          25

<210> 1113
<211> 1962
<212> DNA
<213> Homo sapiens

<400> 1113
atgcctctgt tcctcctgct cttacttgtc ctgctcctgc tgctcgagga cgctggagcc 60
cagcaaggca aatactgtgg tctggggttg caaatgaacc attcaattga atcaaaaggc 120
aatgaaatca cattgctgtt catgagtgga atccatgttt ctggacgcgg atttttggcc 180
tcatactctg ttatagataa acaagatcta attacttgtt tggacactgc atccaatttt 240
ttggaacctg agttcagtaa gtactgccca gctggttgtc tgcttccttt tgctgagata 300
tctggaacaa ttcctcatgg atatagagat tcctcgccat tgtgcatggc tggtgtgcat 360
gcaggagtag tgtcaaacac gttgggcggc caaatcagtg ttgtaattag taaaggtatt 420
ccctattatg aaagttcttt ggctaacaac gtcacatctg tggtgggaca cttatctaca 480
agtcttttta catttaagac aagtggatgt tatggaacac tggggatgga gtctggtgtg 540
atcgcggatc ctcaaataac agcatcatct gtgctggagt ggactgacca cacagggcaa 600
gagaacagtt ggaaacccaa aaaagccagg ctgaaaaaac ctggaccgcc ttgggctgct 660
tttgccactg atgaatacca gtggttacaa atagatttga ataaggaaaa gaaaataaca 720
ggcattataa ccactggatc caccatggtg gagcacaatt actatgtgtc tgcctacaga 780
atcctgtaca gtgatgatgg gcagaaatgg actgtgtaca gagagcctgg tgtggagcaa 840
gataagatat ttcaaggaaa caaagattat caccaggatg tgcgtaataa ctttttgcca 900
ccaattattg cacgttttat tagagtgaat cctacccaat ggcagcagaa aattgccatg 960
aaaatggagc tgctcggatg tcagtttatt cctaaaggtc gtcctccaaa acttactcaa 1020
cctccacctc ctcggaacag caatgacctc aaaaacacta cagcccctcc aaaaatagcc 1080
aaaggtcgtg ccccaaaatt tacgcaacca ctacaacctc gcagtagcaa tgaatttcct 1140
gcacagacag aacaaacaac tgccagtcct gatatcagaa atactaccgt aactccaaat 1200
gtaaccaaag atgtagcgct ggctgcagtt cttgtccctg tgctggtcat ggtcctcact 1260
actctcattc tcatattagt gtgtgcttgg cactggagaa acagaaagaa aaaaactgaa 1320
ggcacctatg acttacctta ctgggaccgg gcaggttggt ggaaaggaat gaagcagttt 1380
cttcctgcaa aagcagtgga ccatgaggaa accccagttc gctatagcag cagcgaagtt 1440
aatcacctga gtccaagaga agtcaccaca gtgctgcagg ctgactctgc agagtatgct 1500
cagccactgg taggaggaat tgttggtaca cttcatcaaa gatctacctt taaaccagaa 1560
gaaggaaaag aagcaggcta tgcagaccta gatccttaca actcaccagg gcaggaagtt 1620
tatcatgcct atgctgaacc actcccaatt acggggcctg agtatgcaac cccaatcatc 1680
atggacatgt cagggcaccc cacaacttca gttggtcagc cctccacatc cactttcaag 1740

gctacgggga accaacctcc cccactagtg ggaacttaca atacacttct ctccaggact 1800
gacagctgct cctcagccca ggcccagtat gataccccga aagctgggaa gccaggtcta 1860
cctgccccag acgaattggt gtaccaggtg ccacagagca cacaagaagt atcaggagca 1920
ggaagggatg gggaatgtga tgtttttaaa gaaatccttt ga 1962

<210> 1114
<211> 653
<212> PRT
<213> Homo sapiens

<400> 1114
Met Pro Leu Phe Leu Leu Leu Leu Leu Val Leu Leu Leu Leu Leu Glu
1               5                   10                  15

Asp Ala Gly Ala Gln Gln Gly Lys Tyr Cys Gly Leu Gly Leu Gln Met
            20                  25                  30

Asn His Ser Ile Glu Ser Lys Gly Asn Glu Ile Thr Leu Leu Phe Met
        35                  40                  45

Ser Gly Ile His Val Ser Gly Arg Gly Phe Leu Ala Ser Tyr Ser Val
    50                  55                  60

Ile Asp Lys Gln Asp Leu Ile Thr Cys Leu Asp Thr Ala Ser Asn Phe
65                  70                  75                  80

Leu Glu Pro Glu Phe Ser Lys Tyr Cys Pro Ala Gly Cys Leu Leu Pro
                85                  90                  95

Phe Ala Glu Ile Ser Gly Thr Ile Pro His Gly Tyr Arg Asp Ser Ser
            100                 105                 110

Pro Leu Cys Met Ala Gly Val His Ala Gly Val Val Ser Asn Thr Leu
        115                 120                 125

Gly Gly Gln Ile Ser Val Val Ile Ser Lys Gly Ile Pro Tyr Tyr Glu
    130                 135                 140

Ser Ser Leu Ala Asn Asn Val Thr Ser Val Val Gly His Leu Ser Thr
145                 150                 155                 160

Ser Leu Phe Thr Phe Lys Thr Ser Gly Cys Tyr Gly Thr Leu Gly Met
                165                 170                 175

Glu Ser Gly Val Ile Ala Asp Pro Gln Ile Thr Ala Ser Ser Val Leu
            180                 185                 190

Glu Trp Thr Asp His Thr Gly Gln Glu Asn Ser Trp Lys Pro Lys Lys
        195                 200                 205

Ala Arg Leu Lys Lys Pro Gly Pro Pro Trp Ala Ala Phe Ala Thr Asp
    210                 215                 220

Glu Tyr Gln Trp Leu Gln Ile Asp Leu Asn Lys Glu Lys Lys Ile Thr
225                 230                 235                 240

Gly Ile Ile Thr Thr Gly Ser Thr Met Val Glu His Asn Tyr Tyr Val
                245                 250                 255

Ser Ala Tyr Arg Ile Leu Tyr Ser Asp Asp Gly Gln Lys Trp Thr Val
            260                 265                 270

Tyr Arg Glu Pro Gly Val Glu Gln Asp Lys Ile Phe Gln Gly Asn Lys
        275                 280                 285

Asp Tyr His Gln Asp Val Arg Asn Asn Phe Leu Pro Pro Ile Ile Ala
    290                 295                 300

Arg Phe Ile Arg Val Asn Pro Thr Gln Trp Gln Gln Lys Ile Ala Met
305                 310                 315                 320

Lys Met Glu Leu Leu Gly Cys Gln Phe Ile Pro Lys Gly Arg Pro Pro
                325                 330                 335

Lys Leu Thr Gln Pro Pro Pro Pro Arg Asn Ser Asn Asp Leu Lys Asn
            340                 345                 350

Thr Thr Ala Pro Pro Lys Ile Ala Lys Gly Arg Ala Pro Lys Phe Thr
        355                 360                 365

Gln Pro Leu Gln Pro Arg Ser Ser Asn Glu Phe Pro Ala Gln Thr Glu
    370                 375                 380

Gln Thr Thr Ala Ser Pro Asp Ile Arg Asn Thr Thr Val Thr Pro Asn
385                 390                 395                 400

Val Thr Lys Asp Val Ala Leu Ala Ala Val Leu Val Pro Val Leu Val
                405                 410                 415

Met Val Leu Thr Thr Leu Ile Leu Ile Leu Val Cys Ala Trp His Trp
            420                 425                 430

Arg Asn Arg Lys Lys Lys Thr Glu Gly Thr Tyr Asp Leu Pro Tyr Trp
        435                 440                 445

Asp Arg Ala Gly Trp Trp Lys Gly Met Lys Gln Phe Leu Pro Ala Lys
    450                 455                 460

Ala Val Asp His Glu Glu Thr Pro Val Arg Tyr Ser Ser Ser Glu Val
465                 470                 475                 480

Asn His Leu Ser Pro Arg Glu Val Thr Thr Val Leu Gln Ala Asp Ser
                485                 490                 495

Ala Glu Tyr Ala Gln Pro Leu Val Gly Gly Ile Val Gly Thr Leu His
            500                 505                 510

Gln Arg Ser Thr Phe Lys Pro Glu Glu Gly Lys Glu Ala Gly Tyr Ala
        515                 520                 525

Asp Leu Asp Pro Tyr Asn Ser Pro Gly Gln Glu Val Tyr His Ala Tyr
    530                 535                 540

Ala Glu Pro Leu Pro Ile Thr Gly Pro Glu Tyr Ala Thr Pro Ile Ile
545                 550                 555                 560

Met Asp Met Ser Gly His Pro Thr Thr Ser Val Gly Gln Pro Ser Thr
                565                 570                 575

Ser Thr Phe Lys Ala Thr Gly Asn Gln Pro Pro Pro Leu Val Gly Thr
            580                 585                 590

Tyr Asn Thr Leu Leu Ser Arg Thr Asp Ser Cys Ser Ser Ala Gln Ala
        595                 600                 605

Gln Tyr Asp Thr Pro Lys Ala Gly Lys Pro Gly Leu Pro Ala Pro Asp
    610                 615                 620

Glu Leu Val Tyr Gln Val Pro Gln Ser Thr Gln Glu Val Ser Gly Ala
625                 630                 635                 640

Gly Arg Asp Gly Glu Cys Asp Val Phe Lys Glu Ile Leu
                645                 650

<210> 1115
<211> 60
<212> DNA
<213> Homo sapiens

<400> 1115
ctgctgctcg aggacgctgg agcccagcaa ggtgatggat gtggacacac tgtactaggc 60

<210> 1116
<211> 20
<212> PRT
<213> Homo sapiens

<400> 1116
Leu Leu Leu Glu Asp Ala Gly Ala Gln Gln Gly Asp Gly Cys Gly His
1               5                   10                  15

Thr Val Leu Gly
            20

<210> 1117
<211> 35
<212> DNA
<213> Homo sapiens

<400> 1117
atggtcctca ctactctcat tctcatatta gtgtg                                35

<210> 1118
<211> 33
<212> DNA
<213> Homo sapiens

<400> 1118
tcaaaggatt tctttaaaaa catcacattc ccc                                  33

<210> 1119
<211> 35
<212> DNA
<213> Homo sapiens

<400> 1119
ctgtactagg ccctgagagt ggaaccctta catcc                                35

<210> 1120
<211> 31
<212> DNA
<213> Homo sapiens

<400> 1120
ggggtttcct catggtccac tgcttttgca g                                    31

<210> 1121
<211> 39
<212> DNA
<213> Homo sapiens

<400> 1121
ccaccatgcc tctgttcctc ctgctcttac ttgtcctgc                            39

<210> 1122
<211> 36
<212> DNA
<213> Homo sapiens

<400> 1122
tcaaaggatt tctttaaaaa catcacattc cccatc                               36

<210> 1123
<211> 33
<212> DNA
<213> Homo sapiens

<400> 1123
ctgcccggtc ccagtaaggt aagtcatagg tgc                                  33